# EUROPEAN PATENT SPECIFICATION

(11) **EP 4 161 643 B1**
(45) Date of publication and mention of the grant of the patent: **14.10.2026**
(21) Application number: 21733525.6
(22) Date of filing: 04.06.2021
(51) Int. Cl.: A61P 9/10, A61P 11/06, A61P 35/00, A61P 37/02, C07D 217/02, C07D 217/24, C07D 413/04, C07D 471/04, C07D 487/04, C07D 487/14, C07D 498/04, A61K 31/47, A61K 31/5365, A61K 31/4738

(54) **N-(PHENYLAMINOCARBONYL) TETRAHYDRO-ISOQUINOLINES AND RELATED COMPOUNDS AS MODULATORS OF GPR65**
N-(PHENYLAMINOCARBONYL) TETRAHYDRO-ISOQUINOLINE UND VERWANDTE VERBINDUNGEN ALS GPR65 MODULATOREN
N-(PHENYLAMINOCARBONYL) TETRAHYDRO-ISOQUINOLINES ET COMPOSÉS APPARANTÉS EN TANT QUE MODULATEURS DU GPR65

(30) Priority: 05.06.2020 GB 202008526; 17.03.2021 GB 202103704
(43) Date of publication of application: 12.04.2023
(73) Proprietor: Pathios Therapeutics Limited, Milton, Abingdon OX14 4RY (GB)
(72) Inventor: MCCARTHY, Tom, Oxford, OX14 4RY (GB); MILNE, Gavin, Oxford, OX14 4RY (GB); MOECHEL, Tobias, Oxford, OX14 4RY (GB); NAYLOR, Alan, Oxford, OX14 4RY (GB)
(74) Representative: D Young & Co LLP
(86) International application number: PCT/GB2021/051396
(87) International publication number: WO 2021/245426

(56) References cited:
- WO-A1-2008/101354
- WO-A1-2008/130581
- WO-A1-2009/000087
- WO-A1-2010/078427
- WO-A1-2019/097479
- US-A1- 2006 154 944
- US-A1- 2017 158 691
- NIE CUNBIN ET AL: "Study on chemical modification and analgesic activity of N-(4-tert-butylphenyl)-4-(3-chloropyridin-2-yl) piperazine-1-carboxamide", EUROPEAN JOURNAL OF MEDICINAL CHEMISTRY, ELSEVIER, AMSTERDAM, NL, vol. 194, 19 March 2020 (2020-03-19), XP086130701, ISSN: 0223-5234, [retrieved on 20200319], DOI: 10.1016/J.EJMECH.2020.112236
- MADAIAH MALAVALLI ET AL: "Synthesis, Characterization, and Evaluation of Difluoropyrido[4,3- b ]indoles as Potential Agents for Acetylcholinesterase and Antiamnesic Activity : Synthesis of Difluoropyrido[4,3- b ]indoles", ARCHIV DER PHARMAZIE, vol. 350, no. 3-4, 22 March 2017 (2017-03-22), Weinheim, pages 1600303, XP055830547, ISSN: 0365-6233, Retrieved from the Internet <URL:https://api.wiley.com/onlinelibrary/tdm/v1/articles/10.1002%2Fardp.201600303> DOI: 10.1002/ardp.201600303
- HUANG XI-PING ET AL: "Allosteric ligands for the pharmacologically dark receptors GPR68 and GPR65", NATURE, vol. 527, no. 7579, 1 November 2015 (2015-11-01), London, pages 477 - 483, XP055830467, ISSN: 0028-0836, Retrieved from the Internet <URL:https://www.ncbi.nlm.nih.gov/pmc/articles/PMC4796946/pdf/nihms760070.pdf> DOI: 10.1038/nature15699

## Description

The present invention relates to compounds that are capable of modulating GPR65. The compounds have potential therapeutic applications in the treatment of a variety of disorders, including proliferative and immune disorders.

### BACKGROUND TO THE INVENTION

GPR65 is a Gs-coupled G protein-coupled receptor (GPCR) that is primarily expressed in immune cells and is activated by acidic extracellular pH to cause increases in cytoplasmic cyclic adenosine monophosphate (cAMP) (Wang, 2004). It has long been known that tumours typically undergo a switch in cellular metabolism from oxidative phosphorylation to aerobic glycolysis, which in turn results in an acidic extracellular microenvironment (Damaghi, 2013). Recently, it has been shown that this acidic microenvironment causes GPR65 activation in tumour-associated macrophages, resulting in an increase in cytoplasmic cAMP leading to transcription of the inducible cAMP early repressor (ICER). This, in turn, suppresses the secretion of tumour necrosis factor alpha (TNFα) to bias the macrophages toward an anti-inflammatory, tumour-permissive phenotype (Bohn, 2018). This GPR65-dependent pathway therefore appears to represent a mechanism by which tumours exploit their acidic microenvironment to evade detection by the immune system.

Autoimmune diseases are also often associated with an acidic local microenvironment (for instance, an inflamed joint). Recent studies also suggest that GPR65 acts through ICER in CD4+ T cells, to suppress IL-2 and hence bias cells toward an inflammatory Th17 phenotype, which is associated with increased pathogenicity in the context of autoimmune disease (Korn, 2009). Supporting this is the recent finding that ICER is required for Th17 differentiation (Yoshida, 2016) as well as that agonism of GPR65 leads to an increase in Th17 differentiation (Hernandez, 2018). Indeed, mutations in the GPR65 locus are associated with several autoimmune diseases, such as multiple sclerosis, ankylosing spondylitis, inflammatory bowel disease, and Crohn's disease (Gaublomme, 2015). One recent study found that mice with CD4+ T cells lacking GPR65 were protected from developing the disease autoimmune encephalomyelitis (EAE) (Gaublomme, 2015).

Thus, GPR65 appears to act through ICER to promote an anti-inflammatory and tumour-permissive phenotype in tumour associated macrophages and an inflammatory Th17 phenotype in CD4+ T cells that is associated with autoimmune disease. GPR65 signalling, therefore, represents an attractive pathway for therapeutic intervention for the treatment of both cancer and autoimmune diseases. There is therefore an ongoing need to develop new small molecule GPR65 modulators.

Various small molecule GPR modulators are known in the art. For example, WO 2009/000087 A1 (Merck Frosst Canada Ltd) discloses substituted fused pyrimidines as antagonists of GPR105 activity. WO 2008/130581 A1 (Schering Corporation) relates to pyrimidinone derivatives for treating a disorder related to the activity of GPR119. Huang et al, Nature 527, 477-483 (2015) discloses allosteric ligands of GPR65. WO 2008/101354 (Merck Frosst Canada Ltd) discloses GPR65-specific antisense oligonucleotides.

The present invention seeks to provide compounds that are capable of modulating GPR65. As made clear from the above discussion, such compounds have potential therapeutic applications in the treatment of a variety of disorders, including proliferative disorders and immune disorders as well as asthma and chronic obstructive pulmonary disease.

### STATEMENT OF INVENTION

Aspects of the invention are described in the accompanying claims.

A first aspect of the invention relates to a compound of formula (Ia), or a pharmaceutically acceptable salt or solvate thereof, 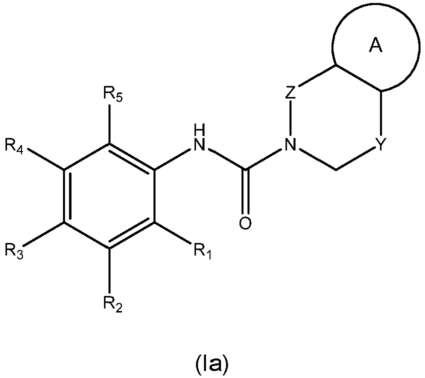 wherein:
ring A is fused to the ring containing N, Z and Y, where ring A is selected from benzene, pyridine, pyridone, pyridine N-oxide, pyridazine, pyrimidine and pyrazine, each of which is optionally substituted with one or more substituents selected from F, CI, Br, I, CN, OH, SO₂-alkyl, alkyl, haloalkyl, aralkyl, and aryl, and wherein said aryl substituent is in turn optionally substituted with one or more substituents each independently selected from F, Cl, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;
   and
R₁₁ and R₁₁' are each independently selected from H, alkyl, haloalkyl, COR₁₂, and SO₂R₁₃, wherein R₁₂ and R₁₃ are both alkyl;
wherein the compound is other than:
*N*-(3,4-Dichlorophenyl)-7,8-dihydropyrido[4,3-d]pyrimidine-6(5H)-carboxamide;
*N*-(3,4-Dichlorophenyl)-5,8-dihydropyrido[3,4-d]pyrimidine-7(6H)-carboxamide;
*N*-(4-Chloro-3-(trifluoromethyl)phenyl)-3,4-dihydroisoquinoline-2(1H)-carboxamide; and
*N*-(3,4-Dichlorophenyl)-3,4-dihydroisoquinoline-2(1H)-carboxamide.

A second aspect of the invention relates to a compound of formula (la"), or a pharmaceutically acceptable salt or solvate thereof, 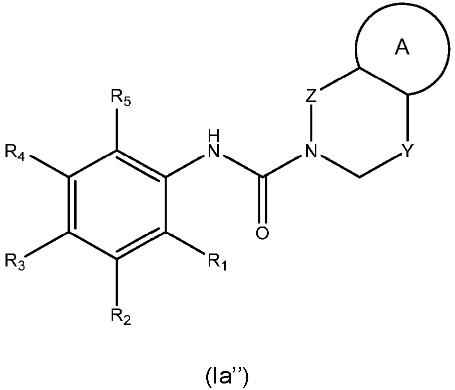 wherein ring A 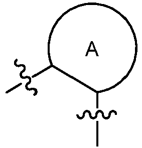 is selected from: 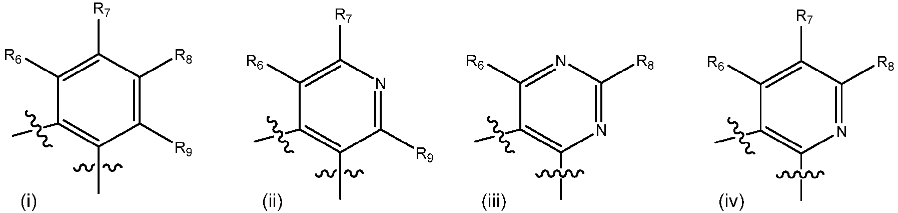 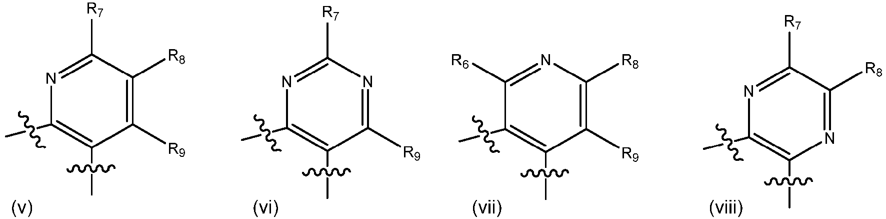 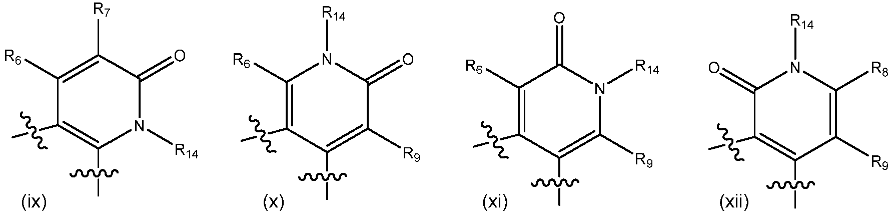 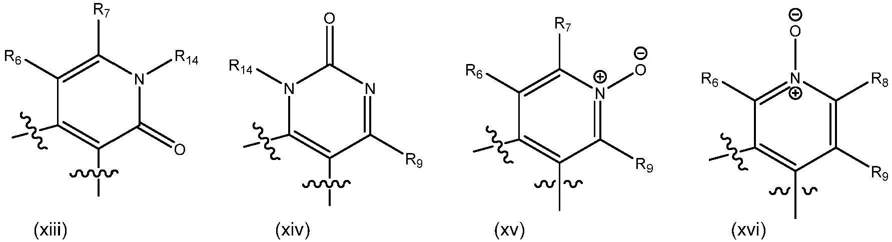 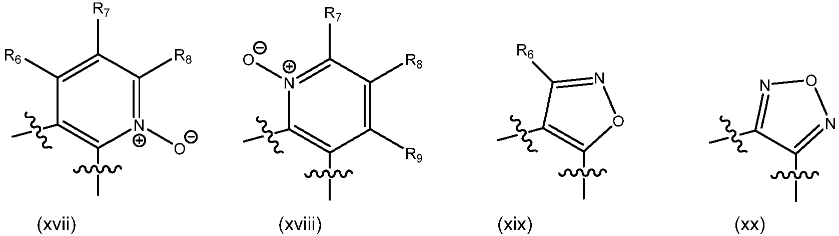 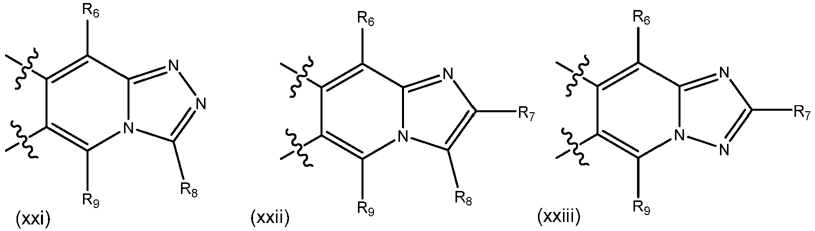 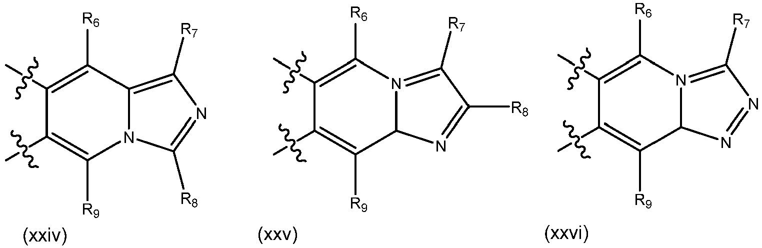 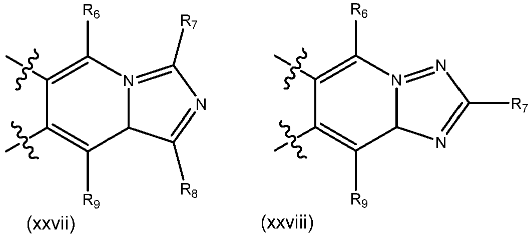 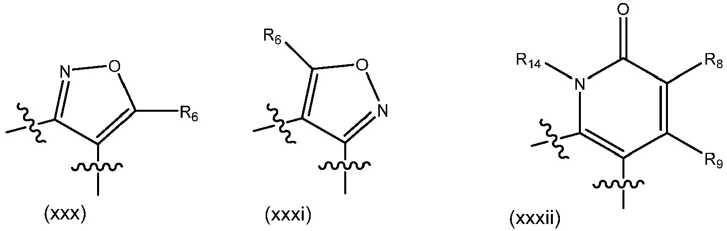 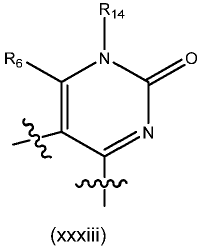 where the wavy lines denote attachment of ring A to the ring containing N, Z and Y: 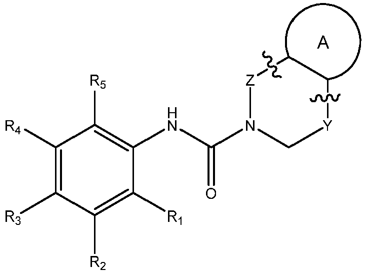 and wherein:
Y and Z are each independently CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;

wherein R₆, R₇, R₈, and R₉ are each independently selected from H, F, CI, Br, I, CN, SO₂-alkyl, OH, C₁-C₆ alkyl, phenyl, and C₁-C₆ haloalkyl, and R₁₄ is H or alkyl; and
wherein the compound is other than:
   *N*-(3,4-Dichlorophenyl)-7,8-dihydropyrido[4,3-d]pyrimidine-6(5H)-carboxamide;
   *N*-(3,4-Dichlorophenyl)-5,8-dihydropyrido[3,4-d]pyrimidine-7(6H)-carboxamide;
   *N*-(4-Chloro-3-(trifluoromethyl)phenyl)-3,4-dihydroisoquinoline-2(1H)-carboxamide;
   *N*-(3,4-dichlorophenyl)-3,4-dihydroisoquinoline-2(1H)-carboxamide; and
   *N*-(3,4-Dichlorophenyl)-6,7-dihydroisoxazolo[4,5-c]pyridine-5(4H)-carboxamide.

Advantageously, the presently claimed compounds are capable of modulating GPR65, thereby rendering the compounds of therapeutic interest in the treatment of various disorders, for example, in the fields of oncology, immuno-oncology, and immunology.

A third aspect of the invention relates to a compound of formula (I), or a pharmaceutically acceptable salt or solvate thereof, 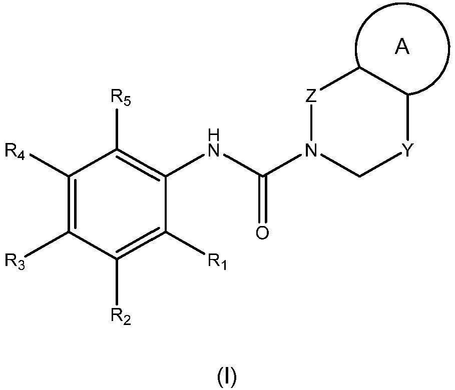 wherein:
ring A is a 5- or 6-membered monocyclic aromatic or heteroaromatic ring, or a 9- or 10-membered bicyclic aromatic or heteroaromatic ring, each of which is optionally substituted with one or more substituents selected from F, CI, Br, I, CN, alkoxy, NR₁₁R₁₁', OH, SO₂-alkyl, CO₂-alkyl, alkyl, haloalkyl, aralkyl, aryl, and heteroaryl, and wherein said aryl and heteroaryl substituents are in turn optionally substituted with one or more substituents each independently selected from F, CI, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;
R₁₁ and R₁₁' are each independently selected from H, alkyl, haloalkyl, COR₁₂, and SO₂R₁₃, wherein R₁₂ and R₁₃ are both alkyl;
for use in treating or preventing a disorder selected from a proliferative disorder, an immune disorder, asthma, chronic obstructive pulmonary disease (COPD) and acute respiratory distress syndrome (ARDS), wherein the compound modulates GPR65, preferably wherein the compound inhibits GPR65 signalling.

Another aspect of the invention relates to a pharmaceutical composition comprising a compound as described above and a pharmaceutically acceptable diluent, excipient, or carrier.

Another aspect of the invention relates to a compound or a pharmaceutical composition as described above for use as a medicament.

Another aspect of the invention relates to a compound or a pharmaceutical composition as described above for use in treating or preventing a disorder selected from a proliferative disorder, an immune disorder, asthma, chronic obstructive pulmonary disease (COPD) and acute respiratory distress syndrome (ARDS).

### DETAILED DESCRIPTION

The present invention relates to compounds that are capable of modulating GPR65.

"Alkyl" is defined herein as a straight-chain or branched alkyl radical, preferably C₁₋₂₀ alkyl, more preferably C₁₋₁₂ alkyl, even more preferably C₁₋₁₀ alkyl or C₁₋₆ alkyl, for example, methyl, ethyl, propyl, isopropyl, butyl, isobutyl, tert-butyl, pentyl, hexyl. More preferably, the alkyl is a C₁₋₃ alkyl.

As used herein, the term "aryl" refers to a C₆₋₁₂ aromatic group, which may be benzocondensed, for example, phenyl or naphthyl. Preferably, the aryl group is phenyl.

"Haloalkyl" is defined herein as a straight-chain or branched alkyl radical as defined above, for example, methyl, ethyl, propyl, isopropyl, butyl, isobutyl, tert-butyl, pentyl, hexyl, that is substituted with one or more halogen atoms (that may be the same or different), such as fluorine, chlorine, bromine, and iodine. Preferably, the haloalkyl is a C₁₋₂₀ haloalkyl, more preferably a C₁₋₁₂ haloalkyl, even more preferably a C₁₋₁₀ haloalkyl or a C₁₋₆ haloalkyl, or a C₁₋₃ haloalkyl. Preferred examples are CF₃ and CHF₂, with CF₃ being particularly preferred.

"Alkoxy" is defined herein as an oxygen atom bonded to an alkyl group as defined above, for example methoxy, ethoxy, propoxy, isopropoxy, butoxy, isobutoxy, tert-butoxy, pentoxy and hexoxy. Preferably, the alkoxy is a C₁₋₂₀ alkoxy, more preferably a C₁₋₁₂ alkoxy, even more preferably C₁₋₁₀ alkoxy or a C₁₋₆ alkoxy, or a C₁₋₃ alkoxy. A preferred example is methoxy (-OCH₃).

"Heteroaryl" is defined herein as a monocyclic or bicyclic C₂₋₁₂ aromatic ring comprising one or more heteroatoms (that may be the same or different), such as oxygen, nitrogen or sulphur. Examples of suitable heteroaryl groups include thienyl, furanyl, pyrrolyl, pyridinyl, oxazolyl, thiazolyl, imidazolyl, pyrazolyl, pyridazinyl, isoxazolyl, pyrimidinyl, pyrazinyl, triazinyl, isothiazolyl, oxadiazolyl, triazolyl, thiadiazolyl etc. and benzo derivatives thereof, such as benzofuranyl, benzothienyl, benzimidazolyl, indolyl, isoindolyl, indazolyl etc.; or pyridyl, pyrazinyl, pyrimidinyl, pyridazinyl, triazinyl etc. and benzo derivatives thereof, such as quinolinyl, isoquinolinyl, cinnolinyl, phthalazinyl, quinazolinyl, quinoxalinyl, naphthyridinyl etc.

"Aralkyl' is defined herein as an alkyl group as defined above substituted by one or more aryl groups as defined above.

### Compounds of Formula (la)

One aspect of the invention relates to compounds of formula (la): 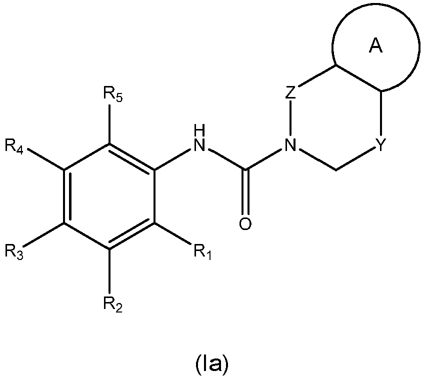 wherein:
ring A is fused to the ring containing N, Z and Y, where ring A is selected from benzene, pyridine, pyridone, pyridine N-oxide, pyridazine, pyrimidine and pyrazine, each of which is optionally substituted with one or more substituents selected from F, CI, Br, I, CN, OH, SO₂-alkyl, alkyl, haloalkyl, aralkyl, and aryl, and wherein said aryl substituent is in turn optionally substituted with one or more substituents each independently selected from F, Cl, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;
   and
R₁₁ and R₁₁' are each independently selected from H, alkyl, haloalkyl, COR₁₂, and SO₂R₁₃, wherein R₁₂ and R₁₃ are both alkyl;
wherein the compound is other than:
*N*-(3,4-Dichlorophenyl)-7,8-dihydropyrido[4,3-d]pyrimidine-6(5H)-carboxamide;
*N*-(3,4-Dichlorophenyl)-5,8-dihydropyrido[3,4-d]pyrimidine-7(6H)-carboxamide;
*N*-(4-Chloro-3-(trifluoromethyl)phenyl)-3,4-dihydroisoquinoline-2(1H)-carboxamide; and
*N*-(3,4-Dichlorophenyl)-3,4-dihydroisoquinoline-2(1H)-carboxamide.

Preferably, the compound of formula (la) is not compound 1, 8, 11, 80 or 81 as defined herein.

In formulae (la), and for other aspects, preferably alkyl is C₁₋C₆ alkyl, haloalkyl is C₁₋C₆ haloalkyl, and alkoxy is C₁₋C₆ alkoxy.

In one embodiment, optional substituents on the A ring are selected from F, CI, Br, I, CN, OH, SO₂-alkyl, alkyl and haloalkyl.

In one preferred embodiment, ring A is optionally substituted by one or more substituents selected from halo, CN, C₁-C₆ OH, C₁-C₆ alkyl, phenyl, SO₂-alkyl, and C₁-C₆ haloalkyl. More preferably, ring A is optionally substituted by one or more substituents selected from Me, CI, F, CN, OH, and SO₂Me.

In some instances, ring A can exist in more than one tautomeric form. By way of illustration, where the heteroaromatic ring is substituted by an OH group, ring A can exist as two possible tautomers as shown below:

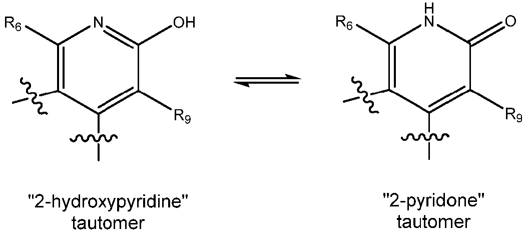

The 2-pyridone tautomer is believed to be the predominant solid state form. In solution, the energy difference between the two tautomeric forms is understood to be very small and is dependent on the polarity of the solvent. The skilled person would appreciate that other hydroxy substituted N-containing heteroaromatic groups (e.g. pyrimidine, other pyridine regioisomers) can be similarly represented in tautomeric form as shown above. The term "heteroaromatic" as used herein encompasses all tautomeric forms of the compounds.

In one preferred embodiment, the monocyclic aromatic or heteroaromatic ring A is a group selected from benzene, pyridine, pyridone, pyridine N-oxide, pyrimidine, pyridazine, and pyrazine, each of which is optionally substituted.

In one preferred embodiment, ring A is a group selected from benzene, pyridine, pyridone, pyridine N-oxide, pyrimidine, pyridazine, and pyrazine, each of which is optionally substituted with one or more substituents selected from F, CI, Br, I, CN, OH, C₁-C₆ alkyl, phenyl, SO₂-alkyl, and C₁-C₆ haloalkyl.

In one preferred embodiment, ring A is a group selected from benzene, pyridine, pyridone, pyridine N-oxide, pyrimidine, pyridazine, and pyrazine, each of which is optionally substituted with one or more substituents selected from F, CI, Br, I, CN, OH, C₁-C₆ alkyl, SO₂-alkyl, I, and C₁-C₆ haloalkyl.

In one preferred embodiment, ring A is a benzene group which is optionally substituted with one or more substituents selected from F, CI, Br, I, CN, OH, C₁-C₆ alkyl, SO₂-alkyl, I, and C₁-C₆ haloalkyl. In one preferred embodiment, ring A is a benzene group which is substituted with one or more substituents selected from F, CI, Br, I, CN, OH, C₁-C₆ alkyl, SO₂-alkyl, I, and C₁-C₆ haloalkyl.

In one preferred embodiment, ring A is a pyridine group which is optionally substituted with one or more substituents selected from F, CI, Br, I, CN, OH, C₁-C₆ alkyl, SO₂-alkyl, I, and C₁-C₆ haloalkyl.

In one preferred embodiment, ring A is a pyridone group which is optionally substituted with one or more substituents selected from F, CI, Br, I, CN, OH, C₁-C₆ alkyl, SO₂-alkyl, I, and C₁-C₆ haloalkyl.

Preferably, ring A is as defined below, where the wavy lines denote attachment to the ring containing N, Z and Y:

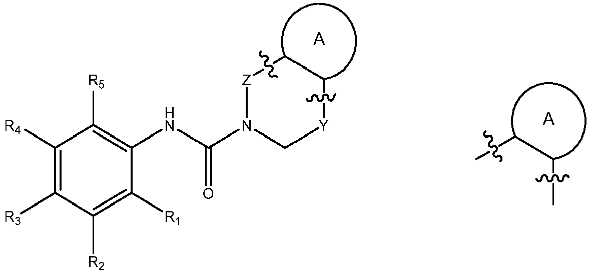

Another aspect of the invention relates to compounds of formula (Ia"), or a pharmaceutically acceptable salt or solvate thereof, 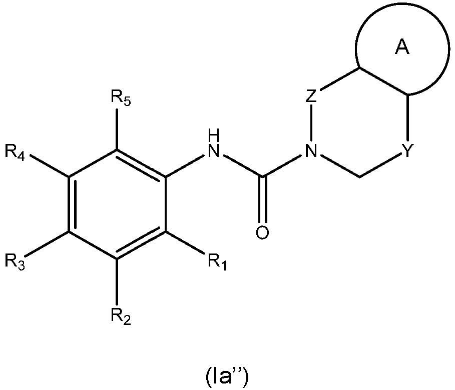 wherein ring A is selected from: 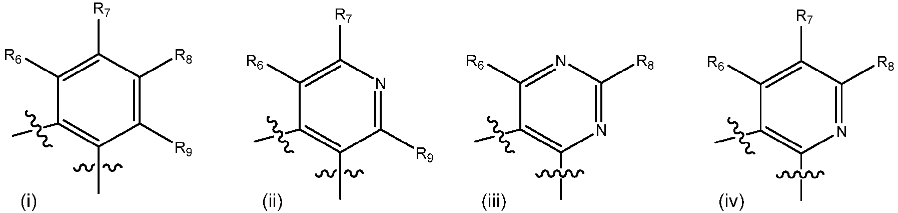 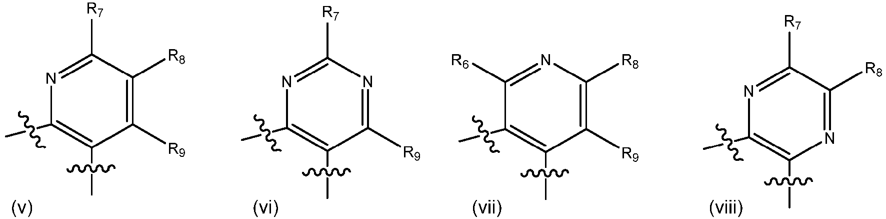 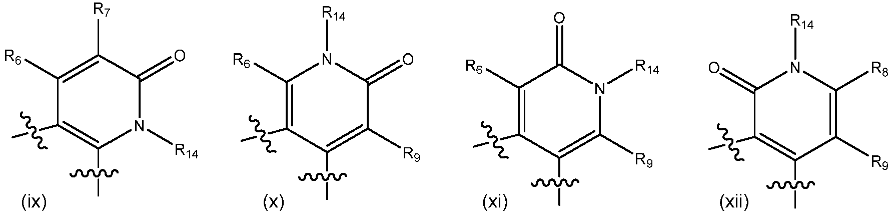 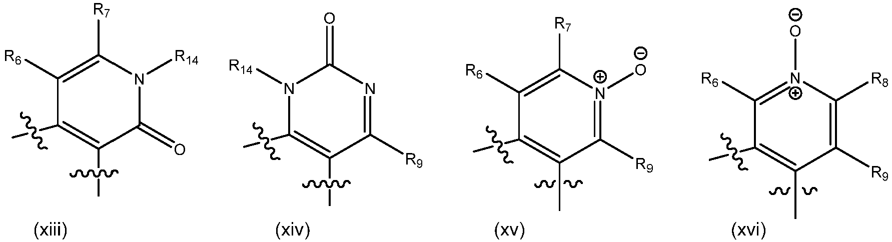 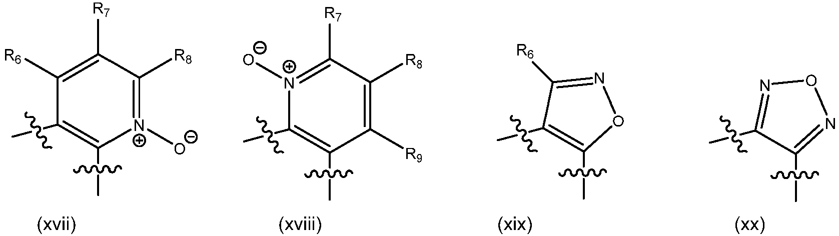 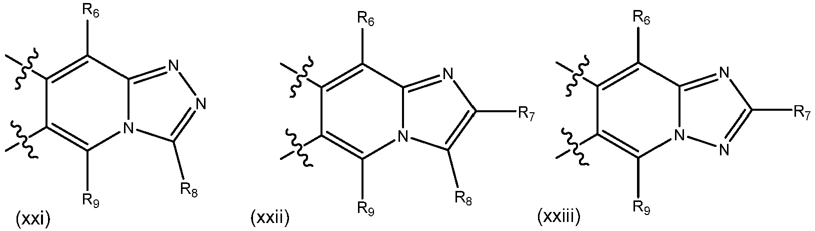 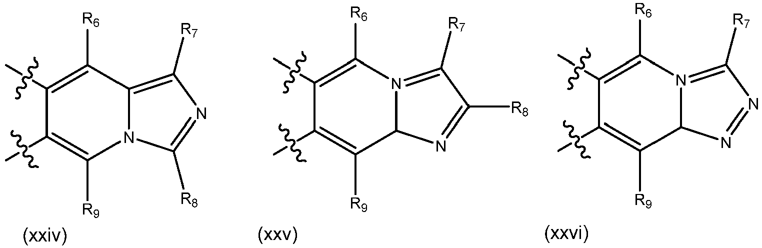 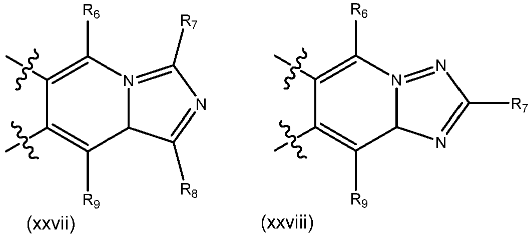 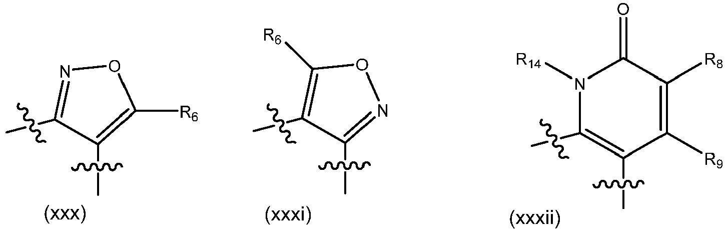 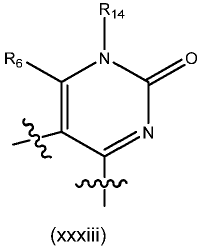 where the wavy lines denote attachment of ring A to the ring containing N, Z and Y: 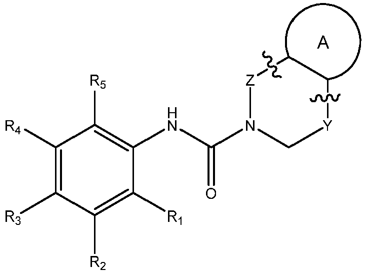
Y and Z are each independently CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;

wherein R₆, R₇, R₈, and R₉ are each independently selected from H, F, CI, Br, I, CN, SO₂-alkyl, OH, C₁-C₆ alkyl, phenyl, and C₁-C₆ haloalkyl, and R₁₄ is H or alkyl; and
wherein the compound is other than:
   *N*-(3,4-Dichlorophenyl)-7,8-dihydropyrido[4,3-d]pyrimidine-6(5H)-carboxamide;
   *N*-(3,4-Dichlorophenyl)-5,8-dihydropyrido[3,4-d]pyrimidine-7(6H)-carboxamide;
   *N*-(4-Chloro-3-(trifluoromethyl)phenyl)-3,4-dihydroisoquinoline-2(1H)-carboxamide;
   *N*-(3,4-dichlorophenyl)-3,4-dihydroisoquinoline-2(1H)-carboxamide; and
   *N*-(3,4-Dichlorophenyl)-6,7-dihydroisoxazolo[4,5-c]pyridine-5(4H)-carboxamide.

In one preferred embodiment, R₆, R₇, R₈, and R₉ are each independently selected from H, F, Cl, Br, I, CN, OH, C₁-C₆ alkyl, phenyl, and C₁-C₆ haloalkyl, and R₁₄ is H or alkyl.

In one preferred embodiment, R₆, R₇, R₈, and R₉ are each independently selected from H, F, Cl, Br, I, CN, OH, C₁-C₆ alkyl, phenyl, and C₁-C₆ haloalkyl, and R₁₄ is H or alkyl.

More preferably, R₁₄ is H or Me, more preferably H.

In one preferred embodiment, ring A is selected from groups (i), (ii) and (iv)-(xxxiii).

In one preferred embodiment, ring A is not (iii).

In one preferred embodiment, ring A is not (vi).

In one preferred embodiment, ring A is selected from groups (ii), (iv), (v), (vii)-(xviii), and (xx)-(xxxiii).

In one preferred embodiment, ring A is selected from groups (i), (ii), (iv), (v), (vii)-(xviii) and (xx)-(xxxiii).

In one preferred embodiment, ring A is selected from groups (i)-(viii), (ix), (xi), and (xxxii).

In one preferred embodiment, ring A is selected from groups (i)-(viii).

In one preferred embodiment, ring A is selected from groups (i), (ii), (vi), (vii) and (x).

In one preferred embodiment, the compound is of formulae (Ia)-(i): 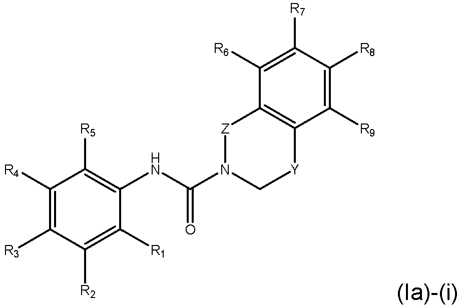 wherein R₁-R₉, R₁₄, Z and Y are as defined above. Preferably, for this embodiment, at least one of R₆-R₉ is other than H. Preferably, at least one of R₆-R₉ is selected from Cl, F, Me, CN, OH, CF₃, and SO₂Me. Preferably, one or two of R₆-R₉ are selected from CI, F, Me, CN, OH, CF₃, and SO₂Me, and the remainder of R₆-R₉ are hydrogen. More preferably, one of R₆-R₉ is selected from CI, F, Me, CN, OH, CF₃, and SO₂Me, and the remainder of R₆-R₉ are hydrogen.

In one preferred embodiment, the compound is of formulae (Ia)-(ii): 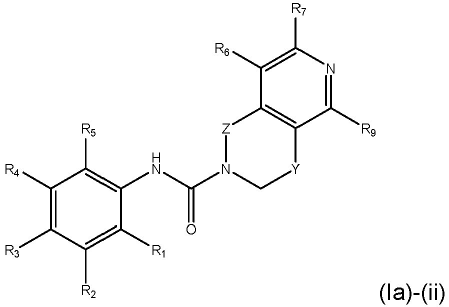 wherein R₁-R₇, R₉, R₁₄, Z and Y are as defined above.

In one preferred embodiment, the compound is of formulae (Ia)-(iv): 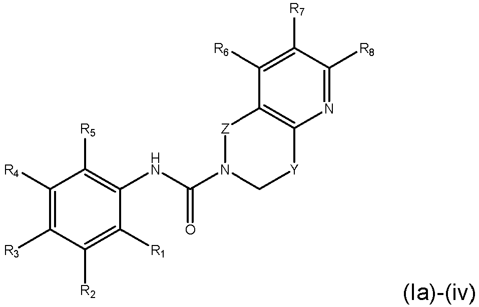 wherein R₁-R₈, R₁₄, Z and Y are as defined above.

In one preferred embodiment, the compound is of formula (Ia)-(vii): 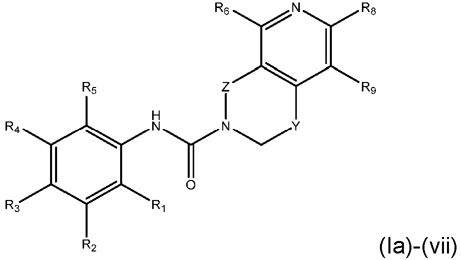 wherein R₁-R₆, R₈, R₉, R₁₄, Z and Y are as defined above.

In one preferred embodiment, the compound is of formula (la)-(x): 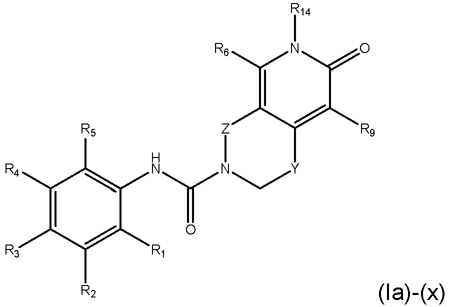 wherein R₁-R₆, R₉, R₁₄, Z and Y are as defined above.

In one preferred embodiment, ring A is selected from groups (i), (ii), (vi) and (vii).

In one preferred embodiment, ring A is selected from groups (i), (ii), (vii) and (x).

In one preferred embodiment, ring A is (i). Preferably, for this embodiment, at least one of R₆-R₉ is other than H.

In one preferred embodiment, ring A is (ii).

In one preferred embodiment, ring A is (iii).

In one preferred embodiment, ring A is (iv).

In one preferred embodiment, ring A is (v).

In one preferred embodiment, ring A is (vi).

In one preferred embodiment, ring A is (vii). In one preferred embodiment, ring A is (viii).

In one preferred embodiment, ring A is (x).

In one preferred embodiment, ring A is a 9- or 10-membered bicyclic heteroaromatic ring containing 1 to 4 nitrogen atoms selected from groups (xxi)-(xxviii).

In one preferred embodiment, ring A is (i) and:
R₇ is Cl or F, and R₆, R₈ and R₉ are all H; or
R₈ is Cl, F, CN, and R₆, R₇ and R₉ are all H; or
R₉ is F, and R₆, R₇ and R₈ are all H.

In one preferred embodiment, ring A is (ii) and:
R₆, R₇ and R₉ are all H; or
R₇ is F or NH₂, and R₆ and R₉ are H; or
R₉ is F, and R₆ and R₇ are H.

In one preferred embodiment, R₁₁ and R₁₁' are selected from H and alkyl, and more preferably selected from H and Me, Even more preferably, R₁₁ and R₁₁' are both H.

In one preferred embodiment, ring A is (x) and R₆, R₉ and R₁₄ are all H.

In one preferred embodiment, ring A is (vii) and R₈ is F, and R₆ and R₉ are H.

In one preferred embodiment, Y and Z are each independently selected from CH₂, CHMe, CHF, CF₂, C(CH₃)₂, C(CF₃)₂, and are more preferably both CH₂. In one preferred embodiment, one of Z and Y is CHMe and the other is CH₂. In one preferred embodiment, Y is CHMe and Z is CH₂. In one preferred embodiment, Z is CHMe and Y is CH₂. In a more preferred embodiment, Z and Y are both CH₂.

In one preferred embodiment, R₁ is selected from H, haloalkyl and F, and is more preferably H.

In one preferred embodiment, R₂ and R₃ are each independently selected from CI, Br, I, and CFₙH₃₋ₙ, where n is 1, 2, or 3.

In one preferred embodiment, R₂ and R₃ are each independently selected from CI, Br, and CFₙH₃₋ₙ, where n is 1, 2, or 3. In one preferred embodiment, n is 3.

In one preferred embodiment, R₂ and R₃ are each independently selected from Cl and CF₃.

In one preferred embodiment, one of R₂ and R₃ is Cl and the other is CF₃.

More preferably, R₂ is Cl and R₃ is CF₃ or R₂ is Cl and R₃ is Cl.

In one preferred embodiment, R₄ is selected from H and CI, and is preferably H.

In one preferred embodiment, R₅ is H or CF₃, more preferably H.

In one preferred embodiment, one of R₂ and R₃ is Cl and the other is CF₃, and R₁, R₄ and R₅ are all H.

In one preferred embodiment, R₂ is Cl, R₃ is CF₃ and R₁, R₄ and R₅ are all H.

In one preferred embodiment, R₃ is Cl, R₂ is CF₃ and R₁, R₄ and R₅ are all H.

In another preferred embodiment, R₂ and R₃ are both Cl, and R₁, R₄ and R₅ are all H.

The following preferred definitions for R₆-R₉ apply to all of the A groups (i)-(xxxiii) defined herein.

In one preferred embodiment, R₆ is selected from H, Me, F, Cl and CN.

In one preferred embodiment, R₆ is selected from H, F, Cl, CN, CH₃, and CF₃. More preferably R₁₁ and R₁₁' are both H.

In one preferred embodiment, R₆ is selected from H, F, Cl, CN and CH₃, and is preferably H.

In one preferred embodiment, R₇ is selected from H, CI, F, Me, CN, CF₃, and OH.

In one preferred embodiment, R₇ is selected from H, F, Cl, CN, CH₃, and CF₃. More preferably, R₁₁ and R₁₁' are both H.

In one preferred embodiment, R₇ is selected from H, F, Cl, CN, CH₃, and CF₃. More preferably R₇ is selected from H, F, and Cl. Even more preferably, R₇ is H.

In one preferred embodiment, R₈ is selected from H, CN, F, Cl, CF₃, OH, SO₂Me, and Me.

In one preferred embodiment, R₈ is selected from H, F, OH, CN, phenyl, CF₃, and CF₂H. More preferably R₁₁ and R₁₁' are both H.

In one preferred embodiment, R₈ is selected from H, F, Cl, CN, CH₃, and CF₃, preferably from H, F, Cl, and CN. More preferably R₈ is selected from F and Cl.

In one preferred embodiment, R₈ is F.

In one preferred embodiment, R₉ is selected from H, F, Cl, Me, CF₃ and CN.

In one preferred embodiment, R₉ is selected from H, F, Cl, CN, CH₃, and CF₃.

In one preferred embodiment, R₉ is selected from H, F, Cl, CN, CH₃, and CF₃, preferably H, F, and CN. More preferably, R₉ is H.

In one preferred embodiment, the compound is selected from the following:

| | | | |
|---|---|---|---|
| 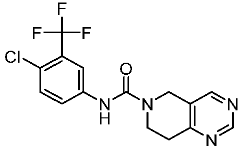 | (5) | 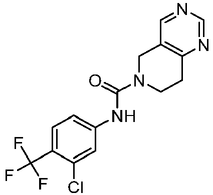 | (6) |
| 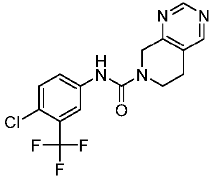 | (7) | 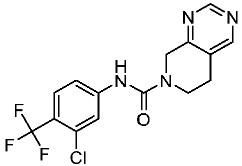 | (9) |
| 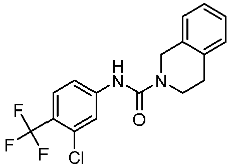 | (10) | 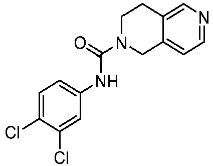 | (12) |
| 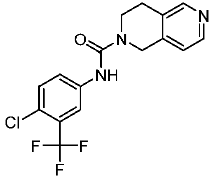 | (13) | 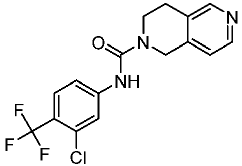 | (14) |
| 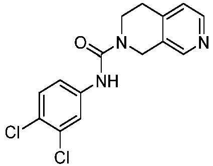 | (15) | 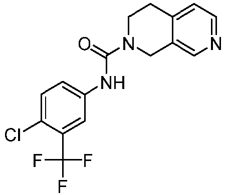 | (16) |
| 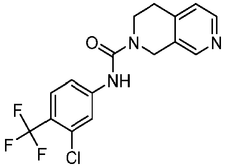 | (17) | 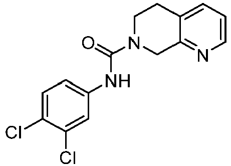 | (18) |
| 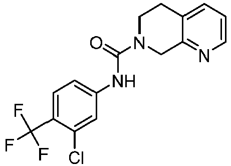 | (19) | 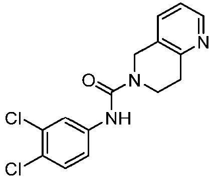 | (20) |
| 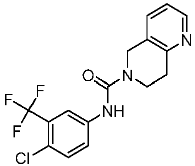 | (21) | 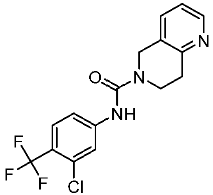 | (22) |
| 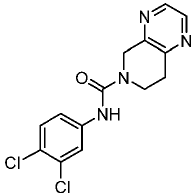 | (23) | 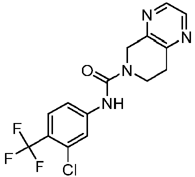 | (24) |
| 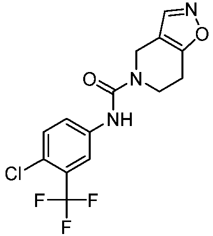 | (25) | 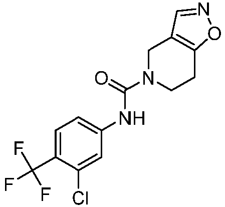 | (26) |
| 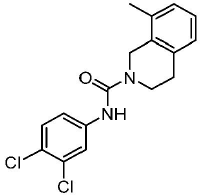 | (27) | 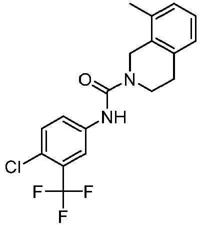 | (28) |
| 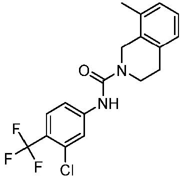 | (29) | 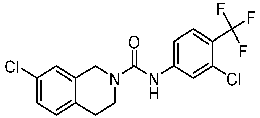 | (30) |
| 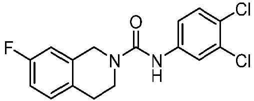 | (31) | 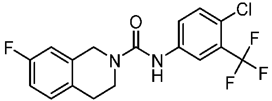 | (32) |
| 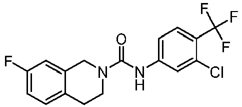 | (33) | 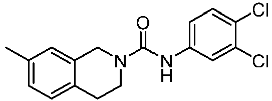 | (34) |
| 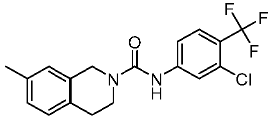 | (35) | 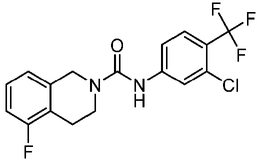 | (36) |
| 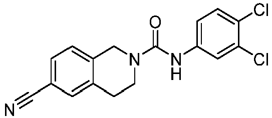 | (37) | 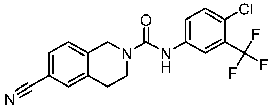 | (38) |
| 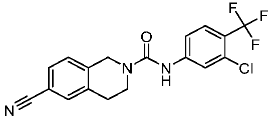 | (39) | 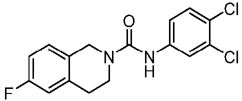 | (42) |
| 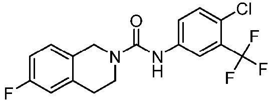 | (43) | 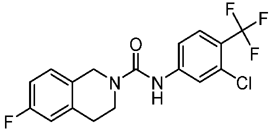 | (44) |
| 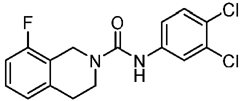 | (45) | 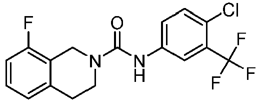 | (46) |
| 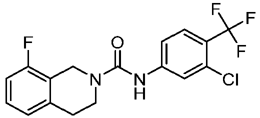 | (47) | | |
| 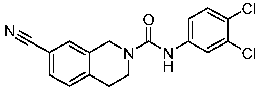 | (49) | 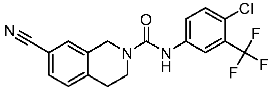 | (50) |
| 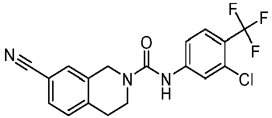 | (51) | 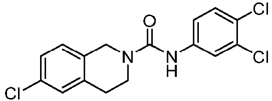 | (52) |
| 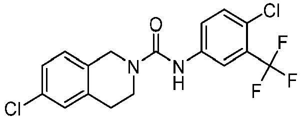 | (53) | 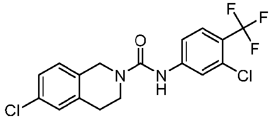 | (54) |
| 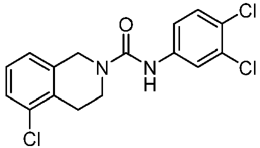 | (55) | 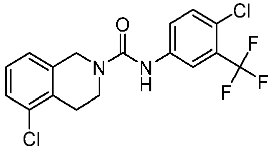 | (56) |
| 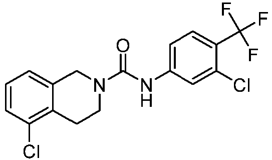 | (57) | 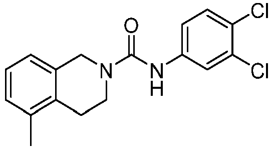 | (58) |
| 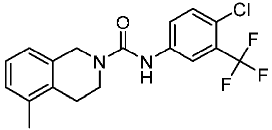 | (59) | 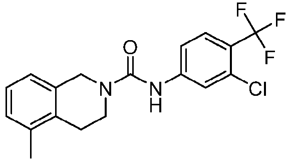 | (60) |
| | | 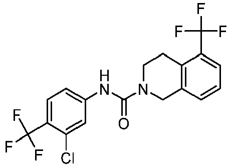 | (70) |
| 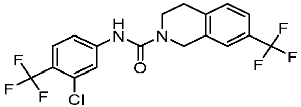 | (71) | 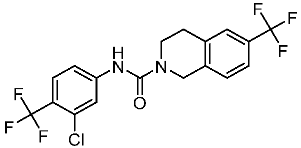 | (72) |
| 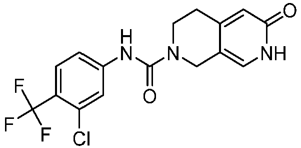 | (74) | 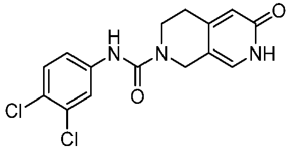 | (76) |
| 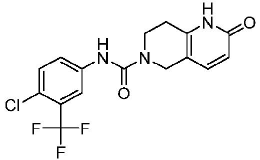 | (77) | 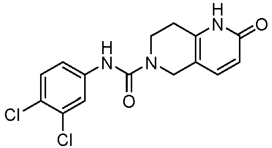 | (78) |
| 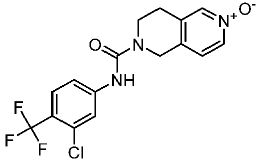 | (79) | | |
| 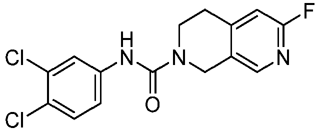 | (85) | 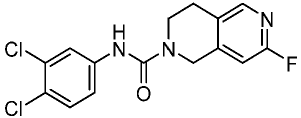 | (86) |
| 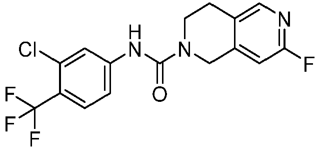 | (87) | 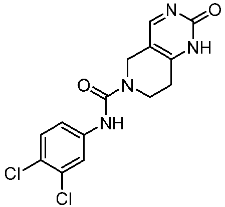 | (88) |
| 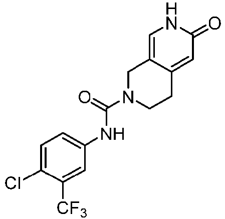 | (89) | 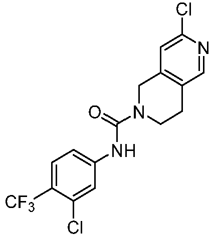 | (90) |
| 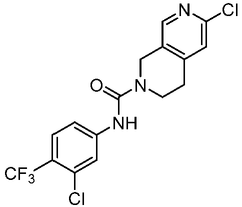 | (91) | 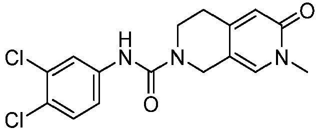 | (92) |
| 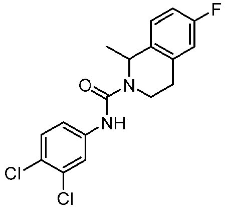 | (93) | 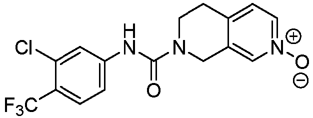 | (94) |
| 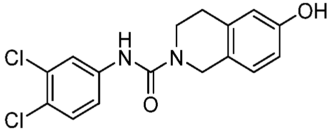 | (95) | 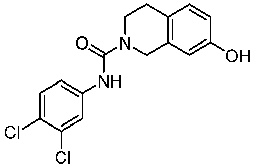 | (96) |
| 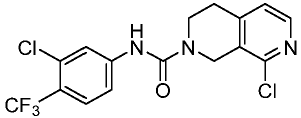 | (97) | 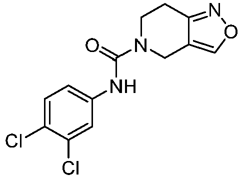 | (98) |
| 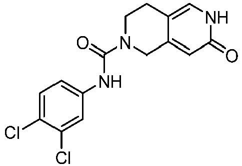 | (99) | 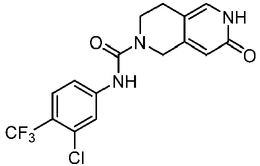 | (100) |
| 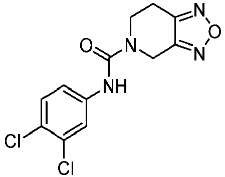 | (101) | 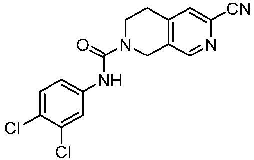 | (102) |
| 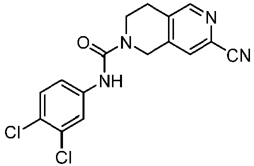 | (103) | 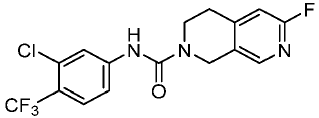 | (104) |
| 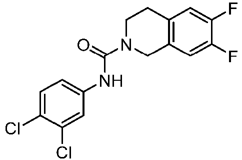 | (106) | 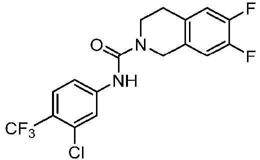 | (108) |
| 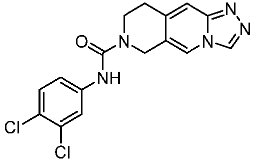 | (109) | 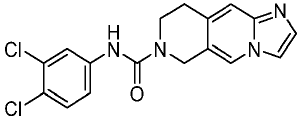 | (110) |
| 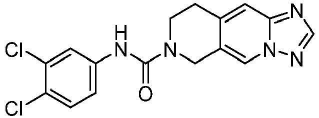 | (111) | 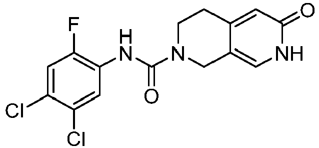 | (112) |
| 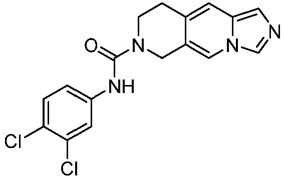 | (113) | | |
| 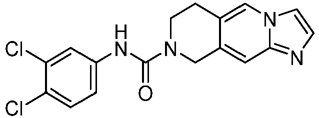 | (115) | 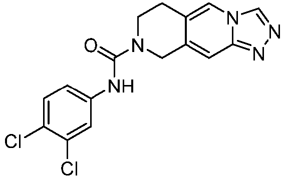 | (116) |
| 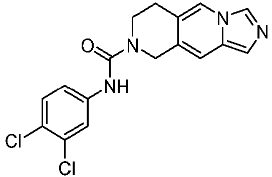 | (117) | 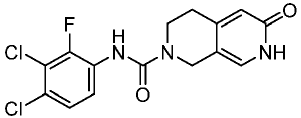 | (118) |
| | | 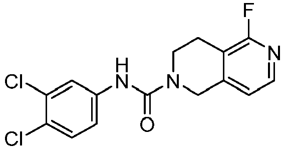 | (120) |
| 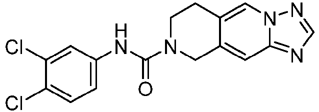 | (121) | 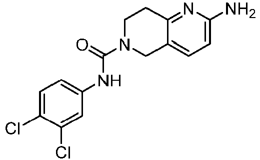 | (122) |
| 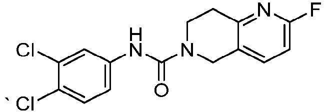 | (123) | 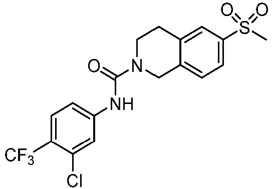 | (124) |
| 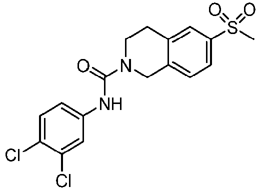 | (125) | | |
| | | 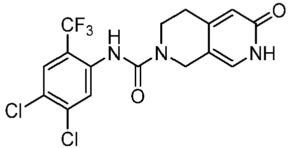 | (130) |
| | | 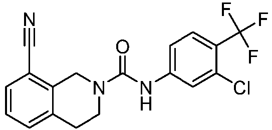 | (132) |
| 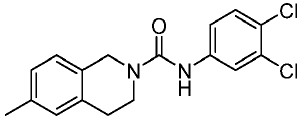 | (135) | 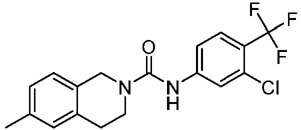 | (136) |
| 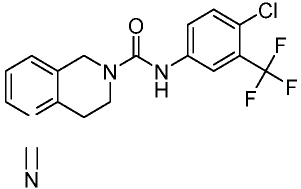 | (137) | 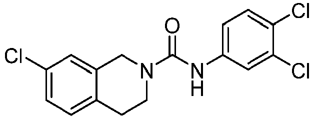 | (138) |
| 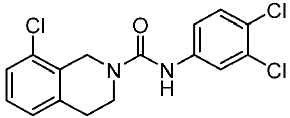 | (139) | | |

and pharmaceutically acceptable salts and solvates thereof.

### Compounds of formula (Ia') and (Ib')

One aspect of the invention relates to a compound of formula (Ia'), or a pharmaceutically acceptable salt or solvate thereof, 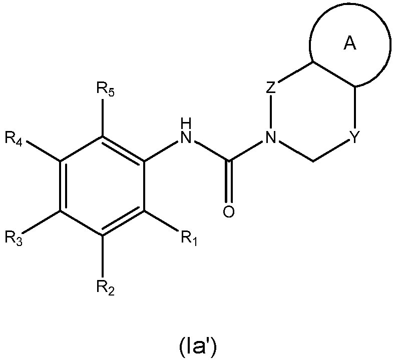 wherein:
ring A is a 6-membered monocyclic aromatic or heteroaromatic ring, wherein said aromatic or heteroaromatic ring is optionally substituted with one or more substituents selected from F, CI, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, and haloalkyl;
wherein at least two of R₂, R₃ and R₄ are other than H; and
R₁₁ and R₁₁' are each independently selected from H, alkyl, haloalkyl, COR₁₂, and SO₂R₁₃, wherein R₁₂ and R₁₃ are both alkyl;
wherein the compound is other than:
*N*-(3,4-Dichlorophenyl)-3,4-dihydroisoquinoline-2(1H)-carboxamide;
*N*-(3-Chlorophenyl)-3,4-dihydro-2(1H)-isoquinolinecarboxamide;
*N*-(4-Chlorophenyl)-3,4-dihydroisoquinoline-2(1H)-carboxamide; and
*N*-(2,4-Dichlorophenyl)-3,4-dihydroisoquinoline-2(1H)-carboxamide.

Another aspect of the invention relates to a compound of formula (Ib'), or a pharmaceutically acceptable salt or solvate thereof, 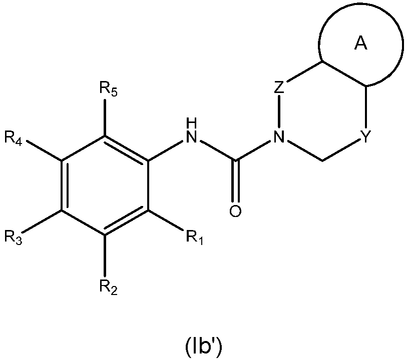 wherein:
ring A is a 6-membered monocyclic aromatic or heteroaromatic ring, wherein said aromatic or heteroaromatic ring is optionally substituted with one or more substituents selected from F, CI, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, and haloalkyl;
wherein at least two of R₂, R₃ and R₄ are other than H; and
R₁₁ and R₁₁' are each independently selected from H, alkyl, haloalkyl, COR₁₂, and SO₂R₁₃, wherein R₁₂ and R₁₃ are both alkyl;
wherein when ring A is: 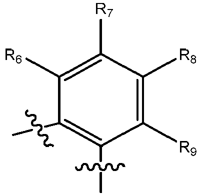
where R₆, R₇, R₈, and R₉ are all H, Z and Y are CH₂,
and R₂ and R₃ are each independently selected from F, CI, Br, I, CN, and haloalkyl, R₂ and R₃ are not both Cl;
wherein the compound is other than:
   *N*-(3,4-Dichlorophenyl)-7,8-dihydropyrido[4,3-d]pyrimidine-6(5H)-carboxamide;
   *N*-(3,4-Dichlorophenyl)-5,8-dihydropyrido[3,4-d]pyrimidine-7(6H)-carboxamide;
   *N*-(4-Chloro-3-(trifluoromethyl)phenyl)-3,4-dihydroisoquinoline-2(1H)-carboxamide; and
   *N*-(3,4-dichlorophenyl)-3,4-dihydroisoquinoline-2(1H)-carboxamide.

In formulae (Ia') and (Ib'), preferably alkyl is C₁₋C₆ alkyl, haloalkyl is C₁₋C₆ haloalkyl, and alkoxy is C₁₋C₆ alkoxy.

In one preferred embodiment, the optionally substituted aromatic or heteroaromatic ring is a benzene, pyridine, pyridine N-oxide, pyridazine, pyrimidine, pyrazine, triazine ring. The term "heteroaromatic" as used herein also encompasses moeties that exist in tautomeric form, such as, but not limited to, pyridone, pyrimidone and the like. The aromatic or heteroaromatic ring A is fused with the adjacent nitrogen-containing heterocyclic group to form a fused bicyclic ring system.

Preferably, the optionally substituted aromatic or heteroaromatic ring is a benzene, pyridine, pyridone, pyridine N-oxide, pyrimidine, pyrimidone, pyridazine, or pyrazine ring.

In one preferred embodiment, ring A is a benzene, pyridine, pyridone, pyridine N-oxide, pyrimidine, pyrimidone, pyridazine, or pyrazine ring that is optionally substituted with one or more substituents selected from F, Cl, Br, I, CN, C₁-C₆ alkoxy, NR₁₁R₁₁', OH, C₁-C₆ alkyl, phenyl, and C₁-C₆ haloalkyl.

In one preferred embodiment, ring A is selected from: 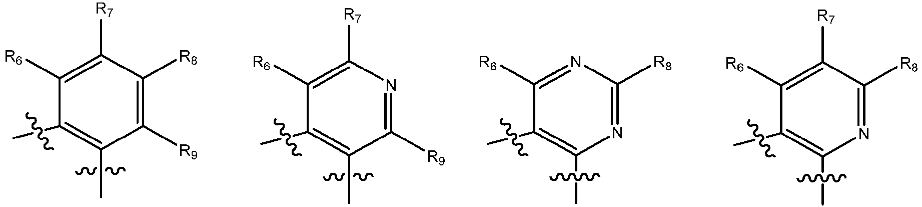 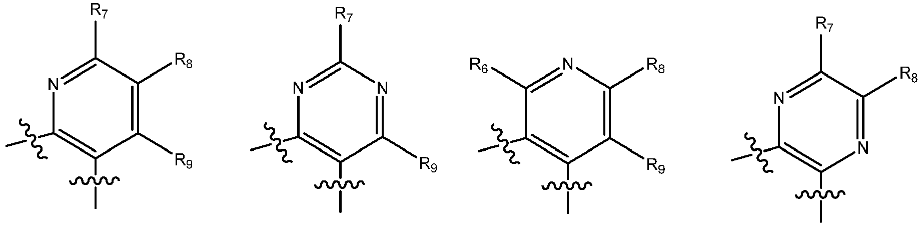 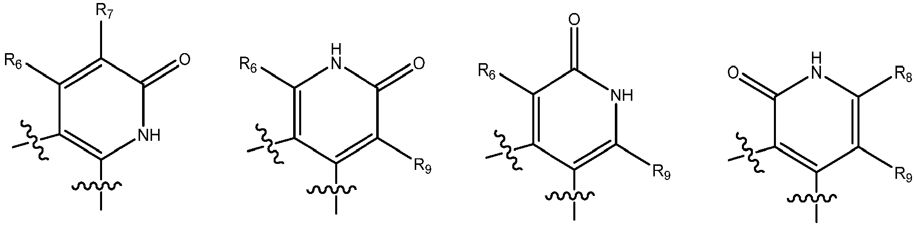 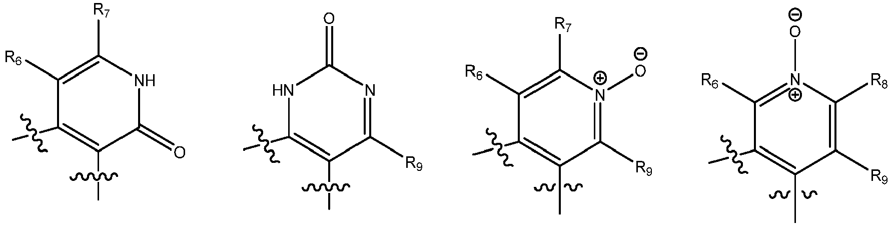 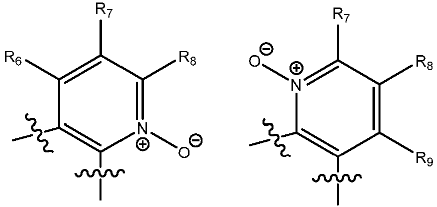 wherein R₆, R₇, R₈, and R₉ are each independently selected from H, F, Cl, Br, I, CN, C₁-C₆ alkoxy, NR₁₁R₁₁', OH, C₁-C₆ alkyl, phenyl, and C₁-C₆ haloalkyl.

In one preferred embodiment, ring A is selected from:

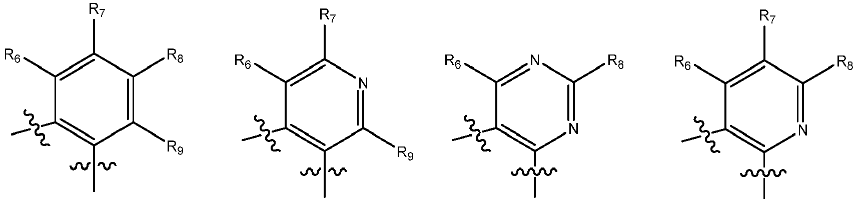

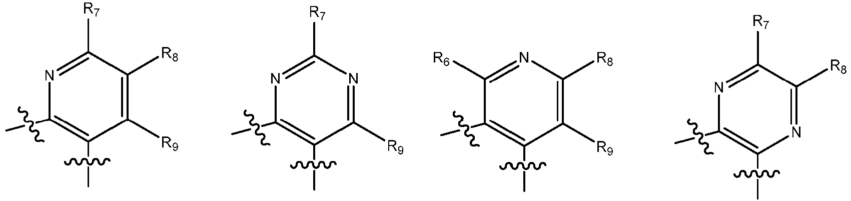

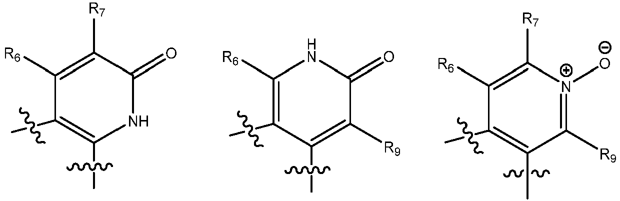

In one preferred embodiment, ring A is selected from:

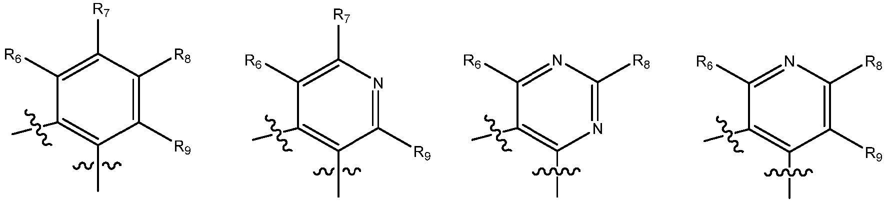

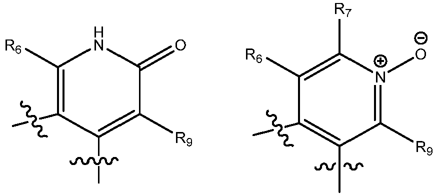

In one preferred embodiment, ring A is selected from:

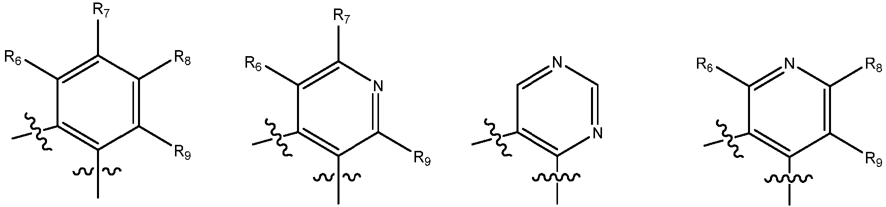

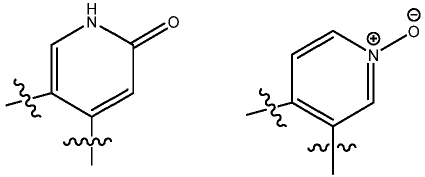

In one preferred embodiment, Y and Z are each independently selected from CH₂, CF₂, C(CH₃)₂, C(CF₃)₂, and are preferably both CH₂.

In one preferred embodiment, Y is CH₂.

In one preferred embodiment, Z is CH₂.

In one preferred embodiment, R₁ is selected from H and F, and is preferably H.

In one preferred embodiment, R₂ and R₃ are each independently selected from F, CI, Br, I, CN, and C₁-C₆ haloalkyl.

In one preferred embodiment, R₂ and R₃ are each independently selected from F, CI, Br, I, CN, and CFₙH₃₋ₙ, where n is 1, 2, or 3, and is preferably 3

In one preferred embodiment, R₂ and R₃ are each independently selected from CI, Br, and CFₙH₃₋ₙ, where n is 1, 2, or 3, and is preferably 3.

In one preferred embodiment, R₂ and R₃ are each independently selected from Cl and CF₃, preferably wherein R₂ and R₃ are not both CF₃, and more preferably wherein R₂ is Cl and R₃ is CF₃ or R₂ is Cl and R₃ is Cl.

In one preferred embodiment, R₄ is selected from H and CI, and is preferably H.

In one preferred embodiment, R₅ is H.

In one preferred embodiment, R₆ is selected from H, F, Cl, CN, methoxy, CH₃, NR₁₁R₁₁', and CF₃, wherein R₁₁ and R₁₁' are each independently selected from H and C₁-C₆ alkyl, and are preferably both H.

In one preferred embodiment, R₆ is selected from H, F, Cl, CN, methoxy, and CH₃, and is preferably H.

In one preferred embodiment, R₇ is selected from H, F, Cl, CN, methoxy, CH₃, NR₁₁R₁₁', and CF₃, wherein R₁₁ and R₁₁' are each independently selected from H and C₁-C₆ alkyl, and are preferably both H.

In one preferred embodiment, R₇ is selected from H, NH₂, F, CI, CN, methoxy, CH₃, and CF₃, preferably H, NH₂, F, or Cl, and is more preferably H.

In one preferred embodiment, R₈ is selected from H, F, OH, CN, methoxy, NR₁₁R₁₁', phenyl, CF₃, CF₂H, NHSO₂CH₃, NHCOCH₃, and NHCHF₂, wherein R₁₁ and R₁₁' are each independently selected from H and C₁-C₆ alkyl and are preferably both H.

In one preferred embodiment, R₈ is selected from H, F, Cl, CN, methoxy, CH₃, and CF₃, preferably from H, F, Cl, and CN, and more preferably from F and Cl.

In one preferred embodiment, R₈ is F.

In one preferred embodiment, R₉ is selected from H, F, Cl, CN, methoxy, CH₃, NR₁₁R₁₁', and CF₃, wherein R₁₁ and R₁₁' are each independently selected from H and C₁-C₆ alkyl, and are preferably both H.

In one preferred embodiment, R₉ is selected from H, F, Cl, CN, methoxy, CH₃, and CF₃, preferably H, F, and CN, and R₉ is more preferably H.

In a particularly preferred embodiment, for the compound of formula (Ia') or (Ib'):
ring A is selected from: 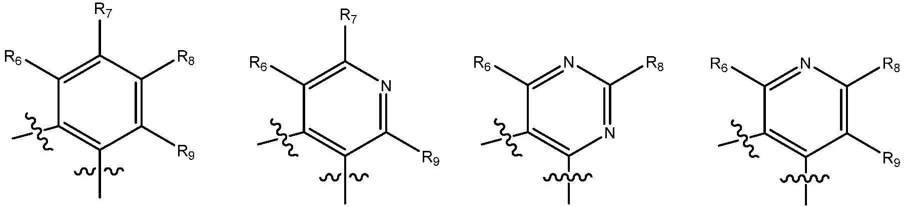 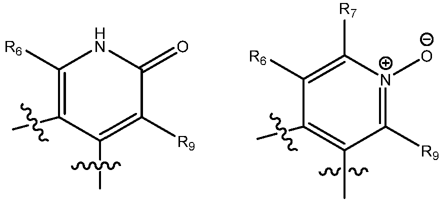
Y and Z are both CH₂;
R₁, R₄, and R₅ are all H;
R₂ and R₃ are each independently selected from Cl and CF₃, preferably wherein R₂ and R₃ are not both CF₃, and more preferably wherein R₂ is Cl and R₃ is CF₃ or R₂ is Cl and R₃ is Cl;
R₆ is selected from H, F, Cl, CN, methoxy, and CH₃, and is preferably H;
R₇ is selected from H, NH₂, F, Cl, CN, methoxy, CH₃, and CF₃, preferably H, NH₂, F, or Cl, and is more preferably H;
R₈ is selected from H, F, Cl, CN, methoxy, CH₃, and CF₃, preferably from H, F, Cl, and CN, and more preferably from F and Cl; and
R₉ is selected from H, F, Cl, CN, methoxy, CH₃, and CF₃, preferably H, F, and CN, and R₉ is more preferably H.

In an even more preferred embodiment, for the compound of formula (Ia') or (Ib'):
ring A is selected from: 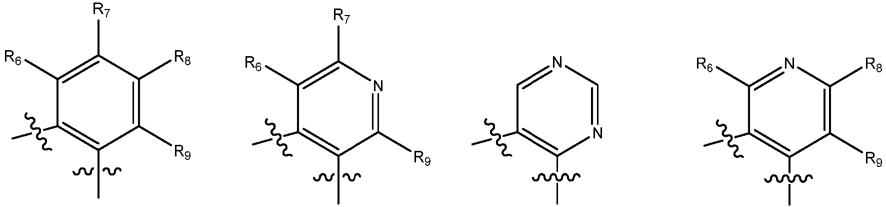 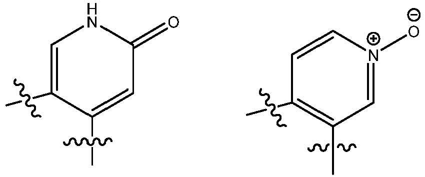
Y and Z are both CH₂;
R₁, R₄, and R₅ are all H;
R₂ and R₃ are each independently selected from Cl and CF₃, preferably wherein R₂ and R₃ are not both CF₃, and more preferably wherein R₂ is Cl and R₃ is CF₃ or R₂ is Cl and R₃ is Cl;
R₆ is selected from H, F, Cl, CN, methoxy, and CH₃, and is preferably H;
R₇ is selected from H, NH₂, F, Cl, CN, methoxy, CH₃, and CF₃, preferably H, NH₂, F, and Cl, and R₇ is more preferably H;
R₈ is selected from H, F, Cl, CN, methoxy, CH₃, and CF₃, preferably from H, F, Cl, and CN, and more preferably from F and Cl; and
R₉ is selected from H, F, Cl, CN, methoxy, CH₃, and CF₃, preferably H, F, and CN, and R₉ is more preferably H.

### Compounds of formula (Ic')

Another aspect of the invention relates to a compound of formula (Ic'), or a pharmaceutically acceptable salt or solvate thereof, 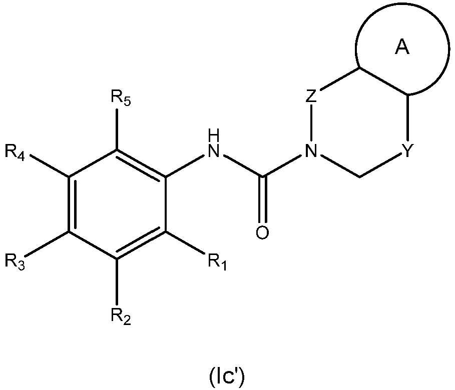 wherein:
ring A is a 6-membered monocyclic aromatic or heteroaromatic ring,
   wherein said aromatic ring is substituted with one or more substituents selected from F, Cl, Br, I, CN, alkoxy, NR₁₁R₁₁', OH, alkyl, haloalkyl, aralkyl, aryl, and heteroaryl, and wherein said aryl or heteroaryl substituents are in turn optionally substituted with one or more substituents each independently selected from F, Cl, Br, I, CN, alkoxy, NR₁₁R₁₁', OH, alkyl, haloalkyl, and aralkyl;
   wherein said heteroaromatic ring is optionally substituted with one or more substituents selected from F, CI, Br, I, CN, alkoxy, NR₁₁R₁₁', OH, alkyl, haloalkyl, aralkyl, aryl, and heteroaryl, and wherein said aryl or heteroaryl substituents are in turn optionally substituted with one or more substituents each independently selected from F, CI, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, and I;
R₂ and R₃ are each independently selected from H, F, Cl, Br, I, CN, and haloalkyl;
wherein at least two of R₂, R₃ and R₄ are other than H; and
R₁₁ and R₁₁' are each independently selected from H, alkyl, haloalkyl, COR₁₂, and SO₂R₁₃, wherein R₁₂ and R₁₃ are both alkyl;
wherein the compound is other than:
*N*-(3,4-Dichlorophenyl)-7,8-dihydropyrido[4,3-d]pyrimidine-6(5H)-carboxamide;
*N*-(3,4-Dichlorophenyl)-5,8-dihydropyrido[3,4-d]pyrimidine-7(6H)-carboxamide;
*N*-(4-Chloro-3-(trifluoromethyl)phenyl)-3,4-dihydroisoquinoline-2(1H)-carboxamide; and
*N*-(3,4-dichlorophenyl)-3,4-dihydroisoquinoline-2(1H)-carboxamide.

In one preferred embodiment, ring A is a substituted benzene group, or an optionally substituted 6-membered heteroaromatic group.

Preferred aspects as defined above for formula (Ia') and (Ib') apply equally to compounds of formula (Ic').

Exemplary compounds of formula (Ic') include the following compounds as described herein: 1-9, 12-24, 27-39, 42-47, 49-67, 70-79 and 82-87 and pharmaceutically acceptable salts and solvates thereof.

### PROCESS

Also disclosed herein is a process for preparing a compound of formula (I), (Ia), (Ia'), (Ib') or (Ic') as defined herein, said process comprising reacting a compound of formula (II) with a compound of formula (III), where R¹⁻⁵, Z, Y and A are as defined above, to form a compound of formula (Ia), (la'), (Ib') or (Ic'):

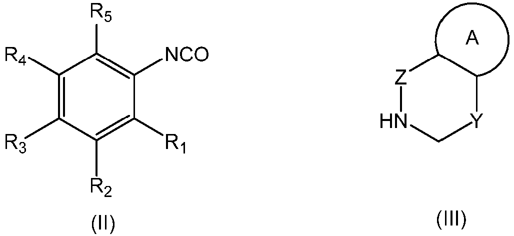

Preferably, the reaction takes place in the presence of a base, preferably, N,N-diisopropylethylamine (DIPEA) or triethylamine. Preferably, the reaction takes place in an organic solvent. Suitable organic solvents include, but are not limited to, dichloromethane, tetrahydrofuran and dimethylformamide, or mixtures of two or more thereof. The skilled person would understand that other bases and solvents would also be suitable.

### THERAPEUTIC APPLICATIONS

A further aspect of the invention relates to compounds as described herein for use in medicine. The compounds have particular use in the field of oncology, immuno-oncology, and immunology as described in more detail below. In a preferred embodiment, the compound of the invention modulates GPR65, and more preferably inhibits GPR65 signalling.

Yet another aspect of the invention relates to compounds as described herein for use as a medicament.

One aspect of the invention relates to a compound of formula (I), or a pharmaceutically acceptable salt or solvate thereof, 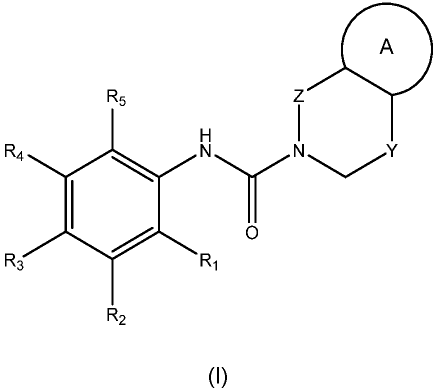 wherein:
ring A is a 5- or 6-membered monocyclic aromatic or heteroaromatic ring, or a 9- or 10-membered bicyclic aromatic or heteroaromatic ring, each of which is optionally substituted with one or more substituents selected from F, CI, Br, I, CN, alkoxy, NR₁₁R₁₁', OH, SO₂-alkyl, CO₂-alkyl, alkyl, haloalkyl, aralkyl, aryl, and heteroaryl, and wherein said aryl and heteroaryl substituents are in turn optionally substituted with one or more substituents each independently selected from F, CI, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, CI, Br, I and haloalkyl;
R₂ and R₃ are each independently selected from CI, Br, I, and haloalkyl;
   and
R₁₁ and R₁₁' are each independently selected from H, alkyl, haloalkyl, COR₁₂, and SO₂R₁₃, wherein R₁₂ and R₁₃ are both alkyl;
for use in treating or preventing a disorder selected from a proliferative disorder, an immune disorder, asthma, chronic obstructive pulmonary disease (COPD) and acute respiratory distress syndrome (ARDS), wherein the compound modulates GPR65, preferably wherein the compound inhibits GPR65 signalling.

Preferred definitions for ring A and groups Y, Z, and R₁₋₁₃ are as set out above for compounds of formula (Ia), (la'), (Ib') and (Ic').

In formulae (I), preferably alkyl is C₁₋C₆ alkyl, haloalkyl is C₁₋C₆ haloalkyl, and alkoxy is C₁₋C₆ alkoxy.

Preferably, the compounds of formula (I) are for use in treating or preventing a disease or disorder selected from a proliferative disorder, an autoimmune disorder, asthma and chronic obstructive pulmonary disease.

One preferred embodiment of the invention relates to compounds as described herein for use in treating or preventing a disorder selected from a proliferative disorder and an immune disorder.

Another preferred embodiment of the invention relates to compounds as described herein for use in treating or preventing asthma and/or chronic obstructive pulmonary disease (COPD). GPR65 variant/SNP (rs6574978) has been shown to be associated with asthma/COPD syndrome with almost GWAS significant p value (1.18x10e-7) (Hardin 2014). Furthermore, GPR65 activation by pH (pH is low/acidic in asthmatic lungs) promotes eosinophil viability in a cAMP-dependent manner, contributing to disease progression/exacerbation. It is further known that GPR65 KO mice have attenuated asthma symptoms (Kottyan 2009).

Another aspect of the invention relates to compounds as described herein for use in treating or preventing acute respiratory distress syndrome (ARDS). GPR65 has been shown to be protective in a model of LPS-induced acute lung injury model (Tsurumaki 2015).

One aspect of the invention relates to a compound as described herein for use in treating a proliferative disorder. Preferably, the proliferative disorder is a cancer or leukemia.

In one preferred embodiment, the cancer is a solid tumour and/or metastases thereof.

In another preferred embodiment, the cancer is selected from melanoma, renal cell carcinoma (RCC), gastric cancer, acute myeloid leukaemia (AML), pancreatic adenocarcinoma, triple negative breast cancer (TNBC), colorectal cancer, head and neck cancer, colorectal adenocarcinoma, lung cancer sarcoma, ovarian cancer, and gliomas, preferably glioblastoma (GBM).

Without wishing to be bound by theory, it is understood that GPR65 modulators are capable of preventing the increase in cytoplasmic cAMP in tumour-associated macrophages (TAMs), natural killer (NK) cells and subsets of T cells that would typically result from their exposure to the acidic tumour microenvironment and concomitant GPR65 activation. This reduction in the level of cytoplasmic cAMP in turn reduces the levels of ICER pro-inflammatory mediators such as CXCL10 and and TNFα, preventing the polarization of TAMs and alteration of other immune cells that are associated with a non-inflammatory and tumour-permissive environment. Therefore, GPR65 modulators are expected to result in an increase in the visibility of the tumour to the immune system leading to increased immune-mediated tumour clearance. This suggests that modulation of GPR65 activity could be an effective treatment for cancer as stand-alone therapy or in combination with cancer immunotherapies (vaccines, agents that promote T cell mediated immune responses) or in patients that do not respond to immunomodulatory approaches such as PD1/PDL-1 blockade.

Another aspect of the invention relates to a compound as described herein for use in treating an immune disorder, preferably an autoimmune disease.

In one embodiment, the autoimmune disease is selected from psoriasis, psoriatic arthritis, rheumatoid arthritis (RA), multiple sclerosis (MS), systemic lupus erythematosus (SLE), autoimmune thyroiditis (Hashimoto's thyroiditis), Graves' disease, uveitis (including intermediate uveitis), ulcerative colitis, Crohn's disease, autoimmune uveoretinitis, systemic vasculitis, polymyositis-dermatomyositis, systemic sclerosis (scleroderma), Sjogren's Syndrome, ankylosing spondylitis and related spondyloarthropathies, sarcoidosis, autoimmune hemolytic anemia, immunological platelet disorders, autoimmune polyendocrinopathies and autoimmune myocarditis, type I diabetes and atopic dermatitis

In a particularly preferred embodiment, the autoimmune disease is selected from psoriasis, psoriatic arthritis, ankylosing spondylitis, Crohn's disease, and multiple sclerosis (MS).

Without wishing to be bound by theory, it is understood that GPR65 modulators will prevent the upregulation of ICER in CD4+ T cells. This, in turn, is expected to prevent the ICER-associated suppression of IL-2 that biases CD4+ T cells toward the inflammatory Th17 phenotype associated with increased pathogenicity in the context of autoimmune disease. This is supported by the fact that mutations in the GPR65 locus are associated with several autoimmune diseases, such as multiple sclerosis, ankylosing spondylitis, inflammatory bowel disease, and Crohn's disease (Gaublomme, 2015). This suggests that modulation of GPR65 activity could be an effective treatment for autoimmune diseases.

Another aspect relates to a compound as described herein for use in treating or preventing a disorder caused by, associated with or accompanied by abnormal activity against GPR65.

Another aspect relates to a compound as described herein for use in treating or preventing a GPR65-associated disease or disorder.

Preferably, the compound as described herein is for use in treating a subject, wherein the subject is a mammal, more preferably a human.

The term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

Herein, the term "treating" includes abrogating, substantially inhibiting, slowing or reversing the progression of a disease or disorder, substantially ameliorating clinical symptoms of a disease or disorder or substantially preventing the appearance of clinical symptoms of a disease or disorder.

Herein, the term "preventing" refers to a method for barring an organism from acquiring a disorder or disease in the first place.

The term "therapeutically effective amount" refers to that amount of the compound being administered which will relieve to some extent one or more of the symptoms of the disease or disorder being treated.

For any compound used in this invention, a therapeutically effective amount, also referred to herein as a therapeutically effective dose, can be estimated initially from cell culture assays. For example, a dose can be formulated in animal models to achieve a circulating concentration range that includes the IC₅₀ or the IC₁₀₀ as determined in cell culture. Such information can be used to more accurately determine useful doses in humans. Initial dosages can also be estimated from *in vivo* data. Using these initial guidelines one of ordinary skill in the art could determine an effective dosage in humans.

Moreover, toxicity and therapeutic efficacy of the compounds described herein can be determined by standard pharmaceutical procedures in cell cultures or experimental animals, e.g., by determining the LD₅₀ and the ED₅₀. The dose ratio between toxic and therapeutic effect is the therapeutic index and can be expressed as the ratio between LD₅₀ and ED₅₀. Compounds which exhibit high therapeutic indices are preferred. The data obtained from these cell cultures assays and animal studies can be used in formulating a dosage range that is not toxic for use in human. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (see, e.g., Fingl *et al,* 1975, The Pharmacological Basis of Therapeutics, chapter 1, page 1).

Dosage amount and interval may be adjusted individually to provide plasma levels of the active compound which are sufficient to maintain therapeutic effect. Usual patient dosages for oral administration range from about 50-2000 mg /day, commonly from about 100-1000 mg/ day, preferably from about 150-700 mg/day and most preferably from about 250-500 mg/day, or from 50-100 mg/day. Preferably, therapeutically effective serum levels will be achieved by administering multiple doses each day. In cases of local administration or selective uptake, the effective local concentration of the drug may not be related to plasma concentration. One skilled in the art will be able to optimize therapeutically effective local dosages without undue experimentation.

As used herein, "GPR65-related disease or disorder" refers to a disease or disorder characterized by inappropriate GPR65 activity. Inappropriate GPR65 activity refers to either an increase or decrease in GPR65 activity as measured by enzyme or cellular assays, for example, compared to the activity in a healthy subject. Inappropriate activity could also be due to overexpression of GPR65 in diseased tissue compared with healthy adjacent tissue.

Preferred diseases or disorders that the compounds described herein may be useful in treating or preventing include proliferative disorders and immune disorders as described hereinbefore, as well as asthma and chronic obstructive pulmonary disease.

The present invention further provides for the use of compounds as defined herein in the preparation of a medicament for the treatment of a disease where it is desirable to modulate GPR65. Such diseases include proliferative disorders and immune disorders as described hereinbefore, as well as asthma and chronic obstructive pulmonary disease.

As used herein the phrase "preparation of a medicament" includes the use of the components of the invention directly as the medicament in addition to their use in any stage of the preparation of such a medicament.

In one preferred embodiment, the compound prevents the increase in cytoplasmic cAMP levels expected following GPR65 activation at acidic pH. This prevention of cAMP accumulation is expected in turn to prevent the undesirable downstream signalling through ICER, as described in the accompanying examples section. The "Human GPR65 cyclic adenosine monophosphate (cAMP) Homogeneous Time Resolved Fluorescence (HTRF) antagonist assay", or simply "cAMP assay", as described below, can be used to measure the potency of GPR65 modulators, which is expressed as the concentration of compound required to reduce the increase in cAMP concentration upon GPR65 activation by 50% (i.e. an IC₅₀).

In one preferred embodiment, the compound exhibits an IC₅₀ value in the cAMP assay of less than about 25 µM. More preferably, the compound exhibits an IC₅₀ value in the cAMP assay of less than about 10 µM, more preferably, less than about 5 µM, even more preferably, less than about 1 µM, even more preferably, less than about 0.1 µM.

In another preferred embodiment, the compound exhibits an hGPR65 IC₅₀ value of less than < 5 µM, more preferably less than < 500 nM in the aforementioned assay.

In another aspect of the invention a compound is provided selected from the following:

| | | | |
|---|---|---|---|
| 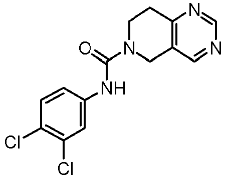 | (1) | 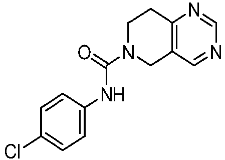 | (2) |
| 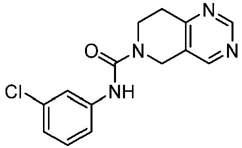 | (3) | 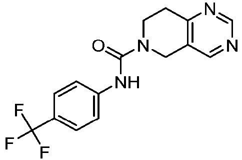 | (4) |
| 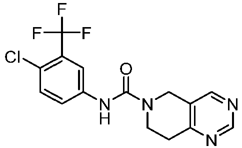 | (5) | 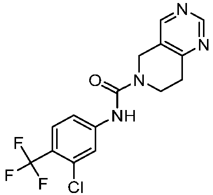 | (6) |
| 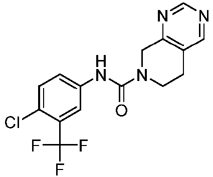 | (7) | 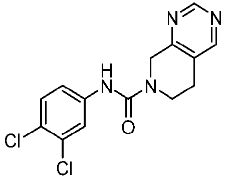 | (8) |
| 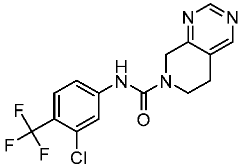 | (9) | 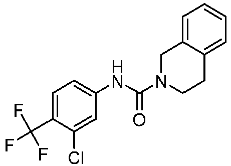 | (10) |
| 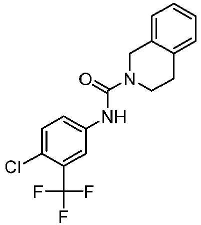 | (11) | 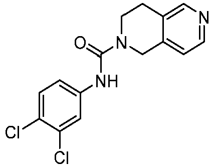 | (12) |
| 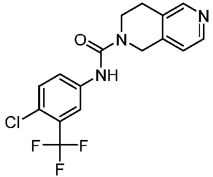 | (13) | 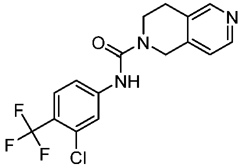 | (14) |
| 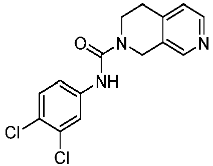 | (15) | 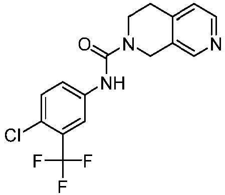 | (16) |
| 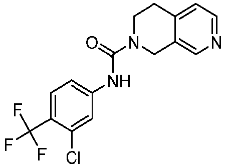 | (17) | 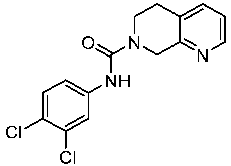 | (18) |
| 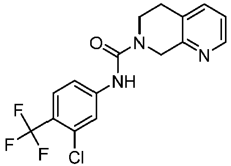 | (19) | 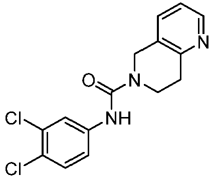 | (20) |
| 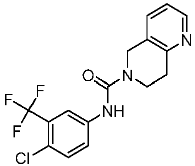 | (21) | 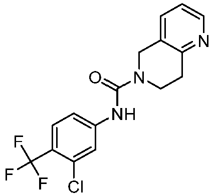 | (22) |
| 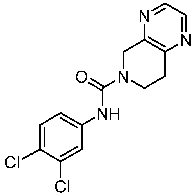 | (23) | 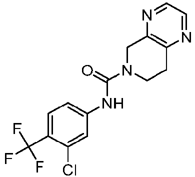 | (24) |
| 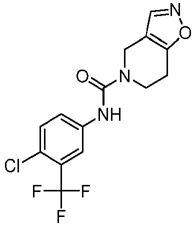 | (25) | 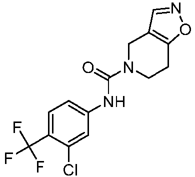 | (26) |
| 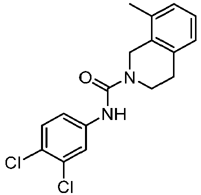 | (27) | 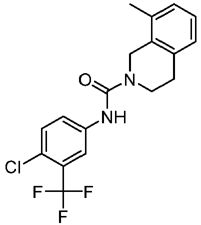 | (28) |
| 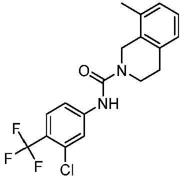 | (29) | 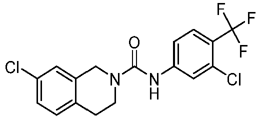 | (30) |
| 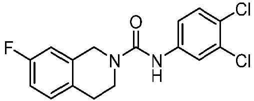 | (31) | 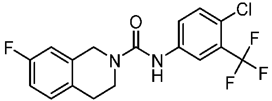 | (32) |
| 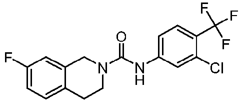 | (33) | 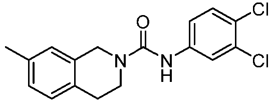 | (34) |
| 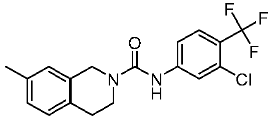 | (35) | 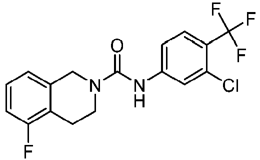 | (36) |
| 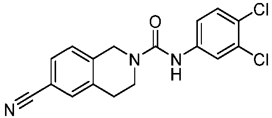 | (37) | 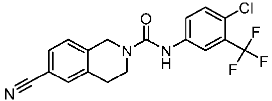 | (38) |
| 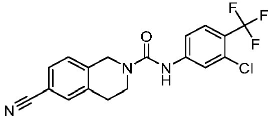 | (39) | 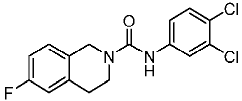 | (42) |
| 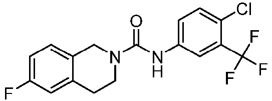 | (43) | 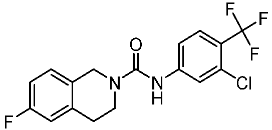 | (44) |
| 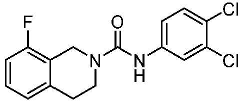 | (45) | 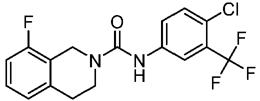 | (46) |
| 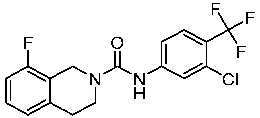 | (47) | | |
| 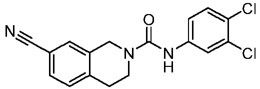 | (49) | 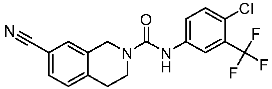 | (50) |
| 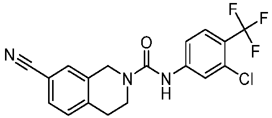 | (51) | 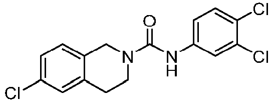 | (52) |
| 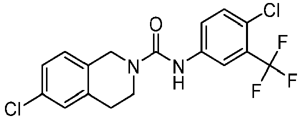 | (53) | 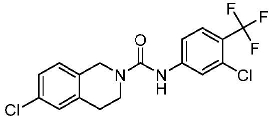 | (54) |
| 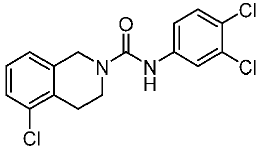 | (55) | 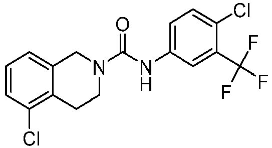 | (56) |
| 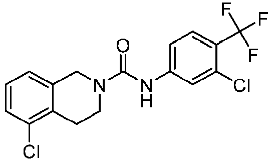 | (57) | 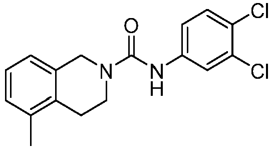 | (58) |
| 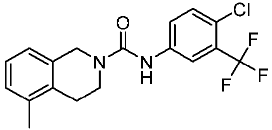 | (59) | 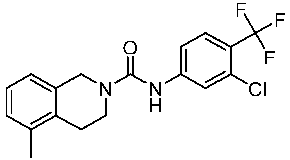 | (60) |
| 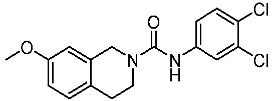 | (61) | 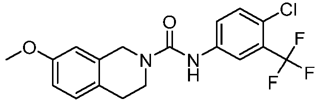 | (62) |
| 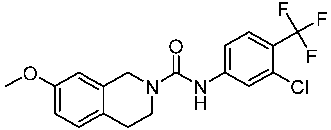 | (63) | 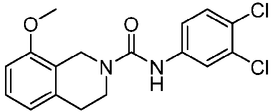 | (64) |
| 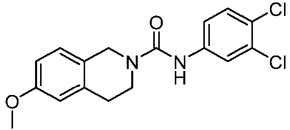 | (65) | 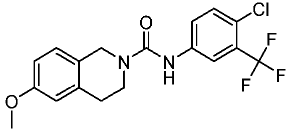 | (66) |
| 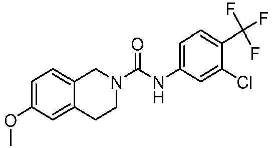 | (67) | 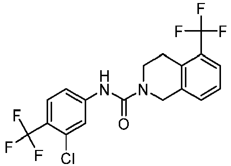 | (70) |
| 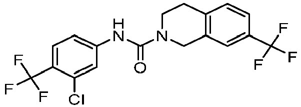 | (71) | 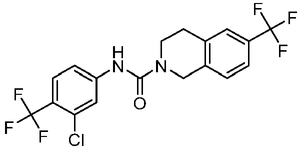 | (72) |
| 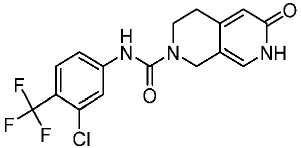 | (74) | 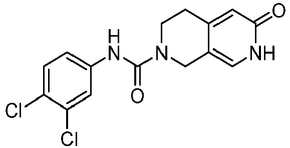 | (76) |
| 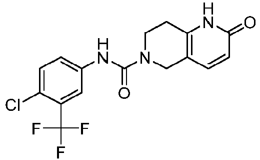 | (77) | 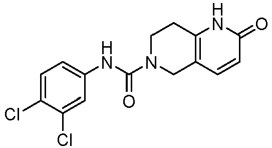 | (78) |
| 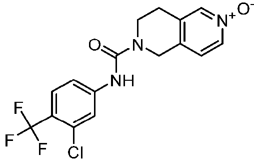 | (79) | 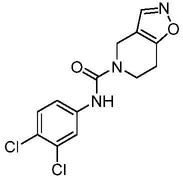 | (80) |
| 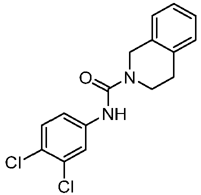 | (81) | 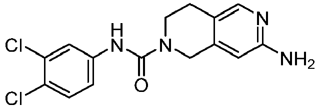 | (82) |
| 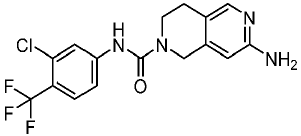 | (83) | 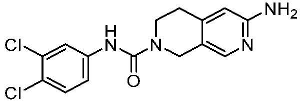 | (84) |
| 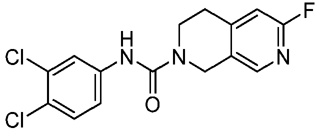 | (85) | 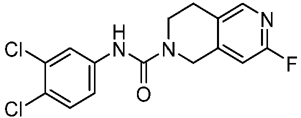 | (86) |
| 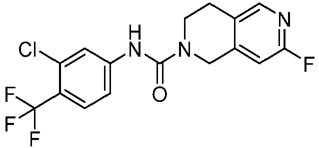 | (87) | 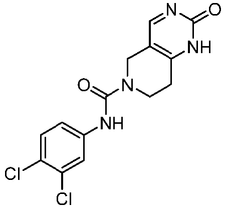 | (88) |
| 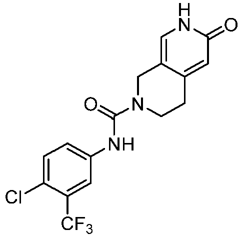 | (89) | 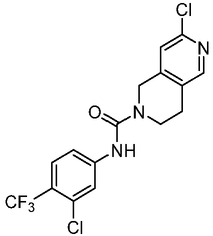 | (90) |
| 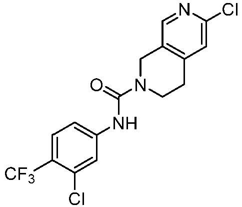 | (91) | 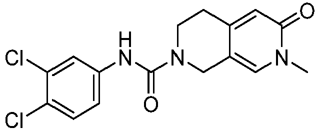 | (92) |
| 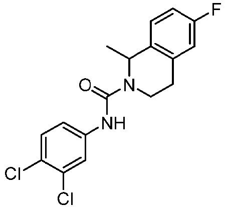 | (93) | 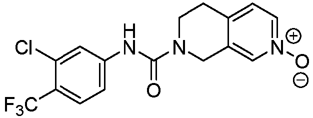 | (94) |
| 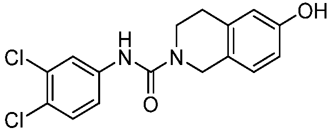 | (95) | 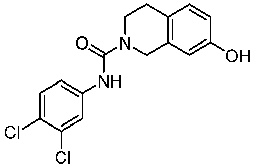 | (96) |
| 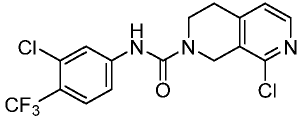 | (97) | 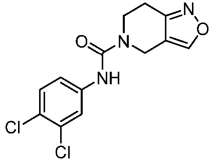 | (98) |
| 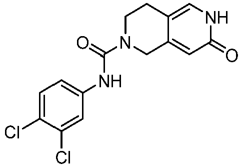 | (99) | 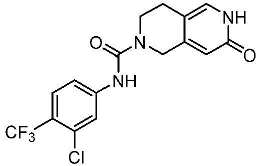 | (100) |
| 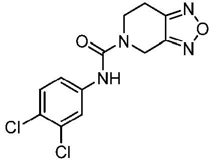 | (101) | 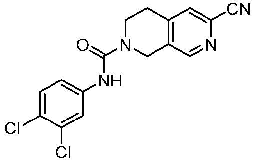 | (102) |
| 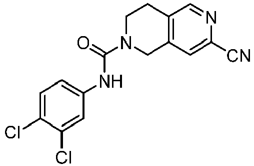 | (103) | 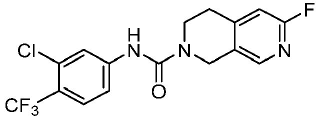 | (104) |
| 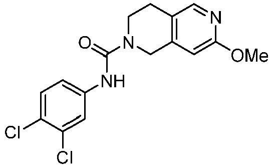 | (105) | 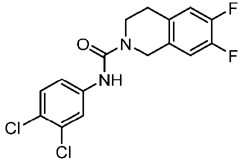 | (106) |
| 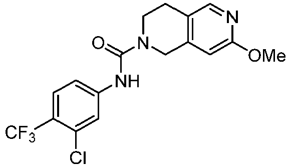 | (107) | 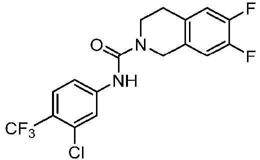 | (108) |
| 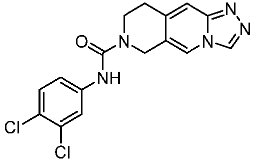 | (109) | 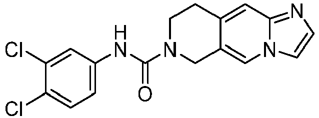 | (110) |
| 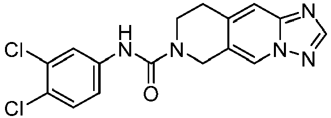 | (111) | 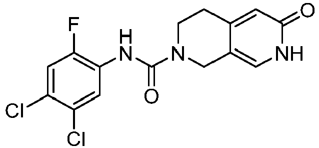 | (112) |
| 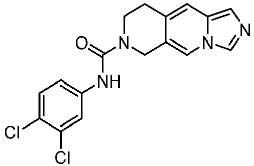 | (113) | 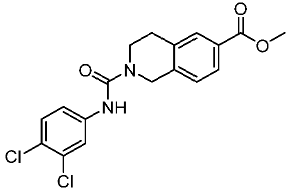 | (114) |
| 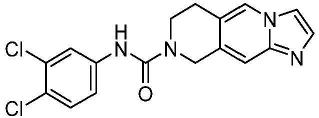 | (115) | 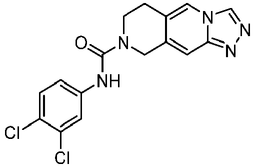 | (116) |
| 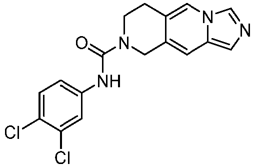 | (117) | 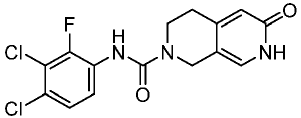 | (118) |
| 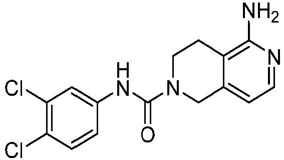 | (119) | 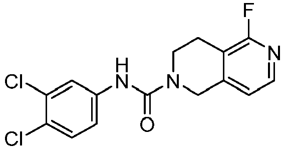 | (120) |
| 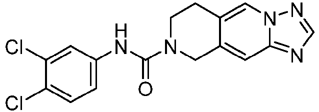 | (121) | 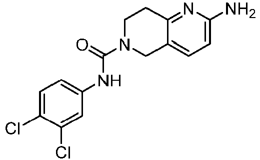 | (122) |
| 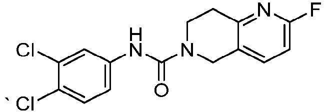 | (123) | 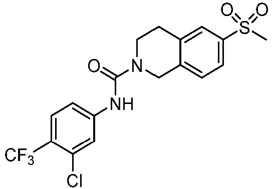 | (124) |
| 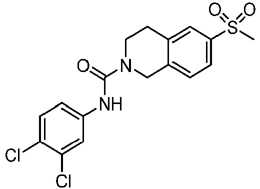 | (125) | 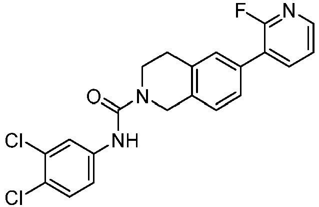 | (126) |
| 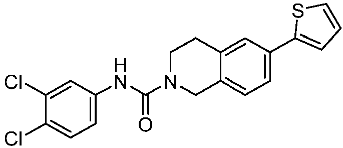 | (127) | 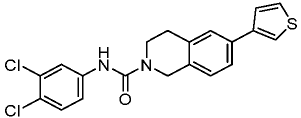 | (128) |
| 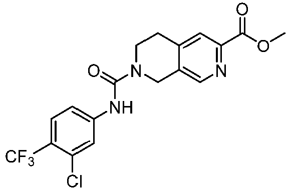 | (129) | 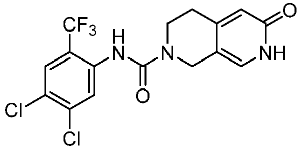 | (130) |
| 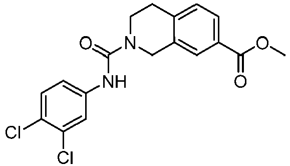 | (131) | 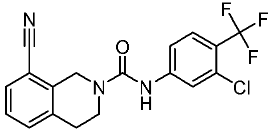 | (132) |
| 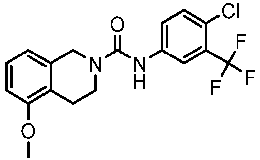 | (133) | 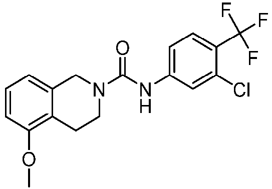 | (134) |
| 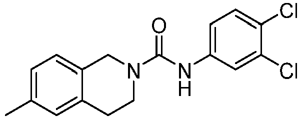 | (135) | 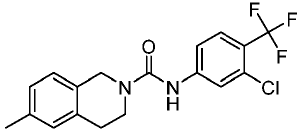 | (136) |
| 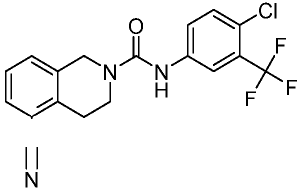 | (137) | 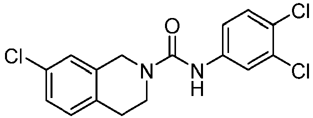 | (138) |
| 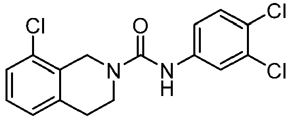 | (139) | 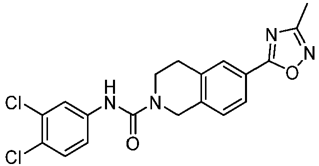 | (140) |

and pharmaceutically acceptable salts and solvates thereof, for use as a medicament.

Preferred compounds according to the invention, or for use according to the invention are alternatively described below:

| | |
|---|---|
| 1 | *N*-(3,4-Dichlorophenyl)-7,8-dihydropyrido[4,3-d]pyrimidine-6(5H)-carboxamide |
| 2 | *N*-(4-Chlorophenyl)-7,8-dihydropyrido[4,3-d]pyrimidine-6(5H)-carboxamide |
| 3 | *N*-(3-Chlorophenyl)-7,8-dihydropyrido[4,3-d]pyrimidine-6(5H)-carboxamide |
| 4 | *N*-(4-(Trifluoromethyl)phenyl)-7,8-dihydropyrido[4,3-d]pyrimidine-6(5H)-carboxamide |
| 5 | *N*-(4-Chloro-3-(trifluoromethyl)phenyl)-7,8-dihydropyrido[4,3-d]pyrimidine-6(5H)-carboxamide |
| 6 | *N*-(3-Chloro-4-(trifluoromethyl)phenyl)-7,8-dihydropyrido[4,3-d]pyrimidine-6(5H)-carboxamide |
| 7 | *N*-(4-Chloro-3-(trifluoromethyl)phenyl)-5,8-dihydropyrido[3,4-d]pyrimidine-7(6H)-carboxamide |
| 8 | *N*-(3,4-Dichlorophenyl)-5,8-dihydropyrido[3,4-d]pyrimidine-7(6H)-carboxamide |
| 9 | *N*-(3-Chloro-4-(trifluoromethyl)phenyl)-5,8-dihydropyrido[3,4-d]pyrimidine-7(6H)-carboxamide |
| 10 | *N*-(3-Chloro-4-(trifluoromethyl)phenyl)-3,4-dihydroisoquinoline-2(1H)-carboxamide |
| 11 | *N*-(4-Chloro-3-(trifluoromethyl)phenyl)-3,4-dihydroisoquinoline-2(1H)-carboxamide |
| 12 | *N*-(3,4-Dichlorophenyl)-3,4-dihydro-2,6-naphthyridine-2(1H)-carboxamide |
| 13 | *N*-(4-Chloro-3-(trifluoromethyl)phenyl)-3,4-dihydro-2,6-naphthyridine-2(1H)-carboxamide |
| 14 | *N*-(3-Chloro-4-(trifluoromethyl)phenyl)-3,4-dihydro-2,6-naphthyridine-2(1H)-carboxamide |
| 15 | *N*-(3,4-Dichlorophenyl)-3,4-dihydro-2,7-naphthyridine-2(1H)-carboxamide |
| 16 | *N*-(4-Chloro-3-(trifluoromethyl)phenyl)-3,4-dihydro-2,7-naphthyridine-2(1H)-carboxamide |
| 17 | *N*-(3-Chloro-4-(trifluoromethyl)phenyl)-3,4-dihydro-2,7-naphthyridine-2(1H)-carboxamide |
| 18 | *N*-(3,4-Dichlorophenyl)-5,8-dihydro-1,7-naphthyridine-7(6H)-carboxamide |
| 19 | *N*-(3-Chloro-4-(trifluoromethyl)phenyl)-5,8-dihydro-1,7-naphthyridine-7(6H)-carboxamide |
| 20 | *N*-(3,4-Dichlorophenyl)-7,8-dihydro-1,6-naphthyridine-6(5H)-carboxamide |
| 21 | *N*-(4-Chloro-3-(trifluoromethyl)phenyl)-7,8-dihydro-1,6-naphthyridine-6(5H)-carboxamide |
| 22 | *N*-(3-Chloro-4-(trifluoromethyl)phenyl)-7,8-dihydro-1,6-naphthyridine-6(5H)-carboxamide |
| 23 | *N*-(3,4-Dichlorophenyl)-7,8-dihydropyrido[3,4-b]pyrazine-6(5H)-carboxamide |
| 24 | *N*-(3-Chloro-4-(trifluoromethyl)phenyl)-7,8-dihydropyrido[3,4-b]pyrazine-6(5H)-carboxamide |
| 25 | *N*-(4-Chloro-3-(trifluoromethyl)phenyl)-6,7-dihydroisoxazolo[4,5-c]pyridine-5(4H)-carboxamide |
| 26 | *N*-(3-Chloro-4-(trifluoromethyl)phenyl)-6,7-dihydroisoxazolo[4,5-c]pyridine-5(4H)-carboxamide |
| 27 | *N*-(3,4-Dichlorophenyl)-8-methyl-3,4-dihydroisoquinoline-2(1H)-carboxamide |
| 28 | *N*-(4-Chloro-3-(trifluoromethyl)phenyl)-8-methyl-3,4-dihydroisoquinoline-2(1H)-carboxamide |
| 29 | *N*-(3-Chloro-4-(trifluoromethyl)phenyl)-8-methyl-3,4-dihydroisoquinoline-2(1H)-carboxamide |
| 30 | 7-Chloro-*N*-(3-chloro-4-(trifluoromethyl)phenyl)-3,4-dihydroisoquinoline-2(1H)-carboxamide |
| 31 | *N*-(3,4-Dichlorophenyl)-7-fluoro-3,4-dihydroisoquinoline-2(1H)-carboxamide |
| 32 | *N*-(4-Chloro-3-(trifluoromethyl)phenyl)-7-fluoro-3,4-dihydroisoquinoline-2(1H)-carboxamide |
| 33 | *N*-(3-Chloro-4-(trifluoromethyl)phenyl)-7-fluoro-3,4-dihydroisoquinoline-2(1H)-carboxamide |
| 34 | *N*-(3,4-Dichlorophenyl)-7-methyl-3,4-dihydroisoquinoline-2(1H)-carboxamide |
| 35 | *N*-(3-Chloro-4-(trifluoromethyl)phenyl)-7-methyl-3,4-dihydroisoquinoline-2(1H)-carboxamide |
| 36 | *N*-(3-Chloro-4-(trifluoromethyl)phenyl)-5-fluoro-3,4-dihydroisoquinoline-2(1H)-carboxamide |
| 37 | 6-Cyano-*N*-(3,4-dichlorophenyl)-3,4-dihydroisoquinoline-2(1H)-carboxamide |
| 38 | *N*-(4-Chloro-3-(trifluoromethyl)phenyl)-6-cyano-3,4-dihydroisoquinoline-2(1H)-carboxamide |
| 39 | *N*-(3-Chloro-4-(trifluoromethyl)phenyl)-6-cyano-3,4-dihydroisoquinoline-2(1H)-carboxamide |
| 42 | *N*-(3,4-Dichlorophenyl)-6-fluoro-3,4-dihydroisoquinoline-2(1H)-carboxamide |
| 43 | *N*-(4-Chloro-3-(trifluoromethyl)phenyl)-6-fluoro-3,4-dihydroisoquinoline-2(1H)-carboxamide |
| 44 | *N*-(3-Chloro-4-(trifluoromethyl)phenyl)-6-fluoro-3,4-dihydroisoquinoline-2(1H)-carboxamide |
| 45 | *N*-(3,4-Dichlorophenyl)-8-fluoro-3,4-dihydroisoquinoline-2(1H)-carboxamide |
| 46 | *N*-(4-Chloro-3-(trifluoromethyl)phenyl)-8-fluoro-3,4-dihydroisoquinoline-2(1H)-carboxamide |
| 47 | *N*-(3-Chloro-4-(trifluoromethyl)phenyl)-8-fluoro-3,4-dihydroisoquinoline-2(1H)-carboxamide |
| 49 | 7-Cyano-*N*-(3,4-dichlorophenyl)-3,4-dihydroisoquinoline-2(1H)-carboxamide |
| 50 | *N*-(4-Chloro-3-(trifluoromethyl)phenyl)-7-cyano-3,4-dihydroisoquinoline-2(1H)-carboxamide |
| 51 | *N*-(3-Chloro-4-(trifluoromethyl)phenyl)-7-cyano-3,4-dihydroisoquinoline-2(1H)-carboxamide |
| 52 | 6-Chloro-*N*-(3,4-dichlorophenyl)-3,4-dihydroisoquinoline-2(1H)-carboxamide |
| 53 | 6-Chloro-*N*-(4-chloro-3-(trifluoromethyl)phenyl)-3,4-dihydroisoquinoline-2(1H)-carboxamide |
| 54 | 6-Chloro-*N*-(3-chloro-4-(trifluoromethyl)phenyl)-3,4-dihydroisoquinoline-2(1H)-carboxamide |
| 55 | 5-Chloro-*N*-(3,4-dichlorophenyl)-3,4-dihydroisoquinoline-2(1H)-carboxamide |
| 56 | 5-Chloro-*N*-(4-chloro-3-(trifluoromethyl)phenyl)-3,4-dihydroisoquinoline-2(1H)-carboxamide |
| 57 | 5-Chloro-*N*-(3-chloro-4-(trifluoromethyl)phenyl)-3,4-dihydroisoquinoline-2(1H)-carboxamide |
| 58 | *N*-(3,4-Dichlorophenyl)-5-methyl-3,4-dihydroisoquinoline-2(1H)-carboxamide |
| 59 | *N*-(4-Chloro-3-(trifluoromethyl)phenyl)-5-methyl-3,4-dihydroisoquinoline-2(1H)-carboxamide |
| 60 | *N*-(3-Chloro-4-(trifluoromethyl)phenyl)-5-methyl-3,4-dihydroisoquinoline-2(1H)-carboxamide |
| 61 | *N*-(3,4-Dichlorophenyl)-7-methoxy-3,4-dihydroisoquinoline-2(1H)-carboxamide |
| 62 | *N*-(4-Chloro-3-(trifluoromethyl)phenyl)-7-methoxy-3,4-dihydroisoquinoline-2(1H)-carboxamide |
| 63 | *N*-(3-Chloro-4-(trifluoromethyl)phenyl)-7-methoxy-3,4-dihydroisoquinoline-2(1H)-carboxamide |
| 64 | *N*-(3,4-Dichlorophenyl)-8-methoxy-3,4-dihydroisoquinoline-2(1H)-carboxamide |
| 65 | *N*-(3,4-Dichlorophenyl)-6-methoxy-3,4-dihydroisoquinoline-2(1H)-carboxamide |
| 66 | *N*-(4-Chloro-3-(trifluoromethyl)phenyl)-6-methoxy-3,4-dihydroisoquinoline-2(1H)-carboxamide |
| 67 | *N*-(3-Chloro-4-(trifluoromethyl)phenyl)-6-methoxy-3,4-dihydroisoquinoline-2(1H)-carboxamide |
| 70 | *N*-(3-Chloro-4-(trifluoromethyl)phenyl)-5-(trifluoromethyl)-3,4-dihydroisoquinoline-2(1H)-carboxamide |
| 71 | *N*-(3-Chloro-4-(trifluoromethyl)phenyl)-7-(trifluoromethyl)-3,4-dihydroisoquinoline-2(1H)-carboxamide |
| 72 | *N*-(3-Chloro-4-(trifluoromethyl)phenyl)-6-(trifluoromethyl)-3,4-dihydroisoquinoline-2(1H)-carboxamide |
| 74 | *N*-(3-Chloro-4-(trifluoromethyl)phenyl)-6-oxo-3,4,6,7-tetrahydro-2,7-naphthyridine-2(1H)-carboxamide |
| 76 | *N*-(3,4-Dichlorophenyl)-6-oxo-3,4,6,7-tetrahydro-2,7-naphthyridine-2(1H)-carboxamide |
| 77 | *N*-(4-Chloro-3-(trifluoromethyl)phenyl)-2-oxo-1,5,7,8-tetrahydro-1,6-naphthyridine-6(2H)-carboxamide |
| 78 | *N*-(3,4-Dichlorophenyl)-2-oxo-1,5,7,8-tetrahydro-1,6-naphthyridine-6(2H)-carboxamide |
| 79 | 6-((3-Chloro-4-(trifluoromethyl)phenyl)carbamoyl)-5,6,7,8-tetrahydro-2,6-naphthyridine 2-oxide |
| 80 | *N*-(3,4-Dichlorophenyl)-6,7-dihydroisoxazolo[4,5-c]pyridine-5(4H)-carboxamide |
| 81 | *N*-(3,4-Dichlorophenyl)-3,4-dihydroisoquinoline-2(1H)-carboxamide |
| 82 | 7-Amino-*N*-(3,4-dichlorophenyl)-3,4-dihydro-2,6-naphthyridine-2(1H)-carboxamide |
| 83 | 7-Amino-*N*-(3-chloro-4-(trifluoromethyl)phenyl)-3,4-dihydro-2,6-naphthyridine-2(1H)-carboxamide |
| 84 | 6-Amino-*N*-(3,4-dichlorophenyl)-3,4-dihydro-2,7-naphthyridine-2(1H)-carboxamide |
| 85 | *N*-(3,4-Dichlorophenyl)-6-fluoro-3,4-dihydro-2,7-naphthyridine-2(1H)-carboxamide |
| 86 | *N*-(3,4-Dichlorophenyl)-7-fluoro-3,4-dihydro-2,6-naphthyridine-2(1H)-carboxamide |
| 87 | *N*-(3-Chloro-4-(trifluoromethyl)phenyl)-7-fluoro-3,4-dihydro-2,6-naphthyridine-2(1H)-carboxamide |
| 88 | *N*-(3,4-Dichlorophenyl)-2-oxo-1,5,7,8-tetrahydropyrido[4,3-d]pyrimidine-6(2H)-carboxamide |
| 89 | *N*-(4-chloro-3-(trifluoromethyl)phenyl)-6-oxo-3,4,6,7-tetrahydro-2,7-naphthyridine-2(1H)-carboxamide |
| 90 | 7-Chloro-*N*-(3-chloro-4-(trifluoromethyl)phenyl)-3,4-dihydro-2,6-naphthyridine-2(1H)-carboxamide |
| 91 | 6-Chloro-*N*-(3-chloro-4-(trifluoromethyl)phenyl)-3,4-dihydro-2,7-naphthyridine-2(1H)-carboxamide |
| 92 | *N*-(3,4-Dichlorophenyl)-7-methyl-6-oxo-3,4,6,7-tetrahydro-2,7-naphthyridine-2(1H)-carboxamide |
| 93 | *N*-(3,4-Dichlorophenyl)-6-fluoro-1-methyl-3,4-dihydroisoquinoline-2(1H)-carboxamide |
| 94 | 7-((3-Chloro-4-(trifluoromethyl)phenyl)carbamoyl)-5,6,7,8-tetrahydro-2,7-naphthyridine 2-oxide |
| 95 | *N*-(3,4-Dichlorophenyl)-6-hydroxy-3,4-dihydroisoquinoline-2(1H)-carboxamide |
| 96 | *N*-(3,4-Dichlorophenyl)-7-hydroxy-3,4-dihydroisoquinoline-2(1H)-carboxamide |
| 97 | 8-Chloro-*N*-(3-chloro-4-(trifluoromethyl)phenyl)-3,4-dihydro-2,7-naphthyridine-2(1H)-carboxamide |
| 98 | *N*-(3,4-Dichlorophenyl)-6,7-dihydroisoxazolo[4,3-c]pyridine-5(4H)-carboxamide |
| 99 | *N*-(3,4-Dichlorophenyl)-7-oxo-3,4,6,7-tetrahydro-2,6-naphthyridine-2(1H)-carboxamide |
| 100 | *N*-(3-Chloro-4-(trifluoromethyl)phenyl)-7-oxo-3,4,6,7-tetrahydro-2,6-naphthyridine-2(1H)-carboxamide |
| 101 | *N*-(3,4-Dichlorophenyl)-6,7-dihydro-[1,2,5]oxadiazolo[3,4-c]pyridine-5(4H)-carboxamide |
| 102 | 6-Cyano-*N*-(3,4-dichlorophenyl)-3,4-dihydro-2,7-naphthyridine-2(1H)-carboxamide |
| 103 | 7-Cyano-*N*-(3,4-dichlorophenyl)-3,4-dihydro-2,6-naphthyridine-2(1H)-carboxamide |
| 104 | *N*-(3-Chloro-4-(trifluoromethyl)phenyl)-6-fluoro-3,4-dihydro-2,7-naphthyridine-2(1H)-carboxamide |
| 105 | *N*-(3,4-Dichlorophenyl)-7-methoxy-3,4-dihydro-2,6-naphthyridine-2(1H)-carboxamide |
| 106 | *N*-(3,4-Dichlorophenyl)-6,7-difluoro-3,4-dihydroisoquinoline-2(1H)-carboxamide |
| 107 | *N*-(3-Chloro-4-(trifluoromethyl)phenyl)-7-methoxy-3,4-dihydro-2,6-naphthyridine-2(1H)-carboxamide |
| 108 | *N-(3-Chloro-4-(trifluoromethyl)phenyl)-6,7-difluoro-3,4-dihydroisoquinoline-2(1H)-*carboxamide |
| 109 | *N*-(3,4-Dichlorophenyl)-8,9-dihydro-[1,2,4]triazolo[4,3-b][2,7]naphthyridine-7(6H)-carboxamide |
| 110 | *N*-(3,4-Dichlorophenyl)-8,9-dihydroimidazo[1,2-b][2,7]naphthyridine-7(6H)-carboxamide |
| 111 | *N*-(3,4-Dichlorophenyl)-8,9-dihydro-[1,2,4]triazolo[1,5-b][2,7]naphthyridine-7(6H)-carboxamide |
| 112 | *N*-(4,5-Dichloro-2-fluorophenyl)-6-oxo-3,4,6,7-tetrahydro-2,7-naphthyridine-2(1H)-carboxamide |
| 113 | *N*-(3,4-Dichlorophenyl)-8,9-dihydroimidazo[1,5-b][2,7]naphthyridine-7(6H)-carboxamide |
| 114 | Methyl 2-((3,4-dichlorophenyl)carbamoyl)-1,2,3,4-tetrahydroisoquinoline-6-carboxylate |
| 115 | *N*-(3,4-Dichlorophenyl)-6,7-dihydroimidazo[1,2-b][2,6]naphthyridine-8(9H)-carboxamide |
| 116 | *N*-(3,4-Dichlorophenyl)-6,7-dihydro-[1,2,4]triazolo[4,3-b][2,6]naphthyridine-8(9H)-carboxamide |
| 117 | *N*-(3,4-Dichlorophenyl)-6,7-dihydroimidazo[1,5-b][2,6]naphthyridine-8(9H)-carboxamide |
| 118 | *N*-(3,4-Dichloro-2-fluorophenyl)-6-oxo-3,4,6,7-tetrahydro-2,7-naphthyridine-2(1H)-carboxamide |
| 119 | 5-Amino-*N*-(3,4-dichlorophenyl)-3,4-dihydro-2,6-naphthyridine-2(1H)-carboxamide |
| 120 | *N*-(3,4-Dichlorophenyl)-5-fluoro-3,4-dihydro-2,6-naphthyridine-2(1H)-carboxamide |
| 121 | *N*-(3,4-Dichlorophenyl)-6,7-dihydro-[1,2,4]triazolo[1,5-b][2,6]naphthyridine-8(9H)-carboxamide |
| 122 | 2-Amino-*N*-(3,4-dichlorophenyl)-7,8-dihydro-1,6-naphthyridine-6(5H)-carboxamide |
| 123 | *N*-(3,4-Dichlorophenyl)-2-fluoro-7,8-dihydro-1,6-naphthyridine-6(5H)-carboxamide |
| 124 | *N*-(3-Chloro-4-(trifluoromethyl)phenyl)-6-(methylsulfonyl)-3,4-dihydroisoquinoline-2(1H)-carboxamide |
| 125 | *N*-(3,4-Dichlorophenyl)-6-(methylsulfonyl)-3,4-dihydroisoquinoline-2(1H)-carboxamide |
| 126 | *N*-(3,4-Dichlorophenyl)-6-(2-fluoropyridin-3-yl)-3,4-dihydroisoquinoline-2(1H)-carboxamide |
| 127 | *N*-(3,4-Dichlorophenyl)-6-(thiophen-2-yl)-3,4-dihydroisoquinoline-2(1H)-carboxamide |
| 128 | *N*-(3,4-Dichlorophenyl)-6-(thiophen-3-yl)-3,4-dihydroisoquinoline-2(1H)-carboxamide |
| 129 | Methyl 7-((3-chloro-4-(trifluoromethyl)phenyl)carbamoyl)-5,6,7,8-tetrahydro-2,7-naphthyridine-3-carboxylate |
| 130 | *N-(4,5-Dichloro-2-(trifluoromethyl)phenyl)-6-oxo-3,4,6,7-tetrahydro-2,7-*naphthyridine-2(1H)-carboxamide |
| 131 | Methyl 2-((3,4-dichlorophenyl)carbamoyl)-1,2,3,4-tetrahydroisoquinoline-7-carboxylate |
| 132 | *N*-(3-Chloro-4-(trifluoromethyl)phenyl)-8-cyano-3,4-dihydroisoquinoline-2(1H)-carboxamide |
| 133 | *N*-(4-Chloro-3-(trifluoromethyl)phenyl)-5-methoxy-3,4-dihydroisoquinoline-2(1H)-carboxamide |
| 134 | *N*-(3-Chloro-4-(trifluoromethyl)phenyl)-5-methoxy-3,4-dihydroisoquinoline-2(1H)-carboxamide |
| 135 | *N*-(3,4-Dichlorophenyl)-6-methyl-3,4-dihydroisoquinoline-2(1H)-carboxamide |
| 136 | *N*-(3-Chloro-4-(trifluoromethyl)phenyl)-6-methyl-3,4-dihydroisoquinoline-2(1H)-carboxamide |
| 137 | *N*-(4-Chloro-3-(trifluoromethyl)phenyl)-5-cyano-3,4-dihydroisoquinoline-2(1H)-carboxamide |
| 138 | 7-Chloro-*N*-(3,4-dichlorophenyl)-3,4-dihydroisoquinoline-2(1H)-carboxamide |
| 139 | 8-Chloro-*N*-(3,4-dichlorophenyl)-3,4-dihydroisoquinoline-2(1H)-carboxamide |
| 140 | *N*-(3,4-Dichlorophenyl)-6-(3-methyl-1,2,4-oxadiazol-5-yl)-3,4-dihydroisoquinoline-2(1H)-carboxamide |

and pharmaceutically acceptable salts and solvates thereof.

In one preferred embodiment, the compound according to the invention, or for use according to the invention is selected from the following: 1, 5-7, 10-17, 19, 25-27, 29-33, 35-45, 47-55, 57-61, 63, 64, 66, 67, 71, 74, 76, 79-83, 85-91, 93, 96, 98, 101-104, 106, 108, 109, 112-114, 117-118, 120, 123, 126, 129 and 131-140.

In a more preferred embodiment, the compound according to the invention, or for use according to the invention is selected from the following: 14, 30, 31, 33, 36, 37, 39-44, 52-54, 74, 76, 79, 82, 83, 85, 93, 104, 106, 108, 109, 112-114, 117, 120, 123 and 140.

A further aspect of the invention relates to a compound of formula (I'), or a pharmaceutically acceptable salt or solvate thereof, 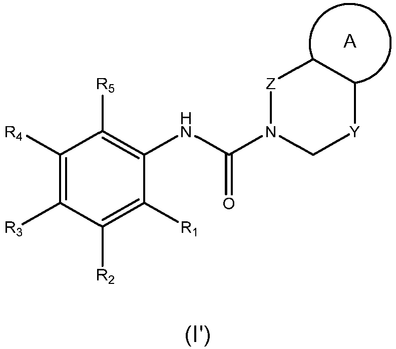 wherein:
ring A is a 5 or 6 membered monocyclic aromatic or heteroaromatic ring, wherein said aromatic or heteroaromatic ring is optionally substituted with one or more substituents selected from F, Cl, Br, I, CN, alkoxy, NR₁₁R₁₁', OH, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y and Z are each independently selected from CH₂ and CR₁₀R₁₀', wherein R₁₀ and R₁₀' are each independently selected from H, F, alkyl, and haloalkyl;
R₁, R₄, and R₅ are each independently selected from H, F, Cl, Br, and I;
R₂ and R₃ are each independently selected from H, F, Cl, Br, I, CN, and haloalkyl;
wherein at least two of R₂, R₃ and R₄ are other than H; and
R₁₁ and R₁₁' are each independently selected from H, alkyl, haloalkyl, COR₁₂, and SO₂R₁₃, wherein R₁₂ and R₁₃ are both alkyl;
for use in treating or preventing a disorder selected from a proliferative disorder, an immune disorder, asthma, chronic obstructive pulmonary disease (COPD) and acute respiratory distress syndrome (ARDS), wherein the compound modulates GPR65, preferably wherein the compound inhibits GPR65 signalling.

Preferred definitions for A, Z, Y, R¹-R⁵ are as set forth above for formulae (I), (la), (Ia') and (Ib').

### PHARMACEUTICAL COMPOSITIONS

For use according to the present invention, the compounds or physiologically acceptable salt, ester or other physiologically functional derivative thereof, described herein, may be presented as a pharmaceutical formulation, comprising the compounds or physiologically acceptable salt, ester or other physiologically functional derivative thereof, together with one or more pharmaceutically acceptable carriers, diluents or excipients therefor and optionally other therapeutic and/or prophylactic ingredients. The carrier(s) must be acceptable in the sense of being compatible with the other ingredients of the formulation and not deleterious to the recipient thereof. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller. The carrier, or, if more than one be present, each of the carriers, must be acceptable in the sense of being compatible with the other ingredients of the formulation and not deleterious to the recipient.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol, sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s), buffer(s), flavouring agent(s), surface active agent(s), thickener(s), preservative(s) (including antioxidants) and the like, and substances included for the purpose of rendering the formulation isotonic with the blood of the intended recipient.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like.

Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

Pharmaceutical formulations include those suitable for oral, topical (including dermal, buccal and sublingual), rectal or parenteral (including subcutaneous, intradermal, intramuscular and intravenous), nasal and pulmonary administration e.g., by inhalation. The formulation may, where appropriate, be conveniently presented in discrete dosage units and may be prepared by any of the methods well known in the art of pharmacy. All methods include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release - controlling matrix, or is coated with a suitable release - controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds. Pharmaceutical formulations suitable for parenteral administration include sterile solutions or suspensions of an active compound in aqueous or oleaginous vehicles.

Injectable preparations may be adapted for bolus injection or continuous infusion. Such preparations are conveniently presented in unit dose or multi-dose containers which are sealed after introduction of the formulation until required for use. Alternatively, an active compound may be in powder form which is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration via the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

Pharmaceutically acceptable carriers are well known to those skilled in the art and include, but are not limited to, 0.1 M and preferably 0.05 M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments. Such preparations may be applied e.g. to a wound or ulcer either directly spread upon the surface of the wound or ulcer or carried on a suitable support such as a bandage, gauze, mesh or the like which may be applied to and over the area to be treated.

Liquid or powder formulations may also be provided which can be sprayed or sprinkled directly onto the site to be treated, e.g. a wound or ulcer. Alternatively, a carrier such as a bandage, gauze, mesh or the like can be sprayed or sprinkle with the formulation and then applied to the site to be treated.

According to a further aspect of the invention, there is provided a process for the preparation of a pharmaceutical or veterinary composition as described above, the process comprising bringing the active compound(s) into association with the carrier, for example by admixture.

In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound as described herein into conjunction or association with a pharmaceutically or veterinarily acceptable carrier or vehicle.

### SALTS/ESTERS

The compounds of the invention can be present as salts or esters, in particular pharmaceutically and veterinarily acceptable salts or esters.

Pharmaceutically acceptable salts of the compounds of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. hydrohalic acids such as hydrochloride, hydrobromide and hydroiodide, sulphuric acid, phosphoric acid sulphate, bisulphate, hemisulphate, thiocyanate, persulphate and sulphonic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane- or p-toluene sulfonic acid. Salts which are not pharmaceutically or veterinarily acceptable may still be valuable as intermediates.

Preferred salts include, for example, acetate, trifluoroacetate, lactate, gluconate, citrate, tartrate, maleate, malate, pantothenate, adipate, alginate, aspartate, benzoate, butyrate, digluconate, cyclopentanate, glucoheptanate, glycerophosphate, oxalate, heptanoate, hexanoate, fumarate, nicotinate, palmoate, pectinate, 3-phenylpropionate, picrate, pivalate, proprionate, tartrate, lactobionate, pivolate, camphorate, undecanoate and succinate, organic sulphonic acids such as methanesulphonate, ethanesulphonate, 2-hydroxyethane sulphonate, camphorsulphonate, 2-naphthalenesulphonate, benzenesulphonate, p-chlorobenzenesulphonate and p-toluenesulphonate; and inorganic acids such as hydrochloride, hydrobromide, hydroiodide, sulphate, bisulphate, hemisulphate, thiocyanate, persulphate, phosphoric and sulphonic acids.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as (C₁-C₄)-alkyl- or aryl-sulfonic acids which are unsubstituted or substituted (for example, by a halogen) such as methane or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

### ENANTIOMERS/TAUTOMERS

In all aspects of the present invention previously discussed, the invention includes, where appropriate all enantiomers, diastereoisomers and tautomers of the compounds of the invention. The person skilled in the art will recognise compounds that possess optical properties (one or more chiral carbon atoms) or tautomeric characteristics. The corresponding enantiomers and/or tautomers may be isolated/prepared by methods known in the art.

Enantiomers are characterised by the absolute configuration of their chiral centres and described by the *R-* and *S*-sequencing rules of Cahn, Ingold and Prelog. Such conventions are well known in the art (e.g. see 'Advanced Organic Chemistry', 3rd edition, ed. March, J., John Wiley and Sons, New York, 1985).

Compounds of the invention containing a chiral centre may be used as a racemic mixture, an enantiomerically enriched mixture, or the racemic mixture may be separated using well-known techniques and an individual enantiomer may be used alone.

### STEREO AND GEOMETRIC ISOMERS

Some of the compounds of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those compounds, and mixtures thereof. The terms used in the claims encompass these forms, provided said forms retain the appropriate functional activity (though not necessarily to the same degree).

The present invention also includes all suitable isotopic variations of the compound or a pharmaceutically acceptable salt thereof. An isotopic variation of a compound of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as ²H, ³H, ¹³C, ¹⁴C, ¹⁵N, ¹⁷O, ¹⁸O, ³¹P, ³²P, ³⁵S, ¹⁸F and ³⁶Cl, respectively. Certain isotopic variations of the agent and pharmaceutically acceptable salts thereof, for example, those in which a radioactive isotope such as ³H or ¹⁴C is incorporated, are useful in drug and/or substrate tissue distribution studies. Tritiated, i.e., ³H, and carbon-14, i.e., ¹⁴C, isotopes are particularly preferred for their ease of preparation and detectability. Further, substitution with isotopes such as deuterium, i.e., ²H, may afford certain therapeutic advantages resulting from greater metabolic stability, for example, increased *in vivo* half-life or reduced dosage requirements and hence may be preferred in some circumstances. For example, the invention includes compounds of general formula (I) where any hydrogen atom has been replaced by a deuterium atom. Isotopic variations of the agent of the present invention and pharmaceutically acceptable salts thereof of this invention can generally be prepared by conventional procedures using appropriate isotopic variations of suitable reagents.

### ATROPISOMERS

Some of the compounds of the invention may exist as atropisomers. Atropisomers are stereoisomers arising because of hindered rotation about a single bond, where energy differences due to steric strain or other contributors create a barrier to rotation that is high enough to allow for isolation of individual conformers. The invention encompasses all such atropisomers. The invention also encompasses rotamers of the compounds.

### PRODRUGS

The invention further includes the compounds of the present invention in prodrug form, i.e. covalently bonded compounds which release the active parent drug *in vivo.* Such prodrugs are generally compounds of the invention wherein one or more appropriate groups have been modified such that the modification may be reversed upon administration to a human or mammalian subject. Reversion is usually performed by an enzyme naturally present in such subject, though it is possible for a second agent to be administered together with such a prodrug in order to perform the reversion *in vivo.* Examples of such modifications include ester (for example, any of those described above), wherein the reversion may be carried out be an esterase etc. Other such systems will be well known to those skilled in the art.

### SOLVATES

The present invention also includes solvate forms of the compounds of the present invention. The terms used in the claims encompass these forms. Preferably the solvate is a hydrate.

### COMBINATIONS

A further aspect of the invention relates to a combination comprising a compound as described herein and one or more additional active agents. In a particularly preferred embodiment, the one or more compounds of the invention are administered in combination with one or more additional active agents, for example, existing drugs available on the market. In such cases, the compounds of the invention may be administered consecutively, simultaneously or sequentially with the one or more other active agents.

Drugs in general are more effective when used in combination. In particular, combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance.

Beneficial combinations may be suggested by studying the activity of the test compounds with agents known or suspected of being valuable in the treatment of a particular disorder. This procedure can also be used to determine the order of administration of the agents, i.e. before, simultaneously, or after delivery. Such scheduling may be a feature of all the active agents identified herein.

In the context of cancer, compounds of the invention can be used in combination with immunotherapies such as cancer vaccines and/or with other immune-modulators such as agents that block the PD1/PDL-1 interaction. Thus, in one preferred embodiment, the additional active agent is an immunotherapy agent, more preferably a cancer immunotherapy agent. An "immunotherapy agent" refers to a treatment that uses the subject's own immune system to fight diseases such as cancer. For other disorders the compounds of the invention can be used in combination agents that block or decrease inflammation such as antibodies that target pro-inflammatory cytokines.

### POLYMORPHS

The invention further relates to the compounds of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

### ADMINISTRATION

The pharmaceutical compositions of the present invention may be adapted for rectal, nasal, intrabronchial, topical (including buccal and sublingual), vaginal or parenteral (including subcutaneous, intramuscular, intravenous, intraarterial and intradermal), intraperitoneal or intrathecal administration. Preferably the formulation is an orally administered formulation. The formulations may conveniently be presented in unit dosage form, i.e., in the form of discrete portions containing a unit dose, or a multiple or sub-unit of a unit dose. By way of example, the formulations may be in the form of tablets and sustained release capsules, and may be prepared by any method well known in the art of pharmacy.

Formulations for oral administration in the present invention may be presented as: discrete units such as capsules, gellules, drops, cachets, pills or tablets each containing a predetermined amount of the active agent; as a powder or granules; as a solution, emulsion or a suspension of the active agent in an aqueous liquid or a non-aqueous liquid; or as an oil-in-water liquid emulsion or a water-in-oil liquid emulsion; or as a bolus etc. Preferably, these compositions contain from 1 to 250 mg and more preferably from 10-100 mg, of active ingredient per dose.

For compositions for oral administration (e.g. tablets and capsules), the term "acceptable carrier" includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropyl-methylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Flavouring agents such as peppermint, oil of wintergreen, cherry flavouring and the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Other forms of administration comprise solutions or emulsions which may be injected intravenously, intraarterially, intrathecally, subcutaneously, intradermally, intraperitoneally or intramuscularly, and which are prepared from sterile or sterilisable solutions. Injectable forms typically contain between 10 - 1000 mg, preferably between 10 - 250 mg, of active ingredient per dose.

The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

### DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

The dosage amount will further be modified according to the mode of administration of the compound. For example, to achieve an "effective amount" for acute therapy, parenteral administration of a compound is typically preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg; preferably between 0.1 and 20 mg, in a manner to maintain the concentration of drug in the plasma at a concentration effective to modulate GPR65. The compounds may be administered one to four times daily at a level to achieve a total daily dose of about 0.4 to about 400 mg. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to achieve one or more of the therapeutic indications disclosed herein. Typically, a pharmaceutical composition containing the compound is administered at an oral dose of between about 0.1 to about 500 mg or about 0.1 to about 50 mg in a manner consistent with the condition of the patient. Preferably the oral dose would be about 0.5 to about 50 mg or about 0.5 to about 20 mg.

No unacceptable toxicological effects are expected when compounds of the present invention are administered in accordance with the present invention. The compounds of this invention, which may have good bioavailability, may be tested in one of several biological assays to determine the concentration of a compound which is required to have a given pharmacological effect.

The invention is further described by the way of the following non-limiting examples.

### EXAMPLES

Where the preparation of starting materials is not described, these are commercially available, known in the literature, or readily obtainable by those skilled in the art using standard procedures. Where it is indicated that compounds were prepared analogously to earlier examples or intermediates, it will be appreciated by the skilled person that the reaction time, number of equivalents of reagents, solvent, concentration and temperature can be modified for each specific reaction and that it may be necessary or desirable to employ different work-up or purification techniques.

### General Schemes

### Abbreviations

A list of some common abbreviations is shown below - where other abbreviations are used which are not listed, these will be understood by the person skilled in the art.
AcOH: Acetic acid; d: doublet; DCM: dichloromethane; DIPEA: *N,N*-diisopropylethylamine; DMF: *N,N*-dimethylformamide; DMSO: dimethylsulfoxide; (ES⁺): electrospray ionization positive mode; h: hours; HPLC: high performance liquid chromatography; Hz: hertz; *J*: coupling constant M: molar; m: multiplet [M+H]+: protonated molecular ion; *m*CPBA: *meta-*chloroperoxybenzoic acid; MeCN: acetonitrile; MHz: megahertz; min: minutes; ml: millilitres; MS: mass spectrometry; m/z: mass-to-charge ratio; NMR: nuclear magnetic resonance; Pd-177: allyl[4,5-bis(diphenylphosphino)-9,9-dimethylxanthene]palladium(ll) chloride; Pd(dppf)Cl₂: [1,1'-Bis(diphenylphosphino)ferrocene]dichloropalladium(II); PDA: photodiode array; RT: room temperaure; Rt: retention time; s: singlet; t:triplet; UPLC: ultra performance liquid chromatography; UV: ultra-violet.

Other abbreviations are intended to convey their generally accepted meaning.

### General experimental conditions

All starting materials and solvents were obtained either from commercial sources or prepared according to literature methods. The appropriate isocyanate starting materials were obtained from Sigma Aldrich, or Enamine store. The appropriate cyclic amine starting materials were obtained from Sigma Aldrich, Enamine store, Fluorochem or Asta Tech Inc. Reaction mixtures were magnetically stirred and reactions performed at room temperature (approximately 20 °C) unless otherwise indicated.

Silica gel chromatography was performed on an automated flash chromatography system, such as CombiFlash Companion, CombiFlash Rf system or Reveleris X2 flash system using RediSep^{®} Rf or Reveleris^{®} or the GraceResolv^{™} pre-packed silica (230-400 mesh, 40-63 µm) cartridges.

Analytical UPLC-MS experiments to determine retention times and associated mass ions were performed using a Waters ACQUITY UPLC^{®} H-Class system, equipped with ACQUITY PDA Detector and ACQUITY QDa mass spectrometer or Waters SQD mass spectrometer, running the analytical method described below.

Preparative HPLC purifications were performed using a Waters X-Bridge BEH C18, 5 µm, 19x50 mm column using a gradient of MeCN and 10 mM aqueous ammonium bicarbonate. Fractions were collected following UV detection across all wavelengths with PDA as well as a SQD2 or ACQUITY QDa mass spectrometer.

NMR spectra were recorded using a Bruker Avance III HD 500 MHz instrument or a Bruker Avance Neo 400 MHz, using either residual non-deuterated solvent or tetra-methylsilane as reference.

### Analytical methods

### Method 1 - Basic 3 min method

| | |
|---|---|
| Column: | Waters ACQUITY UPLC^{®} BEH C18, 1.7 µm, 2.1 x 30 mm at 40 °C |
| Detection: | UV at 210-400 nm unless otherwise indicated, MS by electrospray ionisation |
| Solvents: | A: 10 mM aqueous ammonium bicarbonate, B: MeCN |

### Gradient:

| Time | %A | %B | Flow rate (ml/min) |
|---|---|---|---|
| 0.00 | 95 | 5 | 0.77 |
| 0.11 | 95 | 5 | 0.77 |
| 2.15 | 5 | 95 | 0.77 |
| 2.56 | 5 | 95 | 0.77 |
| 2.83 | 95 | 5 | 0.77 |
| 3.00 | 95 | 5 | 0.77 |

### Method 2 - Basic 4 min method

| | |
|---|---|
| Column: | Waters X-Bridge BEH C18, 2.5 µm, 4.6 x 30 mm at 40 °C |
| Detection: | UV at 254 nm unless otherwise indicated, MS by electrospray ionisation |
| Solvents: | A: 0.1% v/v ammonium hydroxide in water, B: MeCN |

### Gradient:

| Time | %A | %B | Flow rate (ml/min) |
|---|---|---|---|
| 0.0 | 95.0 | 5.0 | 2.5 |
| 3.0 | 5.0 | 95.0 | 2.5 |
| 3.01 | 5.0 | 95.0 | 4.5 |
| 3.6 | 5.0 | 95.0 | 4.5 |
| 3.7 | 95.0 | 5.0 | 2.5 |
| 4.0 | 95.0 | 5.0 | 2.5 |

### Method 3 - Basic 3 min method

| | |
|---|---|
| Column: | Waters ACQUITY UPLC^{®} BEH C18, 1.7 µm, 2.1 x 30 mm at 40 °C |
| Solvents: | A: 0.1% v/v ammonium hydroxide in water, B: MeCN |

| Time | %A | %B | Flow rate (ml/min) |
|---|---|---|---|
| 0.00 | 95 | 5 | 0.77 |
| 0.11 | 95 | 5 | 0.77 |
| 2.15 | 5 | 95 | 0.77 |
| 2.56 | 5 | 95 | 0.77 |
| 2.83 | 95 | 5 | 0.77 |
| 3.00 | 95 | 5 | 0.77 |

### Experimental scheme 1

### Compound 1 N-(3,4-Dichlorophenyl)-7,8-dihydropyrido[4,3-d]pyrimidine-6(5H)-carboxamide

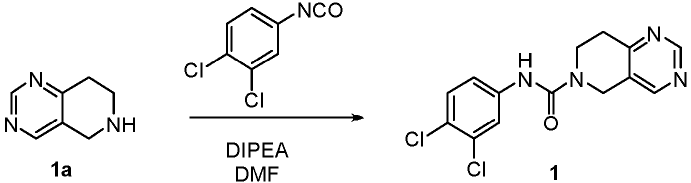

A solution of 5,6,7,8-tetrahydropyrido[4,3-d]pyrimidine **1a** (20 mg, 0.15 mmol) in DMF (1 ml) was added to 1,2-dichloro-4-isocyanatobenzene (34 mg, 0.180 mmol). DIPEA (0.079 ml, 0.450 mmol) was added and the mixture was stirred at RT for 16 h. The reaction mixture was filtered and the product was purified by mass directed HPLC (10-40 % MeCN/10 mM aqueous ammonium bicarbonate solution, C18) to yield *N*-(3,4-dichlorophenyl)-7,8-dihydropyrido[4,3-d]pyrimidine-6(5H)-carboxamide **1** as colourless solid. UPLC-MS (method 1) m/z 323.3, 325.3, 327.2 [M+H]⁺ (ES⁺) at 1.14 min. ¹H NMR (500 MHz, DMSO-d6) δ 9.03 (s, 1H), 8.97 (s, 1H), 8.64 (s, 1H), 7.85 (d, *J =* 2.3 Hz, 1H), 7.50 (d, *J =* 8.8 Hz, 1H), 7.47 (dd, *J* = 8.9, 2.3 Hz, 1H), 4.70 (s, 2H), 3.83 (t, *J =* 5.9 Hz, 2H), 2.95 (t, *J =* 5.9 Hz, 2H).

The following compounds were prepared using appropriate starting materials in an analogous procedure to that described in Experimental Scheme 1. Where the starting materials are not described in the literature, their synthesis is described below.

Key: (a) Reaction performed in THF (b) Reaction performed in DCM (c) Reaction performed in a mixture of DMF/THF (d) Reaction performed in a mixture of DMF/DCM (e) Reaction performed with Et₃N instead of DIPEA (f) Reaction performed without the addition of DIPEA (g) product purified by silica gel chromatography (EtOAc/ isohexane) (h) product purified by silica gel chromatography (DCM/heptane) (i) product purified by RP Flash C18 (MeCN/10 mM aqueous ammonium bicarbonate) (j) product purified by silica gel chromatography (DCM/isohexane) (k) product purified by silica gel chromatography (0.7 M NH₃ in MeOH/DCM) (I) [M+H]⁺ mass not observed [M-H]⁻ reported instead.

| Compound | Structure | UPLC method 1 or 3 Rt [M+H]⁺ | NMR |
|---|---|---|---|
| 2 | 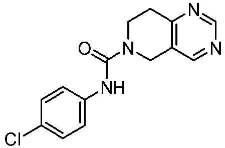 *N*-(4-Chlorophenyl)-7,8-dihydropyrido[4,3-d]pyrimidine-6(5H)-carboxamide | 289.3, 291.2 at 0.98 min | ¹H NMR (500 MHz, DMSO-d6) δ 8.97 (s, 1H), 8.88 (s, 1H), 8.64 (s, 1H), 7.56 - 7.42 (m, 2H), 7.39 - 7.24 (m, 2H), 4.70 (s, 2H), 3.83 (t, *J* = 5.9 Hz, 2H), 2.94 (t, *J* = 5.9 Hz, 2H). |
| 3 | 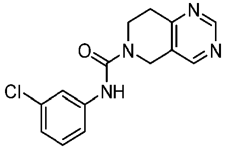 *N*-(3-Chlorophenyl)-7,8-dihydropyrido[4,3-d]pyrimidine-6(5H)-carboxamide | 289.3, 291.2 at 0.99 min | ¹H NMR (500 MHz, DMSO-d6) δ 8.97 (s, 1H), 8.93 (s, 1H), 8.64 (s, 1H), 7.66 (t, *J* = 2.1 Hz, 1H), 7.42 (ddd, *J* = 8.2, 2.1, 1.0 Hz, 1H), 7.28 (t, *J* = 8.1 Hz, 1H), 7.00 (ddd, *J* = 8.0, 2.1, 0.9 Hz, 1H), 4.70 (s, 2H), 3.83 (t, *J* = 5.9 Hz, 2H), 2.95 (t, *J* = 5.9 Hz, 2H). |
| 4 | 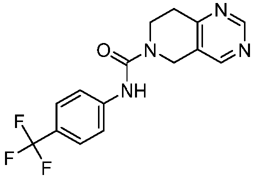 *N*-(4-(Trifluoromethyl)phenyl)-7,8-dihydropyrido[4,3-d]pyrimidine-6(5H)-carboxamide | 323.3 at 1.11 min | ¹H NMR (500 MHz, DMSO-d6) δ 9.14 (s, 1H), 8.97 (s, 1H), 8.65 (s, 1H), 7.70 (d, *J* = 8.5 Hz, 2H), 7.61 (d, *J =* 8.6 Hz, 2H), 4.72 (s, 2H), 3.85 (t, *J* = 5.9 Hz, 2H), 2.96 (t, *J =* 5.9 Hz, 2H). |
| 5 ^{(a)(g)} | 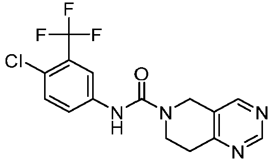 *N*-(4-Chloro-3-(trifluoromethyl)phenyl)-7,8-dihydropyrido[4,3-d]pyrimidine-6(5H)-carboxamide | 357.3, 359.3 at 1.24 min | ¹H NMR (500 MHz, DMSO-d6) δ 9.18 (s, 1H), 8.97 (s, 1H), 8.64 (s, 1H), 8.06 (d, *J* = 2.6 Hz, 1H), 7.82 (dd, *J* = 8.8, 2.6 Hz, 1H), 7.60 (d, *J* = 8.8 Hz, 1H), 4.72 (s, 2H), 3.85 (t, *J* = 6.0 Hz, 2H), 2.96 (t, *J =* 5.9 Hz, 2H). |
| 6^{(b)(e)} | 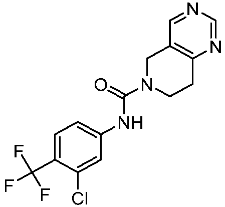 *N*-(3-Chloro-4-(trifluoromethyl)phenyl)-7,8-dihydropyrido[4,3-d]pyrimidine-6(5H)-carboxamide | 357.3, 359.3 at 1.26 min | ¹H NMR (500 MHz, DMSO-d6) δ 9.30 (s, 1H), 8.98 (s, 1H), 8.65 (s, 1H), 7.91 (d, *J* = 2.0 Hz, 1H), 7.74 (d, *J =* 8.8 Hz, 1H), 7.64 (dd, *J =* 8.6, 2.1 Hz, 1H), 4.73 (s, 2H), 3.86 (t, *J* = 5.9 Hz, 2H), 2.96 (t, *J* = 5.9 Hz, 2H). |
| 7^{(b)} | 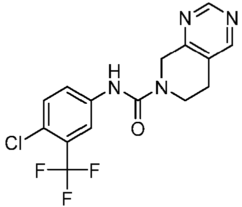 *N*-(4-Chloro-3-(trifluoromethyl)phenyl)-5,8-dihydropyrido[3,4-d]pyrimidine-7(6H)-carboxamide | 357.3, 359.3 at 1.27 min | ¹H NMR (500 MHz, DMSO-d6) δ 9.16 (s, 1H), 9.00 (s, 1H), 8.65 (s, 1H), 8.09 (d, *J* = 2.6 Hz, 1H), 7.85 (dd, *J* = 8.7, 2.5 Hz, 1H), 7.60 (d, *J* = 8.8 Hz, 1H), 4.72 (s, 2H), 3.79 (t, *J* = 5.7 Hz, 2H), 2.89 (t, *J =* 5.8 Hz, 2H). |
| 8^{(b)} | 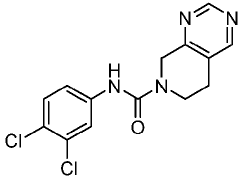 *N*-(3,4-Dichlorophenyl)-5,8-dihydropyrido[3,4-d]pyrimidine-7(6H)-carboxamide | 323.1, 325.3 at 1.2 min | ¹H NMR (500 MHz, DMSO-d6) δ 9.01 (s, 1H), 8.99 (s, 1H), 8.64 (s, 1H), 7.88 (s, 1H), 7.50 (d, *J* = 1.3 Hz, 2H), 4.70 (s, 2H), 3.78 (t, *J* = 5.8 Hz, 2H), 2.88 (t, *J =* 5.7 Hz, 2H), 0.88 - 0.78 (m, 1H). |
| 9^{(b)(e)} | 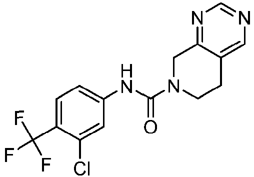 *N*-(3-Chloro-4-(trifluoromethyl)phenyl)-5,8-dihydropyrido[3,4-d]pyrimidine-7(6H)-carboxamide | 357.3, 359.3 at 1.29 min | ¹H NMR (500 MHz, DMSO-d6) δ 9.27 (s, 1H), 9.00 (s, 1H), 8.65 (s, 1H), 7.94 (d, *J* = 2.0 Hz, 1H), 7.74 (d, *J =* 8.8 Hz, 1H), 7.66 (dd, *J =* 8.9, 2.1 Hz, 1H), 4.73 (s, 2H), 3.80 (t, *J* = 5.8 Hz, 2H), 2.89 (t, *J* = 5.8 Hz, 2H). |
| 10^{(b)} | 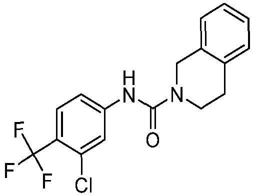 *N*-(3-Chloro-4-(trifluoromethyl)phenyl)-3,4-dihydroisoquinoline-2(1H)-carboxamide | 355.3, 357.3 at 1.71 min | ¹H NMR (500 MHz, DMSO-d6) δ 9.13 (s, 1H), 7.94 (d, *J* = 2.0 Hz, 1H), 7.73 (d, *J* = 8.8 Hz, 1H), 7.66 (dd, *J =* 8.5, 2.0 Hz, 1H), 7.20 (d, *J* = 3.1 Hz, 4H), 4.67 (s, 2H), 3.72 (t, *J* = 5.9 Hz, 2H), 2.87 (t, *J =* 5.9 Hz, 2H). |
| 11^{(b)} | 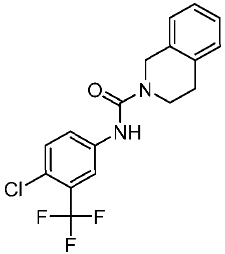 *N*-(4-Chloro-3-(trifluoromethyl)phenyl)-3,4-dihydroisoquinoline-2(1H)-carboxamide | 355.3, 357.3 at 1.69 min | ¹H NMR (500 MHz, DMSO-d6) δ 9.02 (s, 1H), 8.09 (d, *J =* 2.6 Hz, 1H), 7.85 (dd, *J* = 8.8, 2.7 Hz, 1H), 7.59 (d, *J =* 8.8 Hz, 1H), 7.20 (d, *J =* 3.2 Hz, 4H), 4.66 (s, 2H), 3.72 (t, *J* = 5.9 Hz, 2H), 2.87 (t, *J =* 5.9 Hz, 2H) |
| 12^{(b)} | 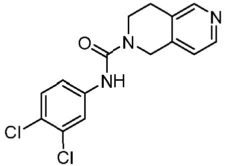 *N*-(3,4-Dichlorophenyl)-3,4-dihydro-2,6-naphthyridine-2(1H)-carboxamide | 322.3, 324.3 at 1.28 min | ¹H NMR (500 MHz, DMSO-d6) δ 8.94 (s, 1H), 8.41 (s, 1H), 8.36 (d, *J =* 5.0 Hz, 1H), 7.86 (t, *J =* 1.4 Hz, 1H), 7.53 - 7.46 (m, 2H), 7.22 (d, *J =* 5.1 Hz, 1H), 4.67 (s, 2H), 3.75 (t, *J* = 5.8 Hz, 2H), 2.87 (t, *J* = 5.8 Hz, 2H). |
| 13^{(b)} | 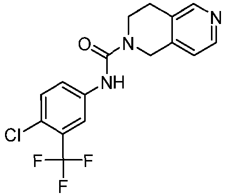 *N*-(4-Chloro-3-(trifluoromethyl)phenyl)-3,4-dihydro-2,6-naphthyridine-2(1H)-carboxamide | 356.3, 358.3 at 1.37 min | ¹H NMR (500 MHz, DMSO-d6) δ 9.09 (s, 1H), 8.41 (s, 1H), 8.36 (d, *J* = 5.1 Hz, 1H), 8.07 (d, *J* = 2.6 Hz, 1H), 7.83 (dd, *J =* 8.8, 2.7 Hz, 1H), 7.60 (d, *J* = 8.8 Hz, 1H), 7.22 (d, *J* = 5.1 Hz, 1H), 4.69 (s, 2H), 3.76 (t, *J =* 5.8 Hz, 2H), 2.88 (t, *J =* 5.8 Hz, 2H) |
| 14^{(b)} | 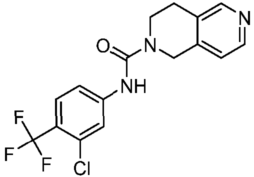 *N*-(3-Chloro-4-(trifluoromethyl)phenyl)-3,4-dihydro-2,6-naphthyridine-2(1H)-carboxamide | 356.3, 358.3 at 1.38 min | ¹H NMR (500 MHz, DMSO-d6) δ 9.21 (s, 1H), 8.42 (s, 1H), 8.37 (d, *J* = 5.1 Hz, 1H), 7.92 (d, *J* = 2.1 Hz, 1H), 7.73 (d, *J* = 8.8 Hz, 1H), 7.65 (dd, *J =* 8.7, 2.1 Hz, 1H), 7.23 (d, *J* = 5.0 Hz, 1H), 4.69 (s, 2H), 3.77 (t, *J =* 5.8 Hz, 2H), 2.88 (t, *J* = 5.9 Hz, 2H). |
| 15^{(b)} | 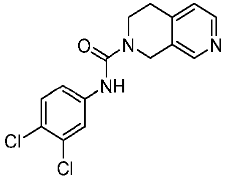 *N*-(3,4-Dichlorophenyl)-3,4-dihydro-2,7-naphthyridine-2(1H)-carboxamide | 322.3, 324.3 at 1.27 min | ¹H NMR (500 MHz, DMSO-d6) δ 8.94 (s, 1H), 8.41 (s, 1H), 8.34 (d, *J* = 5.0 Hz, 1H), 7.86 (dt, *J* = 2.8, 1.4 Hz, 1H), 7.50 (d, *J* = 1.4 Hz, 2H), 7.22 (d, *J* = 5.0 Hz, 1H), 4.69 (s, 2H), 3.72 (t, *J* = 5.9 Hz, 2H), 2.87 (t, *J =* 5.9 Hz, 2H) |
| 16^{(b)} | 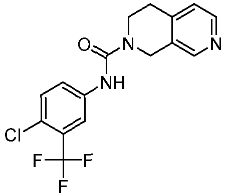 *N*-(4-Chloro-3-(trifluoromethyl)phenyl)-3,4-dihydro-2,7-naphthyridine-2(1H)-carboxamide | 356.4, 358.4 at 1.36 min | ¹H NMR (500 MHz, DMSO-d6) δ 9.09 (s, 1H), 8.41 (s, 1H), 8.35 (d, *J* = 5.0 Hz, 1H), 8.07 (d, *J* = 2.6 Hz, 1H), 7.84 (dd, *J =* 8.8, 2.6 Hz, 1H), 7.60 (d, *J =* 8.8 Hz, 1H), 7.23 (d, *J =* 5.0 Hz, 1H), 4.70 (s, 2H), 3.74 (t, *J* = 5.9 Hz, 2H), 2.88 (t, *J* = 5.9 Hz, 2H) |
| 17^{(b)} | 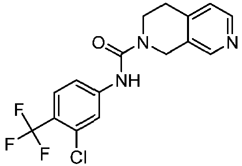 *N*-(3-Chloro-4-(trifluoromethyl)phenyl)-3,4-dihydro-2,7-naphthyridine-2(1H)-carboxamide | 356.3, 358.3 at 1.38 min | ¹H NMR (500 MHz, DMSO-d6) δ 9.21 (s, 1H), 8.42 (s, 1H), 8.35 (d, *J* = 5.0 Hz, 1H), 7.92 (d, *J* = 2.1 Hz, 1H), 7.73 (d, *J* = 8.8 Hz, 1H), 7.65 (dd, *J* = 8.4, 2.0 Hz, 1H), 7.23 (d, *J* = 5.0 Hz, 1H), 4.71 (s, 2H), 3.75 (t, *J* = 5.9 Hz, 2H), 2.89 (t, *J =* 5.9 Hz, 2H) |
| 18^{(b)} | 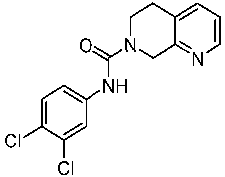 *N*-(3,4-Dichlorophenyl)-5,8-dihydro-1,7-naphthyridine-7(6H)-carboxamide | 322.3, 324.3 at 1.31 min | ¹H NMR (500 MHz, DMSO-d6) δ 8.96 (s, 1H), 8.41 (dd, *J* = 4.8, 1.6 Hz, 1H), 7.89 (d, *J* = 2.2 Hz, 1H), 7.62 (dd, *J =* 7.7, 1.6 Hz, 1H), 7.55 - 7.46 (m, 2H), 7.24 (dd, *J* = 7.7, 4.7 Hz, 1H), 4.70 (s, 2H), 3.75 (t, *J* = 5.9 Hz, 2H), 2.88 (t, *J* = 5.9 Hz, 2H). |
| 19^{(b)} | 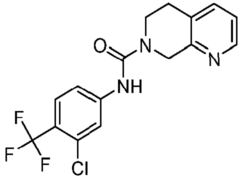 *N*-(3-Chloro-4-(trifluoromethyl)phenyl)-5,8-dihydro-1,7-naphthyridine-7(6H)-carboxamide | 356.3, 358.3 at 1.42 min | ¹H NMR (500 MHz, DMSO-d6) δ 9.23 (s, 1H), 8.41 (dd, *J* = 4.8, 1.7 Hz, 1H), 7.95 (d, *J* = 2.0 Hz, 1H), 7.73 (d, *J* = 8.8 Hz, 1H), 7.67 (dd, *J* = 8.7, 2.0 Hz, 1H), 7.62 (dd, *J* = 7.8, 1.7 Hz, 1H), 7.24 (dd, *J* = 7.7, 4.7 Hz, 1H), 4.73 (s, 2H), 3.77 (t, *J* = 5.9 Hz, 2H), 2.89 (t, *J =* 5.9 Hz, 2H) |
| 20^{(f)} | 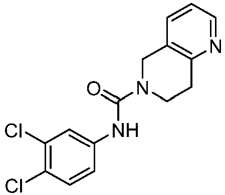 *N*-(3,4-Dichlorophenyl)-7,8-dihydro-1,6-naphthyridine-6(5H)-carboxamide | 322.3, 324.2 at 1.27 min | ¹H NMR (500 MHz, DMSO-d6) δ 8.96 (s, 1H), 8.39 (dd, *J =* 4.7, 1.6 Hz, 1H), 7.86 (t, *J* = 1.4 Hz, 1H), 7.61 (dd, *J* = 7.7, 1.6 Hz, 1H), 7.49 (d, *J* = 1.4 Hz, 2H), 7.24 (dd, *J* = 7.8, 4.7 Hz, 1H), 4.68 (s, 2H), 3.82 (t, *J* = 6.0 Hz, 2H), 2.95 (t, *J* = 6.0 Hz, 2H). |
| 21^{(f)} | 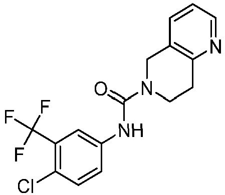 *N*-(4-Chloro-3-(trifluoromethyl)phenyl)-7,8-dihydro-1,6-naphthyridine-6(5H)-carboxamide | 356.3, 358.3 at 1.36 min | ¹H NMR (500 MHz, DMSO-d6) δ 9.11 (s, 1H), 8.40 (dd, *J* = 4.7, 1.6 Hz, 1H), 8.07 (d, *J* = 2.6 Hz, 1H), 7.83 (dd, *J* = 8.9, 2.6 Hz, 1H), 7.65 - 7.55 (m, 2H), 7.25 (dd, *J* = 7.8, 4.8 Hz, 1H), 4.69 (s, 2H), 3.83 (t, *J* = 6.0 Hz, 2H), 2.96 (t, *J =* 5.9 Hz, 2H). |
| 22^{(c)(e)} | 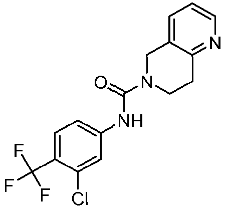 *N*-(3-Chloro-4-(trifluoromethyl)phenyl)-7,8-dihydro-1,6-naphthyridine-6(5H)-carboxamide | 356.4, 358.3 at 1.37 min | ¹H NMR (500 MHz, DMSO-d6) δ 9.23 (s, 1H), 8.40 (dd, *J* = 4.8, 1.7 Hz, 1H), 7.92 (d, *J* = 2.0 Hz, 1H), 7.73 (d, *J* = 8.8 Hz, 1H), 7.69 - 7.56 (m, 2H), 7.25 (dd, *J* = 7.7, 4.8 Hz, 1H), 4.70 (s, 2H), 3.84 (t, *J* = 6.0 Hz, 2H), 2.97 (t, *J* = 6.0 Hz, 2H). |
| 23 | 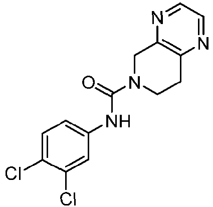 *N*-(3,4-Dichlorophenyl)-7,8-dihydropyrido[3,4-b]pyrazine-6(5H)-carboxamide | 323.3, 325.2 at 1.2 min | ¹H NMR (500 MHz, DMSO-d6) δ 9.05 (s, 1H), 8.49 (s, 2H), 7.88 (t, *J* = 1.4 Hz, 1H), 7.50 (d, *J* = 1.4 Hz, 2H), 4.77 (s, 2H), 3.87 (t, *J* = 5.9 Hz, 2H), 3.02 (t, *J =* 5.9 Hz, 2H) |
| 24^{(d)} | 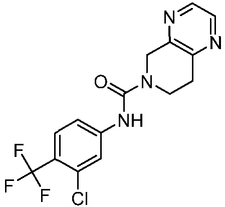 *N*-(3-Chloro-4-(trifluoromethyl)phenyl)-7,8-dihydropyrido[3,4-b]pyrazine-6(5H)-carboxamide | 357.4, 359.3 at 1.32 min | ¹H NMR (500 MHz, DMSO-d6) δ 9.31 (s, 1H), 8.50 (s, 2H), 7.94 (d, *J =* 2.0 Hz, 1H), 7.74 (d, *J =* 8.8 Hz, 1H), 7.69 - 7.63 (m, 1H), 4.80 (s, 2H), 3.90 (t, *J* = 5.9 Hz, 2H), 3.03 (t, *J =* 5.9 Hz, 2H |
| 25 | 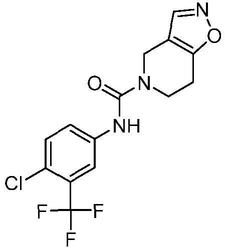 *N*-(4-Chloro-3-(trifluoromethyl)phenyl)-6,7-dihydroisoxazolo[4,5-c]pyridine-5(4H)-carboxamide | 346.3, 348.2 at 1.4 min | ¹H NMR (500 MHz, DMSO-d6) δ 9.16 (s, 1H), 8.54 (s, 1H), 8.04 (d, *J* = 2.6 Hz, 1H), 7.80 (dd, *J* = 8.8, 2.5 Hz, 1H), 7.60 (d, *J* = 8.8 Hz, 1H), 4.47 (d, *J* = 1.7 Hz, 2H), 3.82 (t, *J =* 5.7 Hz, 2H), 2.89 (t, *J* = 5.8 Hz, 2H). |
| 26^{(d)} | 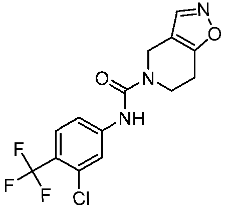 *N*-(3-Chloro-4-(trifluoromethyl)phenyl)-6,7-dihydroisoxazolo[4,5-c]pyridine-5(4H)-carboxamide | 346.3 at 1.43 min | ¹H NMR (500 MHz, DMSO-d6) δ 9.28 (s, 1H), 8.54 (s, 1H), 7.89 (d, *J* = 2.1 Hz, 1H), 7.73 (d, *J* = 8.8 Hz, 1H), 7.64 - 7.59 (m, 1H), 4.47 (d, *J* = 1.7 Hz, 2H), 3.82 (t, *J =* 5.7 Hz, 2H), 2.90 (t, *J =* 5.8 Hz, 2H) |
| 27 | 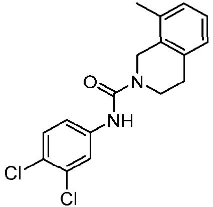 *N*-(3,4-Dichlorophenyl)-8-methyl-3,4-dihydroisoquinoline-2(1H)-carboxamide | 335.3, 337.3 at 1.73 min | ¹H NMR (500 MHz, DMSO-d6) δ 8.91 (s, 1H), 7.87 (t, *J* = 1.4 Hz, 1H), 7.50 (d, *J* = 1.4 Hz, 2H), 7.13 - 6.99 (m, 3H), 4.54 (s, 2H), 3.69 (t, *J =* 5.8 Hz, 2H), 2.84 (t, *J* = 5.8 Hz, 2H), 2.25 (s, 3H) |
| 28 | 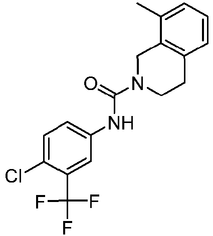 *N*-(4-Chloro-3-(trifluoromethyl)phenyl)-8-methyl-3,4-dihydroisoquinoline-2(1H)-carboxamide | 369.2, 371.3 at 1.78 min | ¹H NMR (500 MHz, DMSO-d6) δ 9.07 (s, 1H), 8.09 (d, *J* = 2.6 Hz, 1H), 7.83 (dd, *J =* 8.9, 2.6 Hz, 1H), 7.60 (d, *J =* 8.8 Hz, 1H), 7.10 (t, *J* = 7.4 Hz, 1H), 7.04 (dd, *J* = 14.5, 7.3 Hz, 2H), 4.55 (s, 2H), 3.70 (t, *J =* 5.9 Hz, 2H), 2.85 (t, *J* = 5.9 Hz, 2H), 2.25 (s, 3H). |
| 29^{(d)} | 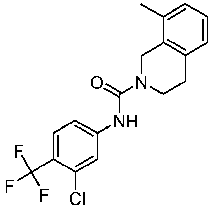 *N*-(3-Chloro-4-(trifluoromethyl)phenyl)-8-methyl-3,4-dihydroisoquinoline-2(1H)-carboxamide | 369.4, 371.3 at 1.80 min | ¹H NMR (500 MHz, DMSO-d6) δ 9.19 (s, 1H), 7.93 (d, *J* = 2.0 Hz, 1H), 7.73 (d, *J* = 8.8 Hz, 1H), 7.66 (dd, *J =* 9.3, 2.0 Hz, 1H), 7.10 (t, *J* = 7.4 Hz, 1H), 7.04 (dd, *J* = 14.9, 7.3 Hz, 2H), 4.56 (s, 2H), 3.71 (t, *J =* 5.8 Hz, 2H), 2.85 (t, *J* = 5.8 Hz, 2H), 2.25 (s, 3H) |
| 30^{(d)} | 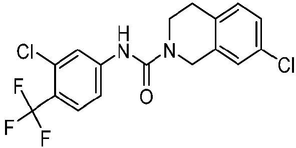 7-Chloro-*N*-(3-chloro-4-(trifluoromethyl)phenyl)-3,4-dihydroisoquinoline-2(1H)-carboxamide | 389.0, 391.2, 393.2 at 1.76 min | ¹H NMR (500 MHz, DMSO-d6) δ 9.16 (s, 1H), 7.92 (d, *J* = 2.0 Hz, 1H), 7.73 (d, *J* = 8.8 Hz, 1H), 7.64 (dd, *J =* 9.1, 1.9 Hz, 1H), 7.30 (d, *J* = 2.0 Hz, 1H), 7.28 - 7.20 (m, 2H), 4.66 (s, 2H), 3.72 (t, *J* = 5.9 Hz, 2H), 2.85 (t, *J* = 5.9 Hz, 2H) |
| 31^{(d)} | 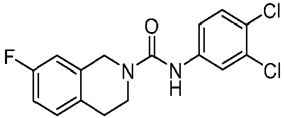 *N*-(3,4-Dichlorophenyl)-7-fluoro-3,4-dihydroisoquinoline-2(1H)-carboxamide | 339.0, 341.1 at 1.59 min | ¹H NMR (500 MHz, DMSO-d6) δ 8.89 (s, 1H), 7.86 (t, *J* = 1.4 Hz, 1H), 7.49 (d, *J* = 1.4 Hz, 2H), 7.23 (dd, *J =* 8.4, 5.9 Hz, 1H), 7.09 - 6.99 (m, 2H), 4.64 (s, 2H), 3.70 (t, *J* = 5.9 Hz, 2H), 2.83 (t, *J =* 5.9 Hz, 2H). |
| 32^{(d)} | 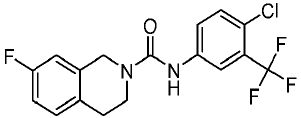 *N*-(4-Chloro-3-(trifluoromethyl)phenyl)-7-fluoro-3,4-dihydroisoquinoline-2(1H)-carboxamide | 373.1 at 1.65 min | ¹H NMR (500 MHz, DMSO-d6) δ 9.04 (s, 1H), 8.07 (d, *J* = 2.6 Hz, 1H), 7.83 (dd, *J* = 8.9, 2.6 Hz, 1H), 7.59 (d, *J* = 8.8 Hz, 1H), 7.24 (dd, *J* = 8.4, 5.8 Hz, 1H), 7.09 - 7.00 (m, 2H), 4.65 (s, 2H), 3.71 (t, *J* = 5.9 Hz, 2H), 2.84 (t, *J =* 5.9 Hz, 2H). |
| 33^{(d)} | 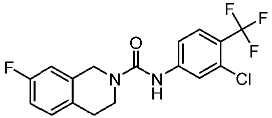 *N*-(3-Chloro-4-(trifluoromethyl)phenyl)-7-fluoro-3,4-dihydroisoquinoline-2(1H)-carboxamide | 372.9 at 1.67 min | ¹H NMR (500 MHz, DMSO-d6) δ 9.16 (s, 1H), 7.92 (d, *J* = 2.0 Hz, 1H), 7.73 (d, *J* = 8.8 Hz, 1H), 7.65 (d, *J =* 9.3 Hz, 1H), 7.24 (dd, *J* = 8.4, 5.9 Hz, 1H), 7.10 - 7.00 (m, 2H), 4.66 (s, 2H), 3.72 (t, *J* = 5.9 Hz, 2H), 2.85 (t, *J =* 5.8 Hz, 2H). |
| 34^{(d)} | 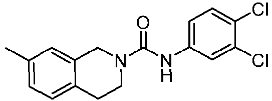 *N*-(3,4-Dichlorophenyl)-7-methyl-3,4-dihydroisoquinoline-2(1H)-carboxamide | 335.1, 337.2 at 1.67 min | ¹H NMR (500 MHz, DMSO-d6) δ 8.85 (s, 1H), 7.87 (dd, *J = 1.9,* 0.9 Hz, 1H), 7.49 (d, *J =* 1.9 Hz, 2H), 7.07 (d, *J =* 7.7 Hz, 1H), 7.02 - 6.97 (m, 2H), 4.59 (s, 2H), 3.68 (t, *J* = 5.9 Hz, 2H), 2.80 (t, *J* = 5.9 Hz, 2H), 2.27 (s, 3H) |
| 35^{(d)} | 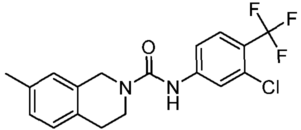 *N*-(3-Chloro-4-(trifluoromethyl)phenyl)-7-methyl-3,4-dihydroisoquinoline-2(1H)-carboxamide | 369.1, 370.0 at 1.75 min | ¹H NMR (500 MHz, DMSO-d6) δ 9.12 (s, 1H), 7.93 (d, *J =* 2.0 Hz, 1H), 7.72 (d, *J* = 8.8 Hz, 1H), 7.68 - 7.61 (m, 1H), 7.08 (d, *J* = 7.6 Hz, 1H), 7.00 (d, *J* = 9.2 Hz, 2H), 4.62 (s, 2H), 3.70 (t, *J* = 5.9 Hz, 2H), 2.81 (t, *J =* 6.0 Hz, 2H), 2.28 (s, 3H). |
| 36^{(d)} | 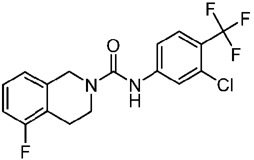 *N-*(3-Chloro-4-(trifluoromethyl)phenyl)-5-fluoro-3,4-dihydroisoquinoline-2(1H)-carboxamide | 373.1, 374.7 at 1.68 min | ¹H NMR (500 MHz, DMSO-d6) δ 9.21 (s, 1H), 7.92 (d, *J* = 2.1 Hz, 1H), 7.73 (d, *J* = 8.8 Hz, 1H), 7.69 - 7.58 (m, 1H), 7.26 (td, *J* = 7.9, 5.8 Hz, 1H), 7.09 - 6.99 (m, 2H), 4.70 (s, 2H), 3.77 (t, *J* = 6.0 Hz, 2H), 2.82 (t, *J =* 6.0 Hz, 2H). |
| 37^{(d)} | 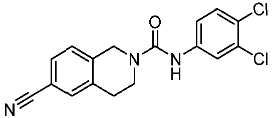 6-Cyano-*N*-(3,4-dichlorophenyl)-3,4-dihydroisoquinoline-2(1H)-carboxamide | 346.2, 348.1 at 1.48 min | ¹H NMR (500 MHz, DMSO-d6) δ 8.92 (s, 1H), 7.87 - 7.82 (m, 1H), 7.71 (s, 1H), 7.67 (d, *J* = 7.9 Hz, 1H), 7.49 (d, *J* = 2.0 Hz, 2H), 7.41 (d, *J* = 8.0 Hz, 1H), 4.72 (s, 2H), 3.72 (t, *J* = 5.9 Hz, 2H), 2.91 (t, *J =* 5.9 Hz, 2H). |
| 38^{(d)} | 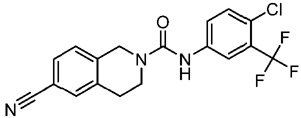 *N*-(4-Chloro-3-(trifluoromethyl)phenyl)-6-cyano-3,4-dihydroisoquinoline-2(1H)-carboxamide | 380.2, 381.9 at 1.55 min | ¹H NMR (500 MHz, DMSO-d6) δ 9.07 (s, 1H), 8.07 (d, *J* = 2.6 Hz, 1H), 7.83 (dd, *J* = 8.9, 2.6 Hz, 1H), 7.71 (d, *J =* 1.7 Hz, 1H), 7.67 (dd, *J* = 7.9, 1.7 Hz, 1H), 7.59 (d, *J* = 8.8 Hz, 1H), 7.41 (d, *J* = 8.0 Hz, 1H), 4.74 (s, 2H), 3.73 (t, *J* = 5.9 Hz, 2H), 2.92 (t, *J =* 5.9 Hz, 2H). |
| 39^{(d)} | 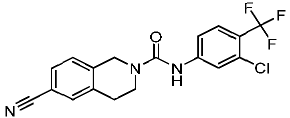 *N*-(3-Chloro-4-(trifluoromethyl)phenyl)-6-cyano-3,4-dihydroisoquinoline-2(1H)-carboxamide | 380.2, 382.2 at 1.56 min | ¹H NMR (500 MHz, DMSO-d6) δ 9.19 (s, 1H), 7.92 (d, *J* = 2.1 Hz, 1H), 7.76 - 7.70 (m, 2H), 7.66 (ddd, *J =* 13.7, 8.3, 1.9 Hz, 2H), 7.42 (d, *J* = 8.0 Hz, 1H), 4.74 (s, 2H), 3.74 (t, *J* = 5.9 Hz, 2H), 2.93 (t, *J* = 5.9 Hz, 2H). |
| 42^{(d)} | 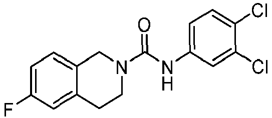 *N*-(3,4-Dichlorophenyl)-6-fluoro-3,4-dihydroisoquinoline-2(1H)-carboxamide | 341.0 at 1.58 min | ¹H NMR (500 MHz, DMSO-d6) δ 8.87 (s, 1H), 7.86 (t, *J* = 1.4 Hz, 1H), 7.49 (d, *J* = 1.4 Hz, 2H), 7.23 (dd, *J =* 8.3, 5.8 Hz, 1H), 7.09 - 7.00 (m, 2H), 4.61 (s, 2H), 3.69 (t, *J* = 5.9 Hz, 2H), 2.87 (t, *J =* 5.9 Hz, 2H) |
| 43^{(d)} | 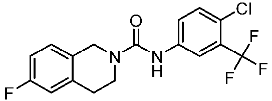 *N*-(4-Chloro-3-(trifluoromethyl)phenyl)-6-fluoro-3,4-dihydroisoquinoline-2(1H)-carboxamide | 373.0, 375.0 at 1.65 min | ¹H NMR (500 MHz, DMSO-d6) δ 9.02 (s, 1H), 8.08 (d, *J* = 2.6 Hz, 1H), 7.84 (dd, *J* = 8.9, 2.6 Hz, 1H), 7.59 (d, *J* = 8.8 Hz, 1H), 7.23 (dd, *J* = 8.3, 5.8 Hz, 1H), 7.06 (ddd, *J* = 13.6, 7.0, 4.3 Hz, 2H), 4.63 (s, 2H), 3.70 (t, *J* = 5.9 Hz, 2H), 2.88 (t, *J* = 6.0 Hz, 2H) |
| 44^{(d)} | 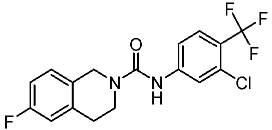 *N*-(3-Chloro-4-(trifluoromethyl)phenyl)-6-fluoro-3,4-dihydroisoquinoline-2(1H)-carboxamide | 373.1, 374.8 at 1.66 min | ¹H NMR (500 MHz, DMSO-d6) δ 9.14 (s, 1H), 7.92 (d, *J =* 2.0 Hz, 1H), 7.72 (d, *J* = 8.8 Hz, 1H), 7.65 (dd, *J =* 8.6, 2.1 Hz, 1H), 7.24 (dd, *J* = 8.3, 5.8 Hz, 1H), 7.10 - 7.01 (m, 2H), 4.64 (s, 2H), 3.71 (t, *J* = 5.9 Hz, 2H), 2.88 (t, *J =* 5.9 Hz, 2H). |
| 45^{(d)} | 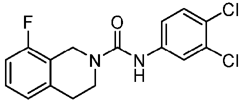 *N*-(3,4-Dichlorophenyl)-8-fluoro-3,4-dihydroisoquinoline-2(1H)-carboxamide | 339.1, 341.1 at 1.62 min | ¹H NMR (500 MHz, DMSO-d6) δ 8.98 (s, 1H), 7.89 - 7.85 (m, 1H), 7.54 - 7.46 (m, 2H), 7.25 (td, *J* = 7.9, 5.9 Hz, 1H), 7.06 (t, *J* = 7.7 Hz, 2H), 4.66 (s, 2H), 3.72 (t, *J* = 5.8 Hz, 2H), 2.88 (t, *J* = 5.8 Hz, 2H). |
| 46^{(d)} | 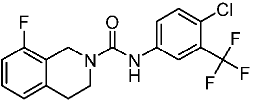 *N*-(4-Chloro-3-(trifluoromethyl)phenyl)-8-fluoro-3,4-dihydroisoquinoline-2(1H)-carboxamide | 373.1, 375.0 at 1.67 min | ¹H NMR (500 MHz, DMSO-d6) δ 9.13 (s, 1H), 8.08 (d, *J* = 2.6 Hz, 1H), 7.84 (dd, *J* = 8.8, 2.6 Hz, 1H), 7.59 (d, *J* = 8.8 Hz, 1H), 7.25 (td, *J* = 7.9, 5.9 Hz, 1H), 7.07 (t, *J* = 7.6 Hz, 2H), 4.67 (s, 2H), 3.74 (t, *J* = 5.8 Hz, 2H), 2.89 (t, *J* = 5.8 Hz, 2H). |
| 47^{(d)} | 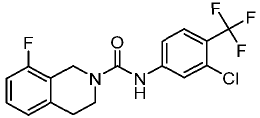 *N*-(3-Chloro-4-(trifluoromethyl)phenyl)-8-fluoro-3,4-dihydroisoquinoline-2(1H)-carboxamide | 373.1, 375.1 at 1.69 min | ¹H NMR (500 MHz, DMSO-d6) δ 9.25 (s, 1H), 7.93 (d, *J =* 2.1 Hz, 1H), 7.73 (d, *J* = 8.8 Hz, 1H), 7.66 (dd, *J =* 8.6, 2.1 Hz, 1H), 7.25 (td, *J* = 7.9, 5.9 Hz, 1H), 7.07 (t, *J* = 7.7 Hz, 2H), 4.68 (s, 2H), 3.74 (t, *J* = 5.8 Hz, 2H), 2.89 (t, *J* = 5.8 Hz, 2H). |
| 49^{(d)} | 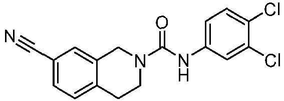 7-Cyano-*N*-(3,4-dichlorophenyl)-3,4-dihydroisoquinoline-2(1H)-carboxamide | 346.0, 348.0 at 1.48 min | ¹H NMR (500 MHz, DMSO-d6) δ 8.93 (s, 1H), 7.85 (dd, *J* = 1.9, 0.8 Hz, 1H), 7.71 (d, *J* = 1.7 Hz, 1H), 7.65 (dd, *J* = 7.9, 1.7 Hz, 1H), 7.53 - 7.43 (m, 2H), 7.41 (d, *J* = 7.9 Hz, 1H), 4.68 (s, 2H), 3.72 (t, *J* = 5.9 Hz, 2H), 2.95 (t, *J* = 5.9 Hz, 2H). |
| 50^{(d)} | 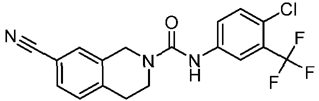 *N*-(4-Chloro-3-(trifluoromethyl)phenyl)-7-cyano-3,4-dihydroisoquinoline-2(1H)-carboxamide | 380.2, 382.2 at 1.55 min | ¹H NMR (500 MHz, DMSO-d6) δ 9.08 (s, 1H), 8.06 (d, *J =* 2.6 Hz, 1H), 7.83 (dd, *J* = 8.8, 2.6 Hz, 1H), 7.71 (d, *J =* 1.7 Hz, 1H), 7.65 (dd, *J* = 7.8, 1.7 Hz, 1H), 7.59 (d, *J =* 8.8 Hz, 1H), 7.42 (d, *J =* 7.9 Hz, 1H), 4.69 (s, 2H), 3.74 (t, *J* = 5.9 Hz, 2H), 2.96 (t, *J =* 5.9 Hz, 2H). |
| 51^{(d)} | 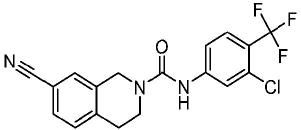 *N*-(3-Chloro-4-(trifluoromethyl)phenyl)-7-cyano-3,4-dihydroisoquinoline-2(1H)-carboxamide | 380.0, 382.1 at 1.56 min | ¹H NMR (500 MHz, DMSO-d6) δ 9.20 (s, 1H), 7.91 (d, *J* = 2.0 Hz, 1H), 7.76 - 7.70 (m, 2H), 7.65 (td, *J* = 9.3, 8.6, 1.9 Hz, 2H), 7.42 (d, *J* = 7.9 Hz, 1H), 4.70 (s, 2H), 3.74 (t, *J* = 5.9 Hz, 2H), 2.96 (t, *J =* 5.9 Hz, 2H). |
| 52^{(d)} | 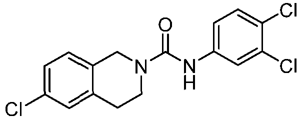 6-Chloro-*N*-(3,4-dichlorophenyl)-3,4-dihydroisoquinoline-2(1H)-carboxamide | 355.0, 357.0 at 1.68 min | ¹H NMR (500 MHz, DMSO-d6) δ 8.88 (s, 1H), 7.86 (t, *J* = 1.4 Hz, 1H), 7.49 (d, *J =* 1.4 Hz, 2H), 7.32 - 7.20 (m, 3H), 4.62 (s, 2H), 3.69 (t, *J* = 5.9 Hz, 2H), 2.87 (t, *J =* 5.9 Hz, 2H). |
| 53^{(d)} | 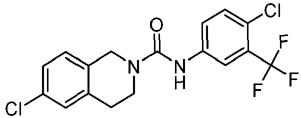 6-Chloro-*N*-(4-chloro-3-(trifluoromethyl)phenyl)-3,4-dihydroisoquinoline-2(1H)-carboxamide | 389.3, 391.2 at 1.74 min | ¹H NMR (500 MHz, DMSO-d6) δ 9.03 (s, 1H), 8.07 (d, *J* = 2.6 Hz, 1H), 7.83 (dd, *J* = 8.8, 2.6 Hz, 1H), 7.59 (d, *J = 8.8 Hz,* 1H), 7.32 - 7.20 (m, 3H), 4.64 (s, 2H), 3.70 (t, *J* = 5.9 Hz, 2H), 2.87 (t, *J* = 5.9 Hz, 2H). |
| 54^{(d)} | 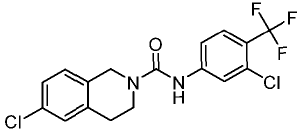 6-Chloro-*N*-(3-chloro-4-(trifluoromethyl)phenyl)-3,4-dihydroisoquinoline-2(1H)-carboxamide | 389.2, 391.0 at 1.75 min | ¹H NMR (500 MHz, DMSO-d6) δ 9.15 (s, 1H), 7.92 (d, *J =* 2.0 Hz, 1H), 7.73 (d, *J* = 8.8 Hz, 1H), 7.67 - 7.62 (m, 1H), 7.32 - 7.21 (m, 3H), 4.64 (s, 2H), 3.71 (t, *J* = 5.9 Hz, 2H), 2.88 (t, *J =* 5.9 Hz, 2H). |
| 55^{(d)} | 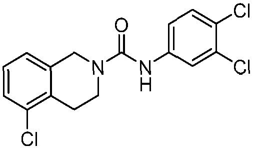 5-Chloro-*N*-(3,4-dichlorophenyl)-3,4-dihydroisoquinoline-2(1H)-carboxamide | 355.0, 357.0, 359.1 at 1.69 min | ¹H NMR (500 MHz, DMSO-d6) δ 8.94 (s, 1H), 7.88 - 7.84 (m, 1H), 7.53 - 7.45 (m, 2H), 7.35 (dd, *J* = 7.9, 1.3 Hz, 1H), 7.25 (t, *J =* 7.8 Hz, 1H), 7.22 - 7.17 (m, 1H), 4.67 (s, 2H), 3.77 (t, *J* = 6.0 Hz, 2H), 2.85 (t, *J =* 6.0 Hz, 2H). |
| 56^{(d)} | 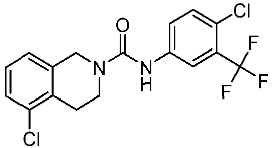 5-Chloro-*N*-(4-chloro-3-(trifluoromethyl)phenyl)-3,4-dihydroisoquinoline-2(1H)-carboxamide | 388.8, 391.2 at 1.75 min | ¹H NMR (500 MHz, DMSO-d6) δ 9.10 (s, 1H), 8.07 (d, *J* = 2.6 Hz, 1H), 7.83 (dd, *J* = 8.8, 2.6 Hz, 1H), 7.59 (d, *J* = 8.8 Hz, 1H), 7.35 (dd, *J* = 7.9, 1.3 Hz, 1H), 7.25 (t, *J* = 7.8 Hz, 1H), 7.22 - 7.17 (m, 1H), 4.69 (s, 2H), 3.78 (t, *J* = 6.0 Hz, 2H), 2.86 (t, *J* = 6.0 Hz, 2H). |
| 57^{(d)} | 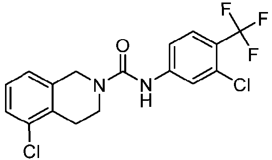 5-Chloro-*N*-(3-chloro-4-(trifluoromethyl)phenyl)-3,4-dihydroisoquinoline-2(1H)-carboxamide | 389.0, 391.2 at 1.76 min | ¹H NMR (500 MHz, DMSO-d6) δ 9.21 (s, 1H), 7.92 (d, *J =* 2.1 Hz, 1H), 7.73 (d, *J* = 8.8 Hz, 1H), 7.64 (dd, *J =* 8.5, 2.1 Hz, 1H), 7.35 (dd, *J* = 7.8, 1.3 Hz, 1H), 7.26 (t, *J* = 7.7 Hz, 1H), 7.20 (dd, *J* = 7.7, 1.3 Hz, 1H), 4.70 (s, 2H), 3.79 (t, *J* = 6.0 Hz, 2H), 2.86 (t, *J* = 6.0 Hz, 2H). |
| 58^{(d)} | 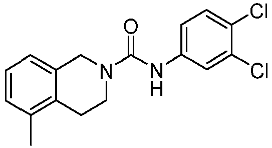 *N*-(3,4-Dichlorophenyl)-5-methyl-3,4-dihydroisoquinoline-2(1H)-carboxamide | 335.1, 337.2 at 1.65 min | ¹H NMR (500 MHz, DMSO-d6) δ 8.89 (s, 1H), 7.87 (dd, *J =* 2.0, 0.8 Hz, 1H), 7.49 (t, *J* = 1.4 Hz, 2H), 7.10 (t, *J =* 7.5 Hz, 1H), 7.05 (d, *J =* 7.2 Hz, 1H), 7.00 (d, *J* = 7.1 Hz, 1H), 4.63 (s, 2H), 3.74 (t, *J* = 6.0 Hz, 2H), 2.73 (t, *J =* 6.0 Hz, 2H), 2.22 (s, 3H). |
| 59^{(d)} | 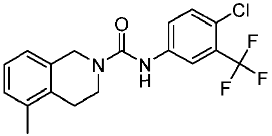 *N*-(4-Chloro-3-(trifluoromethyl)phenyl)-5-methyl-3,4-dihydroisoquinoline-2(1H)-carboxamide | 369.1, 371.1 at 1.71 min | ¹H NMR (500 MHz, DMSO-d6) δ 9.05 (s, 1H), 8.08 (d, *J* = 2.6 Hz, 1H), 7.84 (dd, *J* = 8.8, 2.6 Hz, 1H), 7.58 (d, *J =* 8.8 Hz, 1H), 7.10 (t, *J =* 7.4 Hz, 1H), 7.06 (d, *J =* 7.2 Hz, 1H), 7.01 (d, *J* = 7.5 Hz, 1H), 4.65 (s, 2H), 3.76 (t, *J* = 6.0 Hz, 2H), 2.74 (t, *J* = 6.0 Hz, 2H), 2.22 (s, 3H). |
| 60^{(d)} | 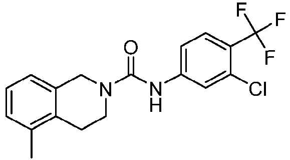 *N*-(3-Chloro-4-(trifluoromethyl)phenyl)-5-methyl-3,4-dihydroisoquinoline-2(1H)-carboxamide | 369.2, 371.4 at 1.73 min | ¹H NMR (500 MHz, DMSO-d6) δ 9.17 (s, 1H), 7.93 (d, *J =* 2.1 Hz, 1H), 7.72 (d, *J =* 8.8 Hz, 1H), 7.68 - 7.62 (m, 1H), 7.11 (t, *J* = 7.4 Hz, 1H), 7.06 (d, *J* = 7.2 Hz, 1H), 7.01 (d, *J* = 7.5 Hz, 1H), 4.66 (s, 2H), 3.76 (t, *J* = 6.0 Hz, 2H), 2.75 (t, *J* = 6.0 Hz, 2H), 2.22 (s, 3H). |
| 61^{(d)} | 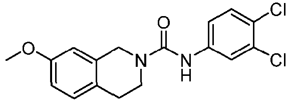 *N*-(3,4-Dichlorophenyl)-7-methoxy-3,4-dihydroisoquinoline-2(1H)-carboxamide | 351.1, 353.1 at 1.56 min | ¹H NMR (500 MHz, DMSO-d6) δ 8.85 (s, 1H), 7.87 (dd, *J* = 2.0, 0.9 Hz, 1H), 7.53 - 7.45 (m, 2H), 7.10 (d, *J =* 8.2 Hz, 1H), 6.81 - 6.74 (m, 2H), 4.61 (s, 2H), 3.73 (s, 3H), 3.68 (t, *J* = 5.9 Hz, 2H), 2.78 (t, *J* = 5.9 Hz, 2H). |
| 62^{(d)} | 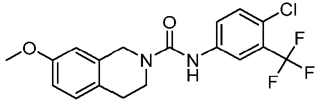 *N*-(4-Chloro-3-(trifluoromethyl)phenyl)-7-methoxy-3,4-dihydroisoquinoline-2(1H)-carboxamide | 385.2, 387.1 at 1.62 min | ¹H NMR (500 MHz, DMSO-d6) δ 9.00 (s, 1H), 8.08 (d, *J* = 2.6 Hz, 1H), 7.84 (dd, *J* = 8.8, 2.6 Hz, 1H), 7.58 (d, *J =* 8.9 Hz, 1H), 7.11 (d, *J* = 8.2 Hz, 1H), 6.81 - 6.74 (m, 2H), 4.62 (s, 2H), 3.74 (s, 3H), 3.69 (t, *J* = 5.9 Hz, 2H), 2.79 (t, *J* = 5.9 Hz, 2H). |
| 63^{(d)} | 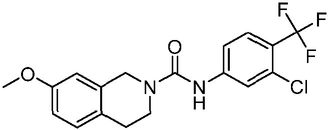 *N*-(3-Chloro-4-(trifluoromethyl)phenyl)-7-methoxy-3,4-dihydroisoquinoline-2(1H)-carboxamide | 385.1, 387.0 at 1.64 min | ¹H NMR (500 MHz, DMSO-d6) δ 9.12 (s, 1H), 7.93 (d, *J =* 2.1 Hz, 1H), 7.72 (d, *J =* 8.8 Hz, 1H), 7.65 (dd, *J =* 8.7, 2.1 Hz, 1H), 7.11 (d, *J* = 8.1 Hz, 1H), 6.81 - 6.75 (m, 2H), 4.63 (s, 2H), 3.74 (s, 3H), 3.70 (t, *J* = 5.9 Hz, 2H), 2.79 (t, *J* = 5.9 Hz, 2H). |
| 64^{(d)} | 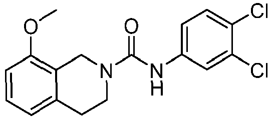 *N*-(3,4-Dichlorophenyl)-8-methoxy-3,4-dihydroisoquinoline-2(1H)-carboxamide | 351.1, 353.1 at 1.62 min | ¹H NMR (500 MHz, DMSO-d6) δ 8.95 (s, 1H), 7.88 - 7.84 (m, 1H), 7.53 - 7.45 (m, 2H), 7.17 (t, *J =* 7.9 Hz, 1H), 6.85 (d, *J* = 8.2 Hz, 1H), 6.78 (d, *J* = 7.6 Hz, 1H), 4.52 (s, 2H), 3.82 (s, 3H), 3.67 (t, *J* = 5.8 Hz, 2H), 2.82 (t, *J* = 5.8 Hz, 2H). |
| 65^{(d)} | 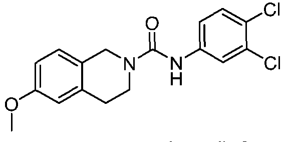 *N*-(3,4-Dichlorophenyl)-6-methoxy-3,4-dihydroisoquinoline-2(1H)-carboxamide | 351.1, 353.1 at 1.55 min | ¹H NMR (500 MHz, DMSO-d6) δ 8.83 (s, 1H), 7.89 - 7.85 (m, 1H), 7.53 - 7.45 (m, 2H), 7.12 - 7.07 (m, 1H), 6.78 (d, *J* = 7.5 Hz, 2H), 4.56 (s, 2H), 3.73 (s, 3H), 3.67 (t, *J* = 5.9 Hz, 2H), 2.83 (t, *J* = 5.9 Hz, 2H). |
| 66^{(d)} | 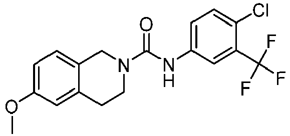 *N*-(4-Chloro-3-(trifluoromethyl)phenyl)-6-methoxy-3,4-dihydroisoquinoline-2(1H)-carboxamide | 385.1, 387.1 at 1.61 min | ¹H NMR (500 MHz, DMSO-d6) δ 8.99 (s, 1H), 8.08 (d, *J* = 2.6 Hz, 1H), 7.84 (dd, *J* = 8.9, 2.6 Hz, 1H), 7.58 (d, *J =* 8.8 Hz, 1H), 7.10 (d, *J =* 8.4 Hz, 1H), 6.79 (d, *J =* 7.3 Hz, 2H), 4.58 (s, 2H), 3.73 (s, 3H), 3.68 (t, *J* = 5.9 Hz, 2H), 2.84 (t, *J* = 5.9 Hz, 2H). |
| 67^{(d)} | 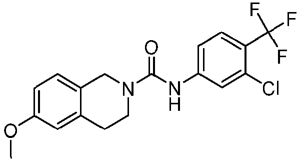 *N*-(3-Chloro-4-(trifluoromethyl)phenyl)-6-methoxy-3,4-dihydroisoquinoline-2(1H)-carboxamide | 385.1, 387.1 at 1.61 min | ¹H NMR (500 MHz, DMSO-d6) δ 9.10 (s, 1H), 7.93 (d, *J =* 2.1 Hz, 1H), 7.72 (d, *J* = 8.8 Hz, 1H), 7.68 - 7.62 (m, 1H), 7.10 (d, *J* = 8.4 Hz, 1H), 6.79 (d, *J* = 7.2 Hz, 2H), 4.59 (s, 2H), 3.74 (s, 3H), 3.69 (t, *J* = 5.9 Hz, 2H), 2.84 (t, *J* = 5.9 Hz, 2H). |
| 70^{(b)} | 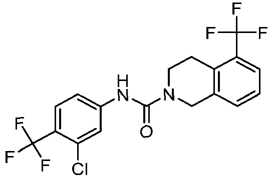 *N*-(3-Chloro-4-(trifluoromethyl)phenyl)-5-(trifluoromethyl)-3,4-dihydroisoquinoline-2(1H)-carboxamide | 423.3, 425.3 at 1.85 min | ¹H NMR (500 MHz, DMSO-d6) δ 9.19 (s, 1H), 7.92 (d, *J* = 2.1 Hz, 1H), 7.73 (d, *J =* 8.8 Hz, 1H), 7.68 - 7.60 (m, 2H), 7.54 (d, *J* = 7.7 Hz, 1H), 7.44 (t, *J* = 7.8 Hz, 1H), 4.75 (s, 2H), 3.76 (t, *J* = 5.9 Hz, 2H), 3.01 (t, *J =* 6.0 Hz, 2H). |
| 71^{(b)} | 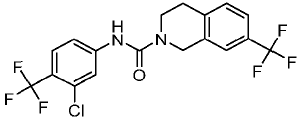 *N*-(3-Chloro-4-(trifluoromethyl)phenyl)-7-(trifluoromethyl)-3,4-dihydroisoquinoline-2(1H)-carboxamide | 423.3, 425.3 at 1.85 min | ¹H NMR (500 MHz, DMSO-d6) δ 9.19 (s, 1H), 7.92 (d, *J=* 2.1 Hz, 1H), 7.73 (d, *J =* 8.8 Hz, 1H), 7.64 (dd, *J =* 8.5, 2.1 Hz, 1H), 7.59 (s, 1H), 7.58 - 7.52 (m, 1H), 7.44 (d, *J =* 8.0 Hz, 1H), 4.75 (s, 2H), 3.76 (t, *J =* 5.9 Hz, 2H), 2.96 (t, *J* = 6.0 Hz, 2H). |
| 72^{(b)} | 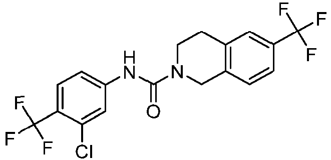 *N*-(3-Chloro-4-(trifluoromethyl)phenyl)-6-(trifluoromethyl)-3,4-dihydroisoquinoline-2(1H)-carboxamide | 423.4, 425.3 at 1.85 min | ¹H NMR (500 MHz, DMSO-d6) δ 9.19 (s, 1H), 7.93 (d, *J=* 2.1 Hz, 1H), 7.73 (d, *J* = 8.8 Hz, 1H), 7.65 (dd, *J =* 8.9, 2.0 Hz, 1H), 7.60 (s, 1H), 7.56 (d, *J =* 8.0 Hz, 1H), 7.44 (d, *J* = 8.0 Hz, 1H), 4.75 (s, 2H), 3.76 (t, *J =* 5.9 Hz, 2H), 2.97 (t, *J* = 5.9 Hz, 2H). |
| 74^{(b)} | 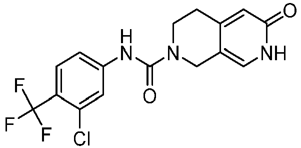 *N*-(3-Chloro-4-(trifluoromethyl)phenyl)-6-oxo-3,4,6,7-tetrahydro-2,7-naphthyridine-2(1H)-carboxamide | 372.3, 374.3 at 1.18 min | ¹H NMR (500 MHz, DMSO-d6) δ 11.39 (s, 1H),9.03 (s, 1H), 7.91 (d, *J* = 2.1 Hz, 1H), 7.72 (d, *J* = 8.8 Hz, 1H), 7.64 (dd, *J* = 8.9, 2.1 Hz, 1H), 7.33 (s, 1H), 6.21 (s, 1H), 4.40 (s, 2H), 3.61 (t, *J* = 6.1 Hz, 2H), 2.78 (t, *J* = 6.1 Hz, 2H). |
| 76^{(b)} | 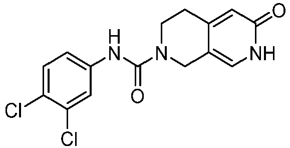 *N*-(3,4-Dichlorophenyl)-6-oxo-3,4,6,7-tetrahydro-2,7-naphthyridine-2(1H)-carboxamide | 338.2, 340.2 at 1.09 min | ¹H NMR (500 MHz, DMSO-d6) δ 11.39 (s, 1H), 8.77 (s, 1H), 7.85 (d, *J* = 1.5 Hz, 1H), 7.48 (d, *J* = 1.4 Hz, 2H), 7.32 (s, 1H), 6.20 (s, 1H), 4.38 (s, 2H), 3.59 (t, *J* = 6.1 Hz, 2H), 2.76 (t, *J =* 6.1 Hz, 2H). |
| 77^{(b)} | 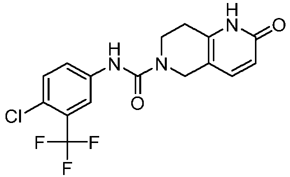 *N*-(4-Chloro-3-(trifluoromethyl)phenyl)-2-oxo-1,5,7,8-tetrahydro-1,6-naphthyridine-6(2H)-carboxamide | 372.3, 374.3 at 1.19 min | ¹H NMR (500 MHz, DMSO-d6) δ 9.05 (s, 1H), 8.05 (d, *J* = 2.6 Hz, 1H), 7.81 (dd, *J* = 8.8, 2.6 Hz, 1H), 7.59 (d, *J =* 8.7 Hz, 1H), 7.25 (d, *J =* 9.2 Hz, 1H), 6.21 (d, *J* = 9.5 Hz, 1H), 4.35 (s, 2H), 3.69 (t, *J* = 5.8 Hz, 2H), 2.66 - 2.60 (m, 2H). (1NH not visible) |
| 78^{(b)} | 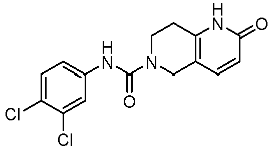 *N*-(3,4-Dichlorophenyl)-2-oxo-1,5,7,8-tetrahydro-1,6-naphthyridine-6(2H)-carboxamide | 338.3, 340.2 at 1.11 min | ¹H NMR (500 MHz, DMSO-d6) δ 11.50 (s, 1H),8.90 (s, 1H), 7.84 (d, *J =* 2.2 Hz, 1H), 7.52 - 7.44 (m, 2H), 7.25 (d, *J =* 9.3 Hz, 1H), 6.21 (d, *J =* 9.3 Hz, 1H), 4.34 (s, 2H), 3.68 (t, *J* = 5.8 Hz, 2H), 2.61 (t, *J =* 5.8 Hz, 2H). |
| 80 | 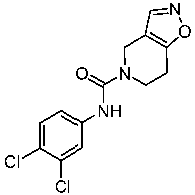 *N*-(3,4-Dichlorophenyl)-6,7-dihydroisoxazolo[4,5-c]pyridine-5(4H)-carboxamide | 312.2, 314.2 at 1.32 min | ¹H NMR (500 MHz, DMSO-d6) δ 9.01 (s, 1H), 8.54 (s, 1H), 7.83 (d, *J =* 2.4 Hz, 1H), 7.50 (d, *J =* 8.8 Hz, 1H), 7.46 (dd, *J* = 8.9, 2.4 Hz, 1H), 4.45 (d, *J =* 1.7 Hz, 2H), 3.80 (t, *J =* 5.7 Hz, 2H), 2.88 (t, *J* = 5.8 Hz, 2H) |
| 81 | 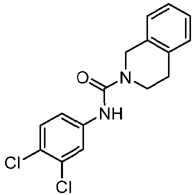 *N*-(3,4-Dichlorophenyl)-3,4-dihydroisoquinoline-2(1H)-carboxamide | 321.3, 323.3 at 1.63 min | ¹H NMR (500 MHz, DMSO-d6) δ 8.86 (s, 1H), 7.87 (d, *J =* 2.0 Hz, 1H), 7.50 (d, *J =* 2.8 Hz, 2H), 7.23 - 7.16 (m, 4H), 4.64 (s, 2H), 3.70 (t, *J =* 5.9 Hz, 2H), 2.86 (t, *J* = 5.9 Hz, 2H). |
| 88⁽ⁱ⁾ | 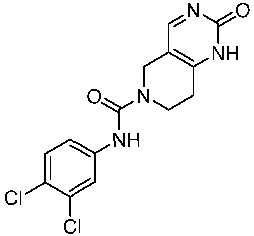 *N*-(3,4-Dichlorophenyl)-2-oxo-1,5,7,8-tetrahydropyrido[4,3-d]pyrimidine-6(2H)-carboxamide | 339.1, 341.2 at 0.79 min | ¹H NMR (500 MHz, DMSO-d6) δ 8.94 (s, 1H), 8.11 (s, 1H), 7.84 (d, *J* = 2.3 Hz, 1H), 7.50 (d, *J* = 8.8 Hz, 1H), 7.46 (dd, *J* = 8.8, 2.4 Hz, 1H), 4.41 (s, 2H), 3.70 (t, *J* = 6.0 Hz, 2H), 2.70 (t, *J* = 5.8 Hz, 2H). (1 exchangeable H not visible) |
| 89⁽ⁱ⁾ | 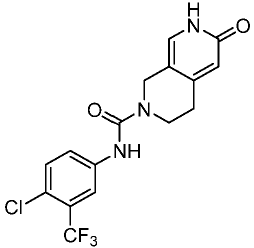 *N*-(4-Chloro-3-(trifluoromethyl)phenyl)-6-oxo-3,4,6,7-tetrahydro-2,7-naphthyridine-2(1H)-carboxamide | 372.3, 374.2 at 1.11 min | ¹H NMR (500 MHz, DMSO-d6) δ 11.39 (s, 1H), 8.92 (s, 1H), 8.06 (d, *J* = 2.6 Hz, 1H), 7.82 (dd, *J* = 8.8, 2.6 Hz, 1H), 7.58 (d, *J =* 8.8 Hz, 1H), 7.32 (s, 1H), 6.21 (s, 1H), 4.40 (s, 2H), 3.61 (t, *J* = 6.1 Hz, 2H), 2.83 - 2.72 (m, 2H). |
| 90 | 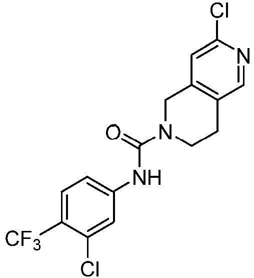 7-Chloro-*N*-(3-chloro-4-(trifluoromethyl)phenyl)-3,4-dihydro-2,6-naphthyridine-2(1H)-carboxamide | 390.3, 392.3 at 1.56 min | ¹H NMR (500 MHz, DMSO-d6) δ 9.24 (s, 1H), 8.26 (s, 1H), 7.91 (d, *J =* 2.1 Hz, 1H), 7.74 (d, *J =* 8.8 Hz, 1H), 7.63 (dd, *J =* 8.6, 2.1 Hz, 1H), 7.42 (s, 1H), 4.70 (s, 2H), 3.76 (t, *J =* 5.8 Hz, 2H), 2.88 (t, *J =* 5.8 Hz, 2H). |
| 91 | 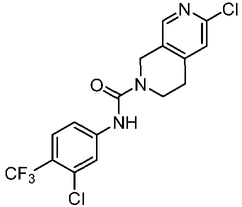 6-Chloro-*N*-(3-chloro-4-(trifluoromethyl)phenyl)-3,4-dihydro-2,7-naphthyridine-2(1H)-carboxamide | 390.3, 392.3 at 1.55 min | ¹H NMR (500 MHz, DMSO-d6) δ 9.22 (s, 1H), 8.28 (s, 1H), 7.91 (d, *J =* 2.1 Hz, 1H), 7.73 (d, *J =* 8.8 Hz, 1H), 7.64 (dd, *J =* 8.8, 2.1 Hz, 1H), 7.41 (s, 1H), 4.69 (s, 2H), 3.73 (t, *J =* 5.9 Hz, 2H), 2.92 (t, *J =* 5.9 Hz, 2H). |
| 93^{(j)} | 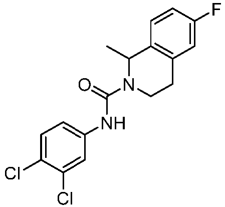 *N*-(3,4-Dichlorophenyl)-6-fluoro-1-methyl-3,4-dihydroisoquinoline-2(1H)-carboxamide | 353.2, 355.3 at 1.73 min | ¹H NMR (500 MHz, DMSO-d6) δ 8.82 (s, 1H), 7.89 - 7.84 (m, 1H), 7.50 (d, *J =* 1.8 Hz, 2H), 7.27 (dd, *J =* 8.2, 5.8 Hz, 1H), 7.04 (t, *J =* 8.7 Hz, 2H), 5.33 (q, *J* = 6.7 Hz, 1H), 4.15 - 4.09 (m, 1H), 3.28 (d, *J =* 3.9 Hz, 1H), 2.89 (ddd, *J =* 16.3, 10.6, 5.4 Hz, 1H), 2.84 - 2.78 (m, 1H), 1.42 (d, *J* = 6.7 Hz, 3H). |
| 96⁽ⁱ⁾ | 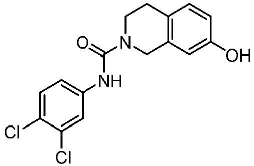 *N*-(3,4-Dichlorophenyl)-7-hydroxy-3,4-dihydroisoquinoline-2(1H)-carboxamide | 337.2, 339.3, 341.2 at 1.40 min | ¹H NMR (500 MHz, DMSO-d6) δ 9.24 (s, 1H), 8.81 (s, 1H), 7.87 (d, *J* = 2.1 Hz, 1H), 7.55 - 7.34 (m, 2H), 6.97 (d, *J* = 8.2 Hz, 1H), 6.60 (dd, *J* = 8.2, 2.6 Hz, 1H), 6.56 (d, *J* = 2.5 Hz, 1H), 4.54 (s, 2H), 3.65 (t, *J* = 5.9, 5.9 Hz, 2H), 2.72 (t, *J* = 5.9, 5.9 Hz, 2H). |
| 98^{(b)(g)} | 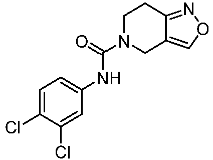 *N*-(3,4-dichlorophenyl)-6,7-dihydroisoxazolo[4,3-c]pyridine-5(4H)-carboxamide | 310.1, 312.1 at 1.29 min^{(l)} | ¹H NMR (500 MHz, DMSO-d6) δ 9.01 (s, 1H), 8.75 (d, *J =* 1.3 Hz, 1H), 7.84 (d, *J* = 2.4 Hz, 1H), 7.50 (d, *J =* 8.8 Hz, 1H), 7.46 (dd, *J =* 8.9, 2.4 Hz, 1H), 4.59 (d, *J =* 1.3 Hz, 2H), 3.77 (t, *J =* 5.9 Hz, 2H), 2.86 (t, *J* = 5.9 Hz, 2H). |
| 99^{(b)} | 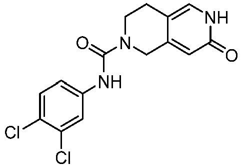 *N*-(3,4-Dichlorophenyl)-7-oxo-3,4,6,7-tetrahydro-2,6-naphthyridine-2(1H)-carboxamide | 338.2, 340.2 at 1.07 min | ¹H NMR (500 MHz, DMSO-d6) δ 11.43 (s, 1H), 8.82 (s, 1H), 7.85 (t, *J* = 1.4 Hz, 1H), 7.49 (d, *J =* 1.5 Hz, 2H), 7.27 (s, 1H), 6.19 (s, 1H), 4.50 (d, *J =* 1.3 Hz, 2H), 3.62 (t, *J =* 6.0 Hz, 2H), 2.63 (t, *J =* 6.0 Hz, 2H). |
| 100^{(b)} | 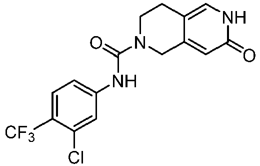 *N*-(3-Chloro-4-(trifluoromethyl)phenyl)-7-oxo-3,4,6,7-tetrahydro-2,6-naphthyridine-2(1H)-carboxamide | 372.3, 374.3 at 1.20 min | ¹H NMR (500 MHz, DMSO-d6) δ 11.44 (s, 1H), 9.10 (s, 1H), 7.91 (d, *J =* 2.1 Hz, 1H), 7.73 (d, *J =* 8.8 Hz, 1H), 7.67 - 7.61 (m, 1H), 7.28 (s, 1H), 6.20 (s, 1H), 4.54 - 4.50 (m, 2H), 3.64 (dd, *J =* 7.4, 4.6 Hz, 2H), 2.65 (t, *J =* 5.8 Hz, 2H). |
| 101^{(b)(g)} | 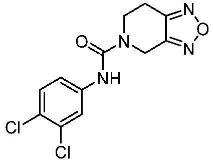 *N*-(3,4-dichlorophenyl)-6,7-dihydro-[1,2,5]oxadiazolo[3,4-c]pyridine-5(4H)-carboxamide | 311.1, 313.1 at 1.39 min^{(l)} | ¹H NMR (500 MHz, DMSO-d6) δ 9.12 (s, 1H), 7.84 (d, *J =* 2.4 Hz, 1H), 7.52 (d, *J =* 8.8 Hz, 1H), 7.47 (dd, *J =* 8.9, 2.4 Hz, 1H), 4.94 (s, 2H), 3.87 (t, *J* = 5.9 Hz, 2H), 3.05 (t, *J =* 5.9 Hz, 2H). |
| 102^{(b)(k)} | 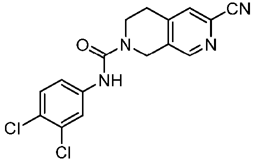 6-Cyano-*N*-(3,4-dichlorophenyl)-3,4-dihydro-2,7-naphthyridine-2(1H)-carboxamide | 347.0, 349.3 at 1.31 min | ¹H NMR (500 MHz, DMSO-d6) δ 8.99 (s, 1H), 8.60 (s, 1H), 7.92 (s, 1H), 7.84 (d, *J =* 2.3 Hz, 1H), 7.53 - 7.45 (m, 2H), 4.77 (s, 2H), 3.74 (t, *J =* 5.9 Hz, 2H), 2.94 (t, *J =* 5.8 Hz, 2H). |
| 103^{(b)(k)} | 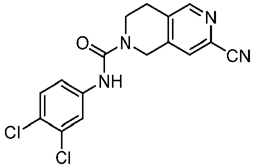 7-Cyano-*N*-(3,4-dichlorophenyl)-3,4-dihydro-2,6-naphthyridine-2(1H)-carboxamide | 347.1, 349.1 at 1.30 min | ¹H NMR (500 MHz, DMSO-d6) δ 9.01 (s, 1H), 8.59 (s, 1H), 7.94 (s, 1H), 7.84 (d, *J =* 2.3 Hz, 1H), 7.54 - 7.44 (m, 2H), 4.72 (s, 2H), 3.76 (t, *J =* 5.8 Hz, 2H), 2.96 (t, *J =* 5.8 Hz, 2H). |
| 105^{(b)(g)} | 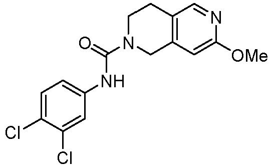 *N*-(3,4-Dichlorophenyl)-7-methoxy-3,4-dihydro-2,6-naphthyridine-2(1H)-carboxamide | 352.4, 354.1 at 1.41 min | ¹H NMR (500 MHz, DMSO-d6) δ 8.90 (s, 1H), 8.01 (s, 1H), 7.85 (dd, *J =* 1.8, 1.0 Hz, 1H), 7.53 - 7.45 (m, 2H), 6.67 (s, 1H), 4.63 (s, 2H), 3.82 (s, 3H), 3.71 (t, *J =* 5.9 Hz, 2H), 2.80 (t, *J* = 5.9 Hz, 2H). |
| 106^{(b)(g)} | 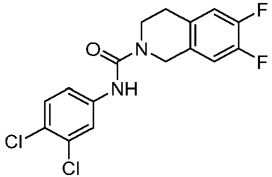 *N*-(3,4-dichlorophenyl)-6,7-difluoro-3,4-dihydroisoquinoline-2(1H)-carboxamide | 355.1, 357.1 at 1.69 min^{(l)} | ¹H NMR (500 MHz, DMSO-d6) δ 8.90 (s, 1H), 7.85 (dd, *J =* 2.0, 0.8 Hz, 1H), 7.53 - 7.45 (m, 2H), 7.30 (dt, *J =* 11.3, 7.5 Hz, 2H), 4.59 (s, 2H), 3.69 (t, *J =* 5.9 Hz, 2H), 2.83 (t, *J =* 5.9 Hz, 2H).1H NMR (500 MHz, DMSO-d6) δ 8.90 (s, 1H), 8.01 (s, 1H), 7.85 (dd, *J* = 1.8, 1.0 Hz, 1H), 7.53 - 7.45 (m, 2H), 6.67 (s, 1H), 4.63 (s, 2H), 3.82 (s, 3H), 3.71 (t, *J =* 5.9 Hz, 2H), 2.80 (t, *J =* 5.9 Hz, 2H). |
| 107^{(b)(g)} | 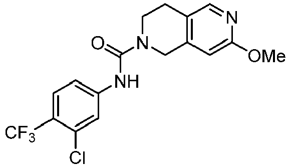 *N*-(3-Chloro-4-(trifluoromethyl)phenyl)-7-methoxy-3,4-dihydro-2,6-naphthyridine-2(1H)-carboxamide | 386.4, 388.4 at 1.54 min | ¹H NMR (500 MHz, DMSO-d6) δ 9.18 (s, 1H), 8.02 (s, 1H), 7.92 (d, *J =* 2.1 Hz, 1H), 7.73 (d, *J =* 8.8 Hz, 1H), 7.67 - 7.61 (m, 1H), 6.68 (s, 1H), 4.65 (s, 2H), 3.82 (s, 3H), 3.73 (t, *J* = 5.9 Hz, 2H), 2.81 (t, *J =* 5.9 Hz, 2H). |
| 108^{(b)(g)} | 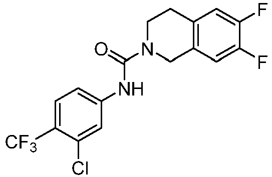 *N*-(3-Chloro-4-(trifluoromethyl)phenyl)-6,7-difluoro-3,4-dihydroisoquinoline-2(1H)-carboxamide | 391.3, 393.0 at 1.78 min | ¹H NMR (500 MHz, DMSO-d6) δ 9.17 (s, 1H), 7.91 (d, *J=* 2.1 Hz, 1H), 7.73 (d, *J =* 8.8 Hz, 1H), 7.64 (dd, *J* = 8.7, 2.1 Hz, 1H), 7.31 (dt, *J =* 11.4, 8.5 Hz, 2H), 4.62 (s, 2H), 3.71 (t, *J =* 5.9 Hz, 2H), 2.85 (t, *J =* 5.9 Hz, 2H). |
| 109^{(b)(g)} | 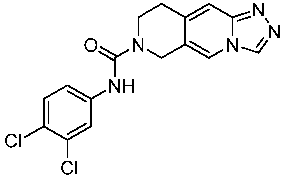 *N*-(3,4-Dichlorophenyl)-8,9-dihydro-[1,2,4]triazolo[4,3-b][2,7]naphthyridine-7(6H)-carboxamide | 362.5, 364.5 at 1.09 min | ¹H NMR (500 MHz, DMSO-d6) δ 9.19 (s, 1H), 8.81 (s, 1H), 8.53 (s, 1H), 7.87 (q, *J =* 1.8 Hz, 1H), 7.66 (s, 1H), 7.50 (d, *J =* 1.4 Hz, 2H), 4.65 (d, *J =* 1.3 Hz, 2H), 3.70 (t, *J =* 6.2 Hz, 2H), 3.05 (t, *J =* 6.2 Hz, 2H). |
| 113^{(b)(k)} | 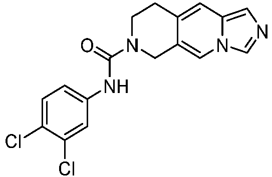 *N*-(3,4-Dichlorophenyl)-8,9-dihydroimidazo[1,5-b][2,7]naphthyridine-7(6H)-carboxamide | 361.3, 363.3 at 1.29 min | ¹H NMR (500 MHz, DMSO-d6) δ 8.76 (s, 1H), 8.32 - 8.27 (m, 2H), 7.87 (dd, *J =* 1.9, 0.9 Hz, 1H), 7.54 - 7.46 (m, 2H), 7.40 (s, 1H), 7.25 (d, *J =* 1.1 Hz, 1H), 4.56 (d, *J =* 1.3 Hz, 2H), 3.66 (t, *J =* 6.2 Hz, 2H), 2.92 (t, *J* = 6.2 Hz, 2H). |
| 114^{(c)(g)} | 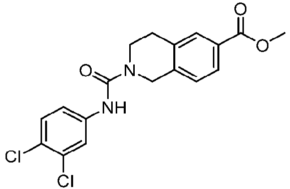 Methyl 2-((3,4-dichlorophenyl)carbamoyl)-1,2,3,4-tetrahydroisoquinoline-6-carboxylate | 379.1, 381.1 at 1.54 min | ¹H NMR (500 MHz, DMSO-d6) δ 8.91 (s, 1H), 7.86 (t, *J* = 1.4 Hz, 1H), 7.82 (d, *J =* 1.8 Hz, 1H), 7.79 (dd, *J =* 7.9, 1.8 Hz, 1H), 7.50 (d, *J* = 1.4 Hz, 2H), 7.34 (d, *J =* 8.0 Hz, 1H), 4.72 (s, 2H), 3.85 (s, 3H), 3.73 (t, *J* = 5.9 Hz, 2H), 2.94 (t, *J =* 5.9 Hz, 2H). |
| 116^{(b)(k)} | 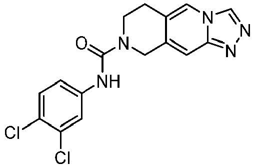 *N*-(3,4-Dichlorophenyl)-6,7-dihydro-[1,2,4]triazolo[4,3-b][2,6]naphthyridine-8(9H)-carboxamide | 362.2, 364.2 at 1.05 min | ¹H NMR (500 MHz, DMSO-d6) δ 9.20 (d, *J =* 0.8 Hz, 1H), 8.84 (s, 1H), 8.48 (d, *J =* 1.2 Hz, 1H), 7.87 (t, *J =* 1.4 Hz, 1H), 7.70 (s, 1H), 7.50 (d, *J =* 1.4 Hz, 2H), 4.73 (d, *J =* 1.4 Hz, 2H), 3.70 (t, *J =* 6.1 Hz, 2H), 2.96 (t, *J =* 6.1 Hz, 2H). |
| 117^{(b)(k)} | 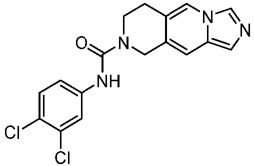 *N*-(3,4-Dichlorophenyl)-6,7-dihydroimidazo[1,5-b][2,6]naphthyridine-8(9H)-carboxamide | 361.3, 363.3 at 1.28 min | ¹H NMR (500 MHz, DMSO-*d*6) δ 8.77 (s, 1H), 8.30 (s, 1H), 8.25 (s, 1H), 7.89 - 7.85 (m, 1H), 7.50 (t, *J =* 1.5 Hz, 1H), 7.43 (s, 1H), 7.28 (s, 1H), 4.59 (s, 2H), 3.66 (t, *J =* 6.2 Hz, 2H), 2.87 (t, *J =* 6.0 Hz, 2H). Urea NH not observed |
| 122^{(b)(k)} | 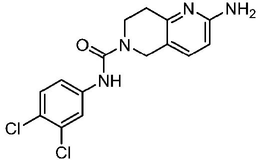 2-Amino-*N*-(3,4-dichlorophenyl)-7,8-dihydro-1,6-naphthyridine-6(5H)-carboxamide | 337.3, 339.3 at 1.19 min | ¹H NMR (500 MHz, DMSO-d6) δ 8.86 (s, 1H), 7.85 (d, *J =* 1.7 Hz, 1H), 7.48 (d, *J =* 1.3 Hz, 2H), 7.16 (d, *J =* 8.4 Hz, 1H), 6.31 (d, *J =* 8.3 Hz, 1H), 5.77 (s, 2H), 4.44 (s, 2H), 3.71 (t, *J =* 5.9 Hz, 2H), 2.67 (t, *J* = 5.9 Hz, 2H). |
| 124^{(b)(g)} | 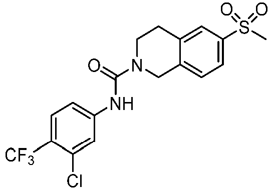 *N*-(3-Chloro-4-(trifluoromethyl)phenyl)-6-(methylsulfonyl)-3,4-dihydroisoquinoline-2(1H)-carboxamide | 433.1, 435.1 at 1.45 min | ¹H NMR (500 MHz, DMSO--d6) δ 8.92 (s, 1H), 7.86 (t, *J =* 1.4 Hz, 1H), 7.79 - 7.71 (m, 1H), 7.51 - 7.44 (m, 3H), 4.74 (s, 2H), 3.74 (t, *J* = 5.9 Hz, 2H), 3.19 (s, 3H), 2.97 (t, *J =* 5.9 Hz, 2H). (Exchangable -NH proton not visible). |
| 125^{(b)(g)} | 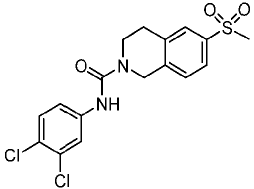 *N*-(3,4-dichlorophenyl)-6-(methylsulfonyl)-3,4-dihydroisoquinoline-2(1H)-carboxamide | 397.2, 399.1 at 1.39 min^{(l)} | ¹H NMR (500 MHz, DMSO-d6) δ 9.19 (s, 1H), 7.92 (d, *J=* 2.1 Hz, 1H), 7.78 (d, *J =* 1.8 Hz, 1H), 7.74 (dd, *J =* 10.4, 8.4 Hz, 2H), 7.64 (d, *J =* 9.5 Hz, 1H), 7.48 (d, *J =* 8.1 Hz, 1H), 4.76 (s, 2H), 3.76 (t, *J* = 5.9 Hz, 2H), 3.19 (s, 3H), 2.99 (t, *J =* 5.9 Hz, 2H) |
| 129^{(c)} | 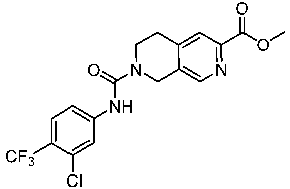 Methyl 7-((3-chloro-4-(trifluoromethyl)phenyl)carbamoyl)-5,6,7,8-tetrahydro-2,7-naphthyridine-3-carboxylate | 414.4, 416.4 at 1.38 min | ¹H NMR (500 MHz, DMSO-d6) δ 9.24 (s, 1H), 8.55 (s, 1H), 7.93 (s, 1H), 7.91 (d, *J* = 2.1 Hz, 1H), 7.73 (d, *J* = 8.8 Hz, 1H), 7.64 (dd, *J =* 8.7, 2.0 Hz, 1H), 4.78 (s, 2H), 3.87 (s, 3H), 3.76 (t, *J* = 5.9 Hz, 2H), 2.97 (t, *J =* 5.9 Hz, 2H). |
| 131^{(g)} | 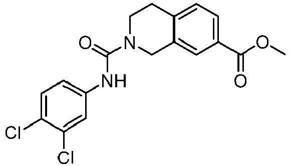 Methyl 2-((3,4-dichlorophenyl)carbamoyl)-1,2,3,4-tetrahydroisoquinoline-7-carboxylate | 379.1, 381.1 at 1.54 min | ¹H NMR (500 MHz, DMSO-d6) δ 8.88 (s, 1H), 7.86 (t, *J* = 1.4 Hz, 1H), 7.78 (d, *J =* 7.7 Hz, 2H), 7.49 (d, *J =* 1.4 Hz, 2H), 7.35 (d, *J =* 7.9 Hz, 1H), 4.71 (s, 2H), 3.85 (s, 3H), 3.72 (t, *J* = 5.9 Hz, 2H), 2.93 (t, *J* = 5.9 Hz, 2H). |
| 132 ^{(d)} | 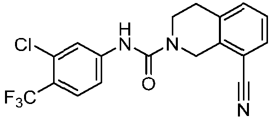 *N*-(3-Chloro-4-(trifluoromethyl)phenyl)-8-cyano-3,4-dihydroisoquinoline-2(1H)-carboxamide | 380.0, 382.2 at 1.59 min | ¹H NMR (500 MHz, DMSO-d6) δ 9.33 (s, 1H), 7.92 (d, *J =* 2.1 Hz, 1H), 7.74 (d, *J =* 8.6 Hz, 2H), 7.64 (dd, J = 8.7, 2.1 Hz, 1H), 7.57 (d, J = 7.7 Hz, 1H), 7.42 (t, J = 7.7 Hz, 1H), 4.82 (s, 2H), 3.78 (t, J = 5.9 Hz, 2H), 2.94 (t, J = 5.9 Hz, 2H). |
| 133^{(d)} | 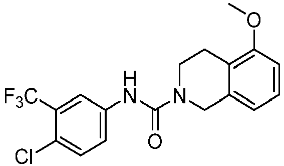 *N*-(4-Chloro-3-(trifluoromethyl)phenyl)-5-methoxy-3,4-dihydroisoquinoline-2(1H)-carboxamide | 365.2, 387.1 at 1.66 min | ¹H NMR (500 MHz, DMSO-d6) δ 9.05 (s, 1H), 8.08 (d, *J* = 2.6 Hz, 1H), 7.84 (dd, *J* = 8.8, 2.6 Hz, 1H), 7.58 (d, *J =* 8.8 Hz, 1H), 7.19 (t, *J =* 7.9 Hz, 1H), 6.84 (d, *J =* 8.2 Hz, 1H), 6.78 (d, *J =* 7.7 Hz, 1H), 4.64 (s, 2H), 3.79 (s, 3H), 3.71 (t, *J* = 6.0 Hz, 2H), 2.71 (t, *J* = 6.0 Hz, 2H). |
| 134^{(d)} | 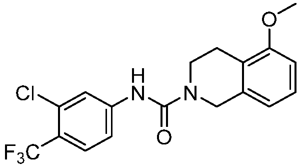 *N*-(3-Chloro-4-(trifluoromethyl)phenyl)-5-methoxy-3,4-dihydroisoquinoline-2(1H)-carboxamide | 365.1, 387.1 at 1.68 min | ¹H NMR (500 MHz, DMSO-d6) δ 9.16 (s, 1H), 7.92 (d, *J=* 2.0 Hz, 1H), 7.72 (d, *J* = 8.8 Hz, 1H), 7.67 - 7.62 (m, 1H), 7.19 (t, *J* = 7.9 Hz, 1H), 6.84 (d, *J* = 8.2 Hz, 1H), 6.78 (d, *J* = 7.7 Hz, 1H), 4.64 (s, 2H), 3.79 (s, 3H), 3.72 (t, *J* = 6.0 Hz, 2H), 2.71 (t, *J* = 6.0 Hz, 2H). |
| 135^{(d)} | 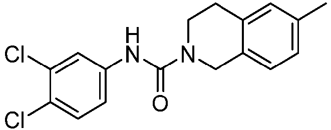 *N*-(3,4-Dichlorophenyl)-6-methyl-3,4-dihydroisoquinoline-2(1H)-carboxamide | 335.1, 337.1 at 1.67 min | ¹H NMR (500 MHz, DMSO-d6) δ 8.84 (s, 1H), 7.87 (d, *J =* 1.9 Hz, 1H), 7.53 - 7.46 (m, 2H), 7.06 (d, *J =* 7.8 Hz, 1H), 7.01 (d, *J* = 7.8 Hz, 2H), 4.59 (s, 2H), 3.68 (t, *J* = 5.9 Hz, 2H), 2.81 (t, *J =* 5.9 Hz, 2H), 2.27 (s, 3H). |
| 136^{(d)} | 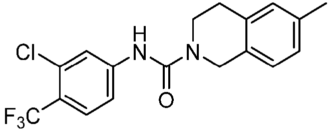 *N*-(3-Chloro-4-(trifluoromethyl)phenyl)-6-methyl-3,4-dihydroisoquinoline-2(1H)-carboxamide | 369.1, 371.1 at 1.74 min | ¹H NMR (500 MHz, DMSO-d6) δ 9.11 (s, 1H), 7.92 (d, *J =* 2.0 Hz, 1H), 7.71 (d, *J =* 8.8 Hz, 1H), 7.65 (dd, *J =* 8.6, 2.0 Hz, 1H), 7.07 (d, *J* = 7.9 Hz, 1H), 7.01 (d, *J =* 7.4 Hz, 2H), 4.61 (s, 2H), 3.69 (t, *J* = 5.9 Hz, 2H), 2.82 (t, *J* = 5.9 Hz, 2H), 2.26 (s, 3H). |
| 137^{(d)} | 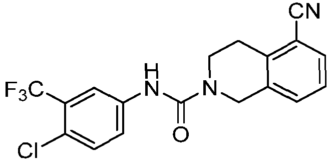 *N*-(4-Chloro-3-(trifluoromethyl)phenyl)-5-cyano-3,4-dihydroisoquinoline-2(1H)-carboxamide | 380.1, 382.1 at 1.56 min | ¹H NMR (500 MHz, DMSO-d6) δ 9.11 (s, 1H), 8.07 (d, *J* = 2.6 Hz, 1H), 7.83 (dd, *J* = 8.8, 2.6 Hz, 1H), 7.73 (d, *J =* 7.6 Hz, 1H), 7.60 (d, *J =* 8.8 Hz, 1H), 7.56 (d, *J* = 7.8 Hz, 1H), 7.42 (t, *J =* 7.7 Hz, 1H), 4.72 (s, 2H), 3.81 (t, *J* = 5.9 Hz, 2H), 3.02 (t, *J* = 5.9 Hz, 2H). |
| 138^{(d)} | 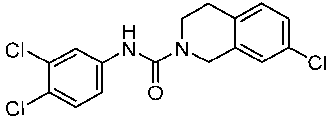 7-Chloro-*N*-(3,4-dichlorophenyl)-3,4-dihydroisoquinoline-2(1H)-carboxamide | 354.9, 356.9, 359.0 at 1.69 min | ¹H NMR (500 MHz, DMSO-d6) δ 8.89 (s, 1H), 7.88 - 7.83 (m, 1H), 7.49 (d, *J =* 1.6 Hz, 2H), 7.29 (s, 1H), 7.27 - 7.20 (m, 2H), 4.63 (s, 2H), 3.70 (t, *J* = 5.9 Hz, 2H), 2.84 (t, *J* = 5.9 Hz, 2H). |
| 139^{(d)} | 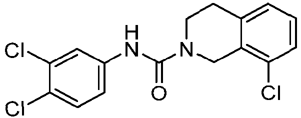 8-Chloro-*N*-(3,4-dichlorophenyl)-3,4-dihydroisoquinoline-2(1H)-carboxamide | 355.0, 357.0, 359.1 at 1.70 min | ¹H NMR (500 MHz, DMSO-d6) δ 9.01 (s, 1H), 7.86 (dd, *J =* 1.9, 0.9 Hz, 1H), 7.49 (d, *J* = 1.7 Hz, 2H), 7.34 (d, *J* = 7.7 Hz, 1H), 7.24 (t, *J =* 7.7 Hz, 1H), 7.20 (d, *J =* 7.6 Hz, 1H), 4.63 (s, 2H), 3.72 (t, *J* = 5.8 Hz, 2H), 2.89 (t, *J* = 5.8 Hz, 2H). |
| 140 ^{(b,e)} | 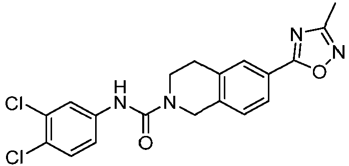 *N*-(3,4-Dichlorophenyl)-6-(3-methyl-1,2,4-oxadiazol-5-yl)-3,4-dihydroisoquinoline-2(1H)-carboxamide | 403.4, 405.5 at 1.59 min | ¹H NMR (400 MHz, DMSO-d6) δ 8.92 (s, 1H), 7.95 (d, *J =* 1.8 Hz, 1H), 7.91 (dd, *J* = 8.0, 1.9 Hz, 1H), 7.86 (t, *J =* 1.4 Hz, 1H), 7.49 (d, *J =* 1.4 Hz, 2H), 7.44 (d, *J =* 8.1 Hz, 1H), 4.74 (s, 2H), 3.74 (t, *J* = 5.9 Hz, 2H), 2.98 (t, *J* = 5.8 Hz, 2H), 2.41 (s, 3H). |

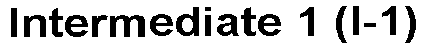

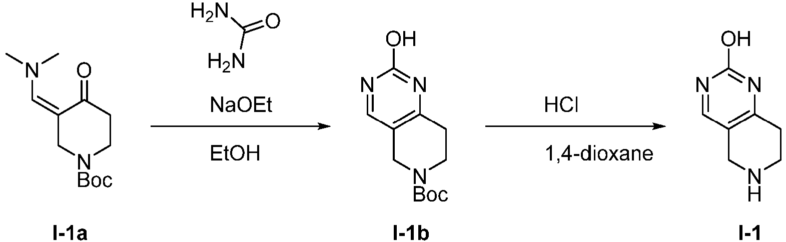

**Step 1:** To a solution of urea (1.03 g, 17.2 mmol) in EtOH (10 ml) was added a solution of sodium ethoxide (21% w/w in EtOH) (5.58 g, 6.4 mL, 17.2 mmol). The mixture was stirred for 5 min before a solution of *tert-*butyl (*Z*)-3-((dimethylamino)methylene)-4-oxopiperidine-1-carboxylate (**I-1a**) (3.65 g, 14.4 mmol) in EtOH (50 ml) was added. The resultant mixture was heated under reflux for 16 h. The reaction was cooled to RT. Saturated ammonium chloride solution (20 ml) was added and the volatiles removed *in vacuo.* The aqueous was extracted with EtOAc (3 x 150 ml). The combined organics were dried over magnesium sulfate and concentrated *in vacuo.* The product was purified by chromatography on silica gel (0-10% (0.7 M NH₃/MeOH)/DCM) to afford *tert-*butyl 2-hydroxy-7,8-dihydropyrido[4,3-d]pyrimidine-6(5H)-carboxylate (**I-1b**) as a light yellow solid. ¹H NMR (500 MHz, DMSO-d6) δ 11.70 (s, 1H), 8.16 (s, 1H), 4.28 (s, 2H), 3.55 (t, J = 6.0 Hz, 2H), 2.62 (t, J = 6.0 Hz, 2H), 1.42 (s, 9H).

**Step 2:** To a solution of *tert-*butyl 2-hydroxy-7,8-dihydropyrido[4,3-d]pyrimidine-6(5H)-carboxylate (**I-1b)** (0.246 g, 979 µmol) in DCM (10 ml) was added a solution of HCl (4M in 1,4-dioxane) (2.45 ml, 9.79 mmol). The resultant mixture was stirred at RT for 16 h. The reaction mixture was filtered and the precipitate was dried *in vacuo.* The solid was suspended in MeOH (50 ml) and SCX (4.9 g, 3.77 mmol) added and the mixture was stirred for 3 h. The SCX was washed with MeOH and the product was eluted with 0.7 M NH₃/MeOH solution and concentrated *in vacuo* to give 5,6,7,8-tetrahydropyrido[4,3-d]pyrimidin-2-ol (**I-1**) as a yellow solid. ¹H NMR (500 MHz, DMSO-d6) δ 8.01 (s, 1H), 5.40 (s, 1H) 3.57 (s, 2H), 2.89 (t, J = 5.9 Hz, 2H), 2.48 (t, J = 5.9 Hz, 2H). (1 exchangable H not visible).

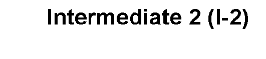

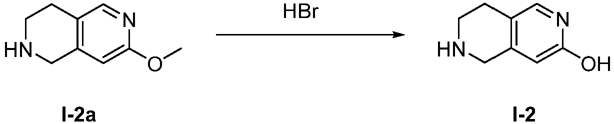

A solution of 7-methoxy-1,2,3,4-tetrahydro-2,6-naphthyridine (**I-2a**) (200 mg, 1.22 mmol) in HBr (2.07 ml, 48% w/w, 18.3 mmol) was heated to 100 °C for 10 h. The reaction mixture was cooled to RT and the reaction mixture concentrated *in vacuo.* The residue was dissolved in MeOH (20 ml ) and loaded onto a SCX cartridge (40g, 30 mmol). The SCX was washed with MeOH and the product was eluted with 0.7 M NH₃ in MeOH and concentrated *in vacuo* to give 5,6,7,8-tetrahydro-2,6-naphthyridin-3-ol (**I-2**) as light brown solid. ¹H NMR (500 MHz, DMSO-*d*₆) δ 11.28 (s, 1H), 7.13 (d, *J =* 3.4 Hz, 1H), 6.02 (d, *J =* 3.5 Hz, 1H), 3.75 - 3.69 (m, 2H), 2.92 - 2.86 (m, 2H), 2.47 (t, *J* = 5.9 Hz, 2H). (1 exchangable H not seen).

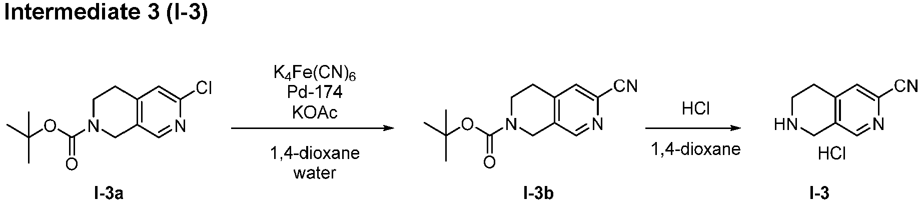

**Step 1:** A solution of *tert-*butyl 6-chloro-3,4-dihydro-2,7-naphthyridine-2(1H)-carboxylate (**I-3a**) (150 mg, 558 µmol), K₄Fe(CN)₆ (212 mg, 502 µmol) and potassium acetate (46.6 mg, 474 µmol) in a mixture of 1,4-dioxane (4 ml) and water (0.8 ml) was degassed for 5 min and Pd-174 (20.1 mg, 27.9 µmol) was added. The mixture was degassed for a further 5 min before being heated to 90 °C for 16 h. The reaction cooled to RT, filtered through a hydrophobic frit and the filtrate was concentrated *in vacuo.* The product was purified by chromatography on silica (0-30% EtOAc/isohexane) to give *tert-*butyl 6-cyano-3,4-dihydro-2,7-naphthyridine-2(1H)-carboxylate (**I-3b**) as a thick colourless oil. LCMS (method 3) m/z 260.1 (M+H)⁺ (ES⁺) at 1.25 min.

**Step 2:** To a solution of *tert-*butyl 6-cyano-3,4-dihydro-2,7-naphthyridine-2(1H)-carboxylate (**I-3b**) (15 mg, 58 µmol) in 1,4-dioxane (2 ml) was added 4 M HCl in dioxane (0.29 ml, 1.2 mmol) at 0 °C. The reaction mixture was stirred at RT for 72 h. The reaction mixture was concentrated *in vacuo* to give 5,6,7,8-tetrahydro-2,7-naphthyridine-3-carbonitrile hydrochloride (**I-3**) as a brown solid. LCMS (method 3) m/z 160.0 (M+H)⁺ (ES⁺) at 0.54 min. ¹H NMR (500 MHz, DMSO-d6) δ 9.25 (s, 2H), 8.64 (s, 1H), 8.00 (s, 1H), 4.42 (s, 2H), 3.44 - 3.40 (m, 2H), 3.07 (t, *J* = 6.3 Hz, 2H).

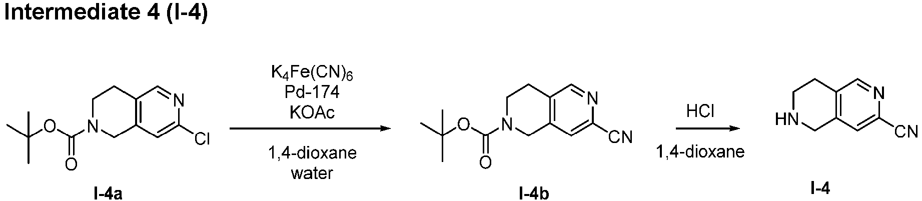

**Step 1:** *tert-*Butyl 7-cyano-3,4-dihydro-2,6-naphthyridine-2(1H)-carboxylate (**I-4b**) was synthesised from *tert-*butyl 7-chloro-3,4-dihydro-2,6-naphthyridine-2(1H)-carboxylate (**I-4a**) using essentially the same procedure as **I-3b.** LCMS (method 2) m/z 260.0 (M+H)⁺ (ES⁺) at 1.93 min.

**Step 2:** 5,6,7,8-Tetrahydro-2,6-naphthyridine-3-carbonitrile hydrochloride (**I-4**) was synthesised from *tert-*butyl 7-cyano-3,4-dihydro-2,6-naphthyridine-2(1H)-carboxylate (**I-4b**) using essentially the same procedure as **I-3**. 1H NMR (500 MHz, DMSO-d6) δ 9.25 (s, 2H), 8.64 (s, 1H), 8.00 (s, 1H), 4.42 (s, 2H), 3.40 (s, 2H), 3.07 (t, *J* = 6.3 Hz, 2H).

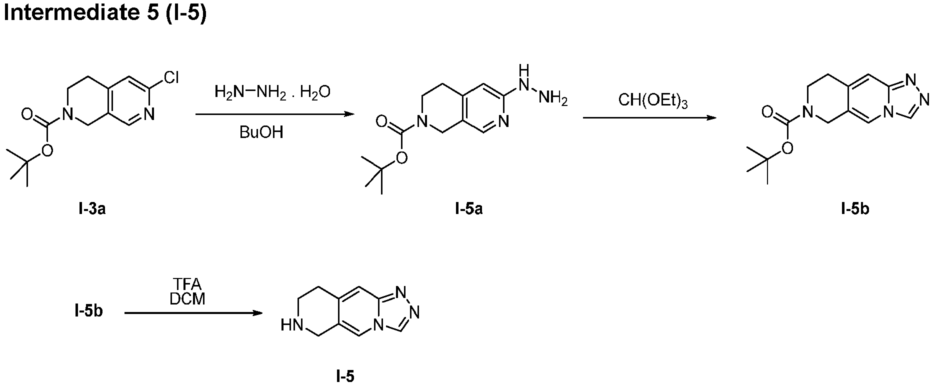

**Step 1:** To a solution of *tert-*butyl 6-chloro-3,4-dihydro-2,7-naphthyridine-2(1H)-carboxylate (**I-3a**) (101 mg, 376 µmol) in ⁿBuOH (2 ml) hydrazine hydrate (35% w/w in water) (266 µl, 1.88 mmol) and the reaction heated to 130 °C for 60 h. The reaction was cooled to room temperature and diluted with water (5 ml) and DCM (10 ml). The layers were separated and the aqueous layer was further extracted with DCM (3 x 10 ml). The combined organic layers were passed through a hydrophobic frit and the filtrate was concentrated *in vacuo* to give *tert-*butyl 6-hydrazineyl-3,4-dihydro-2,7-naphthyridine-2(1H)-carboxylate (**I-5a**) as a colourless oil. LCMS (method 3) m/z 265.1 (M+H)⁺ (ES⁺) at 1.58 min.

**Step 2:** A solution of *tert-*butyl 6-hydrazineyl-3,4-dihydro-2,7-naphthyridine-2(1H)-carboxylate (**I-5a**) in triethyl orthoformate (2 mL, 0.01 mol) was heated to 130 °C for 22 h. The reaction mixture was concentrated *in vacuo* and the product was purified by chromatography on silica gel (0-5% (0.7 M NH₃/MeOH)/DCM) to give *tert-*butyl 8,9-dihydro-[1,2,4]triazolo[4,3-b][2,7]naphthyridine-7(6H)-carboxylate (**I-5b**) as a pale yellow solid. LCMS (method 2) m/z 275.1 (M+H)⁺ (ES⁺) at 1.50 min. ¹H NMR (500 MHz, CDCl₃) δ 8.79 (s, 1H), 8.03 (s, 1H), 7.62 (s, 1H), 4.60 (br s, 2H), 3.67 (t, *J* = 6.5 Hz, 2H), 3.01 (t, *J =* 6.3 Hz, 2H), 1.51 (s, 9H).

**Step 3:** To a solution of *tert*-butyl 8,9-dihydro-[1,2,4]triazolo[4,3-b][2,7]naphthyridine-7(6H)-carboxylate (**I-5b**) (37 mg, 0.13 mmol) in DCM (2 ml) was added TFA (0.1 ml, 1.3 mmol) was added slowly. The reaction mixture was stirred at RT for 3 hours. The reaction mixture was concentrated *in vacuo.* The product was purified ion exchange on SCX (0.6g, 0.42 mmol), eluting with 0.7 M NH₃ in MeOH to give 6,7,8,9-tetrahydro-[1,2,4]triazolo[4,3-b][2,7]naphthyridine (**I-5**) as a colourless solid. LCMS (method 3) m/z 175.1 (M+H)⁺ (ES⁺) at 0.53 min.

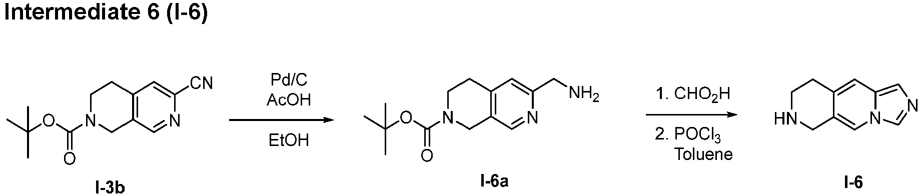

**Step 1:** To a solution of *tert-*butyl 6-cyano-3,4-dihydro-2,7-naphthyridine-2(1H)-carboxylate (**I-3b**) (100 mg, 386 µmol) in EtOH (5 ml) and acetic acid (662 µl, 11.6 mmol) was added Pd/C 10% (50% paste) (50 mg, 23 µmol). The reaction mixture was stirred under a hydrogen atmosphere (5 bar) at RT for 16 h. The catalyst was filtered off and the filtrate was concentrated *in vacuo.* The product was purified on silica gel (0-10% (0.7 M NH₃/MeOH)/DCM) to give *tert-*butyl 6-(aminomethyl)-3,4-dihydro-2,7-naphthyridine-2(1H)-carboxylate (**I-6a**) as a clear colourless tar. ¹H NMR (500 MHz, DMSO-*d*₆) δ 8.29 (s, 1H), 7.22 (s, 1H), 4.51 (s, 2H), 3.73 (s, 2H), 3.56 (t, *J* = 5.9 Hz, 2H), 2.77 (t, *J* = 6.0 Hz, 2H), 2.03 (br s, 2H), 1.43 (s, 9H).

**Step 2:** A solution of *tert*-butyl 6-(aminomethyl)-3,4-dihydro-2,7-naphthyridine-2(1H)-carboxylate (**I-6a**) (25 mg, 95 µmol) in formic acid (1 ml) was heated at 80 °C for 2 h. The reaction mixture was cooled and concentrated *in vacuo.* The residue was combined with toluene (3 ml) and POCl₃ (1 ml, 0.01 mol) and heated to 100 °C for 2 h. The reaction mixture was cooled and concentrated *in vacuo,* the solid residue was combined with saturated NaHCO₃ solution (5 ml) and the product extracted with 20% MeOH in DCM solution (3 x 5 ml). The combined organic layers were concentrated *in vacuo.* The residue was taken up in 1 M NaOH (1 ml) and EtOH (2 ml) and heated to 100 °C for 16 h. The reaction mixture was cooled and concentrated *in vacuo,* the residue was acidified with AcOH and loaded on SCX (20 g, 14 mmol)) and eluted with 0.7 M NH₃ in MeOH to give 6,7,8,9-tetrahydroimidazo[1,5-b][2,7]naphthyridine (**I-6**) as an orange solid. LCMS (method 3) m/z 173.8 (M+H)⁺ (ES⁺) at 0.61 min. ¹H NMR (500 MHz, DMSO-*d*₆) δ 8.19 (s, 1H), 8.12 (s, 1H), 8.06 (s, 1H), 7.24 (d, *J =* 5.5 Hz, 1H), 7.16 (d, *J =* 3.0 Hz, 1H), 3.46 (t, *J =* 6.0 Hz, 2H), 2.91 (t, *J =* 6.2 Hz, 2H), 2.72 - 2.66 (m, 2H).

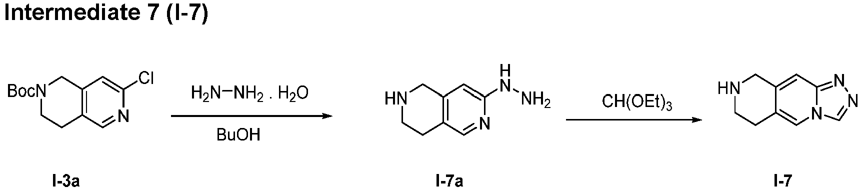

**Step 1:** To a solution of *tert-*butyl 7-chloro-3,4-dihydro-2,6-naphthyridine-2(1H)-carboxylate (**I-3a**) (200 mg, 744 µmol) in EtOH (1 ml), hydrazine monohydrate (1.12 ml , 14.9 mmol) was added and the reaction was heated to 150 °C for 94 h. The reaction mixture was cooled, diluted with DCM (5 ml) and concentrated *in vacuo.* The material was triturated with DCM to give 7-hydrazineyl-1,2,3,4-tetrahydro-2,6-naphthyridine (**I-7a**) as a brown solid. LCMS (method 3) m/z 165.5 (M+H)⁺ (ES⁺) at 0.26 min.

**Step 2:** A solution of 7-hydrazineyl-1,2,3,4-tetrahydro-2,6-naphthyridine (**I-7a**) in triethyl orthoformate (2 ml, 0.01 mol) was heated to 130 °C for 2 h. The reaction mixture was cooled and concentrated *in vacuo* to give 6,7,8,9-tetrahydro-[1,2,4]triazolo[4,3-b][2,6]naphthyridine (**I-7**) as a red solid. LCMS (method 3) m/z 175.3 (M+H)⁺ (ES⁺) at 0.22 min.

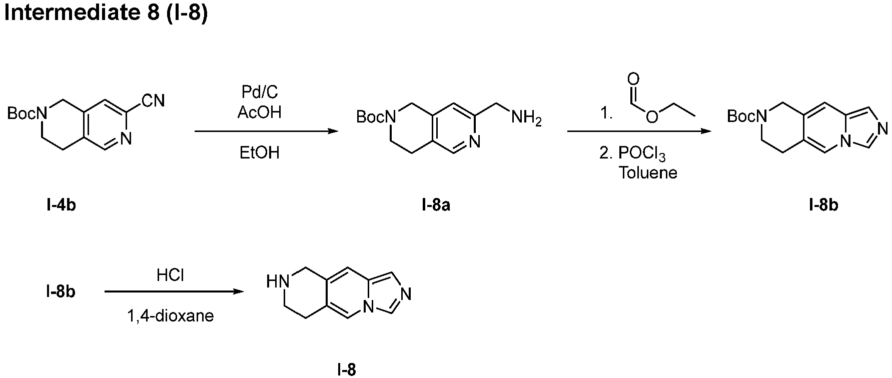

**Step 1:** *tert-*Butyl 7-(aminomethyl)-3,4-dihydro-2,6-naphthyridine-2(1H)-carboxylate (**I-8a**) was synthesised from *tert-*butyl 7-cyano-3,4-dihydro-2,6-naphthyridine-2(1H)-carboxylate (**I-4b**) using essentially the same procedure as (**I-6a**). LCMS (method 3) m/z 264.3 (M+H)⁺ (ES⁺) at 0.99 min. ¹H NMR (500 MHz, DMSO-*d*₆) δ 8.28 (s, 1H), 7.23 (s, 1H), 4.51 (s, 2H), 3.74 (s, 2H), 3.58 (t, *J* = 5.8 Hz, 2H), 2.75 (t, *J =* 5.9 Hz, 2H), 2.16 (s, 2H), 1.43 (s, 9H).

**Step 2:** To a solution of *tert-*butyl 7-(aminomethyl)-3,4-dihydro-2,6-naphthyridine-2(1H)-carboxylate (**I-8a**) (20 mg, 76 µmol) in ethyl formate (2 ml, 0.02 mol) and heated to 65 °C for 3 h. The reaction mixture was cooled and concentrated *in vacuo.* The residue was dissolved in DCM (2 ml) and POCl₃ (11 µl, 0.11 mmol) was added followed by Et₃N (64 µl, 0.46 mmol) and was stirred at RT for 2 h. The material was poured into ice-water (20 ml) and saturated sodium bicarbonate solution (2 ml) was added. The product was extracted with 10% MeOH in DCM solution (2 x 20 ml) and the combined organic layers were concentrated *in vacuo.* The product was purified on silica gel (0-5% (0.7 M NH₃/MeOH)/DCM) to give *tert-*butyl 6,7-dihydroimidazo[1,5-b][2,6]naphthyridine-8(9H)-carboxylate (**I-8b**) as a clear tan oil. LCMS (method 3) m/z 274.3 (M+H)⁺ (ES⁺) at 1.18 min. ¹H NMR (500 MHz, DMSO-*d*₆) δ 8.28 (d, *J* = 0.8 Hz, 1H), 8.22 (s, 1H), 7.42 (s, 1H), 7.26 (d, *J =* 1.0 Hz, 1H), 4.45 (s, 2H), 3.50 (t, *J* = 6.2 Hz, 2H), 2.81 - 2.75 (m, 2H), 1.43 (s, 9H).

**Step 3:** *tert-*Butyl 6,7-dihydroimidazo[1,5-b][2,6]naphthyridine-8(9H)-carboxylate (10 mg, 37 µmol) was dissolved in 4 M HCl in 1,4-dioxane (0.46 mL, 1.8 mmol) and stirred at RT for 1 h. The reaction mixture was concentrated *in vacuo.* The product was purified by ion exchange using SCX washing with MeOH (10 ml) and the product was eluted with 0.7 M NH₃ in MeOH (20 ml) solution and concentrated in vacuo to give 6,7,8,9-tetrahydroimidazo[1,5-b][2,6]naphthyridine (**I-8**) as a brown oil. LCMS (method 3) m/z 173.6 (M-H)⁻ (ES⁻) at 0.59 min.

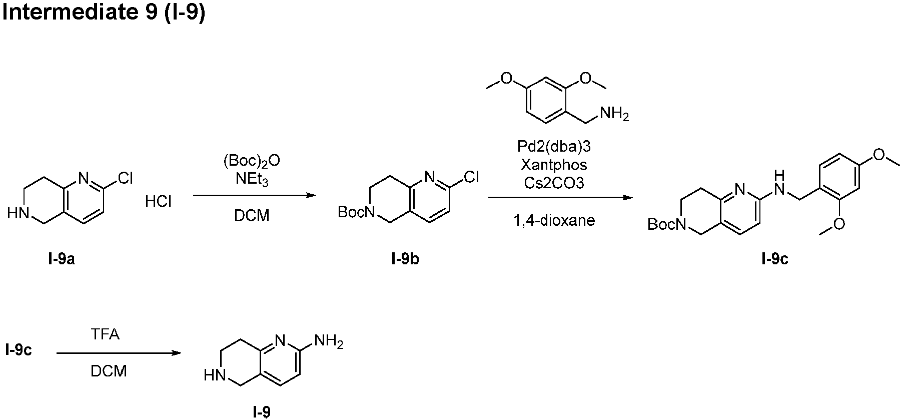

**Step 1:** To a solution of 2-chloro-5,6,7,8-tetrahydro-1,6-naphthyridine HCl (I-9a) (2.00 g, 9.75 mmol) in DCM (10 ml) was added (Boc)₂O (2.46 ml, 10.7 mmol) and Et₃N (1.63 ml, 11.7 mmol). The resultant mixture was stirred at RT for 16 h. Water (20 ml) was added and the product was extracted with EtOAc (3 x 50 ml). The combined organics were dried with magnesium sulfate and concentrated *in vacuo.* The product was purified on silica gel (0-50% EtOAc/isohexane) to afford *tert-*butyl 2-chloro-7,8-dihydro-1,6-naphthyridine-6(5H)-carboxylate (**I-9b**) as a thick colourless oil that solidified upon standing. ¹H NMR (500 MHz, Chloroform-d) δ 7.40 (d, J = 8.1 Hz, 1H), 7.19 (d, J = 8.1 Hz, 1H), 4.58 (s, 2H), 3.75 (t, J = 5.9 Hz, 2H), 2.99 (t, J = 6.0 Hz, 2H), 1.52 (s, 9H).

**Step 2:** To a solution of *tert-*butyl 2-chloro-7,8-dihydro-1,6-naphthyridine-6(5H)-carboxylate (**I-9b**) (1.00 g, 3.72 mmol) in 1,4-dioxane (10 ml) was added (2,4-dimethoxyphenyl)methanamine (727 µl, 4.84 mmol) followed by Cs₂CO₃ (2.44 g, 7.44 mmol). The reaction mixture was purged with nitrogen for 5 min. Xantphos (215 mg, 372 µmol) and Pd₂(dba)₃ (170 mg, 186 µmol) were added and the reaction mixture was purged with nitrogen for a further 5 min. The reaction mixture was heated to 95 °C for 20 h. The reaction mixture was filtered through a plug of celite and the filtrate was concentrated *in vacuo.* The product was purified on silica gel (0-20% EtOAc/isohexane then 0-5% 0.7 M NH₃/MeOH in DCM) to give *tert-*butyl 2-((2,4-dimethoxybenzyl)amino)-7,8-dihydro-1,6-naphthyridine-6(5H)-carboxylate (**I-9c**) as an orange oil. LCMS (method 3) m/z 400.3 (M+H)⁺ (ES⁺) at 1.06 min.

**Step 3:** To a solution of *tert-*butyl 2-((2,4-dimethoxybenzyl)amino)-7,8-dihydro-1,6-naphthyridine-6(5H)-carboxylate (**I-9c**) (270 mg, 676 µmol) in DCM (8 ml) was added TFA (1.04 mL, 13.5 mmol) and the reaction mixture was stirred at RT for 72 h. The reaction mixture was concentrated *in vacuo* and the product was purified by ion exchange using SCX (20 g, 14 mmol) washing with MeOH (5 ml) and the product was eluted with 0.7M NH3 in MeOH to give 5,6,7,8-tetrahydro-1,6-naphthyridin-2-amine (**I-9**) as a brown oil. LCMS (method 3) m/z 150.1 (M+H)⁺ (ES⁺) at 0.23 min. ¹H NMR (500 MHz, DMSO-*d*₆) δ 7.01 (d, *J* = 8.2 Hz, 1H), 6.22 (d, *J* = 8.2 Hz, 1H), 5.59 (s, 2H), 3.65 (s, 2H), 2.94 (dd, *J* = 6.7, 5.4 Hz, 2H), 2.52 (d, *J* = 5.3 Hz, 2H). (NH proton not observed)

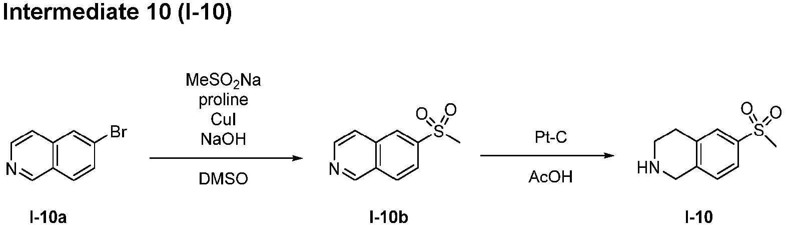

**Step** 1: 6-Bromoisoquinoline (**I-10a**) (2.5 g, 12 mmol), sodium methanesulfinate (1.8 g, 18 mmol), NaOH (96 mg, 2.4 mmol), copper(I) iodide (0.23 g, 1.2 mmol) and proline (0.28 g, 2.4 mmol) were added to a 3-neck round bottom flask and placed under a steady flow of nitrogen. DMSO (25 ml) was added, and the reaction was sparged with nitrogen for 5 min. The reaction mixture was heated to 95 °C for 20 h. The reaction was cooled to RT and diluted with water (100 ml) and EtOAc (200 ml). The layers were separated and the aqueous layer further extracted with EtOAc (2 x 200 ml). The combined organic layers were dried over magnesium sulfate and concentrated *in vacuo.* The product was purified on silica gel (0-10% (0.7 M NH₃/MeOH)/DCM) to give 6-(methylsulfonyl)isoquinoline (**I-10b**) as a brown powder. LCMS (method 2) m/z 208.0 (M+H)⁺ (ES⁺) at 1.16 min. ¹H NMR (500 MHz, DMSO-*d*₆) δ 9.56 (s, 1H), 8.73 (s, 1H), 8.66 (d, *J =* 1.8 Hz, 1H), 8.42 (d, *J =* 8.6 Hz, 1H), 8.17 - 8.11 (m, 2H), 3.35 (s, 3H).

**Step 2:** To a solution of 6-(methylsulfonyl)isoquinoline (**I-10b**) (0.50 g, 2.42 mmol) in AcOH (8 ml) was added 5% Pt-C type 128 (0.5 g, 0.1 mmol).The resultant mixture was stirred under a hydrogen atmosphere (5 Bar) at 25 °C for 72 h. The reaction mixture was cooled to RT and filtered through a pad of celite, washing with EtOAc. The filtrate was concentrated *in vacuo* to give 6-(methylsulfonyl)-1,2,3,4-tetrahydroisoquinoline, AcOH (**I-10**) as a sticky brown oil. LCMS (method 2) m/z 212.0 (M+H)⁺ (ES⁺) at 1.00 min. ¹H NMR (500 MHz, DMSO-*d*₆) δ 7.94 - 7.51 (m, 2H), 7.28 (d, *J* = 8.7 Hz, 1H), 3.92 (s, 2H), 3.16 (s, 3H), 2.96 (t, *J* = 5.9 Hz, 2H), 2.78 (t, *J* = 6.0 Hz, 2H). (Exchangable -NH proton not visible)

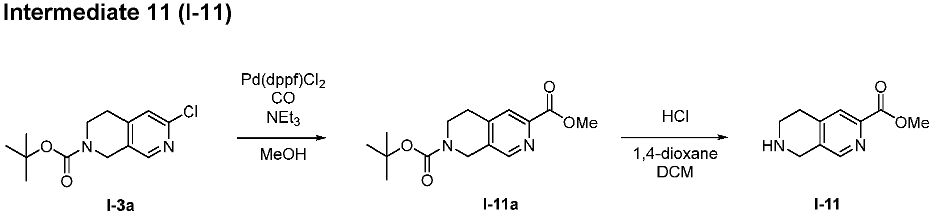

**Step 1:** To a solution of *tert*-butyl 6-chloro-3,4-dihydro-2,7-naphthyridine-2(1H)-carboxylate (**I-3a**) (200 mg, 744 µmol) in MeOH (10 ml) and NEt₃ (1.56 ml, 11.2 mmol) was added Pd-dppf (54.5 mg, 74.4 µmol). The reaction mixture was charged with CO (4.5 bar) and heated to 110 °C for 5 h. The reaction was cooled, filtered through celite and concentrated *in vacuo.* The product was purified on silica gel (0-5% (0.7 M NH₃/MeOH)/DCM) to afford 2-(*tert*-butyl) 6-methyl 3,4-dihydro-2,7-naphthyridine-2,6(1H)-dicarboxylate (**I-11a**) as a clear orange oil. LCMS (method 3) m/z 293.3 (M+H)⁺ (ES⁺) at 1.19 min.

**Step 2:** To a solution of 2-(tert-butyl) 6-methyl 3,4-dihydro-2,7-naphthyridine-2,6(1H)-dicarboxylate (**I-11a**) (225 mg, 770 µmol) in DCM (20 ml) was added a solution 4 M HCl in 1,4-dioxane (1.92 ml, 7.70 mmol). The resultant mixture was stirred at RT for 18 h. The solvent was removed in vacuo to give methyl 5,6,7,8-tetrahydro-2,7-naphthyridine-3-carboxylate hydrochloride (**I-11**) as a brown solid. 1H NMR (500 MHz, DMSO-d6) δ 9.50 (s, 2H), 8.58 (s, 1H), 7.96 (s, 1H), 4.39 (t, *J =* 4.8 Hz, 2H), 3.90 (s, 3H), 3.43 - 3.35 (m, 2H), 3.11 (t, *J =* 6.3 Hz, 2H).

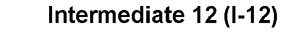

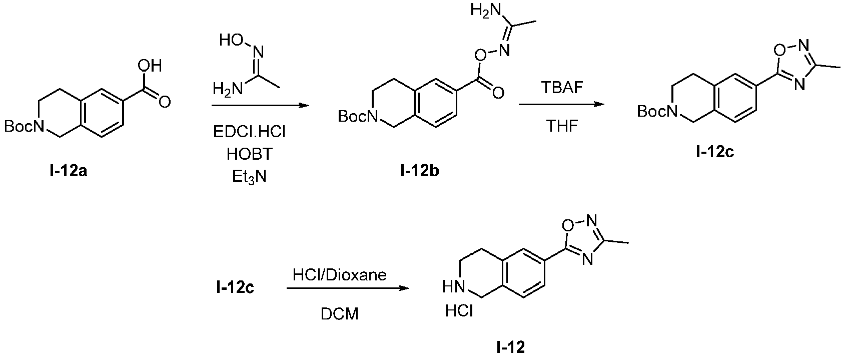

**Step 1:** To a solution of 2-(*tert*-butoxycarbonyl)-1,2,3,4-tetrahydroisoquinoline-6-carboxylic acid (100 mg, 361 µmol), EDCI hydrochloride (104 mg, 541 µmol) and HOBt monohydrate (104 mg, 80% w/w, 541 µmol) in DMF (2 ml) was added *N'*-hydroxyacetimidamide (35 mg, 469 µmol) and the reaction mixture was stirred at RT for 16 h. A further portion of *N*'-hydroxyacetimidamide (35 mg, 469 µmol) and EDCI hydrochloride (104 mg, 541 µmol) was added followed by Et₃N (151 µL, 1.08 mmol) and the reaction mixture was stirred for 18 h. The reaction was diluted with EtOAc (20 ml) and washed with sodium bicarbonate solution. The organics were concentrated *in vacuo.* The product was purified by chromatography on silica gel (0-10% (0.7 M Ammonia/MeOH)/DCM) to afford *tert-*butyl (Z)-6-((((1-aminoethylidene)amino)oxy)carbonyl)-3,4-dihydroisoquinoline-2(1H)-carboxylate **I-12b** as a clear colourless oil. LCMS (method 2) m/z 334.2 (M+H)⁺ (ES⁺) at 1.87 min.

**Step 2:** To a solution of *tert*-butyl-6-((((1-aminoethylidene)amino)oxy)carbonyl)-3,4-dihydroisoquinoline-2(1H)-carboxylate **I-12b** (100 mg, 300 µmol) in THF (2 ml) was added a solution of TBAF (300 µL, 1 M, 300 µmol) in THF. The reaction mixture was stirred at RT for 72 h.The reaction mixture was concentrated *in vacuo.* The product was purified by chromatography on silica gel (0-20% EtOAc/isohexane) to afford *tert-*butyl 6-(3-methyl-1,2,4-oxadiazol-5-yl)-3,4-dihydroisoquinoline-2(1H)-carboxylate **I-12c** as a colourless oil. ¹H NMR (500 MHz, DMSO-d6) δ 7.93 - 7.84 (m, 2H), 7.43 (d, *J =* 7.9 Hz, 1H), 4.60 (s, 2H), 3.59 (t, *J* = 5.9 Hz, 2H), 2.89 (t, *J =* 6.0 Hz, 2H), 2.41 (s, 3H), 1.43 (s, 9H).

**Step 3:** *tert-*butyl 6-(3-methyl-1,2,4-oxadiazol-5-yl)-3,4-dihydroisoquinoline-2(1H)-carboxylate **I-12c** (40 mg, 126 µmol) was dissolved in a solution of HCl in 1,4-dioxane (4 M, 1.5 ml, 6 mmol). The mixture was stirred at RT for 16 h. The reaction mixture was concentrated *in vacuo* to give 3-methyl-5-(1,2,3,4-tetrahydroisoquinolin-6-yl)-1,2,4-oxadiazole hydrochloride **I-12** as a colourless solid. ¹H NMR (500 MHz, DMSO-d6) δ 7.99 (s, 1H), 7.95 (dd, *J* = 8.2, 1.8 Hz, 1H), 7.47 (d, *J* = 8.1 Hz, 1H), 4.36 (s, 2H), 3.40 (t, *J* = 6.3 Hz, 2H), 3.10 (t, *J* = 6.3 Hz, 2H), 2.42 (s, 3H). 2 protons from N-H₂ not observed.

### Experimental Scheme 2

### Compound 79 6-((3-Chloro-4-(trifluoromethyl)phenyl)carbamoyl)-5,6,7,8-tetrahydro-2,6-naphthyridine 2-oxide

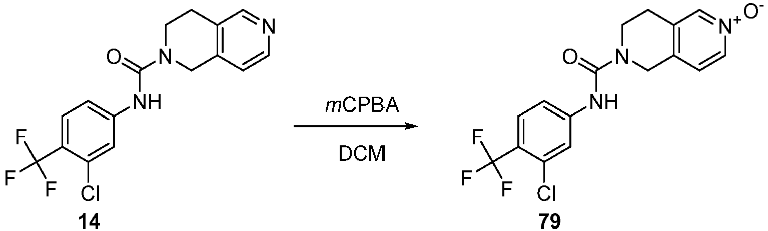

*N*-(3-Chloro-4-(trifluoromethyl)phenyl)-3,4-dihydro-2,6-naphthyridine-2(1H)-carboxamide (10 mg, 28 µmol) was dissolved in DCM (2 ml) and mCPBA (7.6 mg, 77 % w/w, 34 µmol) was added. The reaction mixture was stirred at RT for 16 h. Sodium metabisulfite solution (2 ml) was added and the layers were separated. The solvent was concentrated *in vacuo.* The product was purified by silica gel chromatography (0-10% (0.7 M NH₃/MeOH) in DCM) to afford 6-((3-chloro-4-(trifluoromethyl)phenyl)carbamoyl)-5,6,7,8-tetrahydro-2,6-naphthyridine 2-oxide **79** as a colourless solid. LC-MS (method 1) m/z 372.3, 374.3 [M+H]⁺ (ES⁺) at 1.15 min. ¹H NMR (500 MHz, DMSO-d6) δ 9.21 (s, 1H), 8.16 (s, 1H), 8.06 (dd, *J* = 6.6, 1.9 Hz, 1H), 7.91 (d, *J* = 2.1 Hz, 1H), 7.73 (d, *J =* 8.8 Hz, 1H), 7.64 (d, *J* = 8.9 Hz, 1H), 7.27 (d, *J* = 6.7 Hz, 1H), 4.63 (s, 2H), 3.72 (t, *J* = 5.9 Hz, 2H), 2.82 (t, *J =* 5.9 Hz, 2H).

The following compounds were prepared using appropriate starting materials in an analogous procedure to that described in Experimental Scheme 2.

| Compound | Structure | LCMS method 3 Rt [M+H]⁺ | NMR |
|---|---|---|---|
| 94 | 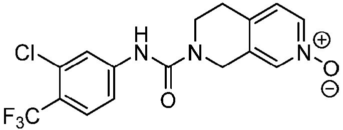 7-((3-Chloro-4-(trifluoromethyl)phenyl)carbamoyl)-5,6,7,8-tetrahydro-2,7-naphthyridine 2-oxide | 372.3, 374.3 at 1.14 | ¹H NMR (500 MHz, DMSO-*d*₆) δ 9.23 (s, 1H), 8.19 (d, *J =* 1.8 Hz, 1H), 8.04 (dd,*J* = 6.6, 1.9 Hz, 1H), 7.90 (d, *J =* 2.1 Hz, 1H), 7.74 (d, *J =* 8.8 Hz, 1H), 7.63 (dd, *J =* 8.7, 2.1 Hz, 1H), 7.26 (d, *J =* 6.6 Hz, 1H), 4.61 (s, 2H), 3.74 (t, *J =* 5.9 Hz, 2H), 2.83 (t, *J* = 5.9 Hz, 2H). |

### Experimental Scheme 3

### Compound 82 7-Amino-N-(3,4-dichlorophenyl)-3,4-dihydro-2,6-naphthyridine-2(1H)-carboxamide

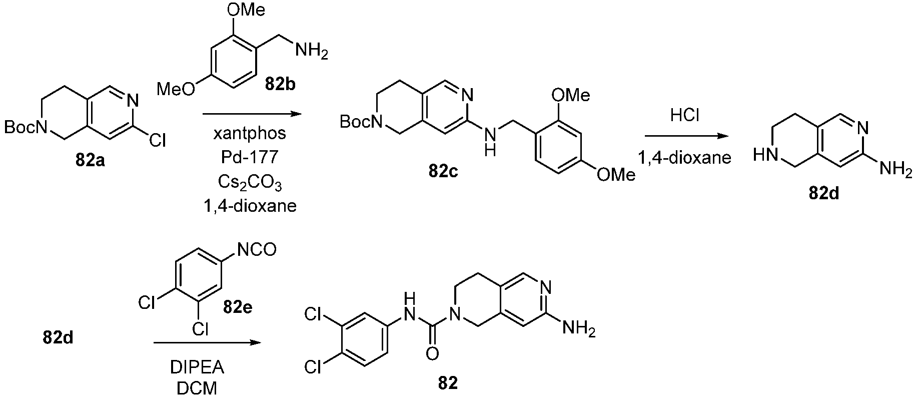

**Step 1:** To a solution of *tert-*butyl 7-chloro-3,4-dihydro-2,6-naphthyridine-2(1H)-carboxylate **82a** (700 mg, 2.60 mmol) in 1,4-dioxane (10 ml) and (2,4-dimethoxyphenyl)methanamine **82b** (1.57 ml, 10.4 mmol) was added followed by Cs₂CO₃ (1.28 g, 3.91 mmol). The reaction mixture was purged with nitrogen for 5 min. Pd-177 (79.3 mg, 104 µmol) and xantphos (60.3 mg, 0.04 Eq, 104 µmol) were added and the reaction mixture was purged with nitrogen for a further 5 min. The reaction mixture was heated to 85 °C for 20 h. The reaction mixture was concentrated *in vacuo.* The product was purified by silica gel chromatography (0-25% EtOAc in isohexane then 0-5% (0.7 M NH₃/MeOH) in DCM) to give *tert-*butyl 7-((2,4-dimethoxybenzyl)amino)-3,4-dihydro-2,6-naphthyridine-2(1H)-carboxylate **82c** as a yellow oil. ¹H NMR (500 MHz, DMSO-d6) δ 7.76 (s, 1H), 7.09 (d, *J =* 8.3 Hz, 1H), 6.54 (d, *J =* 2.4 Hz, 1H), 6.47 (s, 1H), 6.43 (dd, *J =* 8.3, 2.4 Hz, 1H), 6.28 (s, 1H), 4.35 (s, 2H), 4.30 (d, *J =* 6.0 Hz, 2H), 3.80 (s, 3H), 3.73 (s, 3H), 3.50 (t, *J =* 6.0 Hz, 2H), 2.58 (t, *J =* 5.9 Hz, 2H), 1.42 (s, 9H).

**Step 2:** *tert-*Butyl 7-((2,4-dimethoxybenzyl)amino)-3,4-dihydro-2,6-naphthyridine-2(1H)-carboxylate **82c** (360 mg, 901 µmol) was added to a solution of HCl in 1,4-dioxane (4.5 ml, 4 M, 18.0 mmol) and the resultant mixture was stirred at room temperature for 72 h. The solvent was removed *in vacuo.* The residue was dissolved in MeOH and SCX was added. The SCX was washed with MeOH (50 ml) and the product was eluted with 0.7 M NH₃/MeOH (100 ml). The eluant was concentrated *in vacuo* to give 5,6,7,8-tetrahydro-2,6-naphthyridin-3-amine **82d** as a yellow glass. LCMS (method 2) m/z 150.1 [M+H]⁺ (ES⁺) at 0.45 min. ¹H NMR (500 MHz, DMSO-d6) δ 7.64 (s, 1H), 6.09 (s, 1H), 5.49 (s, 2H), 3.69 (s, 2H), 2.89 (t, *J* = 5.9 Hz, 2H), 2.49 (d, *J =* 5.7 Hz, 2H).

**Step 3:** To a solution of 5,6,7,8-tetrahydro-2,6-naphthyridin-3-amine **82d** (60 mg, 0.4 mmol) was added DIPEA (0.21 ml, 1.2 mmol) and 1,2-dichloro-4-isocyanatobenzene **82e** (91 mg, 0.48 mmol) in DCM (2 ml). The reaction mixture was stirred at RT for 1 h. Saturated sodium bicarbonate solution (2 ml) was added and the product was extracted with 10% MeOH in DCM solution (3 x 3 ml) and the combined organics were concentrated *in vacuo.* The product was purified by silica gel chromatography (0-5% (0.7 M NH₃/MeOH)/DCM) to afford 7-amino-*N*-(3,4-dichlorophenyl)-3,4-dihydro-2,6-naphthyridine-2(1H)-carboxamide as a pale yellow solid. LCMS (method 3) m/z 337.3, 379.3 [M+H]⁺ (ES⁺) at 1.19 min. ¹H NMR (500 MHz, DMSO-d6) δ 8.84 (s, 1H), 7.86 (t, *J =* 1.4 Hz, 1H), 7.76 (s, 1H), 7.49 (d, *J =* 1.4 Hz, 2H), 6.24 (d, *J =* 0.9 Hz, 1H), 5.68 (s, 2H), 4.50 (s, 2H), 3.66 (t, *J =* 5.9 Hz, 2H), 2.66 (t, *J* = 5.9 Hz, 2H).

The following compounds were prepared using appropriate starting materials in an analogous procedure to that described in Experimental Scheme 3.

| Compound | Structure | LCMS method 3 Rt [M+H]⁺ | NMR |
|---|---|---|---|
| 83 | 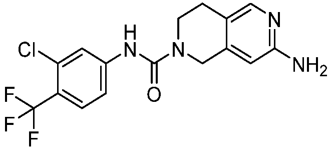 7-Amino-*N*-(3-chloro-4-(trifluoromethyl)phenyl)-3,4-dihydro-2,6-naphthyridine-2(1H)-carboxamide | 371.3, 373.3 at 1.29 min | ¹H NMR (500 MHz, DMSO-d6) δ 9.11 (s, 1H), 7.92 (d, *J* = 2.1 Hz, 1H), 7.76 (s, 1H), 7.72 (d, *J =* 8.8 Hz, 1H), 7.65 (dd, *J* = 8.4, 2.1 Hz, 1H), 6.25 (s, 1H), 5.69 (s, 2H), 4.53 (s, 2H), 3.68 (dd, *J =* 7.4, 4.3 Hz, 2H), 2.68 (t, *J =* 5.8 Hz, 2H). |
| 84 | 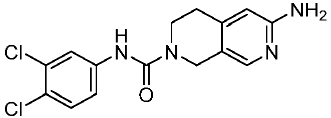 6-Amino-*N*-(3,4-dichlorophenyl)-3,4-dihydro-2,7-naphthyridine-2(1H)-carboxamide | 371.3, 373.3 at 1.18 min | ¹H NMR (500 MHz, DMSO-d6) δ 8.81 (s, 1H), 7.86 (t, *J =* 1.4 Hz, 1H), 7.75 (s, 1H), 7.49 (d, *J =* 1.4 Hz, 2H), 6.26 (s, 1H), 5.71 (s, 2H), 4.48 (s, 2H), 3.62 (t, *J =* 6.0 Hz, 2H), 2.71 (t, *J =* 6.0 Hz, 2H) |

### Experimental Scheme 4

### Compound 85 N-(3,4-dichlorophenyl)-6-fluoro-3,4-dihydro-2,7-naphthyridine-2(1H)-carboxamide

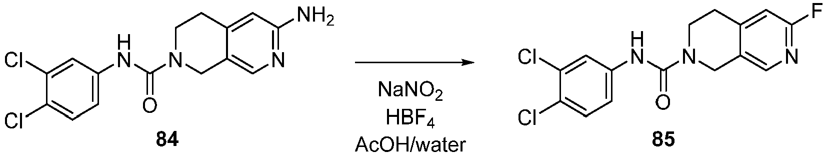

A solution of 6-amino-*N*-(3,4-dichlorophenyl)-3,4-dihydro-2,7-naphthyridine-2(1H)-carboxamide (65 mg, 0.19 mmol) in AcOH (2 ml) was added to a solution of tetrafluoroboric acid in water (3.0 ml, 48% w/w, 19 mmol) at 0 °C. A solution of sodium nitrite (27 mg, 0.39 mmol) in water (1 ml) was added dropwise over 20 min and the reaction mixture was stirred at 0 °C for a further 1 h. The reaction mixture was added to a solution of ice cold NaHCO₃ (10 ml), and the product was extracted using 20% MeOH in DCM solution (3 x 25 ml). The solvent was dried over sodium sulfate and the solvent was removed under reduced pressure. The product was purified by silica gel chromatography (0-0.75% (0.7 M NH₃/MeOH)/DCM to give *N*-(3,4-dichlorophenyl)-6-fluoro-3,4-dihydro-2,7-naphthyridine-2(1H)-carboxamide **85** as a colourless solid. LCMS (method 3) m/z 338.1, 340.1 [M-H]⁻ (ES⁻) at 1.32 min. ¹H NMR (500 MHz, DMSO-d6) δ 8.94 (s, 1H), 8.10 (s, 1H), 7.87 - 7.83 (m, 1H), 7.53 - 7.45 (m, 2H), 7.06 (d, *J =* 1.9 Hz, 1H), 4.67 (s, 2H), 3.71 (t, *J =* 6.0 Hz, 2H), 2.94 (t, *J* = 5.9 Hz, 2H).

The following compounds were prepared using appropriate starting materials in an analogous procedure to that described in Experimental Scheme 4.

| Compound | Structure | LCMS method 3 Rt [M+H]⁺ | NMR |
|---|---|---|---|
| 86 | 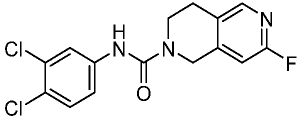 *N*-(3,4-Dichlorophenyl)-7-fluoro-3,4-dihydro-2,6-naphthyridine-2(1H)-carboxamide | 340.1, 342.3 at 1.44 | ¹H NMR (500 MHz, DMSO-d6) δ 8.97 (s, 1H), 8.09 (s, 1H), 7.85 (d, *J =* 2.2 Hz, 1H), 7.53 - 7.45 (m, 2H), 7.07 (d, *J =* 1.9 Hz, 1H), 4.71 (s, 2H), 3.74 (t, *J* = 5.9 Hz, 2H), 2.87 (t, *J =* 5.7 Hz, 2H). |
| 87 | 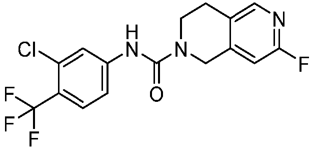 *N*-(3-Chloro-4-(trifluoromethyl)phenyl)-7-fluoro-3,4-dihydro-2,6-naphthyridine-2(1H)-carboxamide | 372.1, 374.2 [M-H]⁻ at 1.44 min | ¹H NMR (500 MHz, DMSO-d6) δ 9.24 (s, 1H), 8.09 (s, 1H), 7.91 (d, *J =* 2.1 Hz, 1H), 7.74 (d, *J =* 8.8 Hz, 1H), 7.66 - 7.62 (m, 1H), 7.08 (s, 1H), 4.74 (s, 2H), 3.76 (t, *J =* 5.9 Hz, 2H), 2.88 (t, *J* = 5.8 Hz, 2H). |
| 120 | 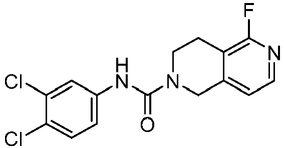 *N*-(3,4-Dichlorophenyl)-5-fluoro-3,4-dihydro-2,6-naphthyridine-2(1H)-carboxamide | 340.5, 342.5 at 1.46 min | ¹H NMR (500 MHz, DMSO-*d*₆) δ 8.99 (s, 1H), 8.02 (d, *J =* 5.2 Hz, 1H), 7.84 (d, *J* = 2.2 Hz, 1H), 7.53 - 7.44 (m, 2H), 7.23 - 7.18 (m, 1H), 4.72 (s, 2H), 3.78 (t, *J =* 5.9 Hz, 2H), 2.78 (t, *J* = 5.9 Hz, 2H). |
| 123 | 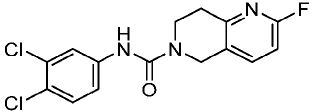 *N*-(3,4-Dichlorophenyl)-2-fluoro-7,8-dihydro-1,6-naphthyridine-6(5H)-carboxamide | 340.5, 342.5 at 1.39 min | ¹H NMR (500 MHz, DMSO-*d*₆) δ 8.98 (s, 1H), 7.84 (d, *J =* 2.1 Hz, 1H), 7.81 (t, *J* = 8.2 Hz, 1H), 7.52 - 7.44 (m, 2H), 7.03 (dd, *J* = 8.3, 2.7 Hz, 1H), 4.66 (s, 2H), 3.80 (t, *J =* 5.9 Hz, 2H), 2.88 (t, *J =* 5.8 Hz, 2H). |

### Experimental Scheme 5

### Compound 92 N-(3,4-Dichlorophenyl)-7-methyl-6-oxo-3,4,6,7-tetrahydro-2,7-naphthyridine-2(1H)-carboxamide

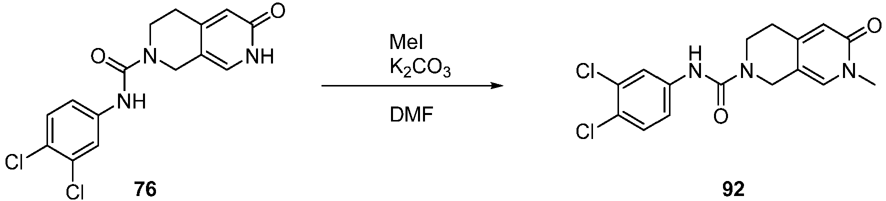

To a solution of *N-*(3,4-dichlorophenyl)-6-oxo-3,4,6,7-tetrahydro-2,7-naphthyridine-2(1H)-carboxamide (**76**) (25 mg, 74 µmol) in DMF (2 ml) was added Mel (4.6 µl, 74 µmol) and potassium carbonate (31 mg, 0.22 mmol) and the reaction mixture was stirred for 36 h at RT. The reaction mixture was partitioned between saturated sodium bicarbonate solution (10 ml) and 20% (0.7M NH₃ in MeOH) in DCM (20 ml) and the organic layer concentrated *in vacuo.* The product was purified via mass directed RP-prep-HPLC (20-50% ammonium bicarbonate in MeCN) to give N-(3,4-dichlorophenyl)-7-methyl-6-oxo-3,4,6,7-tetrahydro-2,7-naphthyridine-2(1H)-carboxamide 92 as a white solid. LCMS (method 1) m/z 352.4, 354.3 [M+H]⁺ (ES⁺) at 1.32 min. ¹H NMR (500 MHz, DMSO-*d*₆) δ 8.79 (s, 1H), 7.85 (d, *J =* 2.0 Hz, 1H), 7.63 (s, 1H), 7.52 - 7.44 (m, 2H), 6.26 (s, 1H), 4.38 (s, 2H), 3.60 (t, *J* = 6.1 Hz, 2H), 3.39 (s, 3H), 2.77 (t, *J* = 6.0 Hz, 2H).

### Experimental Scheme 6

### Compound 95 N-(3,4-Dichlorophenyl)-6-hydroxy-3,4-dihydroisoquinoline-2(1H)-carboxamide

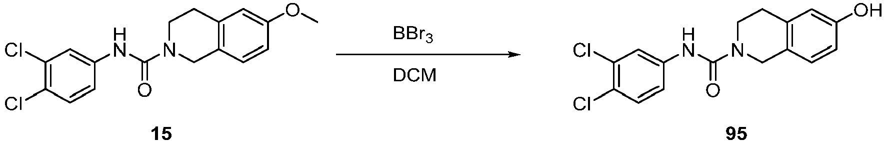

To a solution of *N*-(3,4-dichlorophenyl)-6-methoxy-3,4-dihydroisoquinoline-2(1H)-carboxamide (**15**) (25 mg, 71 µmol) was dissolved in DCM (0.5 ml) and the solution was cooled to -78 °C. A solution of boron tribromide (1M solution in DCM) (93 µl, 93 µmol) was added dropwise and the mixture was stirred at -78 °C for 5 min then warmed to 0 °C for 1 h. The reaction mixture was cooled to -78 °C and anhydrous MeOH (1 ml) was added. The reaction mixture was warmed to RT for 30 min. The reaction mixture was concentrated *in vacuo* and the residue was purified by chromatography on RP Flash C18 (5-65% MeCN/10 mM ammonium bicarbonate) to afford *N*-(3,4-dichlorophenyl)-6-hydroxy-3,4-dihydroisoquinoline-2(1H)-carboxamide (**95**) as a white solid. LCMS (method 3) m/z 337.2, 339.2, 341.2 [M+H]⁺ (ES⁺) at 1.32 min. ¹H NMR (500 MHz, d6-DMSO) δ 1H NMR (500 MHz, DMSO-d6) δ 9.29 (s, 1H), 8.81 (s, 1H), 7.87 (d, *J* = 2.1 Hz, 1H), 7.53 - 7.43 (m, 2H), 6.96 (d, *J* = 8.2 Hz, 1H), 6.61 (dd, *J* = 8.2, 2.5 Hz, 1H), 6.58 (d, *J =* 2.5 Hz, 1H), 4.51 (s, 2H), 3.64 (t, *J* = 5.9, 5.9 Hz, 2H), 2.76 (t, *J* = 5.9, 5.9 Hz, 2H).

### Experimental Scheme 7

### Compound 97 8-Chloro-N-(3-chloro-4-(trifluoromethyl)phenyl)-3,4-dihydro-2,7-naphthyridine-2(1H)-carboxamide

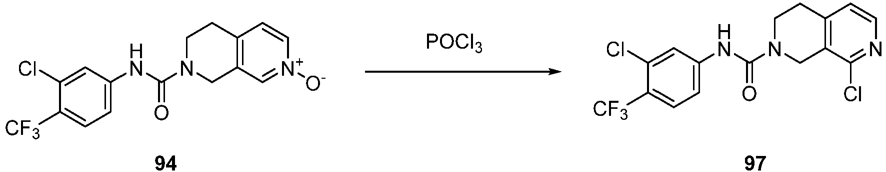

7-((3-Chloro-4-(trifluoromethyl)phenyl)carbamoyl)-5,6,7,8-tetrahydro-2,7-naphthyridine 2-oxide (**94**) (20 mg, 54 µmol) was dissolved in POCl₃ (15 µl, 0.16 mmol) and the reaction mixture was heated under reflux for 16 h. The reaction mixture was cooled to RT and concentrated *in vacuo.* The residue was stirred in ice water and saturated sodium bicarbonate solution (10 ml) was added. The aqueous mixture was extracted with 10% MeOH in DCM (30 ml), dried over sodium sulfate and concentrated *in vacuo.* The product was purified on silica (0-5% MeOH in DCM) to give 8-chloro-*N*-(3-chloro-4-(trifluoromethyl)phenyl)-3,4-dihydro-2,7-naphthyridine-2(1H)-carboxamide as a white solid. LCMS (method 1) m/z 390.0, 392.0 [M+H]⁺ (ES⁺) at 1.50 min. ¹H NMR (500 MHz, DMSO-d6) δ 9.33 (s, 1H), 8.22 (d, *J* = 5.0 Hz, 1H), 7.91 (s, 1H), 7.73-7.75 (m, 1H), 7.64 (d, *J =* 8.9 Hz, 1H), 7.30 (d, *J* = 5.0 Hz, 1H), 4.64 (s, 2H), 3.76 (t, *J =* 5.8 Hz, 2H), 2.93 (t, *J =* 5.8 Hz, 2H).

### Experimental Scheme 8

### Compound 104 N-(3-chloro-4-(trifluoromethyl)phenyl)-6-fluoro-3,4-dihydro-2,7-naphthyridine-2(1H)-carboxamide

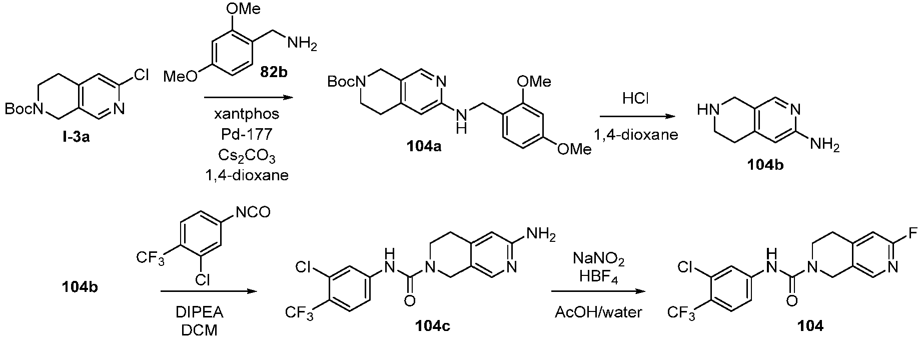

**Step 1:** *tert-*Butyl 6-((2,4-dimethoxybenzyl)amino)-3,4-dihydro-2,7-naphthyridine-2(1H)-carboxylate (**104a**) was synthesised from *tert-*butyl 6-chloro-3,4-dihydro-2,7-naphthyridine-2(1H)-carboxylate (**I-3a**) using essentially the same procedure as **82c.** ¹H NMR (500 MHz, DMSO-d6) δ 7.77 (s, 1H), 7.08 (d, *J* = 8.3 Hz, 1H), 6.56 - 6.50 (m, 2H), 6.43 (dt, *J* = 8.3, 1.9 Hz, 1H), 6.29 (s, 1H), 4.35 - 4.28 (m, 4H), 3.80 (s, 3H), 3.72 (s, 3H), 3.46 (t, *J* = 6.0 Hz, 2H), 2.62 (t, *J* = 6.0 Hz, 2H), 1.42 (d, *J =* 1.3 Hz, 9H).

**Step 2:** 5,6,7,8-Tetrahydro-2,7-naphthyridin-3-amine (**104b**) was synthesised from *tert-*butyl 6-((2,4-dimethoxybenzyl)amino)-3,4-dihydro-2,7-naphthyridine-2(1H)-carboxylate (**104a**) using essentially the same procedure as **82d.** ¹H NMR (500 MHz, DMSO-d6) δ 7.60 (d, *J* = 0.9 Hz, 1H), 6.15 (d, *J =* 1.2 Hz, 1H), 5.50 (s, 2H), 3.69 (s, 2H), 2.88 (t, *J* = 6.0 Hz, 2H), 2.55 (d, *J* = 6.0 Hz, 2H). (1 exchangable H not seen).

**Step 3:** 6-Amino-*N*-(3-chloro-4-(trifluoromethyl)phenyl)-3,4-dihydro-2,7-naphthyridine-2(1H)-carboxamide (**104c**) was synthesised from 5,6,7,8-tetrahydro-2,7-naphthyridin-3-amine (**104b**) using essentially the same procedure as **82.** LCMS (method 3) m/z 371.1, 373.1 [M+H]⁺ (ES⁺) at 1.98 min.

**Step 4:** *N*-(3-Chloro-4-(trifluoromethyl)phenyl)-6-fluoro-3,4-dihydro-2,7-naphthyridine-2(1H)-carboxamide (**104**) was synthesised from 6-amino-*N*-(3-chloro-4-(trifluoromethyl)phenyl)-3,4-dihydro-2,7-naphthyridine-2(1H)-carboxamide (**104c**) using essentially the same procedure as **85.** LCMS (method 3) m/z 374.1, 376.1 [M+H]⁺ (ES⁺) at 1.28 min. ¹H NMR (500 MHz, DMSO-d6) δ 9.21 (s, 1H), 8.11 (s, 1H), 7.91 (d, *J* = 2.1 Hz, 1H), 7.74 (d, *J* = 8.8 Hz, 1H), 7.64 (dd, *J =* 8.6, 2.1 Hz, 1H), 7.09 - 7.05 (m, 1H), 4.70 (s, 2H), 3.74 (t, *J =* 5.9 Hz, 2H), 2.96 (t, *J* = 5.9 Hz, 2H).

### Experimental Scheme 9

### Compound 110 N-(3,4-Dichlorophenyl)-8,9-dihydroimidazo[1,2-b][2,7]naphthyridine-7(6H)-carboxamide

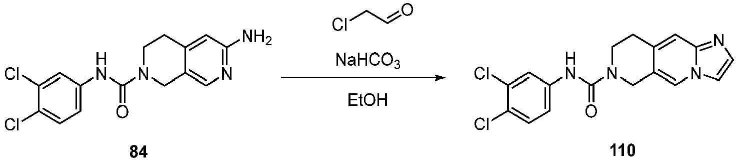

To a solution of 6-amino-*N*-(3,4-dichlorophenyl)-3,4-dihydro-2,7-naphthyridine-2(1H)-carboxamide (**84**) (45 mg, 0.13 mmol) in EtOH (4 ml) was added sodium bicarbonate (22 mg, 0.27 mmol) and 2-chloroacetaldehyde (25 µl, 50% w/w, 0.20 mmol) and the reaction mixture was heated to 85 °C for 16 h. The reaction mixture was concentrated *in vacuo* and the product was purified on silica gel (0-4.5% (0.7 M NH₃/MeOH)/DCM) to give *N*-(3,4-dichlorophenyl)-8,9-dihydroimidazo[1,2-b][2,7]naphthyridine-7(6H)-carboxamide (**110**) as a clear colourless glass. LCMS (method 3) m/z 361.5, 363.3 [M+H]⁺ (ES⁺) at 1.25 min. ¹H NMR (500 MHz, DMSO-d6) δ 8.81 (s, 1H), 8.50 (s, 1H), 7.89 - 7.85 (m, 2H), 7.53 - 7.46 (m, 3H), 7.44 (s, 1H), 4.64 (d, *J =* 1.1 Hz, 2H), 3.69 (t, *J* = 6.2 Hz, 2H), 3.04 - 2.98 (m, 2H).

The following compounds were prepared using appropriate starting materials in an analogous procedure to that described in Experimental Scheme 9.

| Compound | Structure | LCMS method 3 Rt [M+H]⁺ | NMR |
|---|---|---|---|
| 115 | 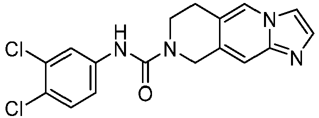 *N*-(3,4-Dichlorophenyl)-6,7-dihydroimidazo[1,2-b][2,6]naphthyridine-8(9H)-carboxamide | 361.3, 363.3 at 1.22 | ¹H NMR (500 MHz, DMSO-d6) δ 8.83 (s, 1H), 8.45 (s, 1H), 7.87 (d, *J =* 2.5 Hz, 2H), 7.54 - 7.47 (m, 3H), 7.45 (s, 1H), 4.70 (d, *J =* 1.3 Hz, 2H), 3.70 (t, *J* = 6.1 Hz, 2H), 2.93 (t, *J* = 6.2 Hz, 2H). |

### Experimental Scheme 10

### Compound 111 N-(3,4-Dichlorophenyl)-8,9-dihydro-[1,2,4]triazolo[1,5-b][2,7]naphthyridine-7(6H)-carboxamide

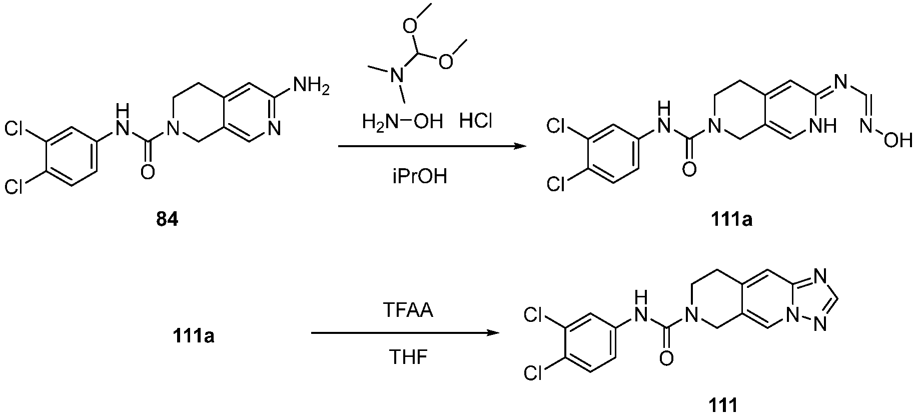

**Step 1:** To a solution of 6-amino-*N*-(3,4-dichlorophenyl)-3,4-dihydro-2,7-naphthyridine-2(1H)-carboxamide (**84**) (45 mg, 0.13 mmol) in 2-propanol (2 ml) was added 1,1-dimethoxy-*N*,*N*-dimethylmethanamine (23 µl, 0.17 mmol) and the reaction mixture was heated to 90 °C for 3 h. The temperature was reduced to 50 °C and hydroxylamine hydrochloride (12 mg, 0.17 mmol) was added. The reaction mixture was stirred at 50 °C for 3 h. The reaction mixture was concentrated *in vacuo* and the product was purified on silica gel (40 g cartridge, 0-5% MeOH/DCM) to give (*Z*)-*N*-(3,4-dichlorophenyl)-6-(((hydroxyamino)methylene)amino)-3,4-dihydro-2,7-naphthyridine-2(1H)-carboxamide (**111a**) as a pale white solid.

**Step 2:** To a solution of (*Z*)-*N*-(3,4-dichlorophenyl)-6-(((hydroxyamino)methylene)amino)-3,4-dihydro-2,7-naphthyridine-2(1H)-carboxamide (**111a**) in THF (5 ml), TFAA (10 µl, 71 µmol) was added at 0 °C. The reaction mixture was stirred at RT for 2 h. The reaction mixture was diluted with DCM (5 ml), washed with saturated sodium bicarbonate solution (5 ml) and the organic layer concentrated *in vacuo.* The product was purified on silica gel (0-4.5% (0.7 M NH₃/MeOH)/DCM) to afford *N*-(3,4-dichlorophenyl)-8,9-dihydro-[1,2,4]triazolo[1,5-b][2,7]naphthyridine-7(6H)-carboxamide (**111**) as a pale white solid. LCMS (method 3) m/z 362.5, 364.3 [M+H]⁺ (ES⁺) at 1.19 min. ¹H NMR (500 MHz, DMSO-d6) δ 8.97 (s, 1H), 8.87 (s, 1H), 8.43 (s, 1H), 7.86 (t, *J =* 1.4 Hz, 1H), 7.73 (s, 1H), 7.50 (d, *J =* 1.4 Hz, 2H), 4.73 (s, 2H), 3.73 (t, *J =* 6.2 Hz, 2H), 3.10 (t, *J =* 6.0 Hz, 2H).

The following compounds were prepared using appropriate starting materials in an analogous procedure to that described in Experimental Scheme 10.

| Compound | Structure | LCMS method 3 Rt [M+H]⁺ | NMR |
|---|---|---|---|
| 121 | 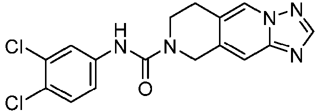 *N*-(3,4-Dichlorophenyl)-6,7-dihydro-[1,2,4]triazolo[1,5-b][2,6]naphthyridine-8(9H)-carboxamide | 362.3, 364.3 at 1.19 | ¹H NMR (500 MHz, DMSO-d6) δ 8.92 - 8.88 (m, 2H), 8.43 (s, 1H), 7.86 (t, *J =* 1.4 Hz, 1H), 7.75 (d, *J =* 1.1 Hz, 1H), 7.50 *(d, J =* 1.4 Hz, 2H), 4.79 (d, *J =* 1.3 Hz, 2H), 3.73 (t, *J =* 6.1 Hz, 2H), 3.01 (t, *J* = 6.0 Hz, 2H). |

### Experimental Scheme 11

### Compound 112 N-(4,5-Dichloro-2-fluorophenyl)-6-oxo-3,4,6,7-tetrahydro-2,7-naphthyridine-2(1H)-carboxamide

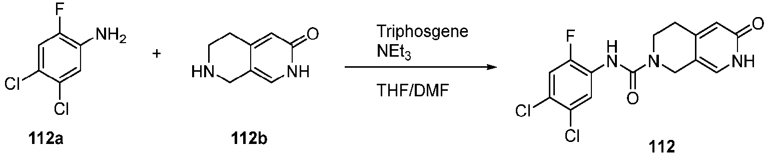

To a solution of triphosgene (28.8 mg, 97.2 µmol) in THF (2 ml) was added a solution of 4,5-dichloro-2-fluoroaniline (**112a**) (50 mg, 278 µmol) and triethylamine (116 µl, 833 µmol) in THF (1 ml) dropwise. The resultant mixture was stirred at RT for 30 min. A solution of 5,6,7,8-tetrahydro-2,7-naphthyridin-3(2H)-one (**112b**) (42 mg, 278 µmol) in THF (1 ml) and DMF (2 ml) was added and the mixture was stirred at RT for 16 h. The reaction mixture was filtered and concentrated *in vacuo.* The product was purified by RP Flash C18 (15-75% MeCN/10 mM ammonium bicarbonate) to afford *N*-(4,5-dichloro-2-fluorophenyl)-6-oxo-3,4,6,7-tetrahydro-2,7-naphthyridine-2(1H)-carboxamide (**112**) as a colourless solid. LCMS (method 3) m/z 356.2, 358.2 [M+H]⁺ (ES⁺) at 1.03 min. ¹H NMR (500 MHz, DMSO-d6) δ 11.38 (s, 1H), 8.48 (s, 1H), 7.82 (d, J = 7.6 Hz, 1H), 7.69 (d, J = 10.3 Hz, 1H), 7.32 (s, 1H), 6.20 (s, 1H), 4.38 (s, 2H), 3.59 (t, J = 6.1 Hz, 2H), 2.77 (t, J = 6.1 Hz, 2H).

The following compounds were prepared using appropriate starting materials in an analogous procedure to that described in Experimental Scheme 11.

### Key: (a) Reaction performed in DCM

| Compound | Structure | LCMS method 3 Rt [M+H]⁺ | NMR |
|---|---|---|---|
| 118^{(a)} | 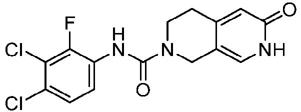 *N*-(3,4-Dichloro-2-fluorophenyl)-6-oxo-3,4,6,7-tetrahydro-2,7-naphthyridine-2(1H)-carboxamide | 356.4, 358.0 at 1.05 min | ¹H NMR (500 MHz, DMSO-d6) δ 11.39 (s, 1H), 8.54 (s, 1H), 7.59 - 7.35 (m, 2H), 7.32 (s, 1H), 6.20 (s, 1H), 4.37 (s, 2H), 3.59 (t, *J =* 6.2 Hz, 2H), 2.77 (t, *J* = 6.0 Hz, 2H). |
| 130 | 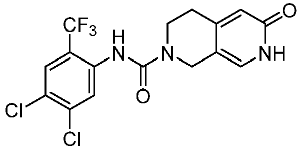 *N*-(4,5-dichloro-2-(trifluoromethyl)phenyl)-6-oxo-3,4,6,7-tetrahydro-2,7-naphthyridine-2(1H)-carboxamide | 406.0, 408.4 at 1.19 min | ¹H NMR (500 MHz, DMSO-d6) δ 11.38 (s, 1H), 8.32 (s, 1H), 7.97 (s, 1H), 7.85 (s, 1H), 7.30 (s, 1H), 6.20 (s, 1H), 4.36 (s, 2H), 3.58 (t, J = 6.2 Hz, 2H), 2.76 (t, J = 6.2 Hz, 2H). |

### Experimental Scheme 12

### Compound 119 5-Amino-N-(3,4-dichlorophenyl)-3,4-dihydro-2,6-naphthyridine-2(1H)-carboxamide

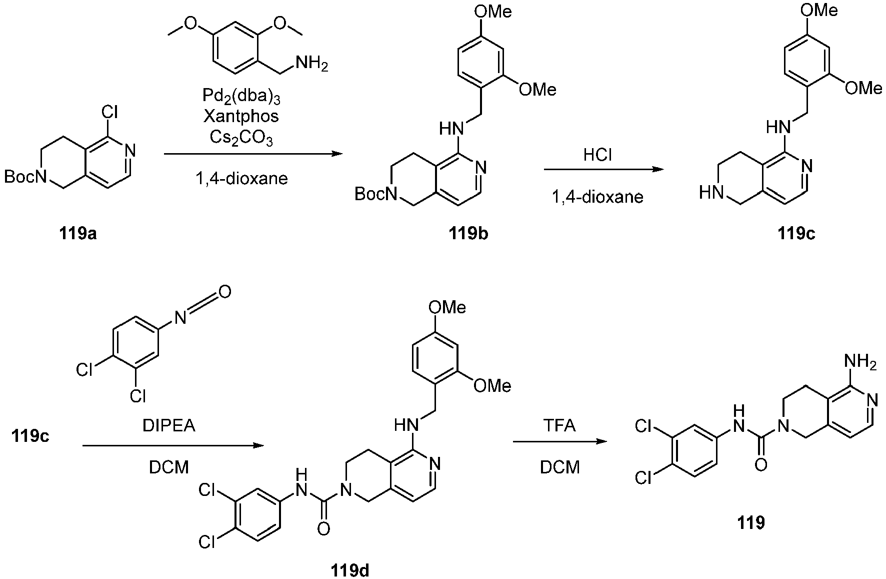

**Step 1:** To a solution of *tert-*butyl 5-chloro-3,4-dihydro-2,6-naphthyridine-2(1H)-carboxylate (**119a**) (750 mg, 2.79 mmol) in 1,4-dioxane (10 ml), (2,4-dimethoxyphenyl)methanamine (545 µl, 3.63 mmol) and Cs₂CO₃ (1.83 g, 5.58 mmol) was added and the reaction mixture was purged with nitrogen for 5 min. Xantphos (161 mg, 279 µmol) and Pd₂(dba)₃ (128 mg, 140 µmol) were added and the reaction mixture was purged with nitrogen for a further 5 min. The reaction mixture was heated to 95 °C for 20 h, cooled and filtered through a plug of celite and concentrated *in vacuo.* The residue was purified on silica (0-20% EtOAc/isohexane) to give *tert-*butyl 5-((2,4-dimethoxybenzyl)amino)-3,4-dihydro-2,6-naphthyridine-2(1H)-carboxylate (**119b**) as a brown oil. ¹H NMR (500 MHz, DMSO-d6) δ 7.73 (d, *J =* 5.2 Hz, 1H), 6.99 (d, *J =* 8.3 Hz, 1H), 6.53 (d, *J =* 2.4 Hz, 1H), 6.40 (dd, *J =* 8.3, 2.4 Hz, 1H), 6.33 (d, *J =* 5.3 Hz, 1H), 6.18 (s, 1H), 4.45 (d, *J* = 5.8 Hz, 2H), 4.37 (s, 2H), 3.80 (s, 3H), 3.71 (s, 3H), 3.61 (d, *J* = 6.0 Hz, 2H), 2.46 (t, *J* = 6.0 Hz, 2H), 1.42 (s, 9H).

**Step 2:** *tert-*Butyl 5-((2,4-dimethoxybenzyl)amino)-3,4-dihydro-2,6-naphthyridine-2(1H)-carboxylate (**119b**) (580 mg, 1.45 mmol) was dissolved in a solution of HCl in 1,4-dioxane (7.26 ml, 4 M, 29.0 mmol) and stirred at RT for 16 h. The reaction mixture was concentrated *in vacuo* and the residue was loaded onto a SCX cartridge in MeOH (50 ml) and the product was eluted with 0.7 M NH₃ in MeOH (100 ml) solution and concentrated *in vacuo* to give *N-*(2,4-dimethoxybenzyl)-5,6,7,8-tetrahydro-2,6-naphthyridin-1-amine (**119c**) as a brown oil. LCMS (method 3) m/z 303.3 [M+H]⁺ (ES⁺) at 1.06 min.

**Step 3:** To a solution of *N*-(2,4-dimethoxybenzyl)-5,6,7,8-tetrahydro-2,6-naphthyridin-1-amine HCl (**119c**) (370 mg, 1.10 mmol) in DCM (5 ml) was added DIPEA (0.58 ml, 3.31 mmol) followed by dropwise addition of 1,2-dichloro-4-isocyanatobenzene (207 mg, 1.10 mmol) in DCM (2 ml). The reaction mixture was stirred at RT for 0.5 h. The reaction mixture was diluted with saturated sodium bicarbonate solution (5 ml), extracted with 10% MeOH in DCM solution (3 ml) and the aqueous layer was extracted with10% MeOH in DCM solution (2 x 3 ml). The combined organic layers were concentrated *in vacuo* and the product was purified on silica gel (0-5% (0.7 M NH₃/MeOH)/DCM) to give *N*-(3,4-dichlorophenyl)-5-((2,4-dimethoxybenzyl)amino)-3,4-dihydro-2,6-naphthyridine-2(1H)-carboxamide (**119d**) as a clear white solid. LCMS (method 3) m/z 487.0, 489.2 [M+H]⁺ (ES⁺) at 1.64 min.

**Step 4:** *N*-(3,4-dichlorophenyl)-5-((2,4-dimethoxybenzyl)amino)-3,4-dihydro-2,6-naphthyridine-2(1H)-carboxamide (**119d**) (295 mg, 605 µmol) were combined with DCM (8 ml) and TFA (1.9 ml, 24.2 mmol) was added and the reaction mixture was stirred at RT for 1 h. The reaction mixture was concentrated *in vacuo* and the product was purified on silica gel (0-5% (0.7 M NH₃/MeOH)/DCM) to afford 5-amino-*N*-(3,4-dichlorophenyl)-3,4-dihydro-2,6-naphthyridine-2(1H)-carboxamide (**119**) as a clear colourless glass. LCMS (method 3) m/z 337.2, 339.2 [M+H]⁺ (ES⁺) at 1.21 min. ¹H NMR (500 MHz, DMSO-d6) δ 8.93 (s, 1H), 7.85 (t, *J =* 1.4 Hz, 1H), 7.73 (d, *J =* 5.2 Hz, 1H), 7.48 (d, *J =* 1.3 Hz, 2H), 6.36 (d, *J =* 5.2 Hz, 1H), 5.76 (s, 2H), 4.50 (s, 2H), 3.73 (t, *J =* 5.9 Hz, 2H), 2.46 (t, *J =* 5.9 Hz, 2H)

### Experimental Scheme 13

### Compound 126 N-(3,4-Dichlorophenyl)-6-(2-fluoropyridin-3-yl)-3,4-dihydroisoquinoline-2(1H)-carboxamide

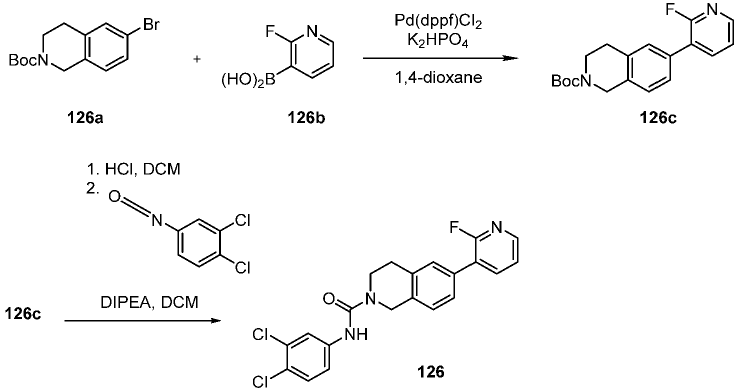

**Step 1:** A vessel was charged with *tert-butyl* 6-bromo-3,4-dihydroisoquinoline-2(1H)-carboxylate (**126a**) (50 mg, 0.16 mmol), Pd(dppf)Cl₂ (12 mg, 16 µmol) and (2-fluoropyridin-3-yl)boronic acid (**126b**) (45 mg, 0.32 mmol) and evacuated and back filled with nitrogen (3 times). 1,4-dioxane (0.5 ml) was added and was purged and backfilled with nitrogen (3 times). An aqueous solution of potassium phosphate, dibasic (0.64 ml, 0.5 M, 0.32 mmol) was added and the reaction was heated to 80 °C for 72 h. The reaction was cooled to RT and filtered through a pad of Celite, washing with EtOAc (20 ml). The product was purified on silica gel (0-50% EtOAc/isohexane) to afford *tert-*butyl 6-(2-fluoropyridin-3-yl)-3,4-dihydroisoquinoline-2(1H)-carboxylate (**126c**) as a sticky white oil. LCMS (method 2) m/z 329.1 [M+H]⁺ (ES⁺) at 2.56 min. ¹H NMR (500 MHz, CDCl₃) δ 8.27 - 8.11 (m, 1H), 7.85 (ddd, *J* = 9.6, 7.4, 1.8 Hz, 1H), 7.38 (d, *J =* 8.2 Hz, 1H), 7.34 (s, 1H), 7.28 (dd, *J =* 5.1, 1.9 Hz, 1H), 7.21 (d, *J =* 8.1 Hz, 1H), 4.63 (s, 2H), 3.69 (s, 2H), 2.90 (t, *J =* 5.8 Hz, 2H), 1.50 (s, 9H).

**Step 2:** To a solution of *tert-*butyl 6-(2-fluoropyridin-3-yl)-3,4-dihydroisoquinoline-2(1H)-carboxylate (**126c**) (39.2 mg, 119 µmol) in DCM (1 ml) was added HCl in 1,4-dioxane (298 µl, 4 M, 1.19 mmol) was added. The reaction was stirred at RT for 17 h. The reaction mixture was concentrated *in vacuo.* The residue was redissolved in DCM (1 ml). 1,2-dichloro-4-isocyanatobenzene (25 mg, 131 µmol) was added, followed by DIPEA (62 µl, 358 µmol). The reaction mixture was stirred at RT for 2 h. The reaction mixture was concentrated *in vacuo* and the product was purified by silica gel (0-40% EtOAc/isohexane) to afford *N*-(3,4-dichlorophenyl)-6-(2-fluoropyridin-3-yl)-3,4-dihydroisoquinoline-2(1H)-carboxamide (**126**) as a light white powder. LCMS (method 3) m/z 414.2 (M-H)- (ES-) at 1.64 min. ¹H NMR (500 MHz, DMSO-d6) δ 8.90 (s, 1H), 8.23 (dd, *J =* 4.0, 2.4 Hz, 1H), 8.10 (ddd, *J =* 10.0, 7.5, 2.0 Hz, 1H), 7.88 (d, *J* = 2.1 Hz, 1H), 7.54 - 7.43 (m, 5H), 7.32 (d, *J =* 8.5 Hz, 1H), 4.70 (s, 2H), 3.74 (t, *J* = 5.9 Hz, 2H), 2.93 (t, *J =* 5.8 Hz, 2H).

The following compounds were prepared using appropriate starting materials in an analogous procedure to that described in Experimental Scheme 13.

### Key: (a) Reaction performed using pinacol boronic ester

| Compound | Structure | LCMS method 3 Rt [M-H]⁻ | NMR |
|---|---|---|---|
| 127 | 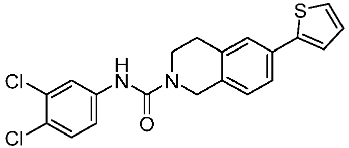 *N*-(3,4-Dichlorophenyl)-6-(thiophen-2-yl)-3,4-dihydroisoquinoline-2(1H)-carboxamide | 401.5, 402.0 at 1.81 min | ¹H NMR (500 MHz, DMSO-*d*6) δ 8.87 (s, 1H), 7.89 - 7.82 (m, 2H), 7.63 (dd, *J* = 5.0, 2.9 Hz, 1H), 7.55 (td, *J =* 3.9, 1.7 Hz, 3H), 7.53 - 7.46 (m, 2H), 7.22 (d, *J =* 8.5 Hz, 1H), 4.65 (s, 2H), 3.72 (t, *J* = 5.9 Hz, 2H), 2.90 (t, *J* = 6.0 Hz, 2H). |
| 128^{(a)} | 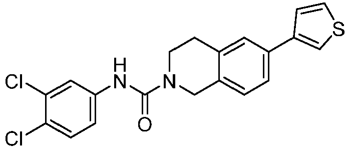 *N*-(3,4-Dichlorophenyl)-6-(thiophen-3-yl)-3,4-dihydroisoquinoline-2(1H)-carboxamide | 401.2, 403.2 at 1.84 min | ¹H NMR (500 MHz, DMSO-*d*6) δ 8.87 (s, 1H), 7.87 (d, *J =* 2.0 Hz, 1H), 7.55 - 7.46 (m, 6H), 7.23 (d, *J =* 7.9 Hz, 1H), 7.13 (dd, *J =* 5.1, 3.6 Hz, 1H), 4.65 (s, 2H), 3.72 (t, *J =* 5.9 Hz, 2H), 2.90 (t, *J =* 5.9 Hz, 2H). |

### Human GPR65 cyclic adenosine monophosphate (cAMP) Homogeneous Time Resolved Fluorescence (HTRF) antagonist assay procedure

IC₅₀ data was obtained by the following procedure:
1321N1 human astrocytoma cells stably expressing human recombinant GPR65 (1321N1-hrGPR65 cells, EuroscreenFast) were cultured according to the vendor's instructions.

Compounds were tested for their ability to antagonise GPR65, through measuring the concentration of cytoplasmic cAMP following treatment of the cells at a pH of 7.2 to activate GPR65 signalling and addition of the compound to be tested. The extent to which the expected rise in cAMP concentration upon GPR65 activation was suppressed by the added compound is indicative of its potency. The assay was carried out according to EuroscreenFast assay methodology as follows.

On the day of the assay, test compounds were added to 384-well, low volume, white microtiter plates by acoustic dispensing. KRH buffer (5 mM KCI, 1.25 mM MgSO₄, 124 mM NaCl, 25 mM HEPES, 13.3 mM Glucose, 1.25 mM KH₂PO₄ and 1.45 mM CaCl₂) was adjusted to pH 6.5, pH 7.6 and pH 8.4 by adding NaOH. 1321N1-hGPR65 cells were rapidly thawed and diluted in KRH, pH 7.6 prior to centrifugation at 300 xg for 5 min and resuspension in assay buffer (KRH, pH 7.6, supplemented with 1 mM 3-isobutyl-1-methylxanthine (IBMX) and 200 µM ethylenediaminetetraacetic acid (EDTA)). Cells were added to assay plates at a density of 2,000 cells per well in a volume of 5 µl. Assay plates were briefly centrifuged at 100 xg and then incubated at room temperature for 30 min. Cells were stimulated by the addition of 5 µL KRH, pH 6.5, to achieve an assay pH of 7.2, while control wells received 5 µl KRH, pH 8.4 to achieve an assay pH of 7.9. Assay plates were briefly centrifuged at 100 xg and then incubated at room temperature for 30 min.

Accumulation of cAMP was detected by cAMP HTRF kit (Cisbio). d2-labeled cAMP and cryptate-labeled anti-cAMP antibody in Lysis and Detection Buffer (Cisbio) were added to assay plates, and the plates were incubated at room temperature for 1 h. HTRF measurements were performed using a Pherastar FSX instrument. Acceptor and donor emission signals were measured at 665 nm and 620 nm, respectively, and HTRF ratios were calculated as signal_{665 nm}/signal₆₂₀ₙₘ x 10⁴. Data were normalised to high and low control values and fitted with 4-parameter logistic regression to determine hGPR65 IC50 values for the test compounds, which are shown in Table 1.

**Table 1: Activity of selected compounds according to the invention**

| | |
|---|---|
| **1** | Medium |
| **2** | Low |
| **3** | Low |
| **4** | Low |
| **5** | Medium |
| **6** | Medium |
| **7** | Medium |
| **8** | Low |
| **9** | Low |
| **10** | Medium |
| **11** | Medium |
| **12** | Medium |
| **13** | Medium |
| **14** | High |
| **15** | Medium |
| **16** | Medium |
| **17** | Medium |
| **18** | Low |
| **19** | Medium |
| **20** | Low |
| **21** | Low |
| **22** | Low |
| **23** | Low |
| **24** | Low |
| **25** | Medium |
| **26** | Medium |
| **27** | Medium |
| **28** | Low |
| **29** | Medium |
| **30** | High |
| **31** | High |
| **32** | Medium |
| **33** | High |
| **34** | Low |
| **35** | Medium |
| **36** | High |
| **37** | High |
| **38** | Medium |
| **39** | High |
| **42** | High |
| **43** | High |
| **44** | High |
| **45** | Medium |
| **46** | Low |
| **47** | Medium |
| **49** | Medium |
| **50** | Medium |
| **51** | Medium |
| **52** | High |
| **53** | High |
| **54** | High |
| **55** | Medium |
| **56** | Low |
| **57** | Medium |
| **58** | Medium |
| **59** | Medium |
| **60** | Medium |
| **61** | Medium |
| **62** | Low |
| **63** | Medium |
| **64** | Medium |
| **65** | Low |
| **66** | Medium |
| **67** | Medium |
| **70** | Low |
| **71** | Medium |
| **72** | Low |
| **74** | High |
| **76** | High |
| **77** | Low |
| **78** | Low |
| **79** | High |
| **80** | Medium |
| **81** | Medium |
| **82** | High |
| **83** | High |
| **84** | Low |
| **85** | High |
| **86** | Medium |
| **87** | Medium |
| **88** | Medium |
| **89** | Medium |
| **90** | Medium |
| **91** | Medium |
| **92** | Low |
| **93** | High |
| **94** | Low |
| **95** | Low |
| **96** | Medium |
| **97** | Low |
| **98** | Medium |
| **99** | Low |
| **100** | Low |
| **101** | Medium |
| **102** | Medium |
| **103** | Medium |
| **104** | High |
| **105** | Low |
| **106** | High |
| **107** | Low |
| **108** | High |
| **109** | High |
| **110** | Low |
| **111** | Low |
| **112** | High |
| **113** | High |
| **114** | High |
| **115** | Low |
| **116** | Low |
| **117** | High |
| **118** | Medium |
| **119** | Low |
| **120** | High |
| **121** | Low |
| **122** | Low |
| **123** | High |
| **124** | Low |
| **125** | Low |
| **126** | Medium |
| **127** | Low |
| **128** | Low |
| **129** | Medium |
| **130** | Low |
| **131** | Medium |
| **132** | Medium |
| **133** | Medium |
| **134** | Medium |
| **135** | Medium |
| **136** | Medium |
| **137** | Medium |
| **138** | Medium |
| **139** | Medium |
| **140** | High |

High = IC50 < 500 nM; Medium = IC50 > 500 nM and < 5 µM; Low = IC50 > 5 µM.

### REFERENCES

Bohn, T. et al. (2018). Tumor immunoevasion via acidosis-dependent induction of regulatory tumor-associated macrophages. Nature Immunology, 1319-1326.
Damaghi, M. et al. (2013). pH Sensing and Regulation in Cancer. Frontiers in Physiology.
Gaublomme, J. et al. (2015). Single-Cell Genomics Unveils Critical Regulators of Th17 Cell Pathogenicity. Cell, 1400-1412.
Hernandez, J. (2018). GPR65, a critical regulator of Th17 cell pathogenicity, is regulated by the CRTC2/CREB pathway. The Journal of Immunology, 200 (Supplement).
Korn, T. et al. (2009). IL-17 and Th17 Cells. Annual Reviews in Immunology, 485-517.
Wang, J. et al. (2004). TDAG8 is a proton-sensing and psychosine-sensitive G-protein-coupled receptor. Journal of Biological Chemistry, 45626-45633.
Yoshida, N. et al. (2016). ICER is requisite for Th17 differentiation. Nature Communications, 12993.
Hardin, M. et al. (2014). The clinical and genetic features of COPD-asthma overlap syndrome. Eur Respir J. 2014 Aug;44(2):341-50.
Kottyan, L. et al. (2009). Eosinophil viability is increased by acidic pH in a cAMP- and GPR65-dependent manner. Blood. 2009 Sep 24;114(13):2774-82.
Tsurumaki, H. et al (2015). Int J Mol Sci. Protective Role of Proton-Sensing TDAG8 in Lipopolysaccharide-Induced Acute Lung Injury. Dec 4;16(12):28931-42

## Claims

1. A compound of formula (la), or a pharmaceutically acceptable salt or solvate thereof, 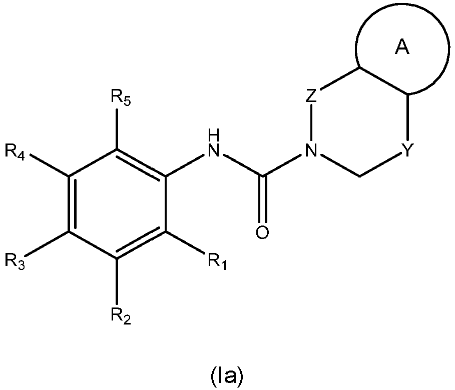 wherein:
ring A is fused to the ring containing N, Z and Y, where ring A is selected from benzene, pyridine, pyridone, pyridine N-oxide, pyridazine, pyrimidine and pyrazine, each of which is optionally substituted with one or more substituents selected from F, Cl, Br, I, CN, OH, SO₂-alkyl, alkyl, haloalkyl, aralkyl, and aryl, and wherein said aryl substituent is in turn optionally substituted with one or more substituents each independently selected from F, Cl, Br, I, CN, alkoxy, NR₁₁R₁₁', OH, alkyl, haloalkyl, and aralkyl;
Y and Z are each independently CR₁₀R₁₀', wherein R₁₀ and R₁₀' are each independently selected from H, F, alkyl, and haloalkyl;
R₁, R₄, and R₅ are each independently selected from H, F, Cl, Br, I and haloalkyl; and
R₂ and R₃ are each independently selected from Cl, Br, I, and haloalkyl;
R₁₁ and R₁₁' are each independently selected from H, alkyl, haloalkyl, COR₁₂, and SO₂R₁₃, wherein R₁₂ and R₁₃ are both alkyl;
wherein the compound is other than:
*N*-(3,4-Dichlorophenyl)-7,8-dihydropyrido[4,3-d]pyrimidine-6(5H)-carboxamide;
*N*-(3,4-Dichlorophenyl)-5,8-dihydropyrido[3,4-d]pyrimidine-7(6H)-carboxamide;
*N*-(4-Chloro-3-(trifluoromethyl)phenyl)-3,4-dihydroisoquinoline-2(1H)-carboxamide; and
*N*-(3,4-dichlorophenyl)-3,4-dihydroisoquinoline-2(1H)-carboxamide.

2. A compound according to claim 1, wherein ring A is optionally substituted with one or more substituents selected from F, Cl, Br, I, CN, OH, C₁-C₆ alkyl, phenyl, SO₂-alkyl, and C₁-C₆ haloalkyl, most preferably wherein the one or more substituents are selected from F, Cl, Br, I, CN, C₁-C₆ OH, C₁-C₆ alkyl, SO₂-alkyl, and C₁-C₆ haloalkyl.

3. A compound of formula (Ia"), or a pharmaceutically acceptable salt or solvate thereof, 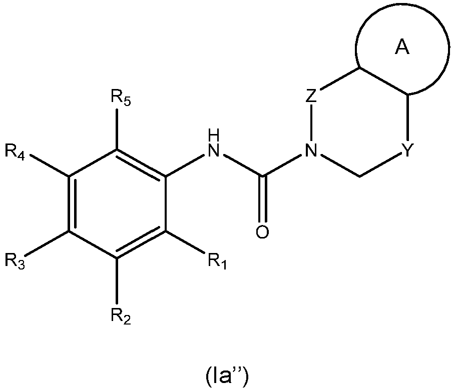 wherein ring A 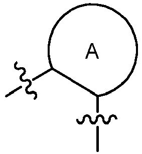 is selected from: 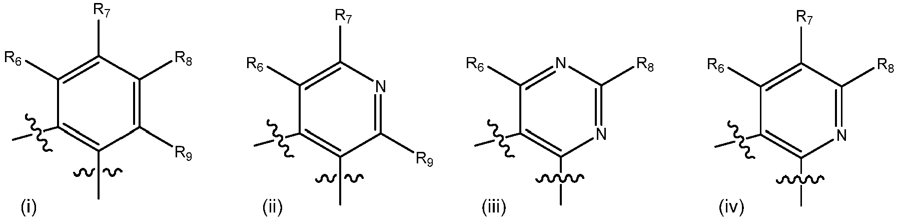 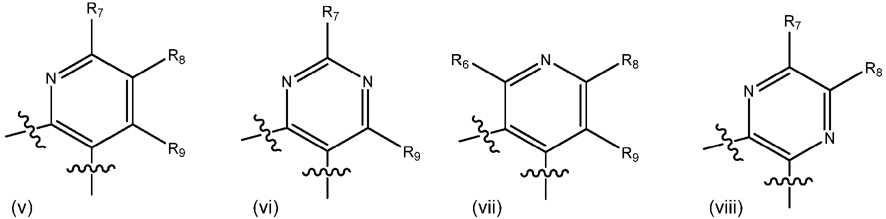 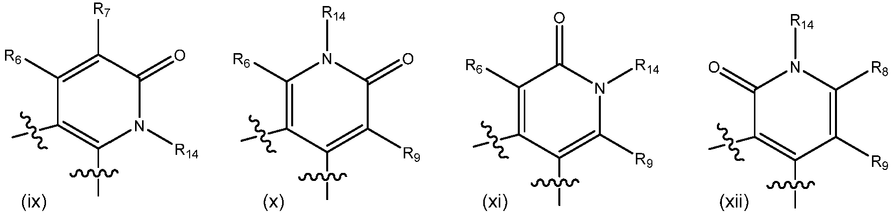 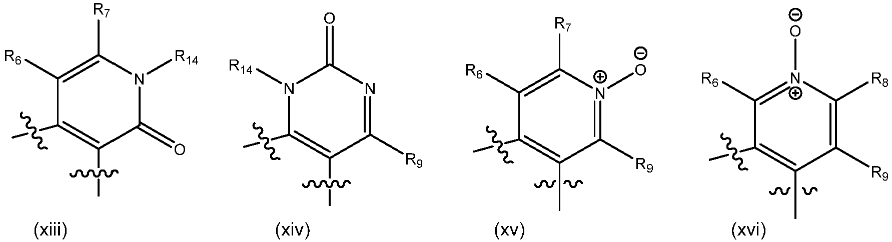 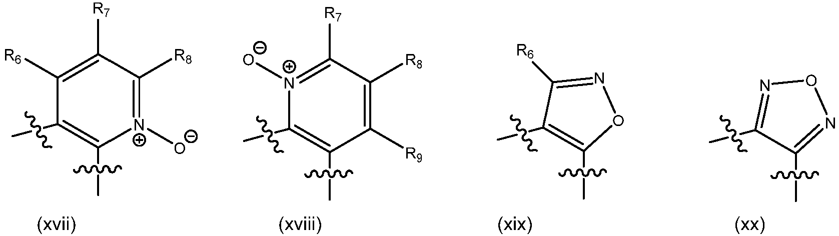 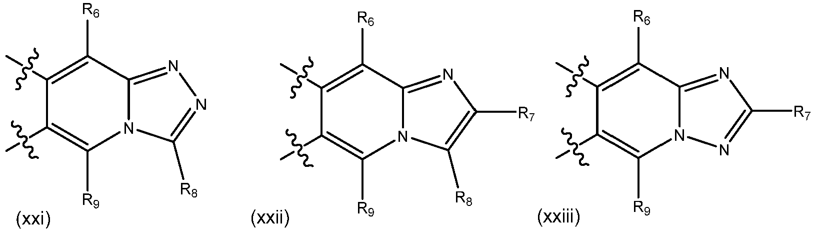 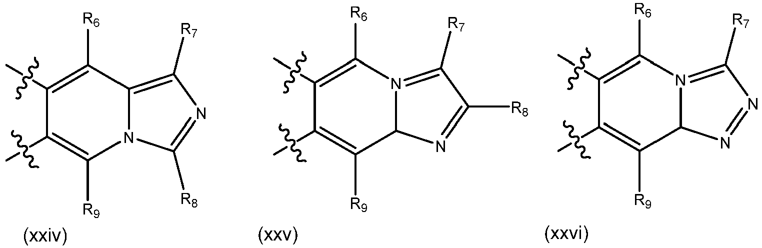 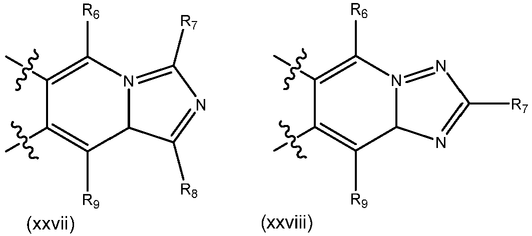 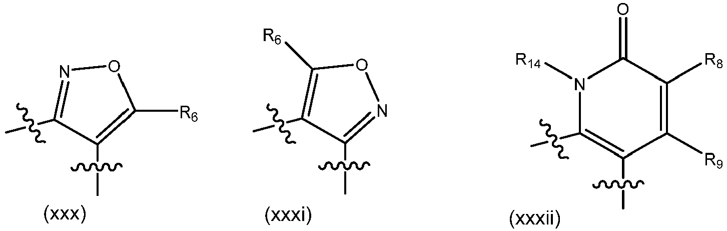 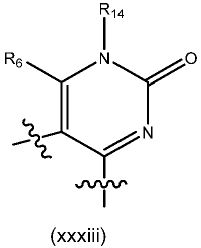 where the wavy lines denote attachment of ring A to the ring containing N, Z and Y: 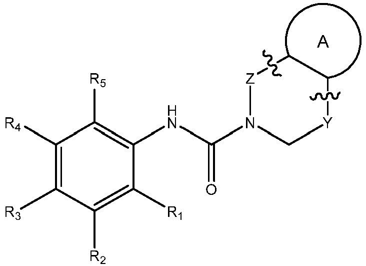 and wherein:
Y and Z are each independently CR₁₀R₁₀', wherein R₁₀ and R₁₀' are each independently selected from H, F, alkyl, and haloalkyl;
R₁, R₄, and R₅ are each independently selected from H, F, Cl, Br, I and haloalkyl;
R₂ and R₃ are each independently selected from Cl, Br, I, and haloalkyl;
wherein R₆, R₇, R₈, and R₉ are each independently selected from H, F, Cl, Br, I, CN, SO₂-alkyl, OH, C₁-C₆ alkyl, phenyl, and C₁-C₆ haloalkyl, and R₁₄ is H or alkyl; and
wherein the compound is other than:
*N*-(3,4-Dichlorophenyl)-7,8-dihydropyrido[4,3-d]pyrimidine-6(5H)-carboxamide;
*N*-(3,4-Dichlorophenyl)-5,8-dihydropyrido[3,4-d]pyrimidine-7(6H)-carboxamide;
*N*-(4-Chloro-3-(trifluoromethyl)phenyl)-3,4-dihydroisoquinoline-2(1H)-carboxamide;
*N*-(3,4-dichlorophenyl)-3,4-dihydroisoquinoline-2(1H)-carboxamide; and
*N*-(3,4-Dichlorophenyl)-6,7-dihydroisoxazolo[4,5-c]pyridine-5(4H)-carboxamide.

4. A compound according to claim 3, wherein ring A is selected from:
- groups (i)-(viii), (ix), (xi), and (xxxii);
- groups (i)-(x); or
- groups (i), (ii), (vii) and (x).

5. A compound according to any preceding claim wherein Y and Z are each independently selected from CH₂, CHMe, CF₂, C(CH₃)₂, C(CF₃)₂, and are preferably both CH₂.

6. A compound according to any preceding claim, wherein R₁ is selected from H, haloalkyl and F, and is preferably H.

7. A compound according to any preceding claim, wherein R₂ and R₃ are each independently selected from Cl, Br, and CFₙH₃₋ₙ, where n is 1, 2, or 3, more preferably wherein R₂ and R₃ are each independently selected from Cl and CF₃, and most preferably wherein one of R₂ and R₃ is Cl and the other is CF₃, or R₂ and R₃ are both Cl.

8. A compound according to any preceding claim, wherein R₄ is selected from H and Cl, and is preferably H.

9. A compound according to any preceding claim, wherein R₅ is H or CF₃, more preferably H.

10. A compound according to any of claims 3-9, wherein R₆ is selected from H, F, Cl, CN, CH₃, and CF₃, preferably wherein R₆ is selected from H, F, Cl, CN, and CH₃, and is more preferably H.

11. A compound according to any of claims 3-10, wherein R₇ is selected from H, F, Cl, CN, CH₃, and CF₃, most preferably H, F, and Cl, and is most preferably H.

12. A compound according to any of claims 3-11, wherein R₈ is selected from H, F, OH, CN, phenyl, CF₃, and CF₂H, preferably wherein R₈ is selected from H, F, Cl, CN, CH₃, and CF₃, more preferably from H, F, Cl, and CN, and most preferably from F and Cl, in particular F.

13. A compound according to any of claims 3-12, wherein R₉ is selected from H, F, Cl, CN, CH₃ and CF₃, more preferably H, F, and CN, and R₉ is most preferably H.

14. A compound according to any one of claims 1 to 13, which is selected from the following:
| | | | |
|---|---|---|---|
| 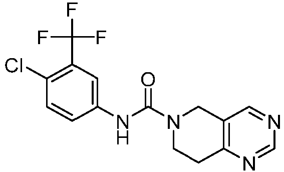 | (5) | 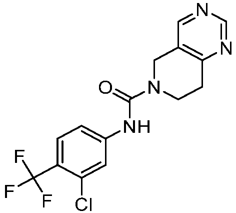 | (6) |
| 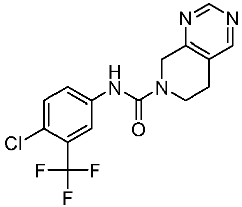 | (7) | 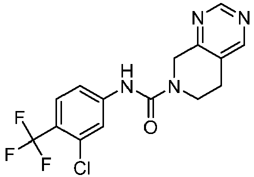 | (9) |
| 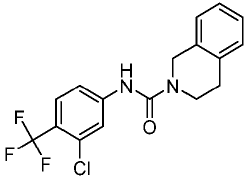 | (10) | 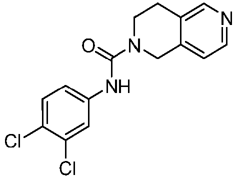 | (12) |
| 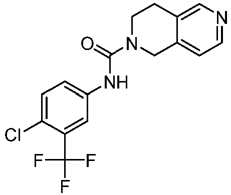 | (13) | 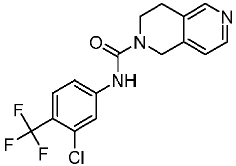 | (14) |
| 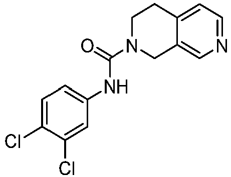 | (15) | 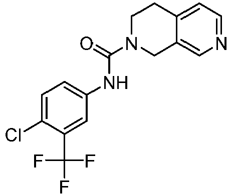 | (16) |
| 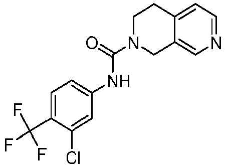 | (17) | 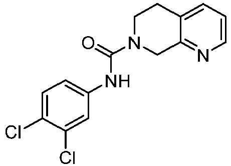 | (18) |
| 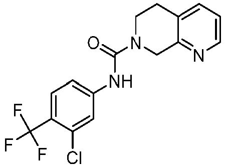 | (19) | 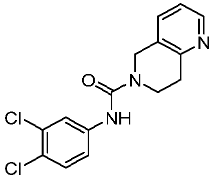 | (20) |
| 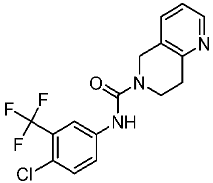 | (21) | 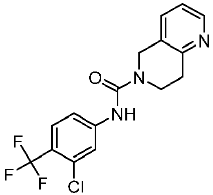 | (22) |
| 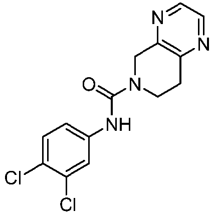 | (23) | 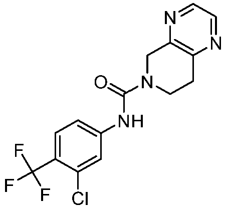 | (24) |
| 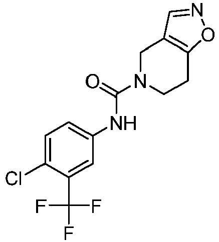 | (25) | 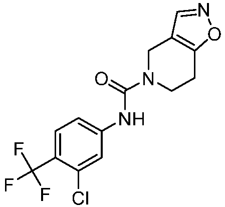 | (26) |
| 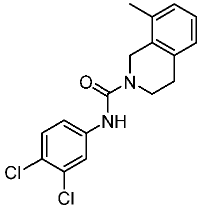 | (27) | 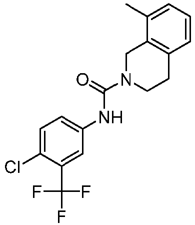 | (28) |
| 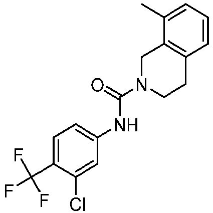 | (29) | 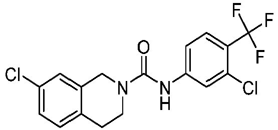 | (30) |
| 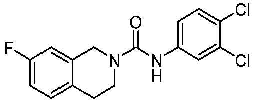 | (31) | 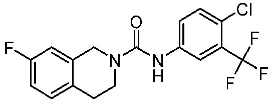 | (32) |
| 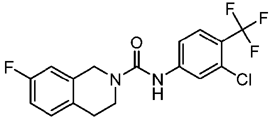 | (33) | 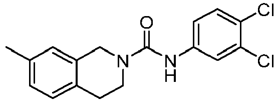 | (34) |
| 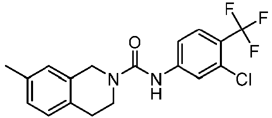 | (35) | 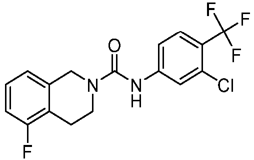 | (36) |
| 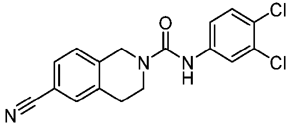 | (37) | 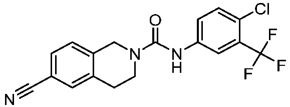 | (38) |
| 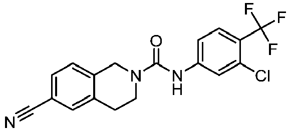 | (39) | 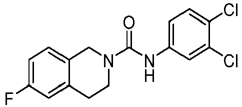 | (42) |
| 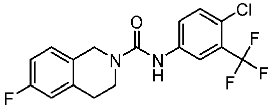 | (43) | 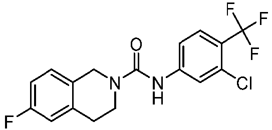 | (44) |
| 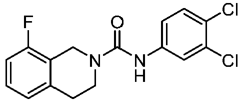 | (45) | 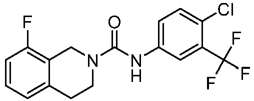 | (46) |
| 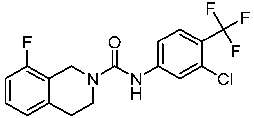 | (47) | | |
| 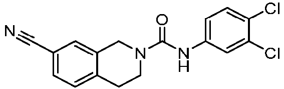 | (49) | 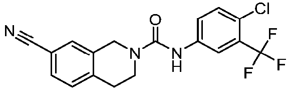 | (50) |
| 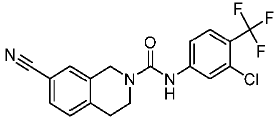 | (51) | 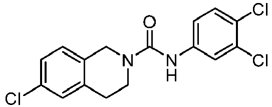 | (52) |
| 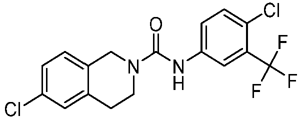 | (53) | 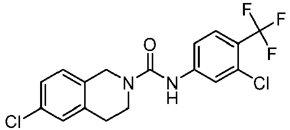 | (54) |
| 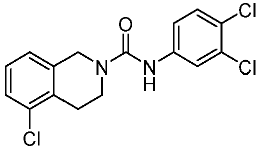 | (55) | 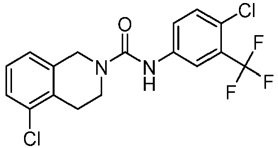 | (56) |
| 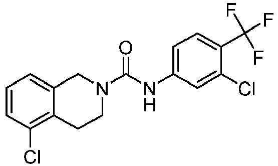 | (57) | 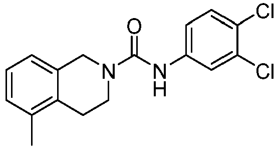 | (58) |
| 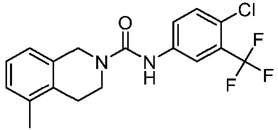 | (59) | 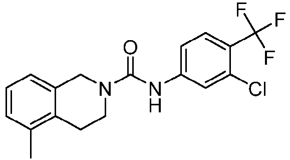 | (60) |
| | | 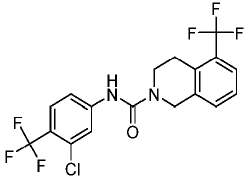 | (70) |
| 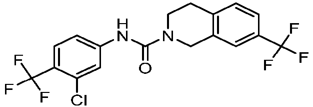 | (71) | 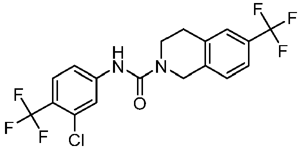 | (72) |
| 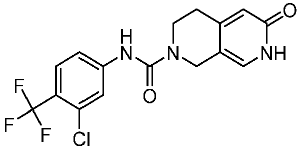 | (74) | 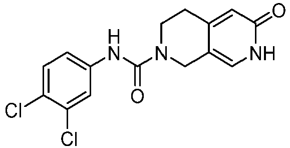 | (76) |
| 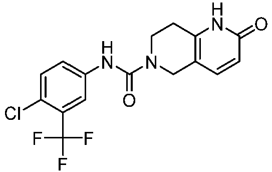 | (77) | 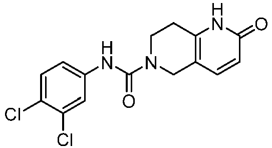 | (78) |
| 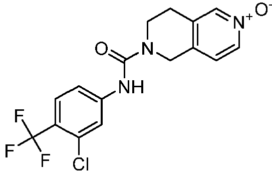 | (79) | | |
| 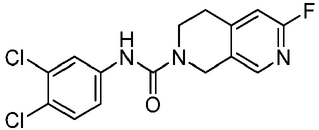 | (85) | 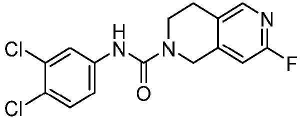 | (86) |
| 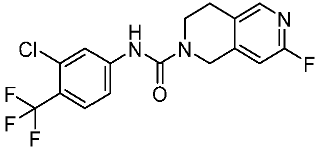 | (87) | 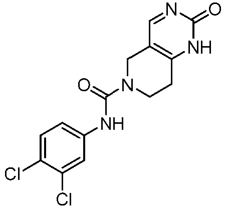 | (88) |
| 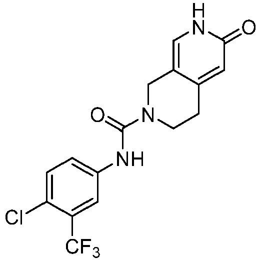 | (89) | 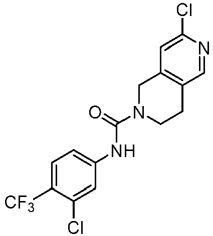 | (90) |
| 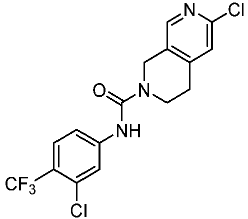 | (91) | 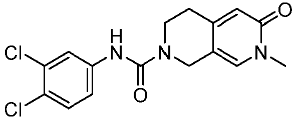 | (92) |
| 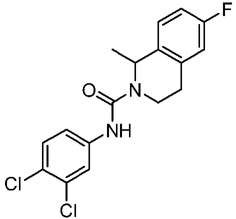 | (93) | 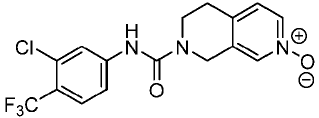 | (94) |
| 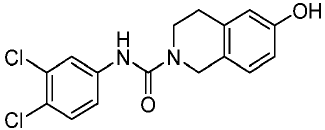 | (95) | 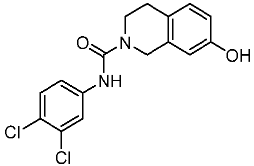 | (96) |
| 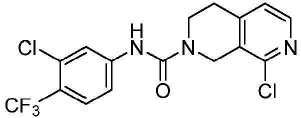 | (97) | 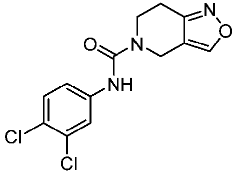 | (98) |
| 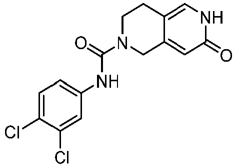 | (99) | 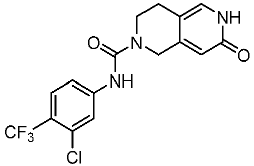 | (100) |
| 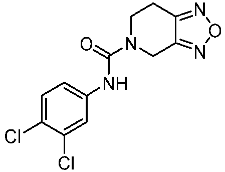 | (101) | 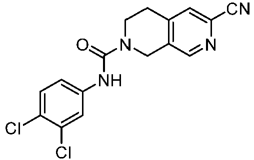 | (102) |
| 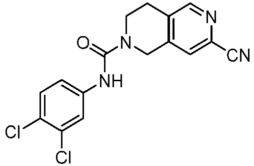 | (103) | 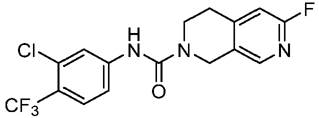 | (104) |
| 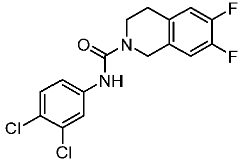 | (106) | 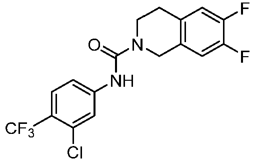 | (108) |
| 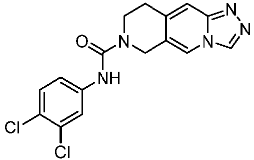 | (109) | 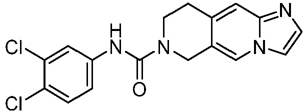 | (110) |
| 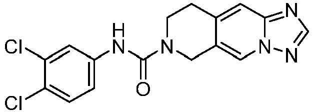 | (111) | 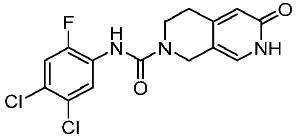 | (112) |
| 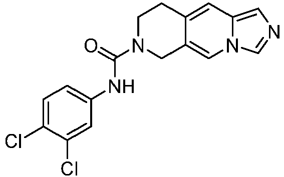 | (113) | | |
| 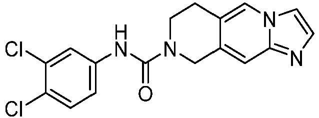 | (115) | 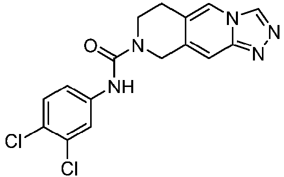 | (116) |
| 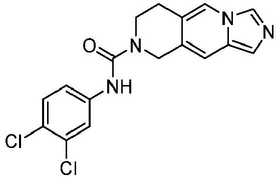 | (117) | 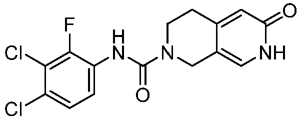 | (118) |
| 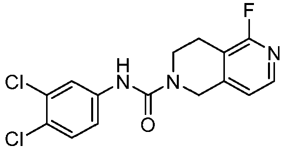 | (120) | 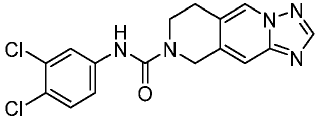 | (121) |
| 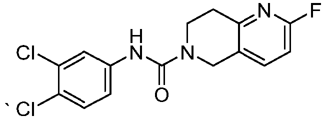 | (123) | 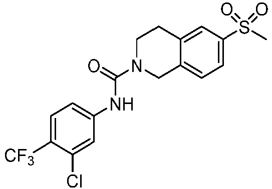 | (124) |
| 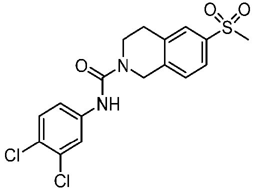 | (125) | | |
| | ) | | |
| | | 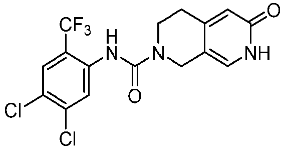 | (130) |
| | | 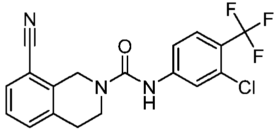 | (132) |
| 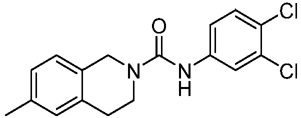 | (135) | 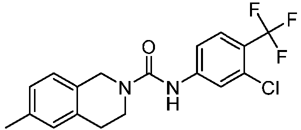 | (136) |
| 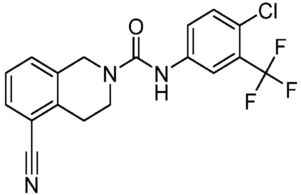 | (137) | 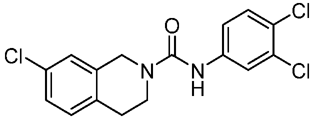 | (138) |
| 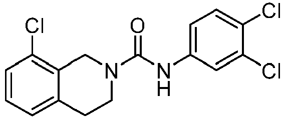 | (139) | | |
and pharmaceutically acceptable salts and solvates thereof.

15. A compound of formula (I), or a pharmaceutically acceptable salt or solvate thereof, 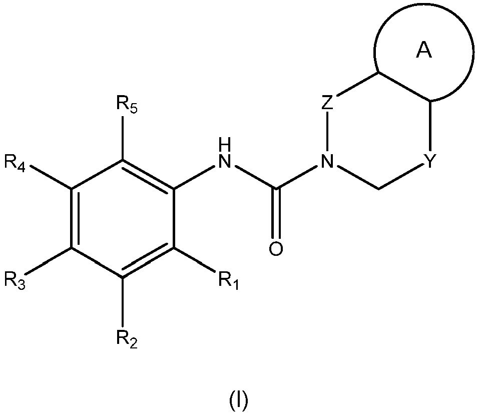 wherein:
ring A is a 5- or 6-membered monocyclic aromatic or heteroaromatic ring, or a 9- or 10-membered bicyclic aromatic or heteroaromatic ring, each of which is optionally substituted with one or more substituents selected from F, Cl, Br, I, CN, alkoxy, NR₁₁R₁₁', OH, SO₂-alkyl, CO₂-alkyl, alkyl, haloalkyl, aralkyl, aryl, and heteroaryl, and wherein said aryl and heteroaryl substituents are in turn optionally substituted with one or more substituents each independently selected from F, Cl, Br, I, CN, alkoxy, NR₁₁R₁₁', OH, alkyl, haloalkyl, and aralkyl;
Y and Z are each independently CR₁₀R₁₀', wherein R₁₀ and R₁₀' are each independently selected from H, F, alkyl, and haloalkyl;
R₁, R₄, and R₅ are each independently selected from H, F, Cl, Br, I and haloalkyl;
R₂ and R₃ are each independently selected from Cl, Br, I, and haloalkyl;
and
R₁₁ and R₁₁' are each independently selected from H, alkyl, haloalkyl, COR₁₂, and SO₂R₁₃, wherein R₁₂ and R₁₃ are both alkyl;
for use in treating or preventing a disorder selected from a proliferative disorder, an immune disorder, asthma, chronic obstructive pulmonary disease (COPD) and acute respiratory distress syndrome (ARDS), wherein the compound modulates GPR65, preferably wherein the compound inhibits GPR65 signalling.

16. A compound for use according to claim 15, further defined according to any one of claims 1 to 14.

17. A compound selected from the following:
| | | | |
|---|---|---|---|
| 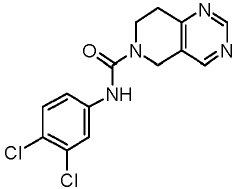 | (1) | 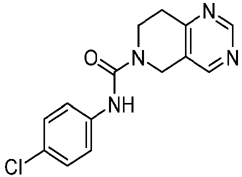 | (2) |
| 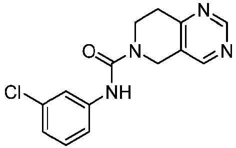 | (3) | 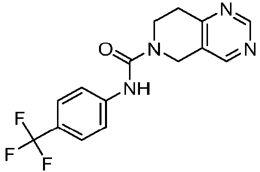 | (4) |
| 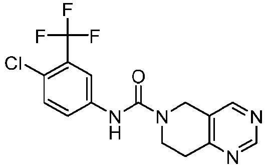 | (5) | 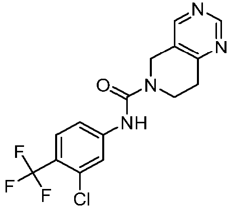 | (6) |
| 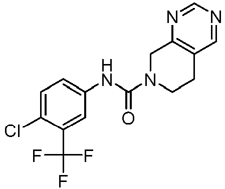 | (7) | 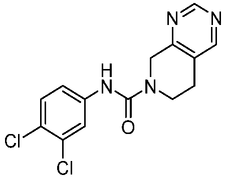 | (8) |
| 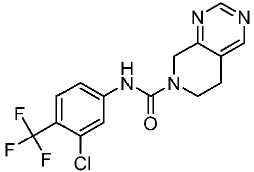 | (9) | 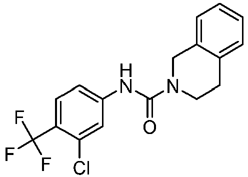 | (10) |
| 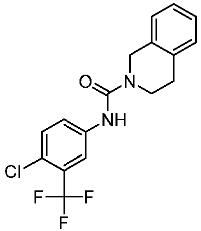 | (11) | 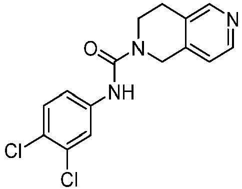 | (12) |
| 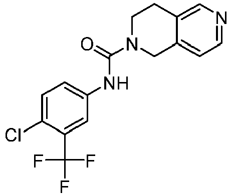 | (13) | 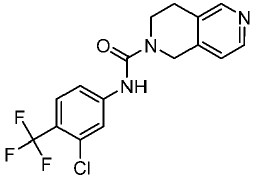 | (14) |
| 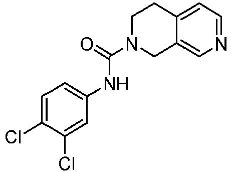 | (15) | 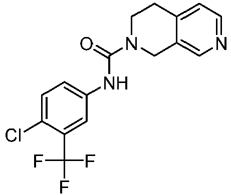 | (16) |
| 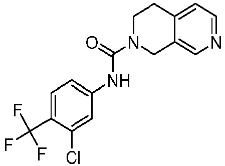 | (17) | 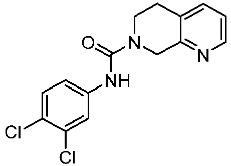 | (18) |
| 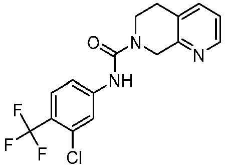 | (19) | 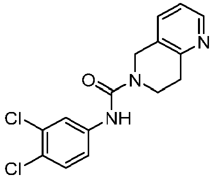 | (20) |
| 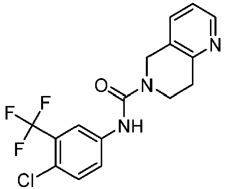 | (21) | 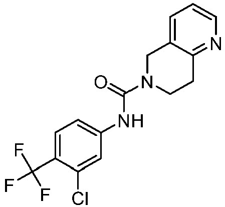 | (22) |
| 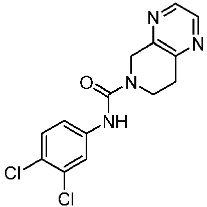 | (23) | 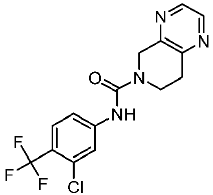 | (24) |
| 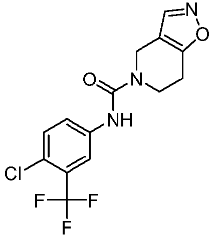 | (25) | 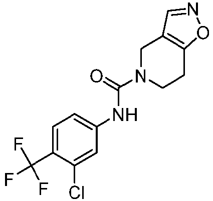 | (26) |
| 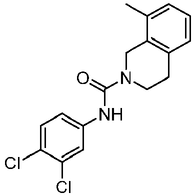 | (27) | 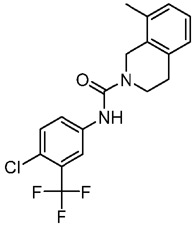 | (28) |
| 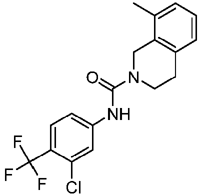 | (29) | 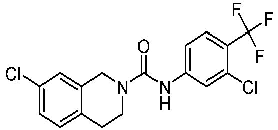 | (30) |
| 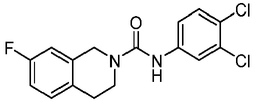 | (31) | 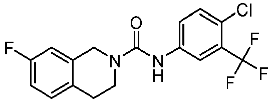 | (32) |
| 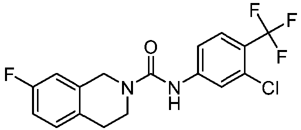 | (33) | 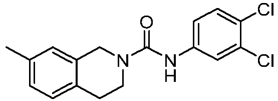 | (34) |
| 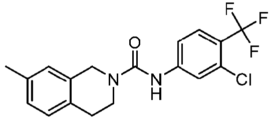 | (35) | 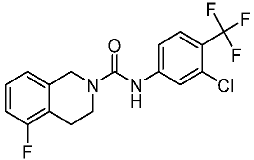 | (36) |
| 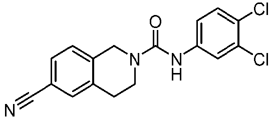 | (37) | 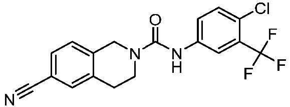 | (38) |
| 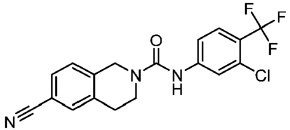 | (39) | 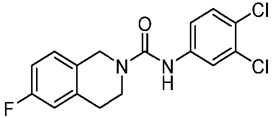 | (42) |
| 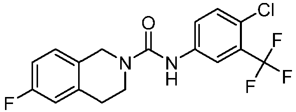 | (43) | 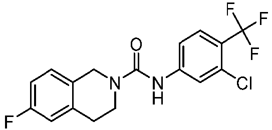 | (44) |
| 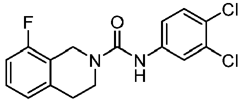 | (45) | 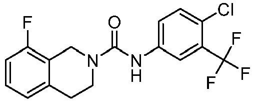 | (46) |
| 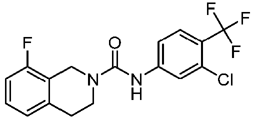 | (47) | | |
| 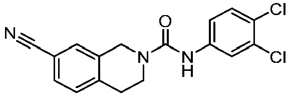 | (49) | 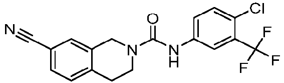 | (50) |
| 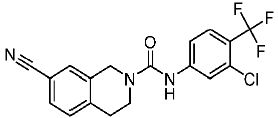 | (51) | 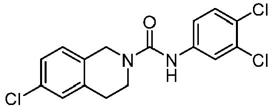 | (52) |
| 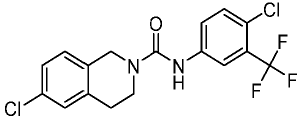 | (53) | 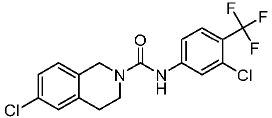 | (54) |
| 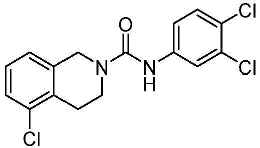 | (55) | 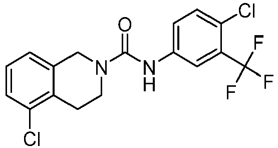 | (56) |
| 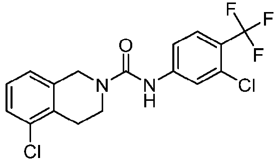 | (57) | 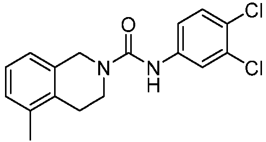 | (58) |
| 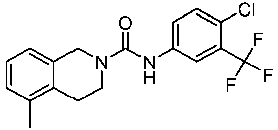 | (59) | 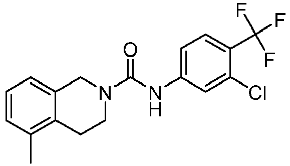 | (60) |
| 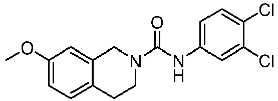 | (61) | 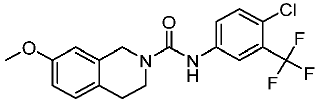 | (62) |
| 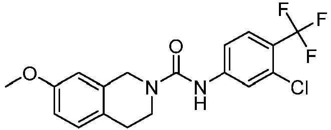 | (63) | 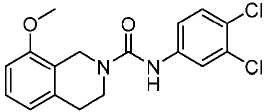 | (64) |
| 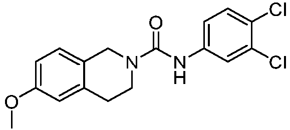 | (65) | 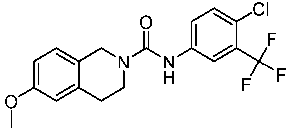 | (66) |
| 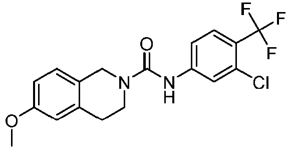 | (67) | 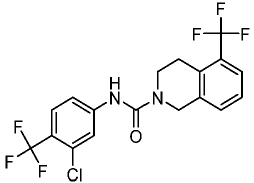 | (70) |
| 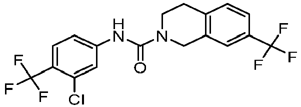 | (71) | 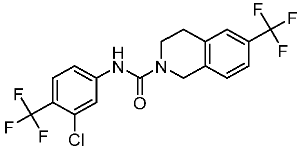 | (72) |
| 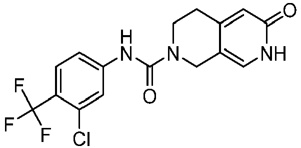 | (74) | 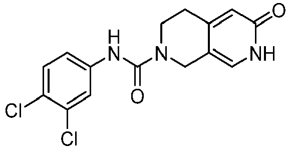 | (76) |
| 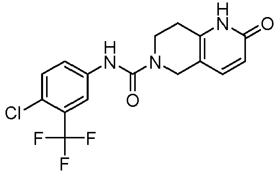 | (77) | 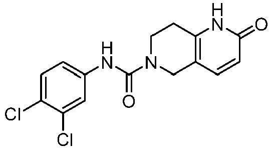 | (78) |
| 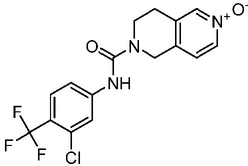 | (79) | 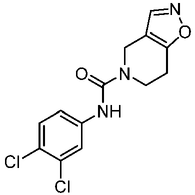 | (80) |
| 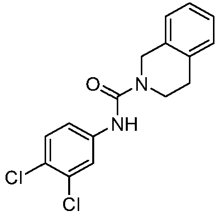 | (81) | 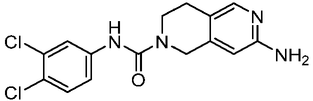 | (82) |
| 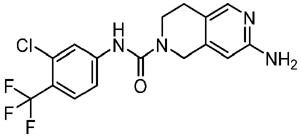 | (83) | 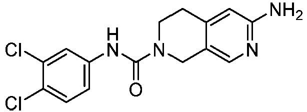 | (84) |
| 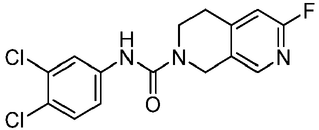 | (85) | 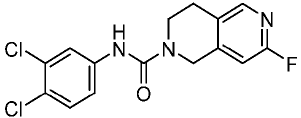 | (86) |
| 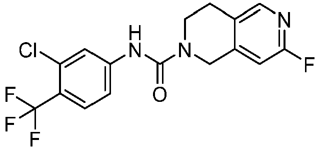 | (87) | 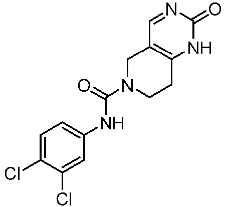 | (88) |
| 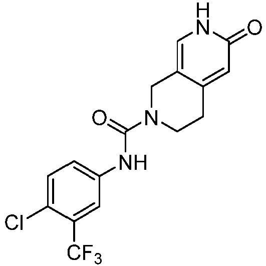 | (89) | 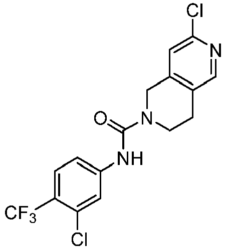 | (90) |
| 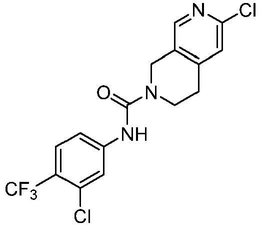 | (91) | 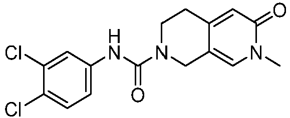 | (92) |
| 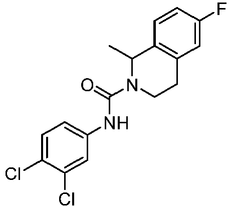 | (93) | 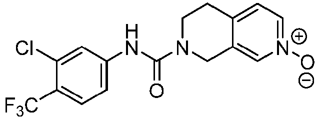 | (94) |
| 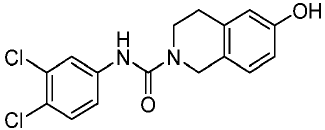 | (95) | 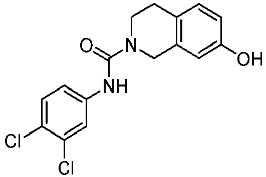 | (96) |
| 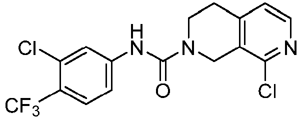 | (97) | 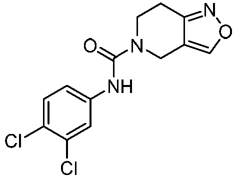 | (98) |
| 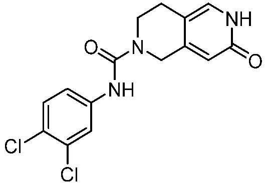 | (99) | 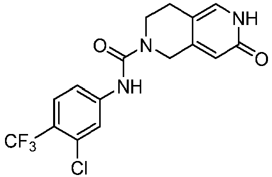 | (100) |
| 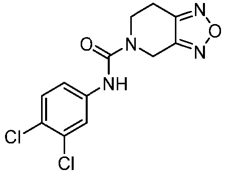 | (101) | 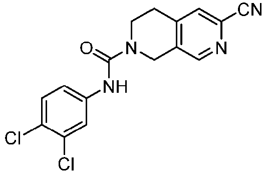 | (102) |
| 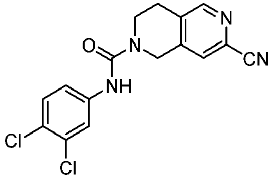 | (103) | 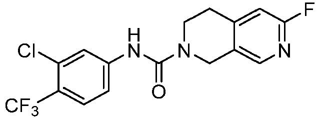 | (104) |
| 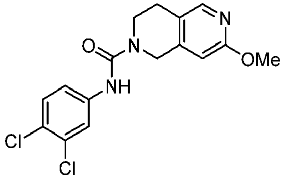 | (105) | 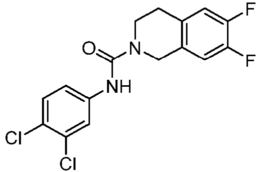 | (106) |
| 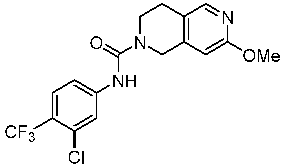 | (107) | 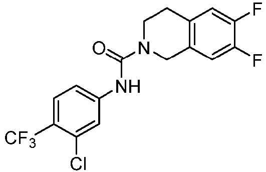 | (108) |
| 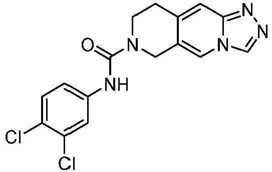 | (109) | 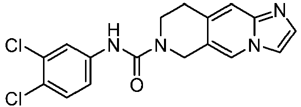 | (110) |
| 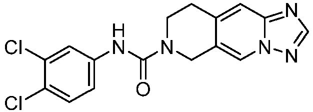 | (111) | 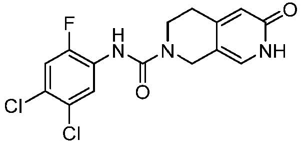 | (112) |
| 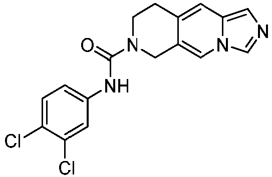 | (113) | 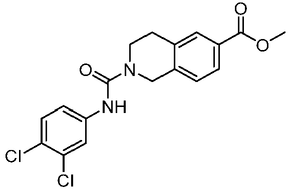 | (114) |
| 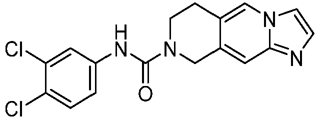 | (115) | 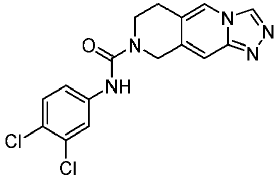 | (116) |
| 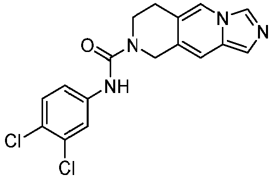 | (117) | 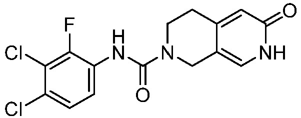 | (118) |
| 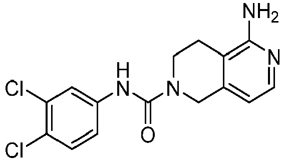 | (119) | 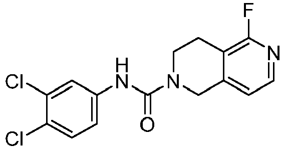 | (120) |
| 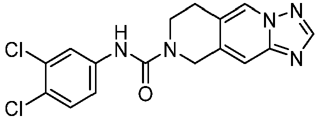 | (121) | 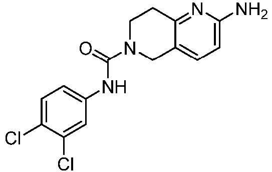 | (122) |
| 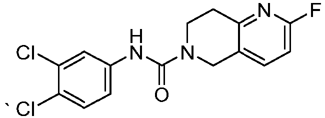 | (123) | 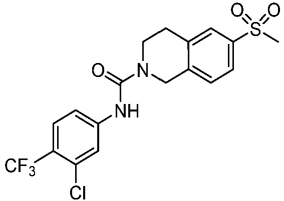 | (124) |
| 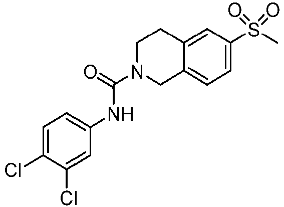 | (125) | 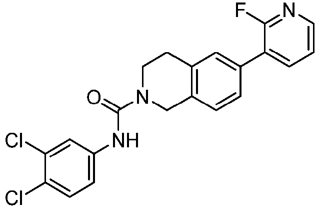 | (126) |
| 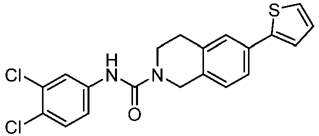 | (127) | 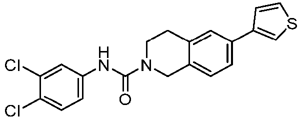 | (128) |
| 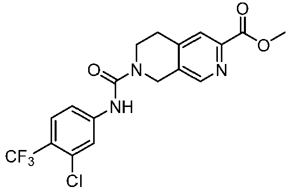 | (129) | 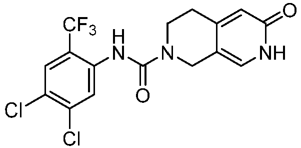 | (130) |
| 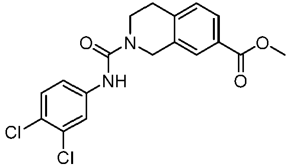 | (131) | 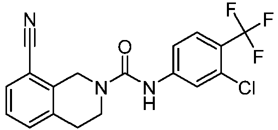 | (132) |
| 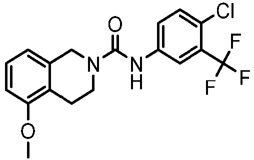 | (133) | 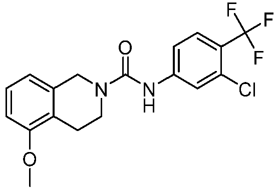 | (134) |
| 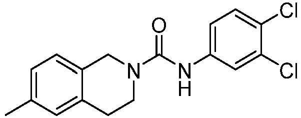 | (135) | 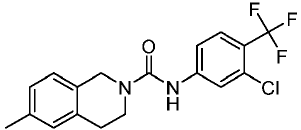 | (136) |
| 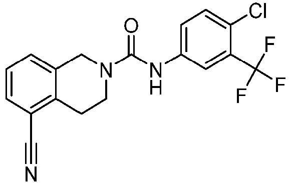 | (137) | 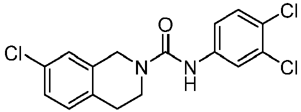 | (138) |
| 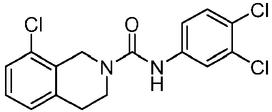 | (139) | 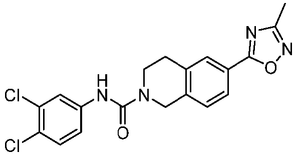 | (140) |
and pharmaceutically acceptable salts and solvates thereof, for use as a medicament.

18. A pharmaceutical composition comprising a compound, or a pharmaceutically acceptable salt or solvate thereof, as defined according to any one of claims 1-14, and a pharmaceutically acceptable diluent, excipient, or carrier.

19. A pharmaceutical composition comprising a compound, or a pharmaceutically acceptable salt or solvate thereof, as defined according to any one of claims 1-14, and a pharmaceutically acceptable diluent, excipient, or carrier, for use in treating or preventing a disorder selected from a proliferative disorder, an immune disorder, asthma, chronic obstructive pulmonary disease (COPD) and acute respiratory distress syndrome (ARDS), wherein the compound modulates GPR65, preferably wherein the compound inhibits GPR65 signalling.

20. A compound for use according to claim 17 or a pharmaceutical composition for use according to claim 19, wherein the disorder is a proliferative disorder, preferably wherein the proliferative disorder is a cancer that is preferably a solid tumour and/or metastases thereof, or a cancer selected from melanoma, renal cell carcinoma (RCC), gastric cancer, acute myeloid leukaemia (AML), pancreatic adenocarcinoma, triple negative breast cancer (TNBC), colorectal cancer, head and neck cancer, colorectal adenocarcinoma, lung cancer, sarcoma, ovarian cancer, and glioma, more preferably glioblastoma (GBM).

21. A compound for use according to claim 17 or a pharmaceutical composition for use according to claim 19, wherein the disorder is an immune disorder, preferably wherein the immune disorder is an autoimmune disease, more preferably wherein the autoimmune disease is selected from psoriasis, psoriatic arthritis, rheumatoid arthritis (RA), multiple sclerosis (MS), systemic lupus erythematosus (SLE), autoimmune thyroiditis (Hashimoto's thyroiditis), Graves' disease, uveitis (including intermediate uveitis), ulcerative colitis, Crohn's disease, autoimmune uveoretinitis, systemic vasculitis, polymyositis-dermatomyositis, systemic sclerosis (scleroderma), Sjogren's Syndrome, ankylosing spondylitis and related spondyloarthropathies, sarcoidosis, autoimmune hemolytic anemia, immunological platelet disorders, and autoimmune polyendocrinopathies, and most preferably wherein the autoimmune disease is selected from psoriasis, psoriatic arthritis, ankylosing spondylitis, Crohn's disease, and multiple sclerosis.

22. A compound for use according to claim 17 or a pharmaceutical composition for use according to claim 19 wherein the use comprises treating or preventing a disorder selected from asthma, chronic obstructive pulmonary disease (COPD) and acute respiratory distress syndrome (ARDS).

23. A compound of formula (la), (la"), (I) as defined in any one of claims 1-17, or a pharmaceutically acceptable salt or solvate thereof, for use in treating or preventing a GPR65-associated disease or disorder.

## Patentansprüche

1. Verbindung der Formel (Ia) oder pharmazeutisch unbedenkliches Salz oder Solvat davon, 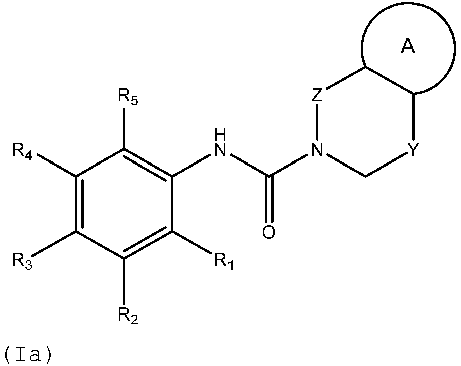 wobei:
Ring A an den N, Z und Y enthaltenden Ring anelliert ist, wobei Ring A aus Benzol, Pyridin, Pyridon, Pyridin-N-oxid, Pyridazin, Pyrimidin und Pyrazin ausgewählt ist, wobei jeder dieser Ringe gegebenenfalls durch einen oder mehrere Substituenten, die aus F, Cl, Br, I, CN, OH, SO₂-Alkyl, Alkyl, Halogenalkyl, Aralkyl und Aryl ausgewählt sind, substituiert ist und wobei der Arylsubstituent wiederum gegebenenfalls durch einen oder mehrere Substituenten, die jeweils unabhängig aus F, Cl, Br, I, CN, Alkoxy, NR₁₁R₁₁', OH, Alkyl, Halogenalkyl und Aralkyl ausgewählt sind, substituiert ist;
Y und Z jeweils unabhängig für CR₁₀R₁₀' stehen, wobei R₁₀ und R₁₀' jeweils unabhängig aus H, F, Alkyl und Halogenalkyl ausgewählt sind;
R₁, R₄ und R₅ jeweils unabhängig aus H, F, Cl, Br, I und Halogenalkyl ausgewählt sind; und
R₂ und R₃ jeweils unabhängig aus Cl, Br, I und Halogenalkyl ausgewählt sind;
R₁₁ und R₁₁' jeweils unabhängig aus H, Alkyl, Halogenalkyl, COR₁₂ und SO₂R₁₃ ausgewählt sind, wobei R₁₂ und R₁₃ beide für Alkyl stehen;
wobei die Verbindung verschieden ist von:
N-(3,4-Dichlorphenyl)-7,8-dihydropyrido[4,3-d]pyrimidin-6(5H)-carboxamid;
N-(3,4-Dichlorphenyl)-5,8-dihydropyrido[3,4-d]pyrimidin-7(6H)-carboxamid;
N-(4-Chlor-3-(trifluormethyl)phenyl)-3,4-dihydroisochinolin-2(1H)-carboxamid und
N-(3,4-Dichlorphenyl)-3,4-dihydroisochinolin-2(1H)-carboxamid.

2. Verbindung nach Anspruch 1, wobei Ring A gegebenenfalls durch einen oder mehrere Substituenten, die aus F, Cl, Br, I, CN, OH, C₁-C₆-Alkyl, Phenyl, SO₂-Alkyl und C₁-C₆-Halogenalkyl ausgewählt sind, substituiert ist, ganz besonders bevorzugt wobei der eine oder die mehreren Substituenten aus F, Cl, Br, I, CN, C₁-C₆ OH, C₁-C₆-Alkyl, SO₂-Alkyl und C₁-C₆-Halogenalkyl ausgewählt sind.

3. Verbindung der Formel (Ia") oder pharmazeutisch unbedenkliches Salz oder Solvat davon, 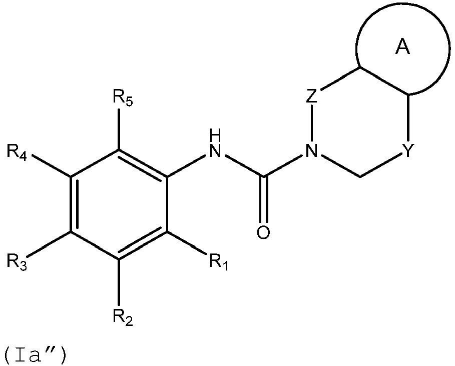 wobei Ring A 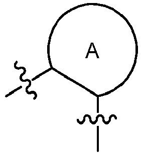 ausgewählt ist aus: 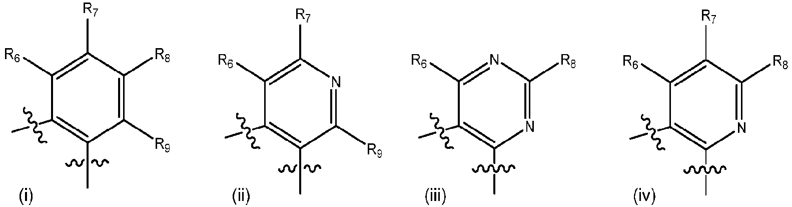 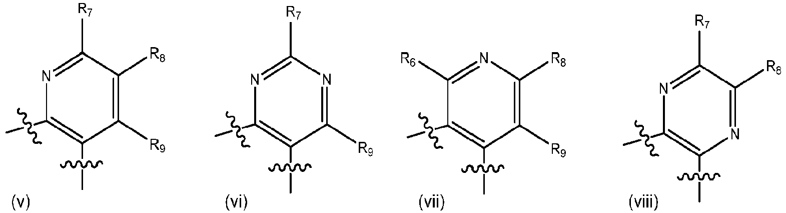 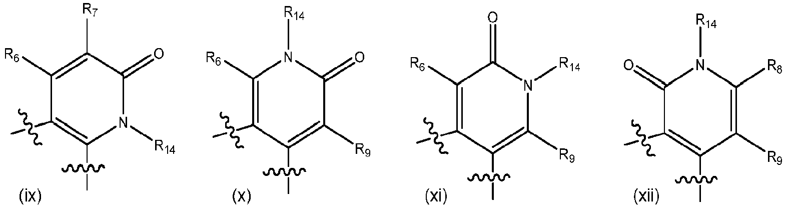 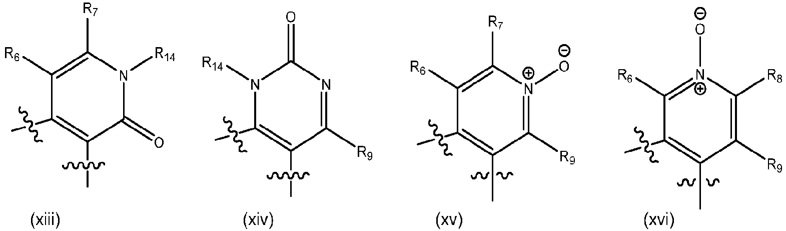 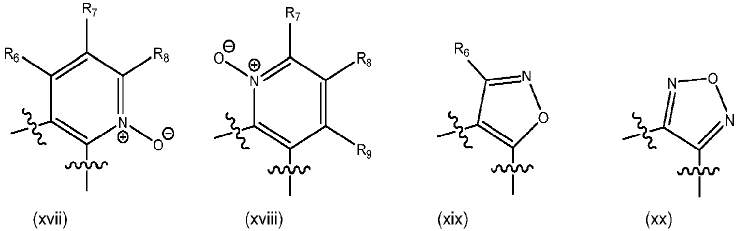 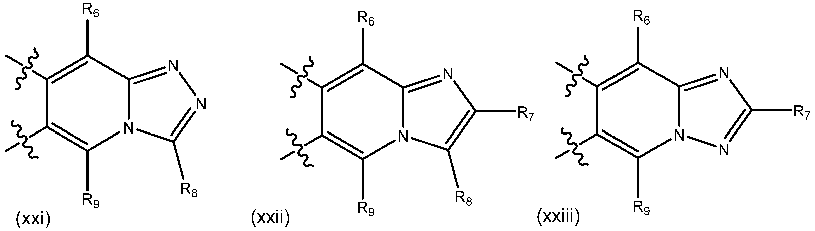 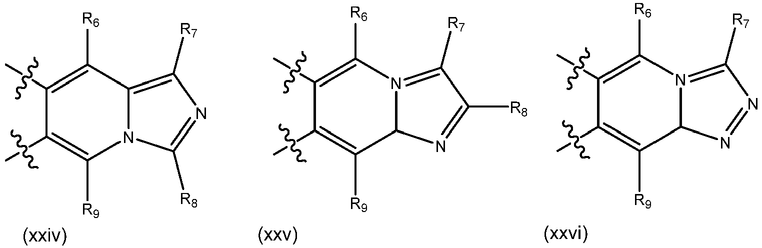 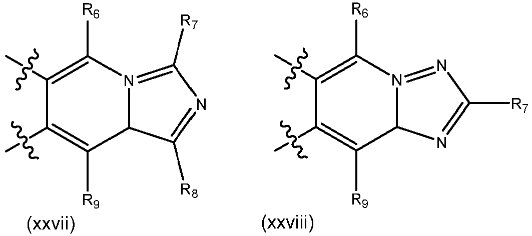 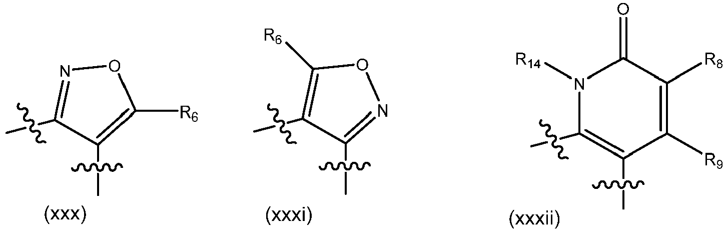 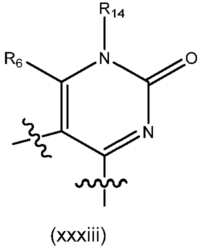 wobei die Wellenlinien die Anbindung von Ring A an den N, Z und Y enthaltenden Ring anzeigen: 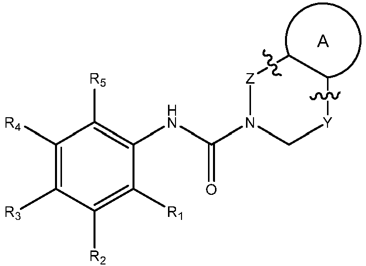 und wobei:
Y und Z jeweils unabhängig für CR₁₀R₁₀' stehen, wobei R₁₀ und R₁₀' jeweils unabhängig aus H, F, Alkyl und Halogenalkyl ausgewählt sind;
R₁, R₄ und R₅ jeweils unabhängig aus H, F, Cl, Br, I und Halogenalkyl ausgewählt sind;
R₂ und R₃ jeweils unabhängig aus Cl, Br, I und Halogenalkyl ausgewählt sind;
wobei R₆, R₇, R₈ und R₉ jeweils unabhängig aus H, F, Cl, Br, I, CN, SO₂-Alkyl, OH, C₁-C₆-Alkyl, Phenyl und C₁-C₆-Halogenalkyl ausgewählt sind und R₁₄ für H oder Alkyl steht; und
wobei die Verbindung verschieden ist von:
N-(3,4-Dichlorphenyl)-7,8-dihydropyrido[4,3-d]pyrimidin-6(5H)-carboxamid;
N-(3,4-Dichlorphenyl)-5,8-dihydropyrido[3,4-d]pyrimidin-7(6H)-carboxamid;
N-(4-Chlor-3-(trifluormethyl)phenyl)-3,4-dihydroisochinolin-2(1H)-carboxamid;
N-(3,4-Dichlorphenyl)-3,4-dihydroisochinolin-2(1H)-carboxamid; und
N-(3,4-Dichlorphenyl)-6,7-dihydroisoxazolo[4,5-c]pyridin-5(4H)-carboxamid.

4. Verbindung nach Anspruch 3, wobei Ring A ausgewählt ist aus:
- Gruppen (i)-(viii), (ix), (xi) und (xxxii);
- Gruppen (i)-(x) oder
- Gruppen (i), (ii), (vii) und (x).

5. Verbindung nach einem der vorhergehenden Ansprüche, wobei Y und Z jeweils unabhängig aus CH₂, CHMe, CF₂, C(CH₃)₂, C(CF₃)₂ ausgewählt sind und vorzugsweise beide für CH₂ stehen.

6. Verbindung nach einem der vorhergehenden Ansprüche, wobei R₁ aus H, Halogenalkyl und F ausgewählt ist und vorzugsweise für H steht.

7. Verbindung nach einem der vorhergehenden Ansprüche, wobei R₂ und R₃ jeweils unabhängig aus Cl, Br und CFₙH₃₋ₙ, wobei n für 1, 2 oder 3 steht, ausgewählt sind, weiter bevorzugt wobei R₂ und R₃ jeweils unabhängig aus Cl und CF₃ ausgewählt sind und am ganz besonders bevorzugt wobei eines von R₂ und R₃ für Cl steht und das andere für CF₃ steht oder R₂ und R₃ beide für Cl stehen.

8. Verbindung nach einem der vorhergehenden Ansprüche, wobei R₄ aus H und Cl ausgewählt ist und vorzugsweise für H steht.

9. Verbindung nach einem der vorhergehenden Ansprüche, wobei R₅ für H oder CF₃, weiter bevorzugt H, steht.

10. Verbindung nach einem der Ansprüche 3-9, wobei R₆ aus H, F, Cl, CN, CH₃ und CF₃ ausgewählt ist, vorzugsweise wobei R₆ aus H, F, Cl, CN und CH₃ ausgewählt ist und weiter bevorzugt für H steht.

11. Verbindung nach einem der Ansprüche 3-10, wobei R₇ ausgewählt ist aus H, F, Cl, CN, CH₃, und CF₃, ganz besonders bevorzugt H, F und Cl, ausgewählt ist und ganz besonders bevorzugt für H steht.

12. Verbindung nach einem der Ansprüche 3-11, wobei R₈ aus H, F, OH, CN, Phenyl, CF₃ und CF₂H ausgewählt ist, vorzugsweise wobei R₈ aus H, F, Cl, CN, CH₃ und CF₃, weiter bevorzugt aus H, F, Cl und CN und ganz besonders bevorzugt aus F und Cl, insbesondere F, ausgewählt ist.

13. Verbindung nach einem der Ansprüche 3-12, wobei R₉ aus H, F, Cl, CN, CH₃ und CF₃, weiter bevorzugt ist H, F und CN, ausgewählt ist und R₉ ganz besonders bevorzugt für H steht.

14. Verbindung nach einem der Ansprüche 1 bis 13, die aus den Folgenden ausgewählt ist:
| | | | |
|---|---|---|---|
| 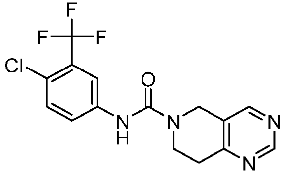 | (5) | 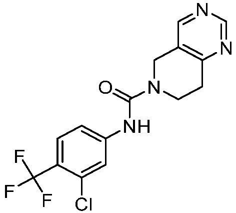 | (6) |
| 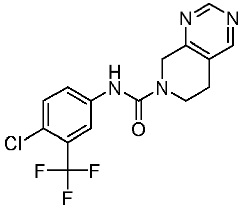 | (7) | 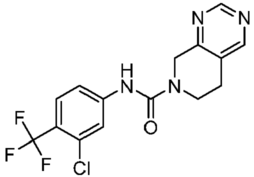 | (9) |
| 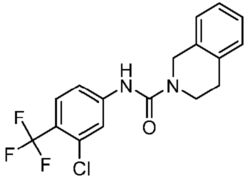 | (10) | 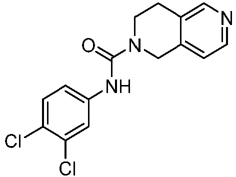 | (12) |
| 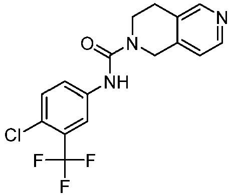 | (13) | 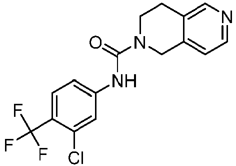 | (14) |
| 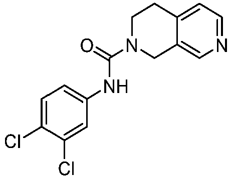 | (15) | 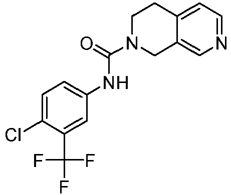 | (16) |
| 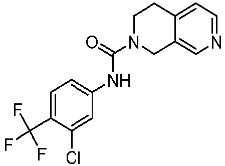 | (17) | 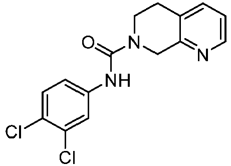 | (18) |
| 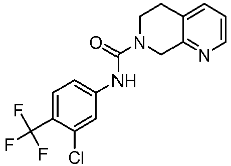 | (19) | 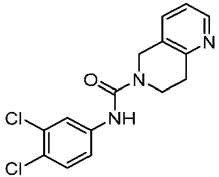 | (20) |
| 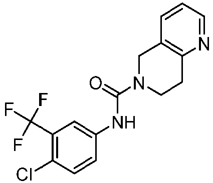 | (21) | 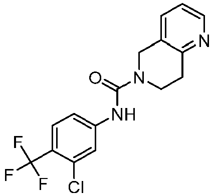 | (22) |
| 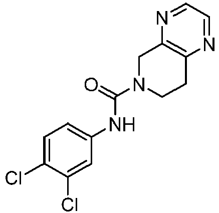 | (23) | 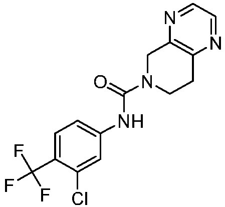 | (24) |
| 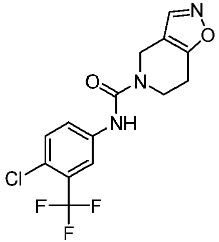 | (25) | 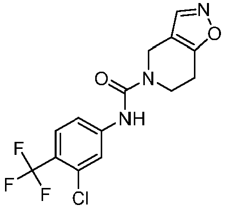 | (26) |
| 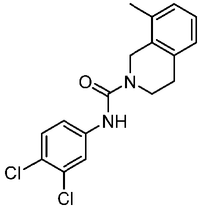 | (27) | 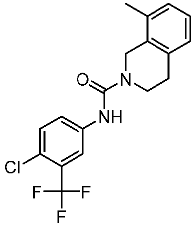 | (28) |
| 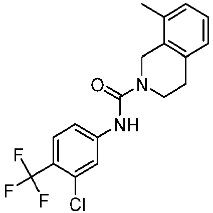 | (29) | 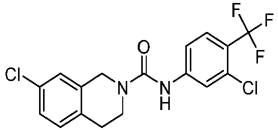 | (30) |
| 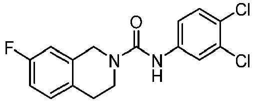 | (31) | 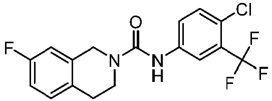 | (32) |
| 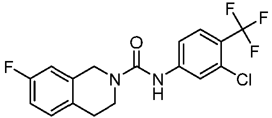 | (33) | 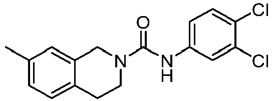 | (34) |
| 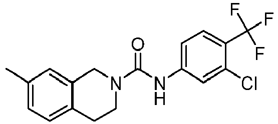 | (35) | 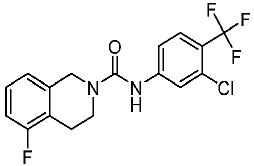 | (36) |
| 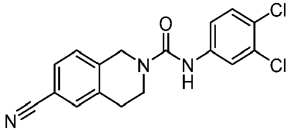 | (37) | 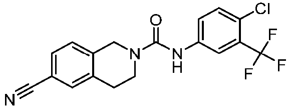 | (38) |
| 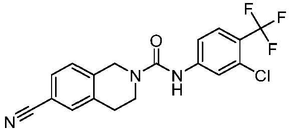 | (39) | 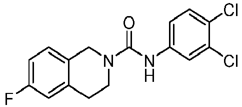 | (42) |
| 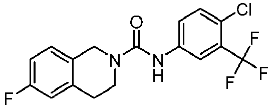 | (43) | 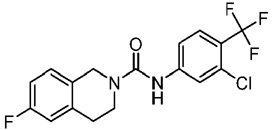 | (44) |
| 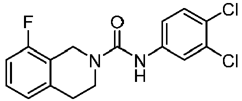 | (45) | 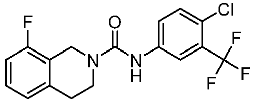 | (46) |
| 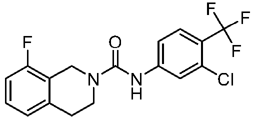 | (47) | | |
| 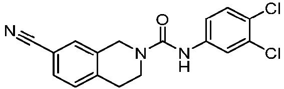 | (49) | 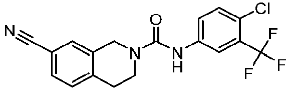 | (50) |
| 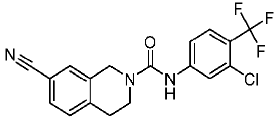 | (51) | 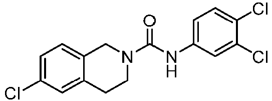 | (52) |
| 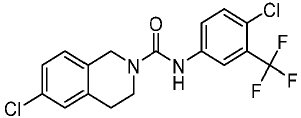 | (53) | 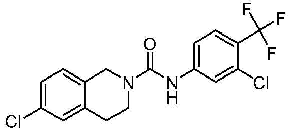 | (54) |
| 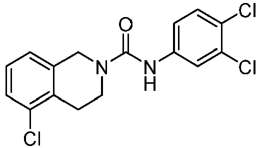 | (55) | 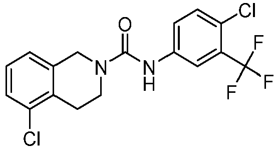 | (56) |
| 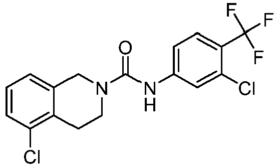 | (57) | 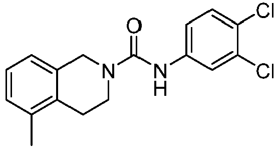 | (58) |
| 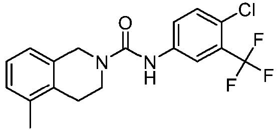 | (59) | 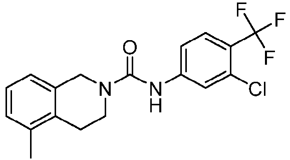 | (60) |
| | | 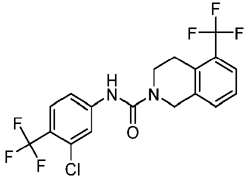 | (70) |
| 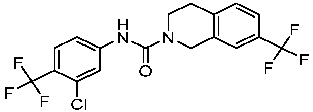 | (71) | 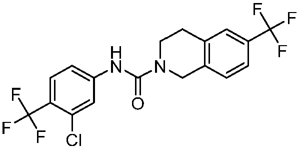 | (72) |
| 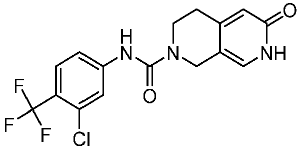 | (74) | 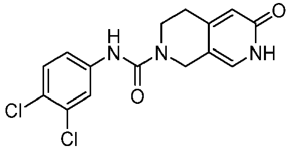 | (76) |
| 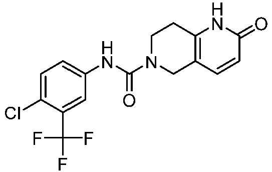 | (77) | 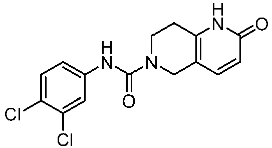 | (78) |
| 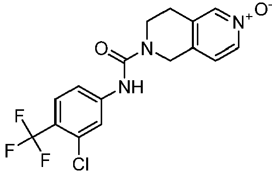 | (79) | | |
| 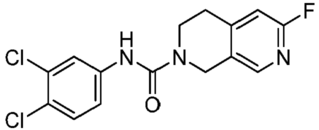 | (85) | 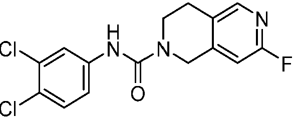 | (86) |
| 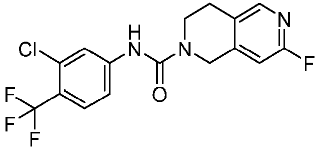 | (87) | 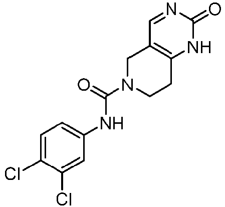 | (88) |
| 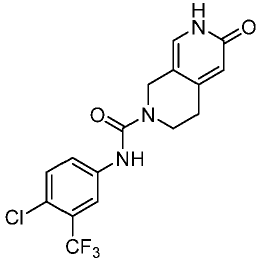 | (89) | 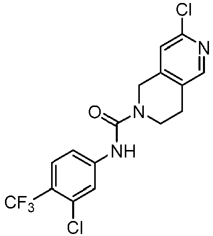 | (90) |
| 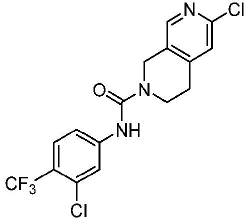 | (91) | 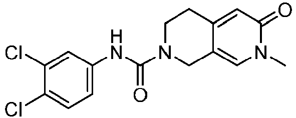 | (92) |
| 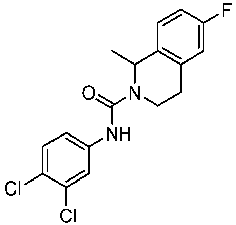 | (93) | 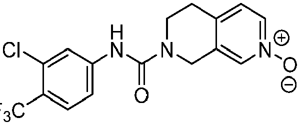 | (94) |
| 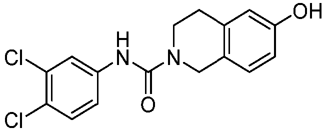 | (95) | 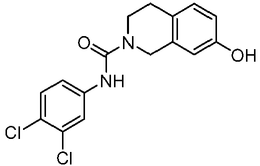 | (96) |
| 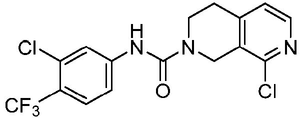 | (97) | 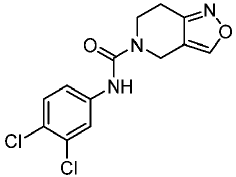 | (98) |
| 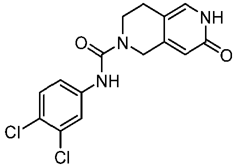 | (99) | 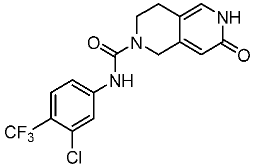 | (100) |
| 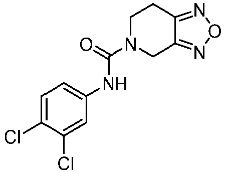 | (101) | 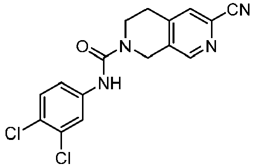 | (102) |
| 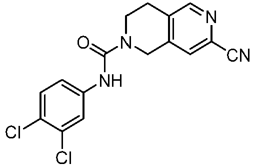 | (103) | 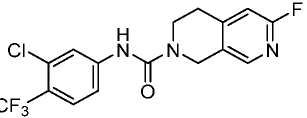 | (104) |
| 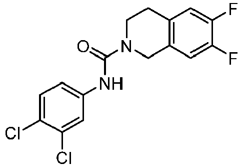 | (106) | 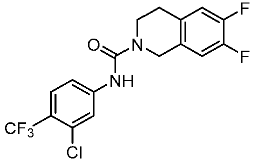 | (108) |
| 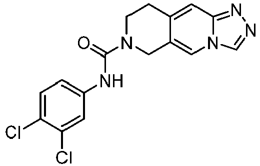 | (109) | 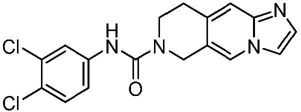 | (110) |
| 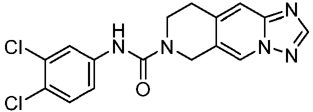 | (111) | 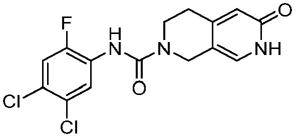 | (112) |
| 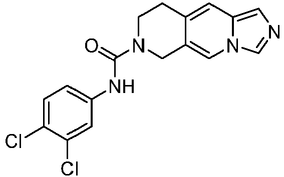 | (113) | | |
| 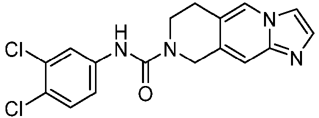 | (115) | 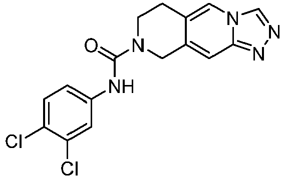 | (116) |
| 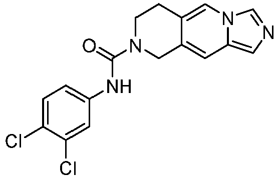 | (117) | 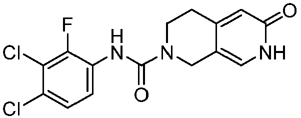 | (118) |
| 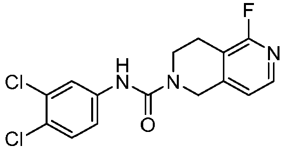 | (120) | 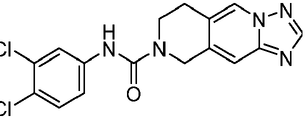 | (121) |
| 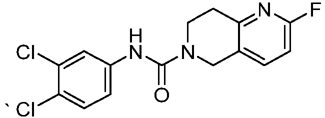 | (123) | 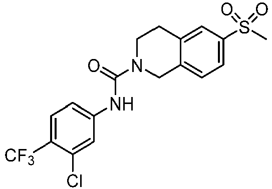 | (124) |
| 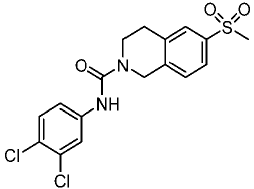 | (125) | | |
| | ) | | |
| | | 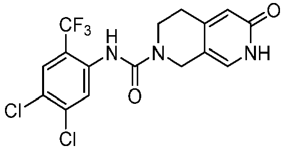 | (130) |
| | | 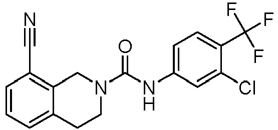 | (132) |
| 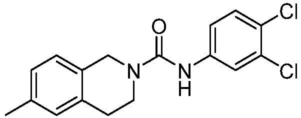 | (135) | 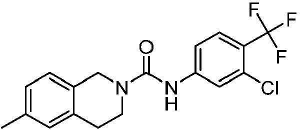 | (136) |
| 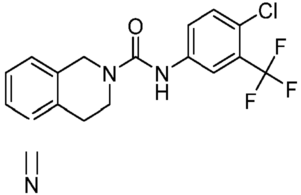 | (137) | 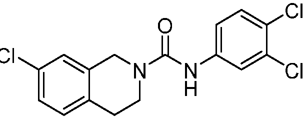 | (138) |
| 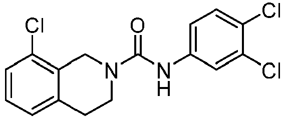 | (139) | | |
und pharmazeutisch unbedenkliche Salze und Solvate davon.

15. Verbindung der Formel (I), oder ein pharmazeutisch unbedenkliches Salz oder Solvat davon, 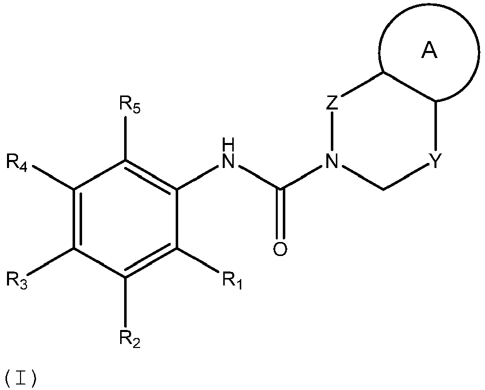 wobei:
Ring A für einen 5- oder 6-gliedrigen monocyclischen aromatischen oder heteroaromatischen Ring oder einen 9- oder 10-gliedrigen bicyclischen aromatischen oder heteroaromatischen Ring steht, der jeweils gegebenenfalls durch einen oder mehreren Substituenten, die aus F, Cl, Br, I, CN, Alkoxy, NR₁₁R₁₁', OH, SO₂-Alkyl, CO₂-Alkyl, Alkyl, Halogenalkyl, Aralkyl, Aryl und Heteroaryl ausgewählt sind, substituiert ist, und wobei die Aryl- und Heteroarylsubstituenten wiederum gegebenenfalls durch einen oder mehrere Substituenten, die jeweils unabhängig aus F, Cl, Br, I, CN, Alkoxy, NR₁₁R₁₁', OH, Alkyl, Halogenalkyl und Aralkyl ausgewählt sind, substituiert sind;
Y und Z jeweils unabhängig für CR₁₀R₁₀' stehen, wobei R₁₀ und R₁₀' jeweils unabhängig aus H, F, Alkyl und Halogenalkyl ausgewählt sind;
R₁, R₄ und R₅ jeweils unabhängig aus H, F, Cl, Br, I und Halogenalkyl ausgewählt sind;
R₂ und R₃ jeweils unabhängig aus Cl, Br, I und Halogenalkyl ausgewählt sind;
und
R₁₁ und R₁₁' jeweils unabhängig aus H, Alkyl, Halogenalkyl, COR₁₂ und SO₂R₁₃ ausgewählt sind, wobei R₁₂ und R₁₃ beide für Alkyl stehen;
zur Verwendung bei der Behandlung oder Prävention einer Störung, die aus einer Proliferationsstörung, einer Immunstörung, Asthma, chronisch-obstruktiver Lungenerkrankung (COPD) und akutem Atemnotsyndrom (ARDS) ausgewählt ist, wobei die Verbindung GPR65 moduliert, vorzugsweise wobei die Verbindung die GPR65-Signalgebung inhibiert.

16. Verbindung zur Verwendung nach Anspruch 15, weiter definiert nach einem der Ansprüche 1 bis 14.

17. Verbindung, ausgewählt aus den Folgenden:
| | | | |
|---|---|---|---|
| 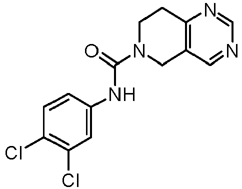 | (1) | 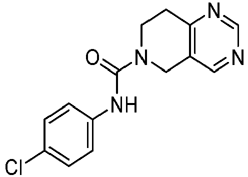 | (2) |
| 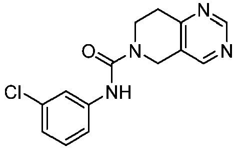 | (3) | 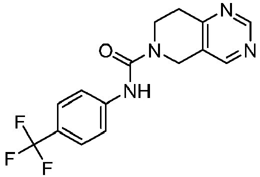 | (4) |
| 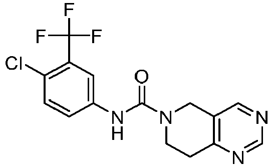 | (5) | 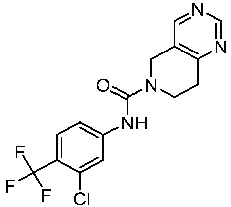 | (6) |
| 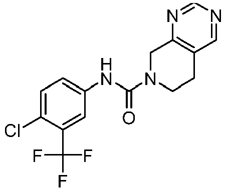 | (7) | 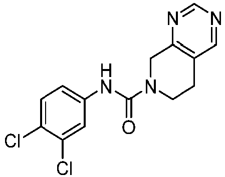 | (8) |
| 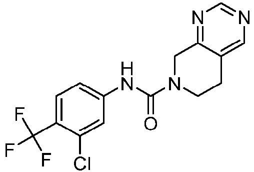 | (9) | 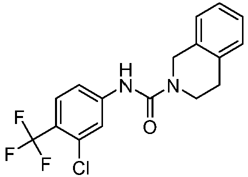 | (10) |
| 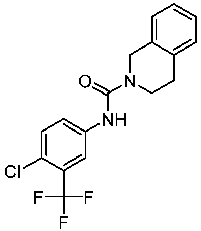 | (11) | 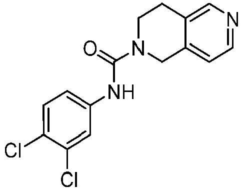 | (12) |
| 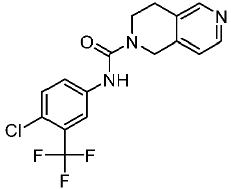 | (13) | 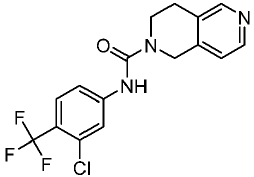 | (14) |
| 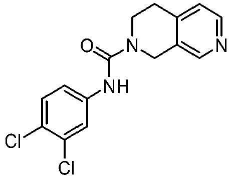 | (15) | 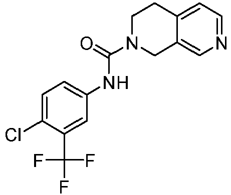 | (16) |
| 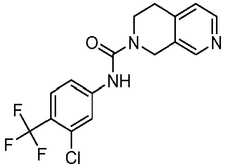 | (17) | 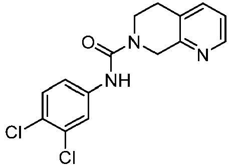 | (18) |
| 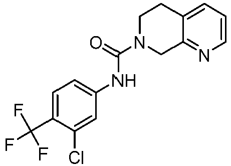 | (19) | 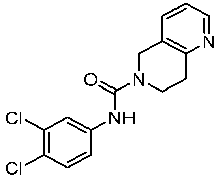 | (20) |
| 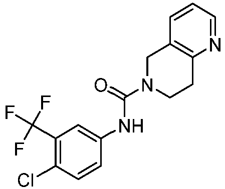 | (21) | 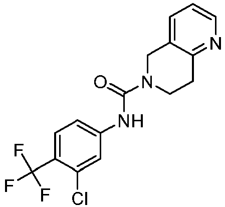 | (22) |
| 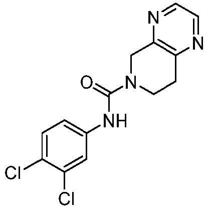 | (23) | 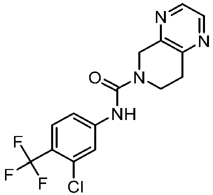 | (24) |
| 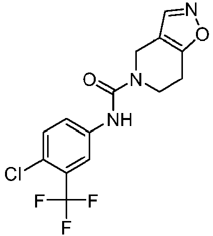 | (25) | 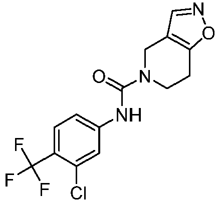 | (26) |
| 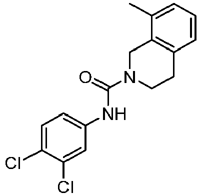 | (27) | 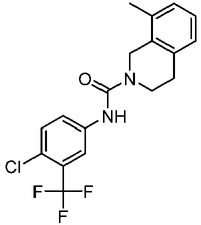 | (28) |
| 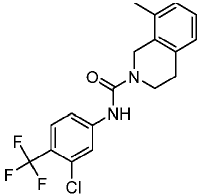 | (29) | 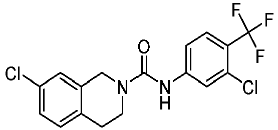 | (30) |
| 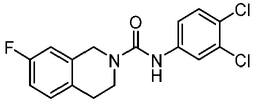 | (31) | 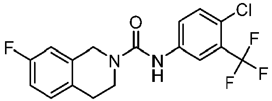 | (32) |
| 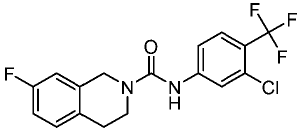 | (33) | 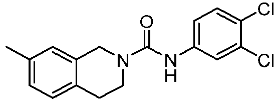 | (34) |
| 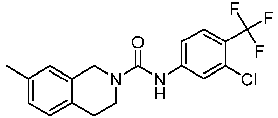 | (35) | 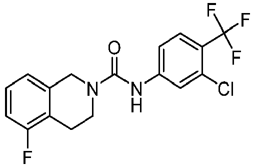 | (36) |
| 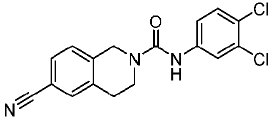 | (37) | 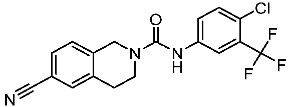 | (38) |
| 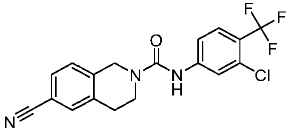 | (39) | 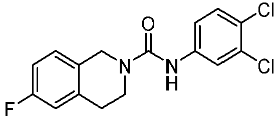 | (42) |
| 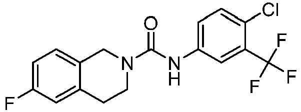 | (43) | 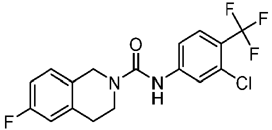 | (44) |
| 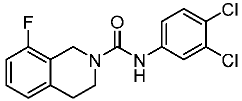 | (45) | 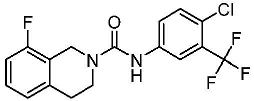 | (46) |
| 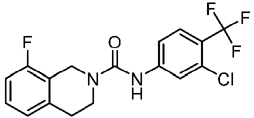 | (47) | | |
| 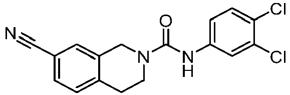 | (49) | 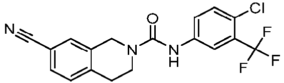 | (50) |
| 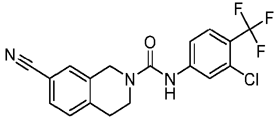 | (51) | 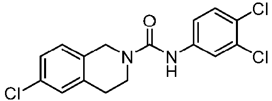 | (52) |
| 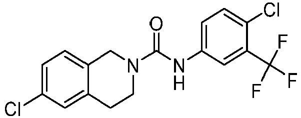 | (53) | 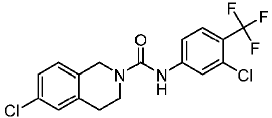 | (54) |
| 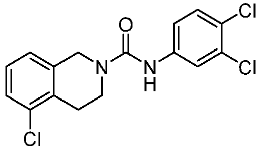 | (55) | 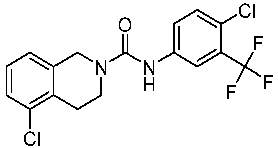 | (56) |
| 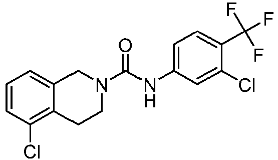 | (57) | 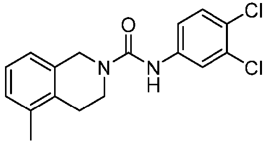 | (58) |
| 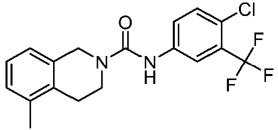 | (59) | 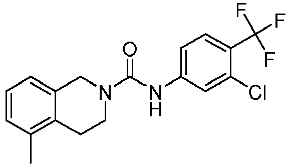 | (60) |
| 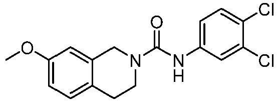 | (61) | 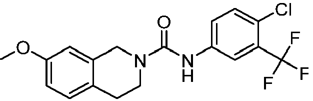 | (62) |
| 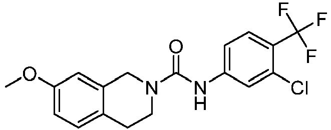 | (63) | 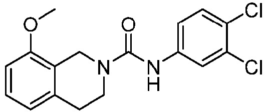 | (64) |
| 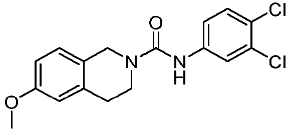 | (65) | 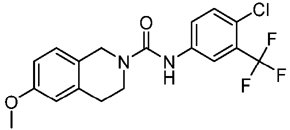 | (66) |
| 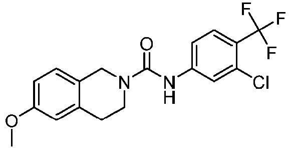 | (67) | 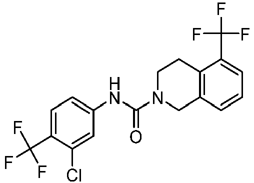 | (70) |
| 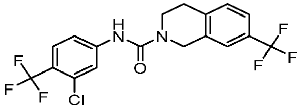 | (71) | 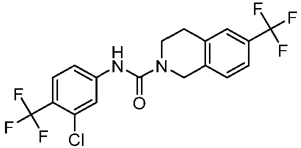 | (72) |
| 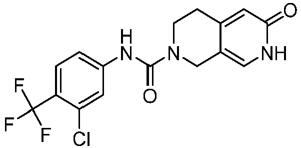 | (74) | 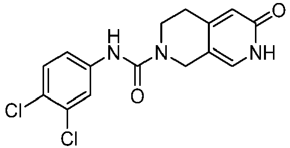 | (76) |
| 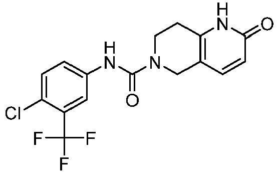 | (77) | 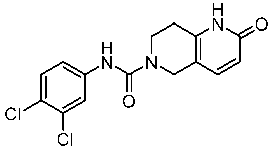 | (78) |
| 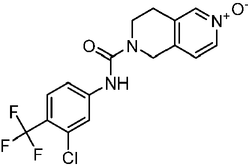 | (79) | 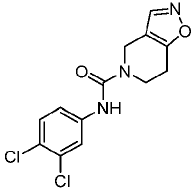 | (80) |
| 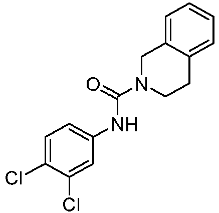 | (81) | 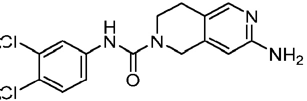 | (82) |
| 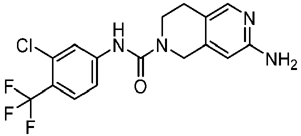 | (83) | 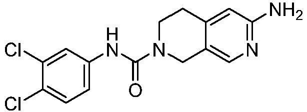 | (84) |
| 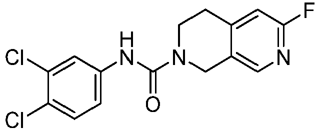 | (85) | 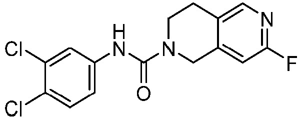 | (86) |
| 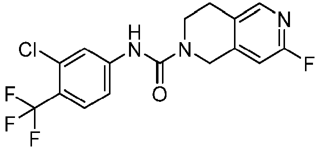 | (87) | 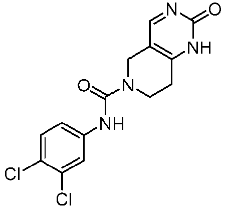 | (88) |
| 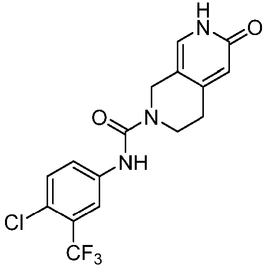 | (89) | 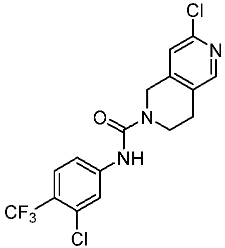 | (90) |
| 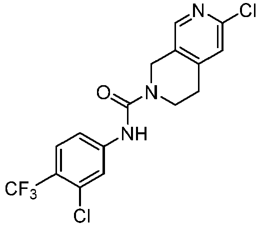 | (91) | 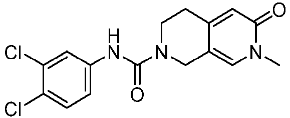 | (92) |
| 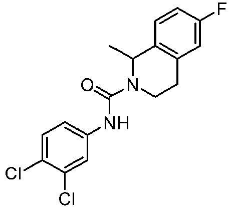 | (93) | 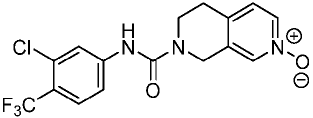 | (94) |
| 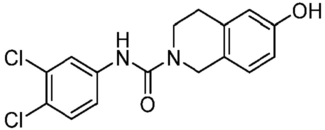 | (95) | 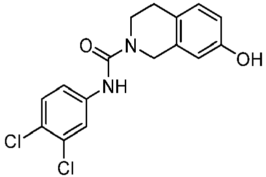 | (96) |
| 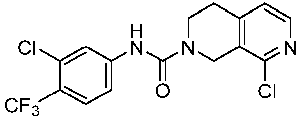 | (97) | 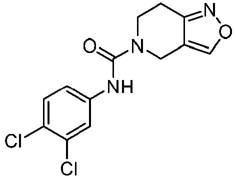 | (98) |
| 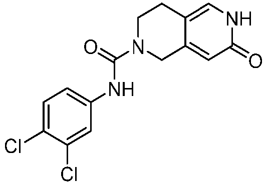 | (99) | 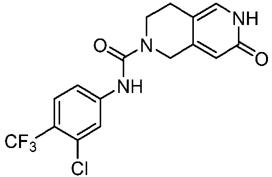 | (100) |
| 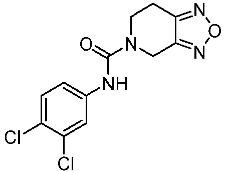 | (101) | 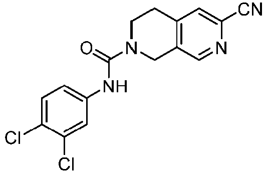 | (102) |
| 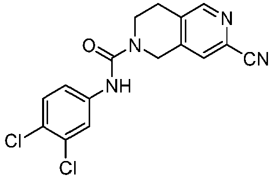 | (103) | 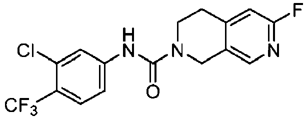 | (104) |
| 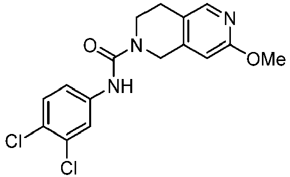 | (105) | 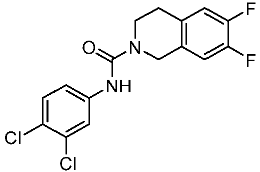 | (106) |
| 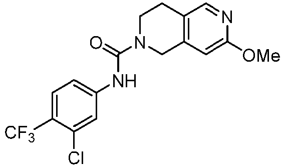 | (107) | 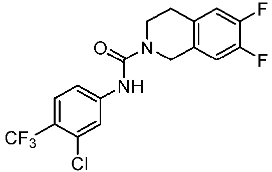 | (108) |
| 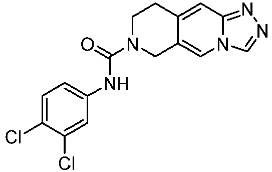 | (109) | 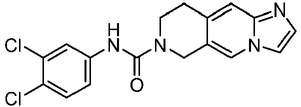 | (110) |
| 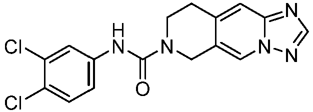 | (111) | 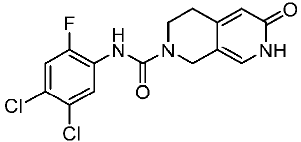 | (112) |
| 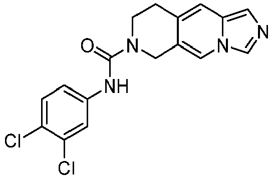 | (113) | 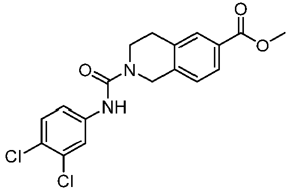 | (114) |
| 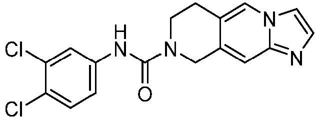 | (115) | 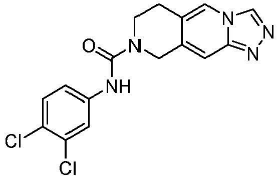 | (116) |
| 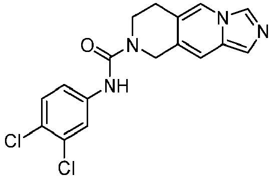 | (117) | 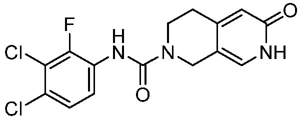 | (118) |
| 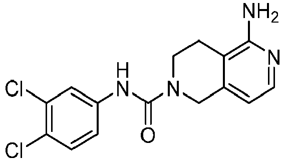 | (119) | 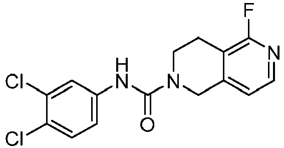 | (120) |
| 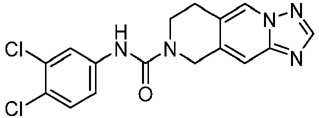 | (121) | 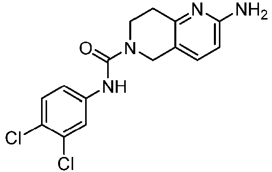 | (122) |
| 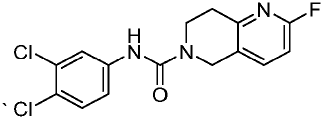 | (123) | 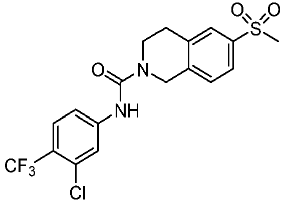 | (124) |
| 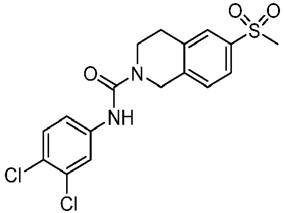 | (125) | 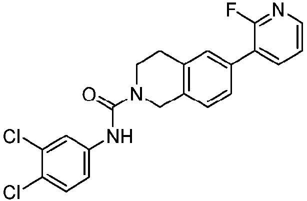 | (126) |
| 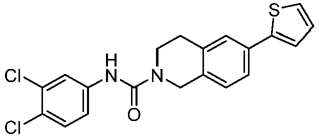 | (127) | 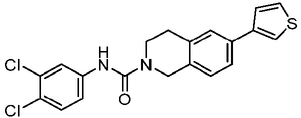 | (128) |
| 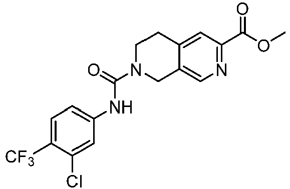 | (129) | 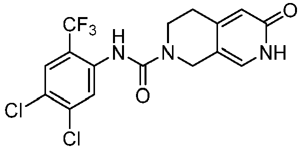 | (130) |
| 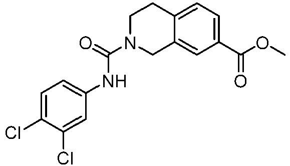 | (131) | 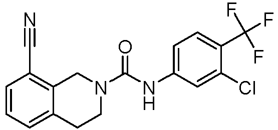 | (132) |
| 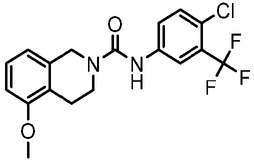 | (133) | 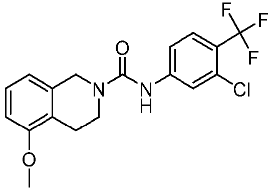 | (134) |
| 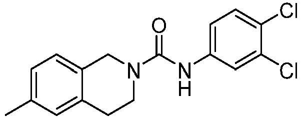 | (135) | 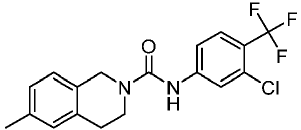 | (136) |
| 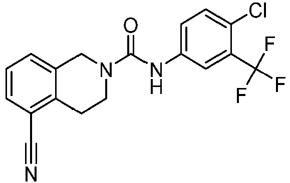 | (137) | 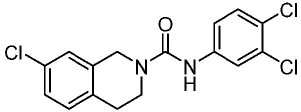 | (138) |
| 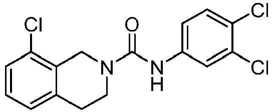 | (139) | 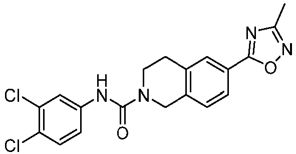 | (140) |
und pharmazeutisch unbedenkliche Salze und Solvate davon zur Verwendung als Medikament.

18. Pharmazeutische Zusammensetzung, umfassend die Verbindung oder ein pharmazeutisch unbedenkliches Salz oder Solvat davon gemäß einem der Ansprüche 1-14 und ein pharmazeutisch unbedenkliches Verdünnungsmittel, einen pharmazeutisch unbedenklichen Hilfsstoff oder einen pharmazeutisch unbedenklichen Träger.

19. Pharmazeutische Zusammensetzung, umfassend eine Verbindung oder ein pharmazeutisch unbedenkliches Salz oder Solvat davon gemäß einem der Ansprüche 1-14 und ein pharmazeutisch unbedenkliches Verdünnungsmittel, einen pharmazeutisch unbedenklichen Hilfsstoff oder einen pharmazeutisch unbedenklichen Träger, zur Verwendung bei der Behandlung oder Prävention einer Störung, die aus einer Proliferationsstörung, einer Immunstörung, Asthma, chronisch-obstruktiver Lungenerkrankung (COPD) und akutem Atemnotsyndrom (ARDS) ausgewählt ist, wobei die Verbindung GPR65 moduliert, vorzugsweise wobei die Verbindung die GPR65-Signalgebung inhibiert.

20. Verbindung zur Verwendung nach Anspruch 17 oder pharmazeutische Zusammensetzung zur Verwendung nach Anspruch 19, wobei es sich bei der Störung um eine Proliferationsstörung handelt, vorzugsweise wobei es sich bei der Proliferationsstörung um eine Krebserkrankung handelt, bei der es sich vorzugsweise um einen soliden Tumor und/oder Metastasen davon oder eine Krebserkrankung, die aus Melanom, Nierenzellkarzinom (Renal Cell Carcinoma, RCC), Magenkrebs, akuter myeloischer Leukämie (AML), Pankreas-Adenokarzinom, dreifach negativem Brustkrebs (Triple Negative Breast Cancer, TNBC), Kolorektalkrebs, Kopf- und Halskrebs, kolorektalem Adenokarzinom, Lungenkrebs, Sarkom, Eierstockkrebs und Gliom, weiter bevorzugt Glioblastom (GBM), ausgewählt ist, handelt.

21. Verbindung zur Verwendung nach Anspruch 17 oder pharmazeutische Zusammensetzung zur Verwendung nach Anspruch 19, wobei es sich bei der Störung um eine Immunstörung handelt, vorzugsweise wobei es sich bei der Immunstörung um eine Autoimmunstörung handelt, weiter bevorzugt wobei die Autoimmunstörung aus Psoriasis, Arthritis psoriatica, rheumatoider Arthritis (RA), multipler Sklerose (MS), systemischem Lupus erythematodes (SLE), Autoimmunthyroiditis (Hashimoto-Thyroiditis), Morbus Basedow, Uveitis (einschließlich intermediärer Uveitis), Colitis ulcerosa, Morbus Crohn, Autoimmunuveoretinitis, systemischer Vaskulitis, Polymyositis-Dermatomyositis, systemischer Sklerose (Sklerodermie), Sjogren-Syndrom, Spondylitis ankylosans und verwandten Spondyloarthropathien, Sarkoidose, autoimmunhämolytischer Anämie, immunologischen Thrombozytenstörungen und Autoimmunpolyendokrinopathien ausgewählt ist und ganz besonders bevorzugt wobei die Autoimmunerkrankung aus Psoriasis, Arthritis psoriatica, Spondylitis ankylosans, Morbus Crohn und multipler Sklerose ausgewählt ist.

22. Verbindung zur Verwendung nach Anspruch 17 oder pharmazeutische Zusammensetzung zur Verwendung nach Anspruch 19, wobei die Verwendung die Behandlung oder Prävention einer Störung, die aus Asthma, chronisch-obstruktiver Lungenerkrankung (COPD) und akutem Atemnotsyndrom (ARDS) ausgewählt ist, umfasst.

23. Verbindung der Formel (Ia), (Ia"), (I) gemäß einem der Ansprüche 1-17 oder pharmazeutisch unbedenkliches Salz oder Solvat davon zur Verwendung bei der Behandlung oder Prävention einer mit GPR65 assoziierten Erkrankung oder Störung.

## Revendications

1. Composé de formule (Ia), ou sel ou solvate pharmaceutiquement acceptable correspondant, 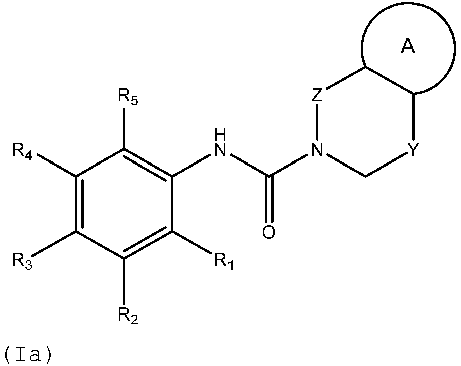
le cycle A étant condensé au cycle contenant N, Z et Y, le cycle A étant choisi parmi benzène, pyridine, pyridone, pyridine N-oxyde, pyridazine, pyrimidine et pyrazine, chacun desquels étant éventuellement substitué par un ou plusieurs substituants choisis parmi F, Cl, Br, I, CN, OH, SO₂-alkyle, alkyle, halogénoalkyle, aralkyle, et aryle, et ledit substituant aryle étant à son tour éventuellement substitué par un ou plusieurs substituants chacun indépendamment choisi parmi F, Cl, Br, I, CN, alcoxy, NR₁₁R₁₁', OH, alkyle, halogénoalkyle, et aralkyle ;
Y et Z étant chacun indépendamment CR₁₀R₁₀', R₁₀ et R₁₀' étant chacun indépendamment choisis parmi H, F, alkyle, et halogénoalkyle ;
R₁, R₄, et R₅ étant chacun indépendamment choisis parmi H, F, Cl, Br, I et halogénoalkyle ; et
R₂ et R₃ étant chacun indépendamment choisis parmi Cl, Br, I, et halogénoalkyle ;
R₁₁ et R₁₁' étant chacun indépendamment choisis parmi H, alkyle, halogénoalkyle, COR₁₂, et SO₂R₁₃, R₁₂ et R₁₃ étant tous deux alkyle ;
le composé étant autre que :
N-(3,4-Dichlorophényl)-7,8-dihydropyrido[4,3-d]pyrimidine-6(5H)-carboxamide ;
N-(3,4-Dichlorophényl)-5,8-dihydropyrido[3,4-d]pyrimidine-7(6H)-carboxamide ;
N-(4-Chloro-3-(trifluorométhyl)phényl)-3,4-dihydroisoquinoline-2(1H)-carboxamide ; et
N-(3,4-dichlorophényl)-3,4-dihydroisoquinoline-2(1H)-carboxamide.

2. Composé selon la revendication 1, le cycle A étant éventuellement substitué par un ou plusieurs substituants choisis parmi F, Cl, Br, I, CN, OH, C₁-C₆ alkyle, phényle, SO₂-alkyle, et C₁-C₆ halogénoalkyle, le plus préférablement l'un ou plusieurs substituants étant choisis parmi F, Cl, Br, I, CN, C₁-C₆ OH, C₁-C₆ alkyle, SO₂-alkyle, et C₁-C₆ halogénoalkyle.

3. Composé de formule (Ia"), ou sel ou solvate pharmaceutiquement acceptable correspondant, 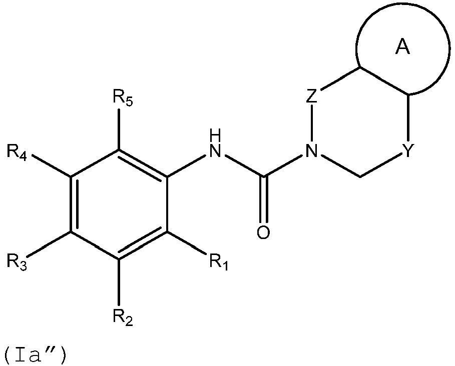 le cycle A 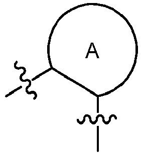 étant choisi parmi : 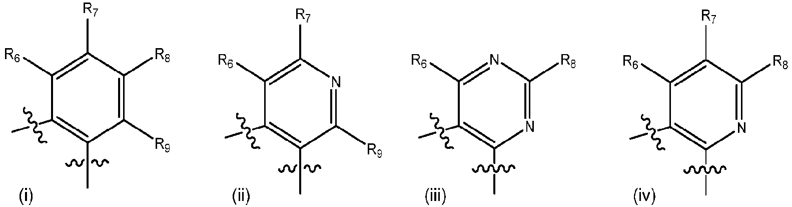 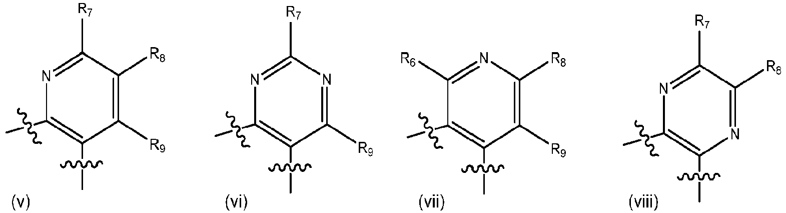 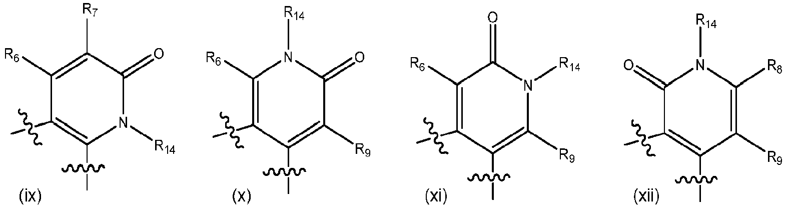 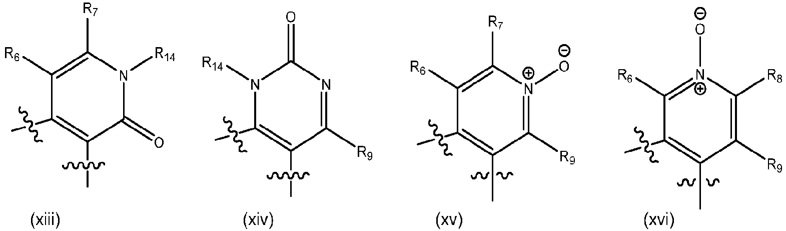 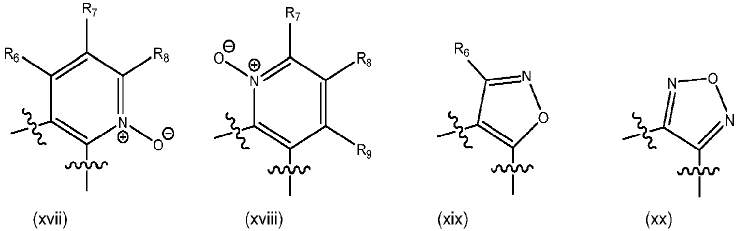 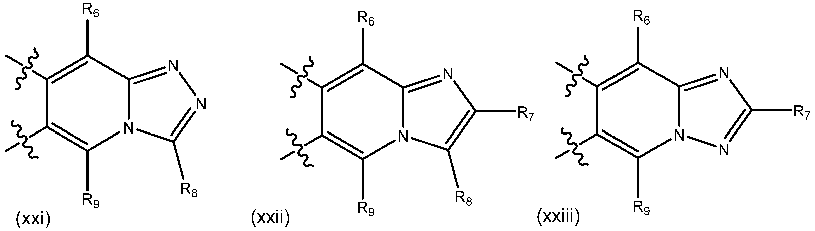 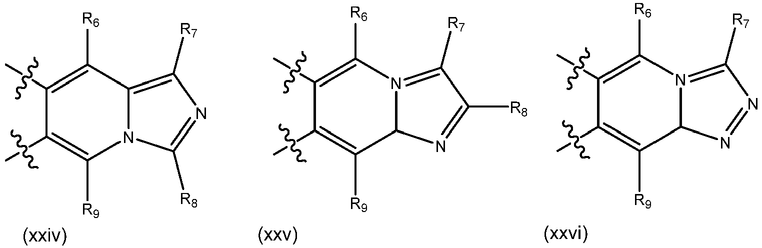 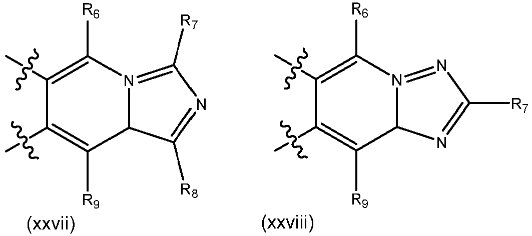 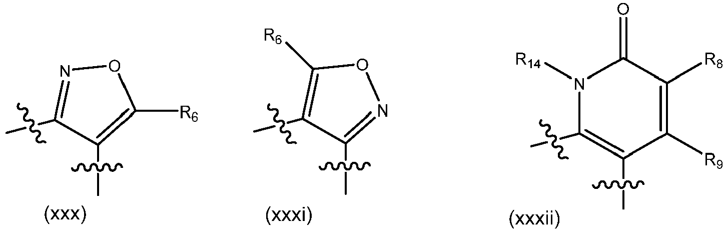 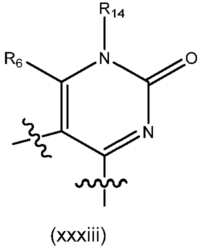 les lignes ondulées désignant la fixation du cycle A au cycle contenant N, Z et Y : 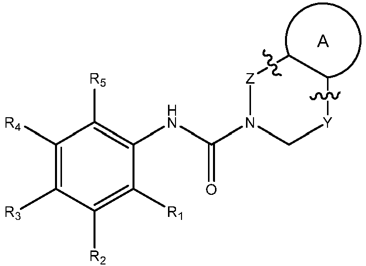 et :
Y et Z étant chacun indépendamment CR₁₀R₁₀', R₁₀ et R₁₀' étant chacun indépendamment choisis parmi H, F, alkyle, et halogénoalkyle ;
R₁, R₄, et R₅ étant chacun indépendamment choisis parmi H, F, Cl, Br, I et halogénoalkyle ;
R₂ et R₃ étant chacun indépendamment choisis parmi Cl, Br, I, et halogénoalkyle ;
R₆, R₇, R₈, et R₉ étant chacun indépendamment choisis parmi H, F, Cl, Br, I, CN, SO₂-alkyle, OH, C₁-C₆ alkyle, phényle, et C₁-C₆ halogénoalkyle, et R₁₄ étant H ou alkyle ; et
le composé étant autre que :
N-(3,4-Dichlorophényl)-7,8-dihydropyrido[4,3-d]pyrimidine-6(5H)-carboxamide ;
N-(3,4-Dichlorophényl)-5,8-dihydropyrido[3,4-d]pyrimidine-7(6H)-carboxamide ;
N-(4-chloro-3-(trifluorométhyl)phényl)-3,4-dihydroisoquinoline-2(1H)-carboxamide ;
N-(3,4-dichlorophényl)-3,4-dihydroisoquinoline-2(1H)-carboxamide ; et
N-(3,4-dichlorophényl)-6,7-dihydroisoxazolo[4,5-c]pyridine-5(4H)-carboxamide.

4. Composé selon la revendication 3, le cycle A étant choisi parmi :
- groupes (i)-(viii), (ix), (xi), et (xxxii) ;
- groupes (i)-(x) ; ou
- groupes (i), (ii), (vii) et (x).

5. Composé selon l'une quelconque des revendications précédentes Y et Z étant chacun indépendamment choisis parmi CH₂, CHMe, CF₂, C(CH₃)₂, C(CF₃)₂, et étant préférablement tous deux CH₂.

6. Composé selon l'une quelconque des revendications précédentes, R₁ étant choisi parmi H, halogénoalkyle et F, et étant préférablement H.

7. Composé selon l'une quelconque des revendications précédentes, R₂ et R₃ étant chacun indépendamment choisis parmi Cl, Br, et CFₙH₃₋ₙ, n étant 1, 2, ou 3, plus préférablement R₂ et R₃ étant chacun indépendamment choisis parmi Cl et CF₃, et le plus préférablement l'un parmi R₂ et R₃ étant Cl et l'autre étant CF₃, ou R₂ et R₃ étant tous deux Cl.

8. Composé selon l'une quelconque des revendications précédentes, R₄ étant choisi parmi H et Cl, et étant préférablement H.

9. Composé selon l'une quelconque des revendications précédentes, R₅ étant H ou CF₃, plus préférablement H.

10. Composé selon l'une quelconque des revendications 3 à 9, R₆ étant choisi parmi H, F, Cl, CN, CH₃, et CF₃, préférablement R₆ étant choisi parmi H, F, Cl, CN, et CH₃, et étant plus préférablement H.

11. Composé selon l'une quelconque des revendications 3 à 10, R₇ étant choisi parmi H, F, Cl, CN, CH₃, et CF₃, le plus préférablement H, F, et Cl, et étant le plus préférablement H.

12. Composé selon l'une quelconque des revendications 3 à 11, R₈ étant choisi parmi H, F, OH, CN, phényle, CF₃, et CF₂H, préférablement R₈ étant choisi parmi H, F, Cl, CN, CH₃, et CF₃, plus préférablement parmi H, F, Cl, et CN, et le plus préférablement parmi F et Cl, en particulier F.

13. Composé selon l'une quelconque des revendications 3 à 12, R₉ étant choisi parmi H, F, Cl, CN, CH₃ et CF₃, plus préférablement H, F, et CN, et R₉ étant le plus préférablement H.

14. Composé selon l'une quelconque des revendications 1 à 13, qui est choisi parmi les suivants :
| | | | |
|---|---|---|---|
| 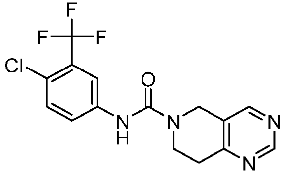 | (5) | 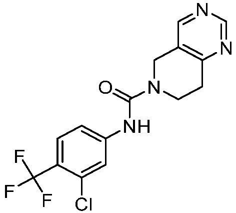 | (6) |
| 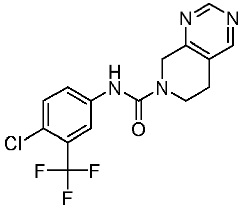 | (7) | 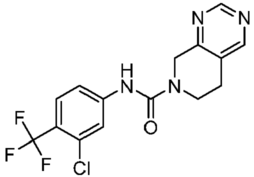 | (9) |
| 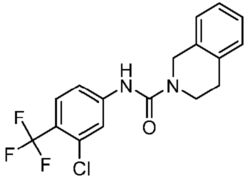 | (10) | 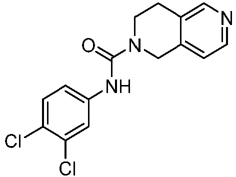 | (12) |
| 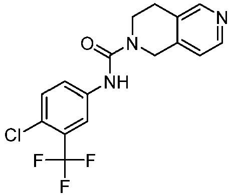 | (13) | 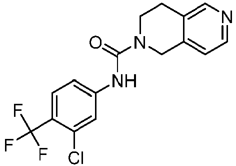 | (14) |
| 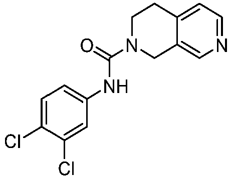 | (15) | 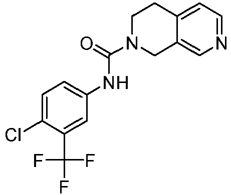 | (16) |
| 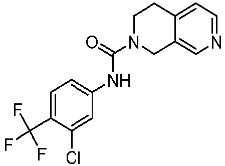 | (17) | 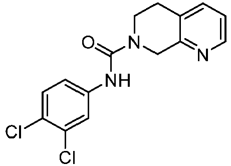 | (18) |
| 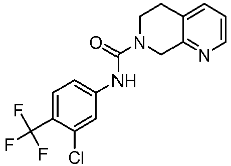 | (19) | 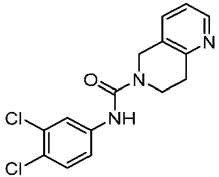 | (20) |
| 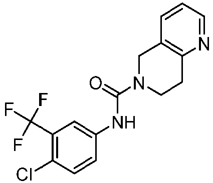 | (21) | 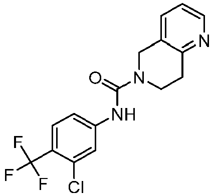 | (22) |
| 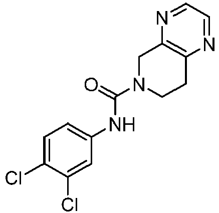 | (23) | 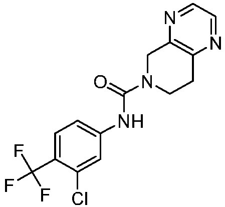 | (24) |
| 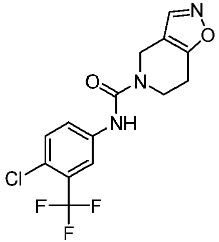 | (25) | 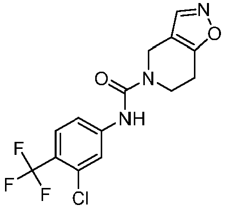 | (26) |
| 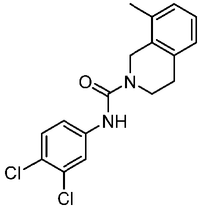 | (27) | 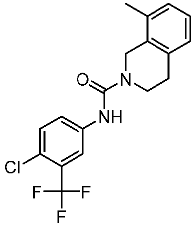 | (28) |
| 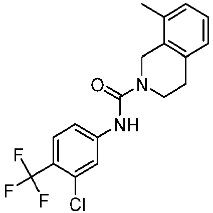 | (29) | 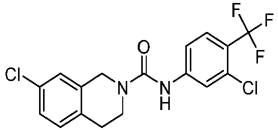 | (30) |
| 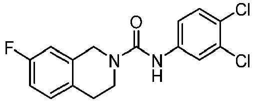 | (31) | 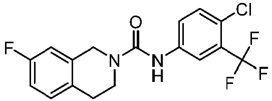 | (32) |
| 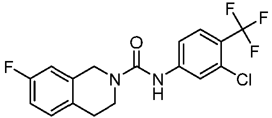 | (33) | 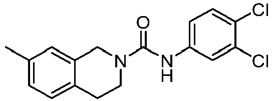 | (34) |
| 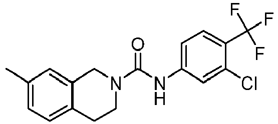 | (35) | 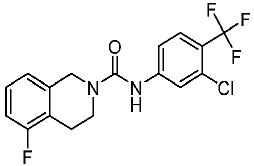 | (36) |
| 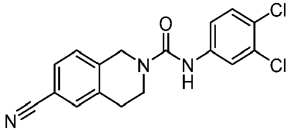 | (37) | 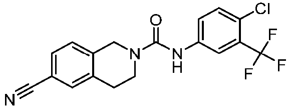 | (38) |
| 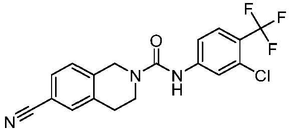 | (39) | 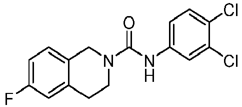 | (42) |
| 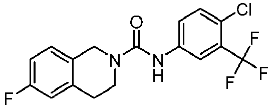 | (43) | 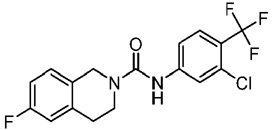 | (44) |
| 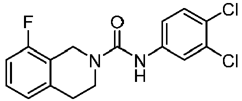 | (45) | 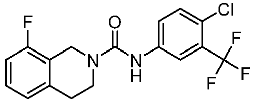 | (46) |
| 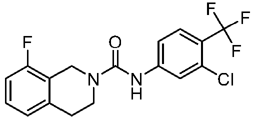 | (47) | | |
| 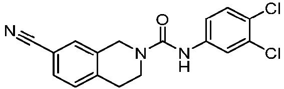 | (49) | 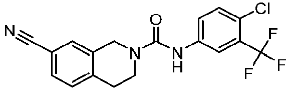 | (50) |
| 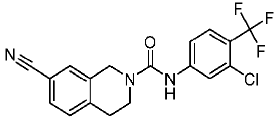 | (51) | 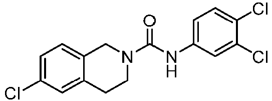 | (52) |
| 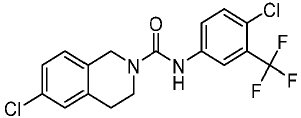 | (53) | 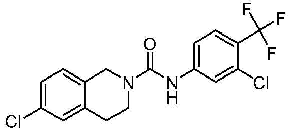 | (54) |
| 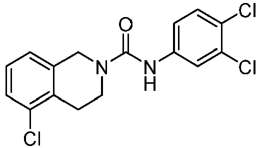 | (55) | 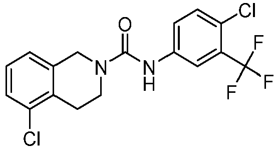 | (56) |
| 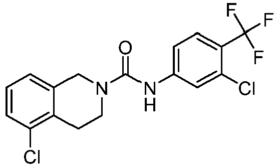 | (57) | 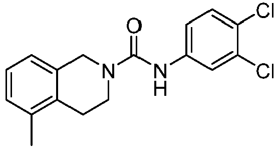 | (58) |
| 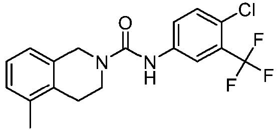 | (59) | 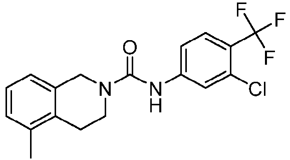 | (60) |
| | | 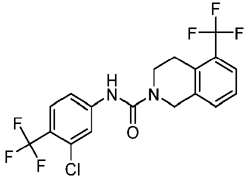 | (70) |
| 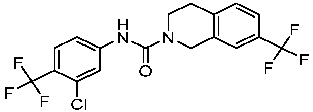 | (71) | 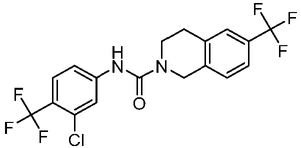 | (72) |
| 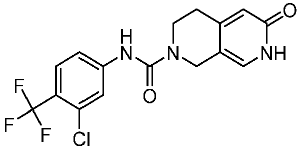 | (74) | 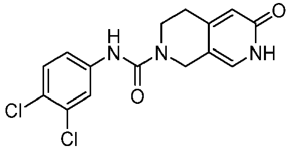 | (76) |
| 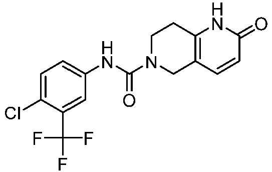 | (77) | 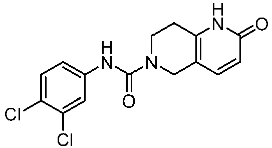 | (78) |
| 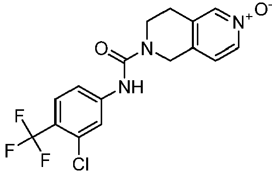 | (79) | | |
| 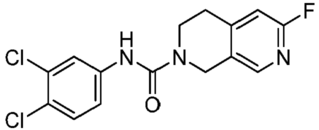 | (85) | 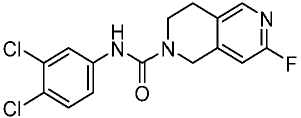 | (86) |
| 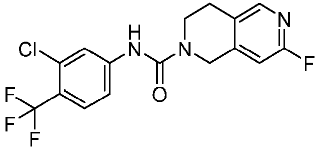 | (87) | 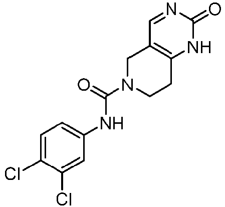 | (88) |
| 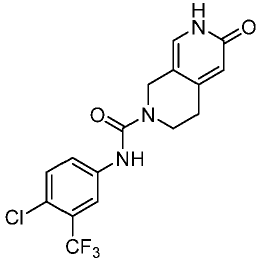 | (89) | 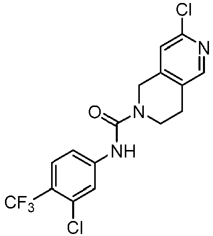 | (90) |
| 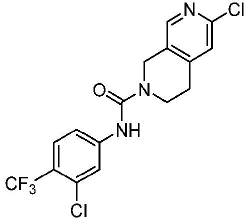 | (91) | 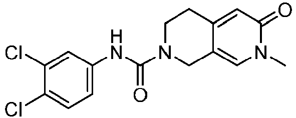 | (92) |
| 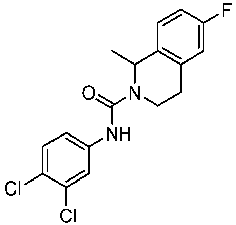 | (93) | 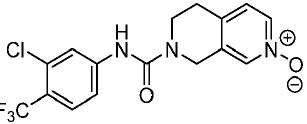 | (94) |
| 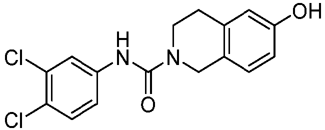 | (95) | 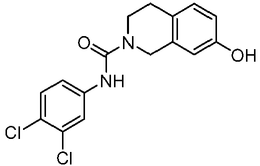 | (96) |
| 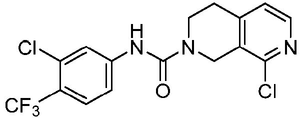 | (97) | 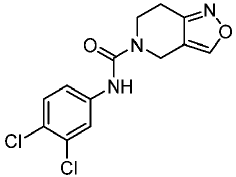 | (98) |
| 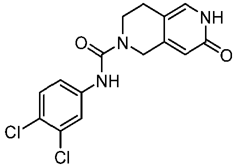 | (99) | 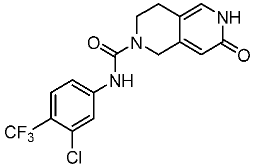 | (100) |
| 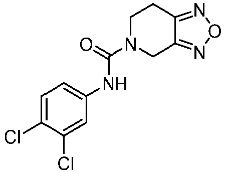 | (101) | 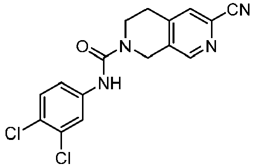 | (102) |
| 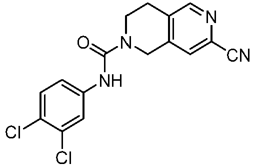 | (103) | 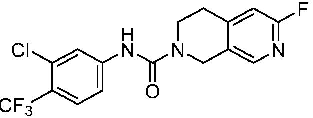 | (104) |
| 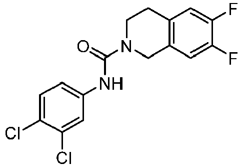 | (106) | 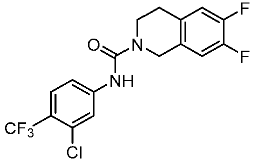 | (108) |
| 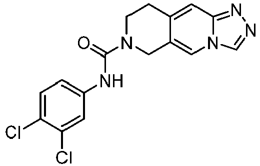 | (109) | 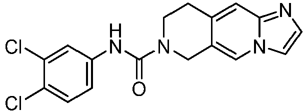 | (110) |
| 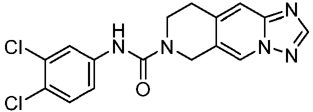 | (111) | 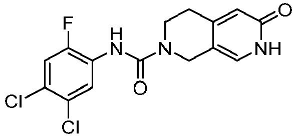 | (112) |
| 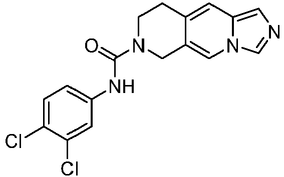 | (113) | | |
| 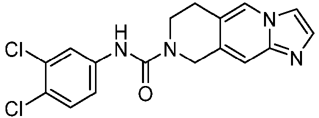 | (115) | 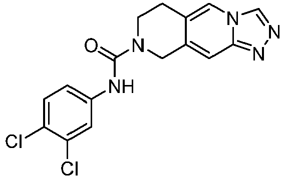 | (116) |
| 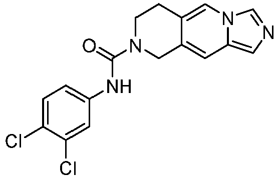 | (117) | 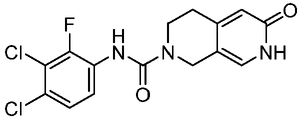 | (118) |
| 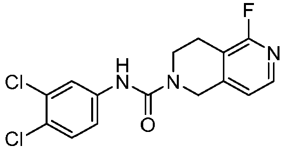 | (120) | 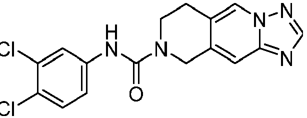 | (121) |
| 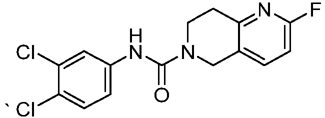 | (123) | 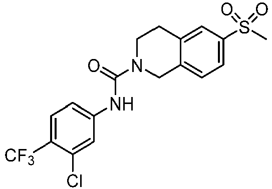 | (124) |
| 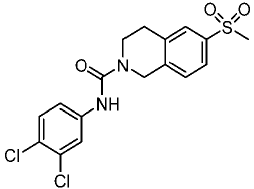 | (125) | | |
| | ) | | |
| | | 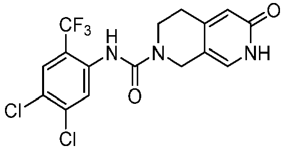 | (130) |
| | | 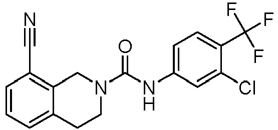 | (132) |
| 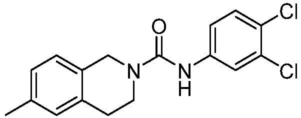 | (135) | 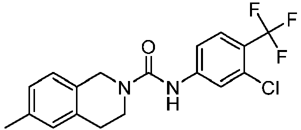 | (136) |
| 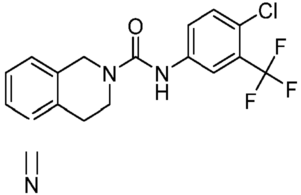 | (137) | 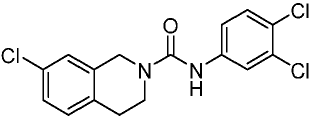 | (138) |
| 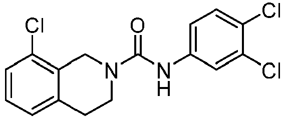 | (139) | | |
et sels et solvates pharmaceutiquement acceptables correspondants.

15. Composé de formule (I), ou sel ou solvate pharmaceutiquement acceptable correspondant, 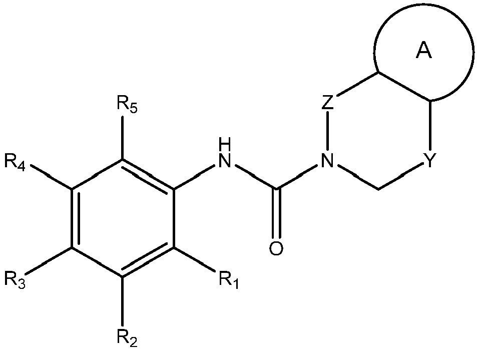 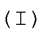
le cycle A étant un cycle aromatique ou hétéroaromatique monocyclique à 5 ou 6 chaînons, ou un cycle aromatique ou hétéroaromatique bicyclique à 9 ou 10 chaînons, chacun desquels étant éventuellement substitué par un ou plusieurs substituants choisis parmi F, Cl, Br, I, CN, alcoxy, NR₁₁R₁₁', OH, SO₂-alkyle, CO₂-alkyle, alkyle, halogénoalkyle, aralkyle, aryle, et hétéroaryle, et lesdits substituants aryle et hétéroaryle étant à leur tour éventuellement substitués par un ou plusieurs substituants chacun indépendamment choisi parmi F, Cl, Br, I, CN, alcoxy, NR₁₁R₁₁', OH, alkyle, halogénoalkyle, et aralkyle ;
Y et Z étant chacun indépendamment CR₁₀R₁₀', R₁₀ et R₁₀' étant chacun indépendamment choisis parmi H, F, alkyle, et halogénoalkyle ;
R₁, R₄, et R₅ étant chacun indépendamment choisis parmi H, F, Cl, Br, I et halogénoalkyle ;
R₂ et R₃ étant chacun indépendamment choisis parmi Cl, Br, I, et halogénoalkyle ;
et
R₁₁ et R₁₁' étant chacun indépendamment choisis parmi H, alkyle, halogénoalkyle, COR₁₂, et SO₂R₁₃, R₁₂ et R₁₃ étant tous deux alkyle ;
pour une utilisation dans le traitement ou la prévention d'un trouble sélectionné parmi un trouble prolifératif, un trouble immunitaire, l'asthme, la bronchopneumopathie chronique obstructive (BPCO) et le syndrome de détresse respiratoire aiguë (SDRA), le composé modulant GPR65, de préférence le composé inhibant la signalisation de GPR65.

16. Composé pour une utilisation selon la revendication 15, défini en outre selon l'une quelconque des revendications 1 à 14.

17. Composé choisi parmi les suivants :
| | | | |
|---|---|---|---|
| 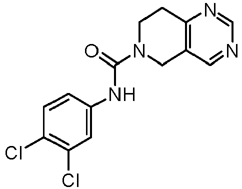 | (1) | 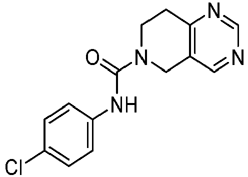 | (2) |
| 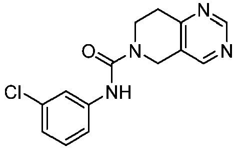 | (3) | 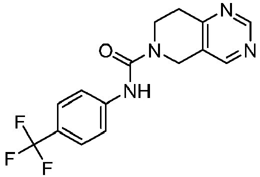 | (4) |
| 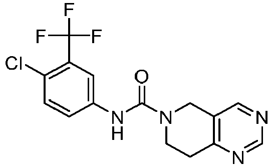 | (5) | 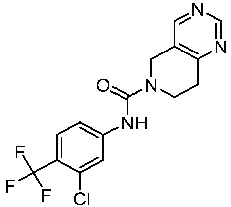 | (6) |
| 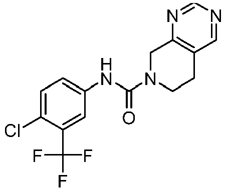 | (7) | 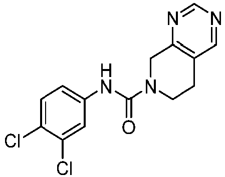 | (8) |
| 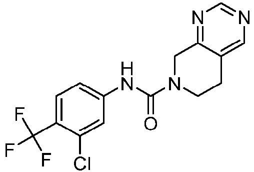 | (9) | 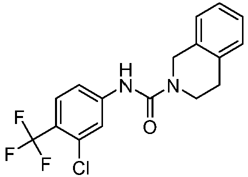 | (10) |
| 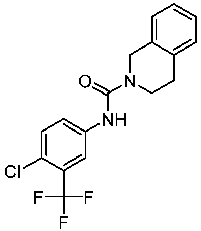 | (11) | 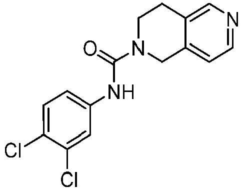 | (12) |
| 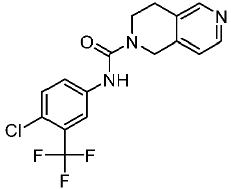 | (13) | 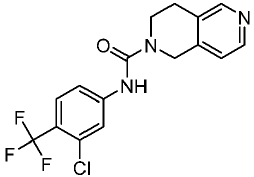 | (14) |
| 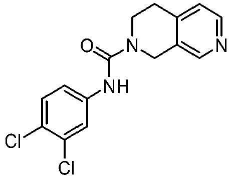 | (15) | 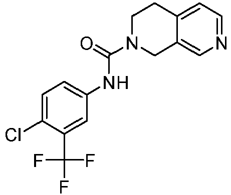 | (16) |
| 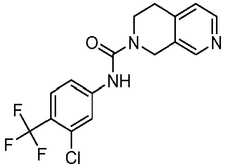 | (17) | 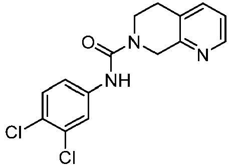 | (18) |
| 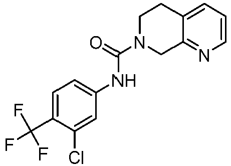 | (19) | 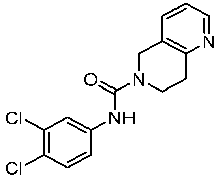 | (20) |
| 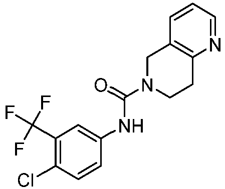 | (21) | 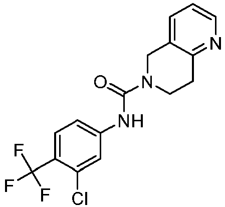 | (22) |
| 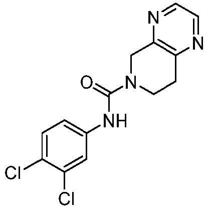 | (23) | 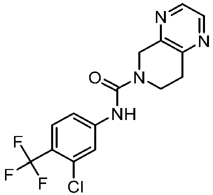 | (24) |
| 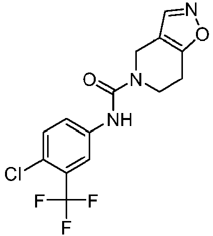 | (25) | 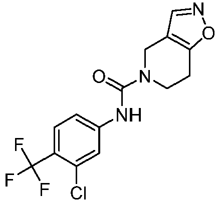 | (26) |
| 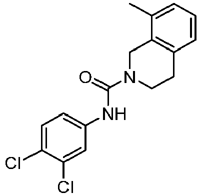 | (27) | 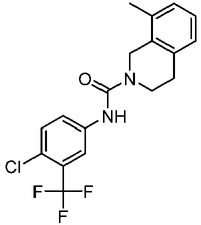 | (28) |
| 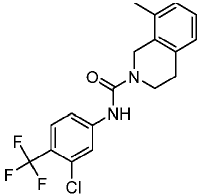 | (29) | 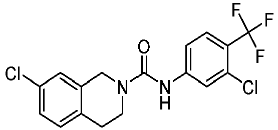 | (30) |
| 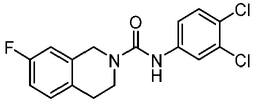 | (31) | 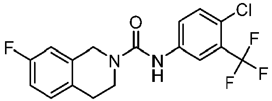 | (32) |
| 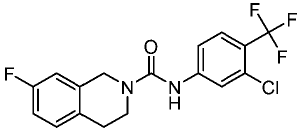 | (33) | 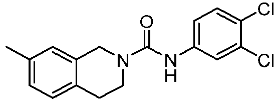 | (34) |
| 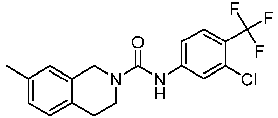 | (35) | 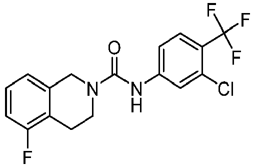 | (36) |
| 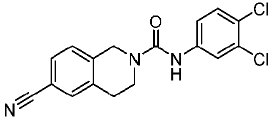 | (37) | 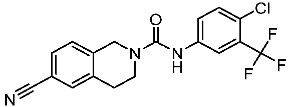 | (38) |
| 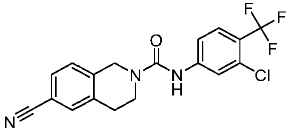 | (39) | 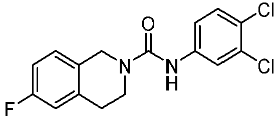 | (42) |
| 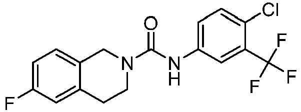 | (43) | 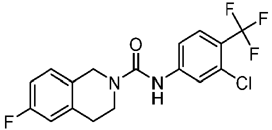 | (44) |
| 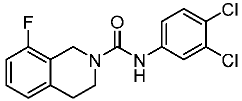 | (45) | 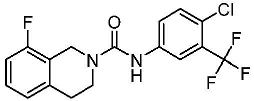 | (46) |
| 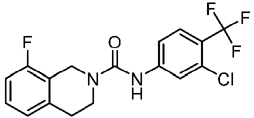 | (47) | | |
| 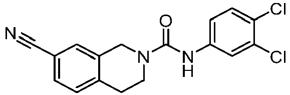 | (49) | 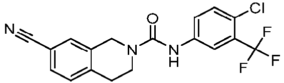 | (50) |
| 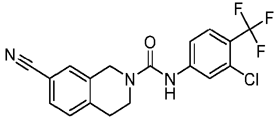 | (51) | 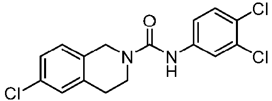 | (52) |
| 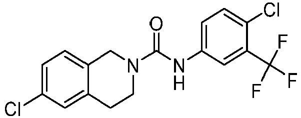 | (53) | 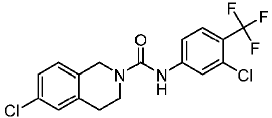 | (54) |
| 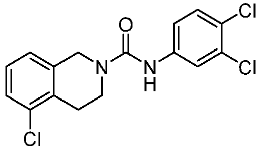 | (55) | 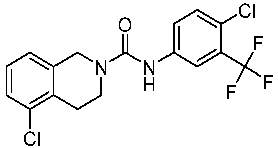 | (56) |
| 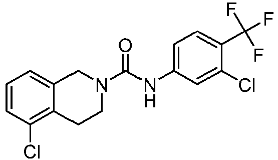 | (57) | 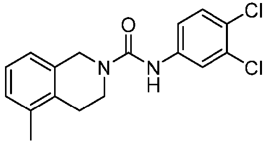 | (58) |
| 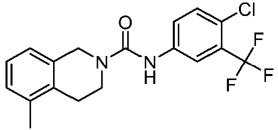 | (59) | 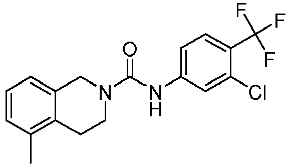 | (60) |
| 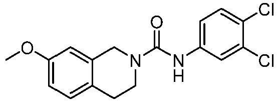 | (61) | 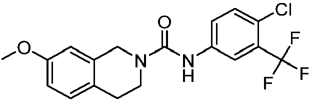 | (62) |
| 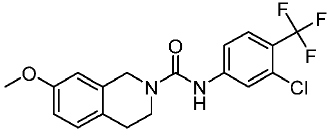 | (63) | 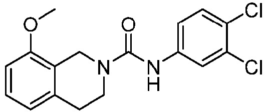 | (64) |
| 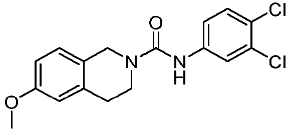 | (65) | 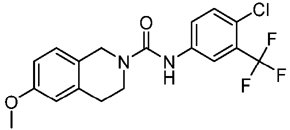 | (66) |
| 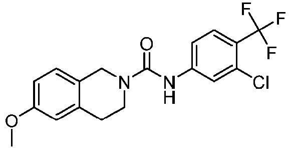 | (67) | 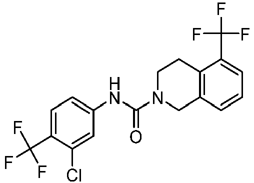 | (70) |
| 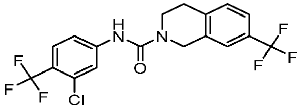 | (71) | 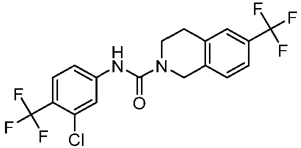 | (72) |
| 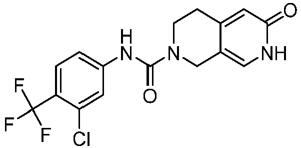 | (74) | 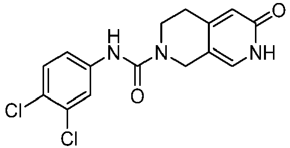 | (76) |
| 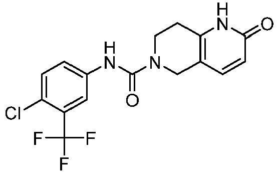 | (77) | 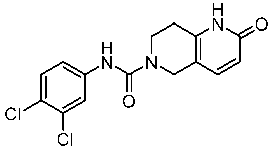 | (78) |
| 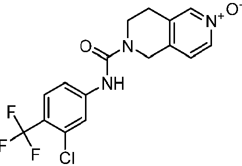 | (79) | 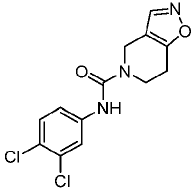 | (80) |
| 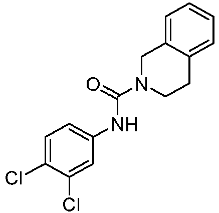 | (81) | 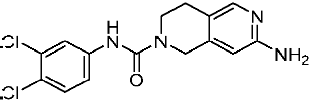 | (82) |
| 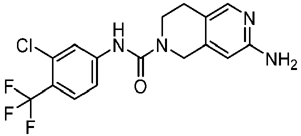 | (83) | 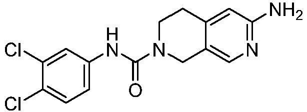 | (84) |
| 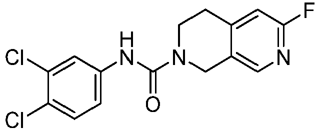 | (85) | 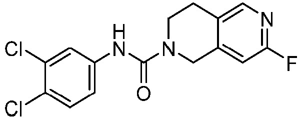 | (86) |
| 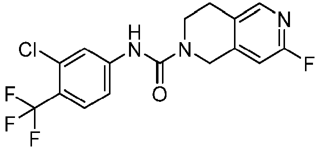 | (87) | 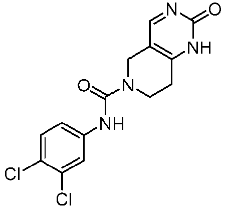 | (88) |
| 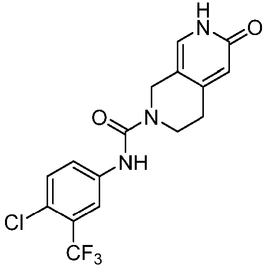 | (89) | 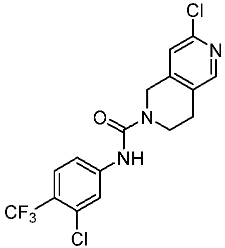 | (90) |
| 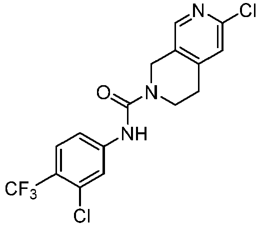 | (91) | 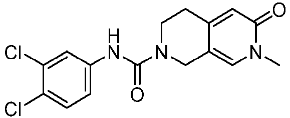 | (92) |
| 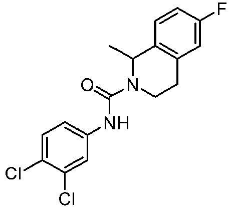 | (93) | 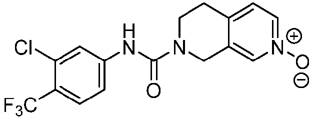 | (94) |
| 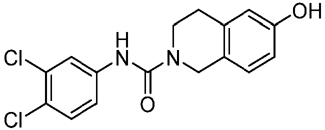 | (95) | 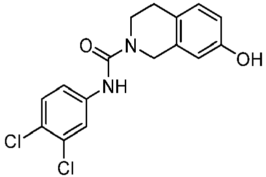 | (96) |
| 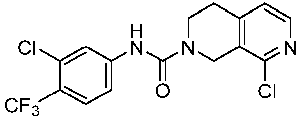 | (97) | 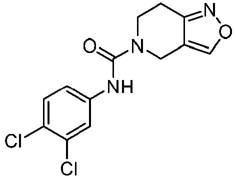 | (98) |
| 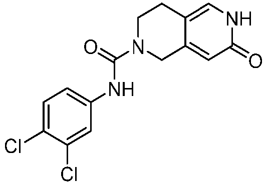 | (99) | 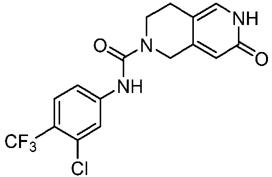 | (100) |
| 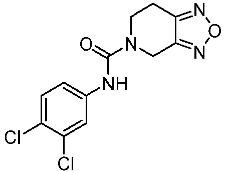 | (101) | 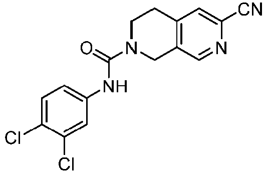 | (102) |
| 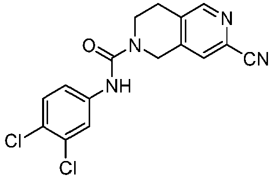 | (103) | 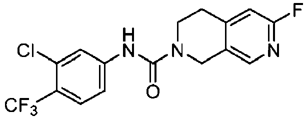 | (104) |
| 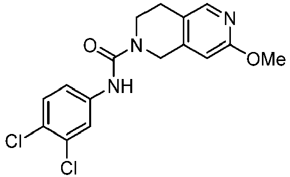 | (105) | 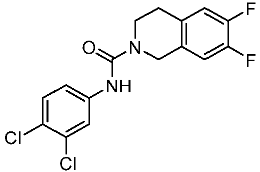 | (106) |
| 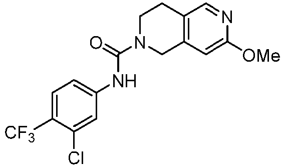 | (107) | 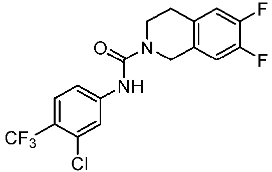 | (108) |
| 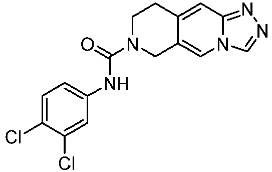 | (109) | 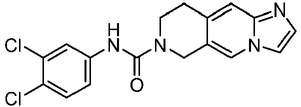 | (110) |
| 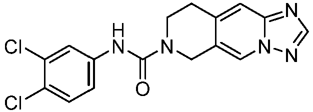 | (111) | 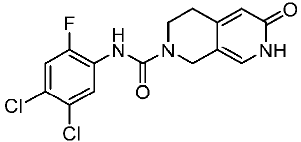 | (112) |
| 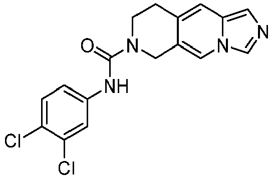 | (113) | 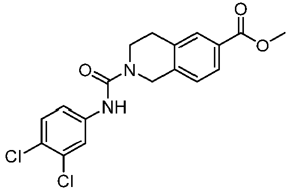 | (114) |
| 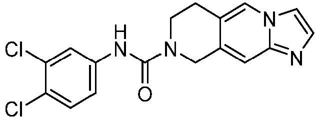 | (115) | 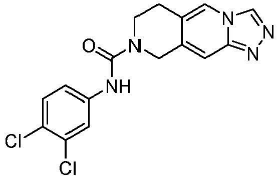 | (116) |
| 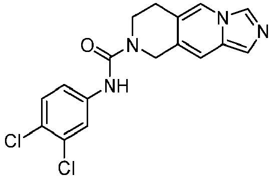 | (117) | 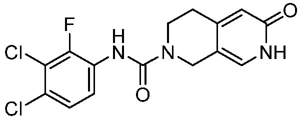 | (118) |
| 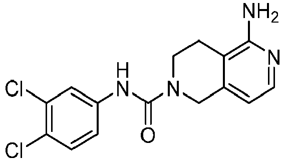 | (119) | 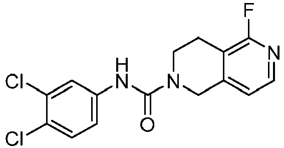 | (120) |
| 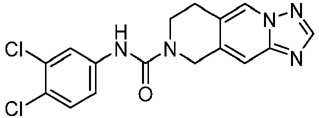 | (121) | 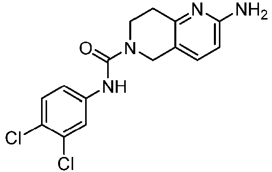 | (122) |
| 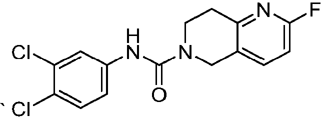 | (123) | 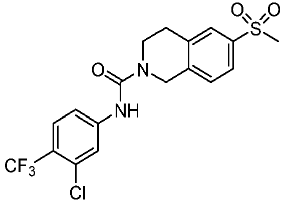 | (124) |
| 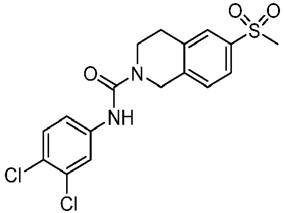 | (125) | 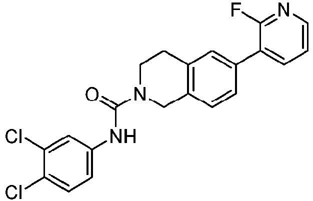 | (126) |
| 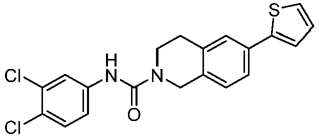 | (127) | 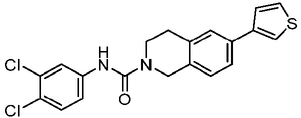 | (128) |
| 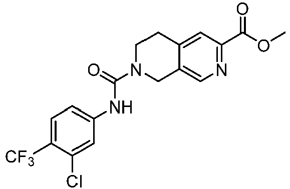 | (129) | 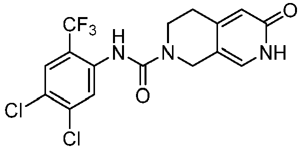 | (130) |
| 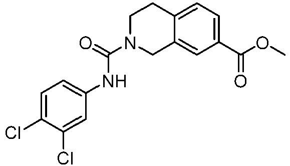 | (131) | 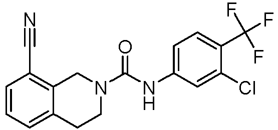 | (132) |
| 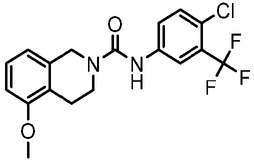 | (133) | 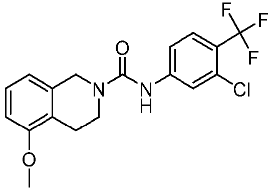 | (134) |
| 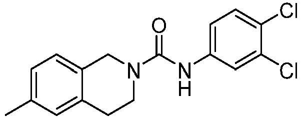 | (135) | 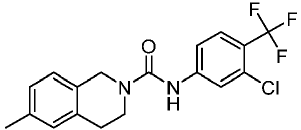 | (136) |
| 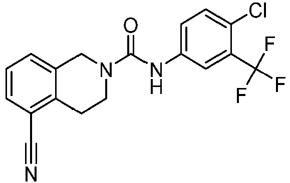 | (137) | 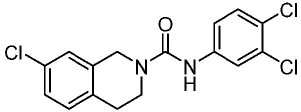 | (138) |
| 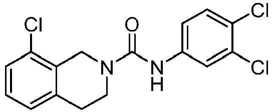 | (139) | 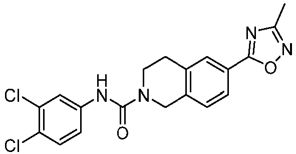 | (140) |
et sels et solvates pharmaceutiquement acceptables correspondants, pour une utilisation en tant que médicament.

18. Composition pharmaceutique comprenant un composé ou un sel ou solvate pharmaceutiquement acceptable de celui-ci, tel que défini selon l'une quelconque des revendications 1 à 14, et un diluant, excipient ou support pharmaceutiquement acceptable.

19. Composition pharmaceutique comprenant un composé, ou un sel ou solvate pharmaceutiquement acceptable de celui-ci, tel que défini selon l'une quelconque des revendications 1 à 14, et un diluant, excipient ou support pharmaceutiquement acceptable, pour une utilisation dans le traitement ou la prévention d'un trouble choisi parmi un trouble prolifératif, un trouble immunitaire, l'asthme, la bronchopneumopathie chronique obstructive (BPCO) et le syndrome de détresse respiratoire aiguë (SDRA), le composé modulant GPR65, de préférence le composé inhibant la signalisation de GPR65.

20. Composé pour une utilisation selon la revendication 17 ou composition pharmaceutique pour une utilisation selon la revendication 19, le trouble étant un trouble prolifératif, de préférence le trouble prolifératif étant un cancer qui est de préférence une tumeur solide et/ou des métastases de celle-ci, ou un cancer choisi parmi le mélanome, le carcinome à cellules rénales (CCR), le cancer gastrique, la leucémie myéloïde aiguë (LMA), l'adénocarcinome pancréatique, le cancer du sein triple négatif (CSTN), le cancer colorectal, le cancer de la tête et du cou, l'adénocarcinome colorectal, le cancer du poumon, le sarcome, le cancer de l'ovaire, et le gliome, plus préférablement le glioblastome (GBM).

21. Composé pour une utilisation selon la revendication 17 ou composition pharmaceutique pour une utilisation selon la revendication 19, le trouble étant un trouble immunitaire, de préférence le trouble immunitaire étant une maladie auto-immune, plus préférablement la maladie auto-immune étant choisie parmi le psoriasis, le rhumatisme psoriasique, la polyarthrite rhumatoïde (PR), la sclérose en plaques (SEP), le lupus érythémateux disséminé (LED), la thyroïdite auto-immune (thyroïdite de Hashimoto), la maladie de Graves, l'uvéite (y compris l'uvéite intermédiaire), la rectocolite hémorragique, la maladie de Crohn, l'uvéorétinite auto-immune, la vascularite systémique, la polymyosite-dermatomyosite, la sclérose (sclérodermie) systémique, le syndrome de Sjögren, la spondylarthrite ankylosante et les spondylarthropathies apparentées, la sarcoïdose, l'anémie hémolytique auto-immune, les troubles plaquettaires immunologiques, et les polyendocrinopathies auto-immunes, et le plus préférablement la maladie auto-immune étant choisie parmi le psoriasis, le rhumatisme psoriasique, la spondylarthrite ankylosante, la maladie de Crohn, et la sclérose en plaques.

22. Composé pour une utilisation selon la revendication 17 ou composition pharmaceutique pour une utilisation selon la revendication 19, l'utilisation comprenant le traitement ou la prévention d'un trouble choisi parmi l'asthme, une bronchopneumopathie chronique obstructive (BPCO) et un syndrome de détresse respiratoire aiguë (SDRA).

23. Composé de formule (Ia), (Ia"), (I) tel que défini dans l'une quelconque des revendications 1 à 17, ou sel ou solvate pharmaceutiquement acceptable correspondant, pour une utilisation dans le traitement ou la prévention d'une maladie ou d'un trouble associé(e) à GPR65.
